(19) Europäisches Patentamt
European Patent Office
Office européen des brevets

(11) EP 2 050 734 A1

(12) **EUROPEAN PATENT APPLICATION**
published in accordance with Art. 153(4) EPC

(43) Date of publication:
**22.04.2009 Bulletin 2009/17**

(21) Application number: **07790787.1**

(22) Date of filing: **13.07.2007**

(51) Int Cl.:
***C07C 233/11*** (2006.01) ***A61K 31/167*** (2006.01)
***A61K 31/18*** (2006.01) ***A61K 31/277*** (2006.01)
***A61K 31/416*** (2006.01) ***A61K 31/4184*** (2006.01)
***A61K 31/4245*** (2006.01) ***A61K 31/428*** (2006.01)
***A61K 31/433*** (2006.01) ***A61K 31/44*** (2006.01)
***A61K 31/4406*** (2006.01) ***A61K 31/4409*** (2006.01)
***A61K 31/4427*** (2006.01) ***A61K 31/443*** (2006.01)
***A61K 31/47*** (2006.01) ***A61K 31/4709*** (2006.01)
***A61K 31/472*** (2006.01) ***A61K 31/4725*** (2006.01)
***A61K 31/496*** (2006.01) ***A61K 31/506*** (2006.01)
***A61K 31/536*** (2006.01)

(86) International application number:
**PCT/JP2007/064007**

(87) International publication number:
**WO 2008/007780 (17.01.2008 Gazette 2008/03)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **13.07.2006 JP 2006193044**

(71) Applicant: **Kyowa Hakko Kirin Co., Ltd.**
**Tokyo 100-8185 (JP)**

(72) Inventors:
- **NAKASATO, Yoshisuke**
**Nagaizumi-cho, Sunto-gun. Shizuoka 411- 8731 (JP)**
- **SAKU, Osamu**
**Nagaizumi-cho, Sunto-gun. Shizuoka 411- 8731 (JP)**
- **ATSUMI, Eri**
**Nagaizumi-cho, Sunto-gun. Shizuoka 411- 8731 (JP)**
- **SUGIMOTO, Yoshiyuki**
**6-6,Asahi-machi 3-chome,Machida-shi,Tokyo 194-8533 (JP)**
- **ISHIDA, Hiroshi**
**Philadelphia, PA 19107 (US)**

(74) Representative: **Vossius & Partner**
**Siebertstraße 4**
**81675 München (DE)**

(54) **PENTADIENAMIDE DERIVATIVE**

(57) The present invention provides a pentadienamide derivative represented by the formula (I):

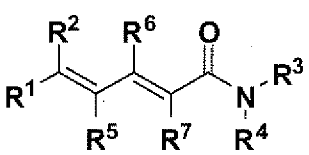

(I)

(wherein $R^1$ represents substituted or unsubstituted aryl or a substituted or unsubstituted aromatic heterocyclic group;
$R^2$ represents substituted or unsubstituted aryl, a substituted or unsubstituted aromatic heterocyclic group, a substituted or unsubstituted heteroalicyclic group, or the like;
$R^3$ represents a hydrogen atom or is combined together with $R^4$ and the adjacent nitrogen atom thereto to form a substituted or unsubstituted heterocyclic group;
$R^4$ represents substituted or unsubstituted aryl, a substituted or unsubstituted aromatic heterocyclic group, a substituted or unsubstituted heteroalicyclic group, or the like, or is combined together with $R^3$ and the adjacent nitrogen atom thereto to form a substituted or unsubstituted heterocyclic group; and
$R^5$, $R^6$, and $R^7$ may be the same or different, and each represents a hydrogen atom or methyl)
or a pharmaceutically acceptable salt thereof, and the like.

EP 2 050 734 A1

## Description

Technical Field

The present invention relates to a pentadienamide derivative or a pharmaceutically acceptable salt thereof having an activity to modify the function of a vanilloid receptor (TRPV1), and the like.

Background Art

Capsaicin (trans-8-methyl-N-vanillyl-6-nonenamide) which is the main component of hot peppers acts on receptors present in primary afferent sensory neurons (mainly C-fibers) thereby inducing stimulation (pain) and thereafter exhibiting an analgesic activity and an antiinflammatory activity. Recently, this receptor has been cloned and named vanilloid receptor subtype 1 (VR1) (Nature, vol. 389, p. 816 (1997)). Thereafter, it has been revealed that the receptor belongs to one of the subfamilies of ion channels having six transmembrane domains and showing a high $Ca^{2+}$ permeability which is structurally related to a TRP (transient receptor potential) channel family, and therefore, it is called TRPV1 at present. TRPV1 is activated not only by capsaicin, but also by heat stimulation or proton stimulation. Further, as an endogenous ligand, anandamide which is an endogenous cannabinoid, lipoxygenase (LOX) products such as leukotriene $B_4$, and N-arachidonyl dopamine (NADA) are presumed. Further, it has also been revealed that inflammation-related substances such as ATP or bradykinin act on metabotropic receptors and control the activity of TRPV1 via the activation of phospholipase C (PKC). Further, it has been known that in TRPV1-deficient mice, not only a pain response to capsaicin is lost, but also hyperalgesia during inflammation is attenuated (Nature, vol. 405, p. 183 (2000)). These suggest that there is a possibility that TRPV1 is associated with a pain in various pathologies.

When capsaicin acts on TRPV1 on a primary afferent sensory neuron, a cation channel is opened, and the membrane is depolarized so that neuropeptides such as substance P are released to induce a pain. However, it has been known that continuous activation of TRPV1 makes an afferent neuron unresponsive (desensitization) to pain stimulation and an analgesic activity is exhibited instead (Pharmacological Reviews, vol. 51, p. 159 (1999)). In fact, a capsaicin cream has been used for the treatment of neuropathic pain such as postherpetic neuralgia or diabetic neuropathy, or inflammatory pain such as rheumatoid arthritis. Further, intravesical injection of capsaicin or resiniferatoxin (RTX) which is a related substance of capsaicin alleviates bladder dysfunction seen in spinal cord injury patients and the like due to desensitization similar to an analgesic activity (Life Sciences, vol. 51, p. 1777 (1992); Journal of Urology, vol. 158, p. 2087 (1997)).

Not only a TRPV1 agonist shows desensitization, but also a TRPV1 antagonist blocks the release of a neurotransmitter thereby showing an analgesic activity. It has been known that a TRPV1 antagonist, capsazepine or N-(4-tert-butylphenyl)-4-(3-chloropyridin-2-yl)tetrahydropyrazine-1(2H)-carboxamide (BCTC) shows an effect on neuropathic pain or inflammatory pain in animal models (The Journal of Pharmacology and Experimental Therapeutics, vol. 304, p. 56 (2003); The Journal of Pharmacology and Experimental Therapeutics, vol. 306, p. 387 (2003)). As described above, it is expected that inhibition of activation of TRPV1 will lead to prevention or treatment of symptoms or diseases associated with the activation of TRPV1 as well as pain relief.

Accordingly, it is believed that an agonist or an antagonist having an activity to modify the function of TRPV1 is useful for various pains such as neuropathic pain and inflammatory pain, headaches such as migraine and cluster headache, bladder diseases such as overactive bladder and interstitial cystitis, ulcerative colitis, pruritus, allergic and nonallergic rhinitis, and respiratory diseases such as asthma and chronic obstructive pulmonary disease.
As a TRPV1 ligand, a compound represented by the formula (a):

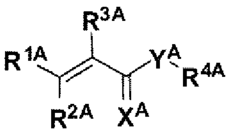 (a)

(wherein $R^{1A}$ is substituted or unsubstituted phenyl or the like;
$R^{2A}$ is substituted or unsubstituted phenyl or the like;
$R^{3A}$ is a hydrogen atom, lower alkyl, or the like;
$R^{4A}$ is a saturated or unsaturated 5- or 6-membered heterocyclic group or the like;
$X^A$ is an oxygen atom or the like; and
$Y^A$ is NH or the like)
is described in Patent document 1.
On the other hand, a pentadienamide derivative represented by the formula (b):

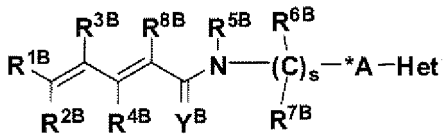

(b)

[wherein $Y^B$ is an oxygen atom or a sulfur atom;
*A is p-phenylene or *-$(CH_2)_n$- $(X^B)_m$-$(CH_2)_r$-
$X^B$ is an oxygen atom, a sulfur atom or -CH=CH-
n and r are independently 0 to 3;
s is 0 or 1;
m is 0 or 1;
with the proviso that when m is 1, n+s is at least 2;
$R^{1B}$ and $R^{2B}$ are independently a hydrogen atom, lower alkyl, cycloalkyl, lower alkenyl, Het or aryl;
$R^{3B}$, $R^{4B}$, and $R^{8B}$ are independently a hydrogen atom, lower alkyl, or aryl;
$R^{5B}$ and $R^{6B}$ are independently a hydrogen atom or lower alkyl;
$R^{7B}$ is a hydrogen atom, lower alkyl, cycloalkyl, Het-lower alkyl, or aryl;
Het is a monocyclic 5- or 6-membered heteroaromatic group or a bicyclic heteroaromatic group containing one or two heteroatoms selected from a nitrogen atom and a sulfur atom, which may be substituted with lower alkyl, halogen, or phenyl; and
the asterisk means a bonding point]
has been reported as a compound showing an activity as a platelet-activating factor antagonist (see Patent document 2).

Further, an antihypercholesterolemic agent (see Patent document 3), an agent for improving arteriosclerosis (see Patent document 4), and an antimalarial agent (see Non-patent documents 1 and 2) all of which contain a 5,5-diphenylpenta-2,4-dienamide derivative have been known.
As described above, an agonist or an antagonist having an activity to modify the function of TRPV1 can be expected as a therapeutic agent for various pains such as neuropathic pain and inflammatory pain, headaches such as migraine and cluster headache, bladder diseases such as overactive bladder and interstitial cystitis, ulcerative colitis, pruritus, allergic and nonallergic rhinitis, and respiratory diseases such as asthma and chronic obstructive pulmonary disease.

Patent document 1: WO 03/049702
Patent document 2: US Patent No. 4788206
Patent document 3: Japanese Published Unexamined Patent Application No. 316144/1995
Patent document 4: EP Patent No. 0124791
Non-Patent document 1: Journal of Medicinal Chemistry, vol. 12, p. 946 (1969)
Non-Patent document 2: Journal of Medicinal Chemistry, vol. 11, p. 749 (1968)

Disclosure of the invention

Problems to be solved by the Invention

An object of the present invention is to provide a pentadienamide derivative or a pharmaceutically acceptable salt thereof having an activity to modify the function of TRPV1, and the like.

Means for Solving the Problems

The present invention relates to the following (1) to (36).

(1) A pentadienamide derivative represented by the formula (I):

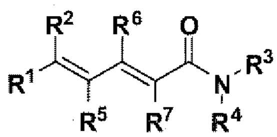

(I)

(wherein $R^1$ represents substituted or unsubstituted aryl or a substituted or unsubstituted aromatic heterocyclic group; $R^2$ represents substituted or unsubstituted cycloalkyl, substituted or unsubstituted cycloalkenyl, substituted or unsubstituted aryl, a substituted or unsubstituted aromatic heterocyclic group, or a substituted or unsubstituted heteroalicyclic group;
$R^3$ represents a hydrogen atom or is combined together with $R^4$ and the adjacent nitrogen atom thereto to form a substituted or unsubstituted heterocyclic group;
$R^4$ represents substituted or unsubstituted lower alkyl, substituted or unsubstituted cycloalkyl, substituted or unsubstituted aryl, a substituted or unsubstituted aromatic heterocyclic group, or a substituted or unsubstituted heteroalicyclic group, or is combined together with $R^3$ and the adjacent nitrogen atom thereto to form a substituted or unsubstituted heterocyclic group; and
$R^5$, $R^6$, and $R^7$ may be the same or different, and each represents a hydrogen atom or methyl), or a pharmaceutically acceptable salt thereof.

(2) The pentadienamide derivative or the pharmaceutically acceptable salt thereof according to the above (1), wherein each of $R^5$, $R^6$, and $R^7$ is a hydrogen atom.
(3) The pentadienamide derivative or the pharmaceutically acceptable salt thereof according to the above (1) or (2), wherein $R^3$ and $R^4$ are combined together with the adjacent nitrogen atom thereto to form a substituted or unsubstituted heterocyclic group.
(4) The pentadienamide derivative or the pharmaceutically acceptable salt thereof according to the above (1) or (2), wherein $R^3$ and $R^4$ are combined together with the adjacent nitrogen atom thereto to form substituted or unsubstituted thiomorpholino.
(5) The pentadienamide derivative or the pharmaceutically acceptable salt thereof according to the above (1) or (2), wherein $R^3$ and $R^4$ are combined together with the adjacent nitrogen atom thereto to form substituted or unsubstituted piperidino, or substituted or unsubstituted piperazinyl.
(6) The pentadienamide derivative or the pharmaceutically acceptable salt thereof according to the above (1) or (2), wherein $R^3$ is a hydrogen atom.
(7) The pentadienamide derivative or the pharmaceutically acceptable salt thereof according to the above (6), wherein $R^4$ is substituted or unsubstituted aryl.
(8) The pentadienamide derivative or the pharmaceutically acceptable salt thereof according to the above (6), wherein $R^4$ is substituted or unsubstituted dihydrobenzoxazinyl.
(9) The pentadienamide derivative or the pharmaceutically acceptable salt thereof according to the above (6), wherein $R^4$ is substituted or unsubstituted tetrahydroisoquinolyl.
(10) The pentadienamide derivative or the pharmaceutically acceptable salt thereof according to the above (6), wherein $R^4$ is a substituted or unsubstituted aromatic heterocyclic group.

(11) The pentadienamide derivative or the pharmaceutically acceptable salt thereof according to the above (6), wherein $R^4$ is substituted or unsubstituted indazolyl.
(12) The pentadienamide derivative or the pharmaceutically acceptable salt thereof according to the above (6), wherein $R^4$ is substituted or unsubstituted isoquinolyl.
(13) The pentadienamide derivative or the pharmaceutically acceptable salt thereof according to any one of the above (1) to (12), wherein $R^1$ is substituted or unsubstituted aryl.
(14) The pentadienamide derivative or the pharmaceutically acceptable salt thereof according to any one of the above (1) to (12), wherein $R^1$ is 4-(trifluoromethyl)phenyl.
(15) The pentadienamide derivative or the pharmaceutically acceptable salt thereof according to any one of the above (1) to (14), wherein $R^2$ is substituted or unsubstituted aryl.
(16) The pentadienamide derivative or the pharmaceutically acceptable salt thereof according to any one of the above (1) to (14), wherein $R^2$ is substituted or unsubstituted phenyl.
(17) The pentadienamide derivative or the pharmaceutically acceptable salt thereof according to any one of the above (1) to (14), wherein $R^2$ is phenyl substituted with substituted or unsubstituted lower alkoxy.

(18) The pentadienamide derivative or the pharmaceutically acceptable salt thereof according to any one of the above (1) to (14), wherein $R^2$ is 4-(trifluoromethyl)phenyl.
(19) The pentadienamide derivative or the pharmaceutically acceptable salt thereof according to any one of the above (1) to (14), wherein $R^2$ is a substituted or unsubstituted aromatic heterocyclic group.
(20) The pentadienamide derivative or the pharmaceutically acceptable salt thereof according to any one of the above (1) to (14), wherein $R^2$ is substituted or unsubstituted pyridyl or substituted or unsubstituted pyrimidinyl.
(21) A pharmaceutical composition, which comprises, as an active ingredient, the pentadienamide derivative or the pharmaceutically acceptable salt thereof described in any one of the above (1) to (20).
(22) A vanilloid receptor (TRPV1) agonist, which comprises, as an active ingredient, the pentadienamide derivative or the pharmaceutically acceptable salt thereof described in any one of the above (1) to (20).
(23) A vanilloid receptor (TRPV1) antagonist, which comprises, as an active ingredient, the pentadienamide derivative or the pharmaceutically acceptable salt thereof described in any one of the above (1) to (20).
(24) A preventive and/or therapeutic agent for a disease associated with the function of a vanilloid receptor (TRPV1),

which comprises, as an active ingredient, the pentadienamide derivative or the pharmaceutically acceptable salt thereof described in any one of the above (1) to (20).

(25) A preventive and/or therapeutic agent for a pain, which comprises, as an active ingredient, the pentadienamide derivative or the pharmaceutically acceptable salt thereof described in any one of the above (1) to (20).

(26) The preventive and/or therapeutic agent according to the above (25), wherein the pain is neuropathic pain.

(27) A method for agonizing a vanilloid receptor (TRPV1), which comprises the step of administering an effective amount of the pentadienamide derivative or the pharmaceutically acceptable salt thereof described in any one of the above (1) to (20).

(28) A method for antagonizing a vanilloid receptor (TRPV1), which comprises the step of administering an effective amount of the pentadienamide derivative or the pharmaceutically acceptable salt thereof described in any one of the above (1) to (20).

(29) A method for preventing and/or treating a disease associated with the function of a vanilloid receptor (TRPV1), which comprises the step of administering an effective amount of the pentadienamide derivative or the pharmaceutically acceptable salt thereof described in any one of the above (1) to (20).

(30) A method for preventing and/or treating a pain, which comprises the step of administering an effective amount of the pentadienamide derivative or the pharmaceutically acceptable salt thereof described in any one of the above (1) to (20).

(31) The method for preventing and/or treating according to the above (30), wherein the pain is neuropathic pain.

(32) Use of the pentadienamide derivative or the pharmaceutically acceptable salt thereof described in any one of the above (1) to (20), for the manufacture of a vanilloid receptor (TRPV1) agonist.

(33) Use of the pentadienamide derivative or the pharmaceutically acceptable salt thereof described in any one of the above (1) to (20), for the manufacture of a vanilloid receptor (TRPV1) antagonist.

(34) Use of the pentadienamide derivative or the pharmaceutically acceptable salt thereof described in any one of the above (1) to (20), for the manufacture of a preventive and/or therapeutic agent for a disease associated with the function of a vanilloid receptor (TRPV1).

(35) Use of the pentadienamide derivative or the pharmaceutically acceptable salt thereof described in any one of the above (1) to (20), for the manufacture of a preventive and/or therapeutic agent for a pain.

(36) The use according to the above (35), wherein the pain is neuropathic pain.

## Best Mode for Carrying Out the Invention

Hereinafter, the compound represented by the formula (I) is referred to as Compound (I). The compounds having the other formula numbers are referred to in the same manner.

The definitions of the respective groups in the formula (I) are as follows.

Examples of the lower alkyl include linear or branched alkyl having 1 to 10 carbon atoms. More specific examples thereof include methyl, ethyl, propyl, isopropyl, butyl, isobutyl, sec-butyl, tert-butyl, pentyl, neopentyl, hexyl, heptyl, octyl, nonyl, decyl, and the like.

Examples of the cycloalkyl include cycloalkyl having 1 to 10 carbon atoms. More specific examples thereof include cyclopropyl, cyclobutyl, cyclopentyl, cyclohexyl, cycloheptyl, cyclooctyl, cyclodecyl, noradamantyl, adamantyl, bicyclo[2.2.1]heptyl, bicyclo[2.2.2]octyl, bicyclo[3.3.0]octyl, bicyclo[3.3.1]nonyl, and the like.

Examples of the cycloalkenyl include cycloalkenyl having 3 to 10 carbon atoms. More specific examples thereof include cyclopropenyl, cyclobutenyl, cyclopentenyl, cyclohexenyl, cycloheptenyl, cyclooctenyl, cyclononenyl, cyclodecenyl, and the like.

Examples of the aryl include aryl having 6 to 14 carbon atoms. More specific examples thereof include phenyl, naphthyl, indenyl, anthryl, and the like. The aryl may be aryl formed by condensation with a heteroalicyclic group such as 1,2,3,4-tetrahydroquinolyl, 1,2,3,4-tetrahydroisoquinolyl, dihydroquinoxalinyl, tetrahydroquinoxalinyl, dihydrobenzofuranyl, chromanyl, isochromanyl, and dihydrobenzoxazinyl.

Examples of the aromatic heterocyclic group include a 5- or 6-membered monocyclic aromatic heterocyclic group which contains at least one atom selected from a nitrogen atom, an oxygen atom, and a sulfur atom; a bicyclic or tricyclic condensed aromatic heterocyclic group which is formed by condensation of 3- to 8-membered rings and contains at least one atom selected from a nitrogen atom, an oxygen atom, and a sulfur atom; and the like. More specific examples thereof include pyridyl, pyridonyl, pyrazinyl, pyrimidinyl, pyridazinyl, quinolyl, isoquinolyl, phthalazinyl, quinazolinyl, quinoxalinyl, naphthyridinyl, cinnolinyl, pyrrolyl, pyrazolyl, imidazolyl, triazolyl, tetrazolyl, thienyl, furyl, thiazolyl, oxazolyl, indolyl, indazolyl, benzimidazolyl, isooxazolyl, benzotriazolyl, benzothiazolyl, benzoxazolyl, purinyl, acridinyl, carbazolyl, pyranyl, furazanyl, benzofrazanyl, thiadiazolyl, and the like.

Examples of the heteroalicyclic group include a 5-or 6-membered monocyclic heteroalicyclic group which contains at least one atom selected from a nitrogen atom, an oxygen atom, and a sulfur atom; a bicyclic or tricyclic condensed heteroalicyclic group which is formed by condensation of 3- to 8-membered rings and contains at least one

atom selected from a nitrogen atom, an oxygen atom, and a sulfur atom; and the like. More specific examples thereof include pyrrolidinyl, pyrrolidonyl, piperidino, piperidyl, piperazinyl, morpholino, morpholinyl, thiomorpholino, thiomorpholinyl, homopiperidino, homopiperidyl, homopiperazinyl, tetrahydropyridyl, tetrahydroquinolyl, tetrahydroisoquinolyl, tetrahydrofuranyl, tetrahydropyranyl, dihydrobenzofuranyl, indolinyl, isoindolinyl, dihydroquinolyl, dihydroisoquinolyl, dihydropyranyl, oxiranyl, oxetanyl, thietanyl, aziridinyl, azetidinyl, azepanyl, oxazepanyl, and the like.

Examples of the heterocyclic group formed together with the adjacent nitrogen atom thereto include an aromatic heterocyclic group formed together with the adjacent nitrogen atom thereto and a heteroalicyclic group formed together with the adjacent nitrogen atom thereto.

Examples of the aromatic heterocyclic group formed together with the adjacent nitrogen atom thereto include a 5- or 6-membered monocyclic aromatic heterocyclic group which contains at least one nitrogen atom (the monocyclic aromatic heterocyclic group may further contain another nitrogen atom, an oxygen atom, or a sulfur atom), a bicyclic or tricyclic condensed aromatic heterocyclic group which is formed by condensation of 3- to 8-membered rings and contains at least one nitrogen atom (the condensed aromatic heterocyclic group may further contain another nitrogen atom, an oxygen atom, or a sulfur atom), and the like. More specific examples thereof include pyrrolyl, imidazolyl, indolyl, indazolyl, carbazolyl, and the like.

Examples of the heteroalicyclic group formed together with the adjacent nitrogen atom thereto include 5- or 6-membered monocyclic heteroalicyclic group which contains at least one nitrogen atom (the monocyclic heteroalicyclic group may further contain another nitrogen atom, an oxygen atom, or a sulfur atom), a bicyclic or tricyclic condensed heteroalicyclic group which is formed by condensation of 3- to 8-membered rings and contains at least one nitrogen atom (the condensed heteroalicyclic group may further contain another nitrogen atom, an oxygen atom, or a sulfur atom) and the like. More specific examples thereof include pyrrolidinyl, pyrrolidonyl, piperidino, piperazinyl, morpholino, thiomorpholino, homopiperidino, homopiperazinyl, tetrahydropyridyl, tetrahydroquinolyl, tetrahydroisoquinolyl, indolinyl, isoindolinyl, dihydroquinolyl, dihydroisoquinolyl, and the like.

More specific examples of the substituents of the substituted lower alkyl, the substituted cycloalkyl, the substituted cycloalkenyl, the substituted aryl, the substituted aromatic heterocyclic group, the substituted heteroalicyclic group, and the substituted heterocyclic group formed together with the adjacent nitrogen atom thereto, which may be the same or different and in number of, for example, 1 to 3, include halogen, hydroxy, amino, hydroxyimino, amidino, hydroxyamidino, nitro, mercapto, cyano, carboxy, methylenedioxy, ethylenedioxy, carbamoyl, sulfamoyl, sulfamoyloxy, lower alkenyl, cycloalkenyl, lower alkynyl, lower alkoxycarbonyl, mono- or di-lower alkylcarbamoyl, lower alkylsulfinyl, lower alkylthio, aryloxy, aralkyloxy, aroyl, lower alkoxycarbonylamino, mono- or di-(substituted or unsubstituted lower alkyl)amino [more specific examples of the substituents of the substituted lower alkylamino, the lower alkyl(substituted lower alkyl)amino and di-substituted lower alkylamino, which may be the same or different and in number of, for example, 1 to 3, include halogen, hydroxy, lower alkoxy, and the like], substituted or unsubstituted lower alkanoylamino (more specific examples of the substituent of the substituted lower alkanoylamino, which may be the same or different and in number of, for example, 1 to 3, include halogen, hydroxy, cyano, and the like), substituted or unsubstituted lower alkylsulfonyl (more specific examples of the substituent of the substituted lower alkylsulfonyl, which may be the same or different and in number of, for example, 1 to 3, include halogen, hydroxy, cyano, and the like), substituted or unsubstituted lower alkoxy [more specific examples of the substituent of the substituted lower alkoxy, which may be the same or different and in number of, for example, 1 to 3, include halogen, amino, mono- or di-(lower alkyl)amino, hydroxy, cyano, lower alkoxy, lower alkoxycarbonyl, lower alkoxycarbonylamino, lower alkanoyl, cycloalkyl, lower alkyl substituted cycloalkyl, and the like], substituted or unsubstituted cycloalkyloxy (more specific examples of the substituent of the substituted cycloalkyloxy, which may be the same or different and in number of, for example, 1 to 3, include halogen, hydroxy, lower alkyl, lower alkoxy, lower alkanoyl, and the like), substituted or unsubstituted lower alkanoyl (more specific examples of the substituent of the substituted lower alkanoyl, which may be the same or different and in number of, for example, 1 to 3, include halogen and the like), substituted or unsubstituted aryl (more specific examples of the substituent of the substituted aryl, which may be the same or different and in number of, for example, 1 to 3, include halogen, nitro, hydroxy, lower alkoxy, and the like), substituted or unsubstituted aralkyl (more specific examples of the substituent of the substituted aralkyl, which may be the same or different and in number of, for example, 1 to 3, include halogen, hydroxy, lower alkoxy, and the like), a substituted or unsubstituted aromatic heterocyclic group (more specific examples of the substituent of the substituted aromatic heterocyclic group, which may be the same or different and in number of, for example, 1 to 3, include halogen, hydroxy, lower alkyl, lower alkoxy, lower alkanoyl, and the like), substituted or unsubstituted aromatic heterocyclic aminosulfonyl (more specific examples of the substituent of the substituted aromatic heterocyclic aminosulfonyl, which may be the same or different and in number of, for example, 1 to 3, include halogen, hydroxy, lower alkyl, lower alkoxy, lower alkanoyl, and the like), a substituted or unsubstituted heteroalicyclic group (more specific examples of the substituent of the substituted heteroalicyclic group, which may be the same or different and in number of, for example, 1 to 3, include halogen, hydroxy, lower alkyl, lower alkoxy, lower alkanoyl, and the like), substituted or unsubstituted heteroalicyclic oxy (more specific examples of the substituent of the substituted heteroalicyclic oxy, which may be the same or different and in number of, for example, 1 to 3, include halogen, hydroxy, lower

alkyl, lower alkoxy, lower alkanoyl, and the like), substituted or unsubstituted heteroalicyclic alkyloxy (more specific examples of the substituent of the substituted heteroalicyclic alkyloxy, which may be the same or different and in number of, for example, 1 to 3, include halogen, hydroxy, lower alkyl, lower alkoxy, lower alkanoyl, and the like), and the like. Incidentally, the substituents of the substituted aryl, the substituted aromatic heterocyclic group, the substituted heteroalicyclic group, the substituted aromatic heterocyclic group formed together with the adjacent nitrogen atom thereto, and the substituted heteroalicyclic group formed together with the adjacent nitrogen atom thereto may be substituted or unsubstituted lower alkyl (more specific examples of the substituent of the substituted lower alkyl, which may be the same or different and in number of, for example, 1 to 3, include halogen, amino, hydroxy, hydroxyimino, cyano, mono- or di-lower alkylamino, an aromatic heterocyclic group, a heteroalicyclic group, and the like). Further, the substituents of the substituted heteroalicyclic group and the substituted heteroalicyclic group formed together with the adjacent nitrogen atom thereto may be oxo. Further, the substituent in the case where the substituted aryl is aryl formed by condensation with a substituted heteroalicyclic group also includes oxo. Further, in the case where the heteroalicyclic group, the aromatic heterocyclic group, and the heterocyclic group formed together with the adjacent nitrogen atom thereto have a nitrogen atom and/or a sulfur atom in its ring, the nitrogen atom and/or the sulfur atom may be oxidized.

Here, the lower alkyl, the cycloalkyl, the cycloalkenyl, the aryl, the aromatic heterocyclic group, and the heteroalicyclic group have the same definitions as described above, respectively.
Examples of the halogen include each atom of fluorine, chlorine, bromine, and iodine.
The lower alkyl moiety of the lower alkoxy, the lower alkoxycarbonyl, the lower alkoxycarbonylamino, the mono- or di-lower alkylamino, the mono- or di-lower alkylcarbamoyl, the lower alkyl substituted cycloalkyl, the lower alkyl sulfinyl, the lower alkyl sulfonyl, and the lower alkylthio has the same definition as the lower alkyl described above. Incidentally, the two lower alkyl moieties of the di-lower alkylamino and the di-lower alkylcarbamoyl may be the same or different, respectively.

The cycloalkylene moiety of the lower alkyl substituted cycloalkyl has the same definition as cycloalkylene formed by removing one hydrogen atom from the cycloalkyl described above.
The cycloalkyl moiety of the cycloalkyloxy has the same definition as the cycloalkyl described above.
The alkylene moiety of the aralkyl, the aralkyloxy, and the heteroalicyclic alkyloxy has the same definition as alkylene formed by removing one hydrogen atom from the lower alkyl described above.

Examples of the lower alkenyl include linear or branched alkenyl having 3 to 10 carbon atoms. More specific examples thereof include allyl, 2-butenyl, 3-butenyl, 4-pentenyl, 5-hexenyl, 6-heptenyl, 6-octenyl, 2,6-octadienyl, 9-decenyl, and the like.
Examples of the lower alkynyl include linear or branched alkynyl having 3 to 6 carbon atoms. More specific examples thereof include propargyl, 3-butynyl, 3-hexynyl, 4-methyl-2-pentynyl, and the like.

Examples of the lower alkanoyl and the lower alkanoyl moiety of the lower alkanoylamino include linear or branched alkanoyl having 1 to 8 carbon atoms. More specific examples thereof include formyl, acetyl, propionyl, butyryl, isobutyryl, valeryl, isovaleryl, pivaloyl, hexanoyl, heptanoyl, octanoyl, and the like.
The aryl moiety of the aralkyl, the aryloxy, the aralkyloxy, and aroyl has the same definition as the aryl described above.

The heteroalicyclic group moiety of the heteroalicyclic oxy and the heteroalicyclic alkyloxy has the same definition as the heteroalicyclic group described above.
The aromatic heterocyclic group moiety of the aromatic heterocyclic aminosulfonyl has the same definition as the aromatic heterocyclic group described above.
Examples of the pharmaceutically acceptable salt of Compound (I) include pharmaceutically acceptable metal salts, ammonium salts, organic amine addition salts, amino acid addition salts, acid addition salts, and the like. Examples of the pharmaceutically acceptable metal salts include alkali metal salts such as sodium salts and potassium salts, alkaline earth metal salts such as magnesium salts and calcium salts, aluminum salts, zinc salts, and the like. Examples of the pharmaceutically acceptable ammonium salts include salts of ammonium, tetramethylammonium, and the like. Examples of the pharmaceutically acceptable organic amine addition salts include addition salts of morpholine, piperidine, and the like. Examples of the pharmaceutically acceptable amino acid addition salts include addition salts of amino acids such as lysine, glycine, and phenylalanine. Examples of the pharmaceutically acceptable acid addition salts include inorganic acid salts such as hydrochlorides, sulfates, and phosphates, organic acid salts such as acetates, maleates, fumarates, tartrates, and citrates, and the like.

Among Compounds (I) of the present invention, some may include various stereoisomers, regioisomers, tautomers, enantiomers, and the like. All these possible isomers and mixtures thereof are included in the invention, and the mixing ratio thereof may be any ratio.
Examples of the disease associated with the function of TRPV1 include various pains such as neuropathic pain, trigeminal neuralgia, diabetic pain, postherpetic neuralgia, HIV-related pain, fibromyalgia, inflammatory pain, and cancer pain, headaches such as migraine and cluster headache, bladder diseases such as overactive bladder and interstitial cystitis, ulcerative colitis, pruritus, allergic and nonallergic rhinitis, respiratory diseases such as asthma and chronic obstructive pulmonary disease, and the like.

Hereinafter, production methods for Compound (I) will be described.
In the production methods described below, when a defined group changes under the conditions of the implementation method or is not suitable for carrying out the method, it is possible to easily perform production using a method commonly used in synthetic organic chemistry, for example, by means of protection of a functional group and deprotection thereof [see, for example, Protective Groups in Organic Synthesis, 3rd edition, T. W. Greene, John Wiley & Sons Inc. (1999), etc.] or the like. If necessary, the order of reaction steps such as introduction of a substituent can also be changed.
Compound (I) can be obtained, for example, according to the reaction steps described in the following Production methods 1 to 3. The symbols Et and Bu in the following production methods represent ethyl and butyl, respectively.

Production method 1

Among Compounds (I), Compound (Ia) in which $R^5$, $R^6$, and $R^7$ are hydrogen atoms can be obtained by, for example, the following Production method 1.

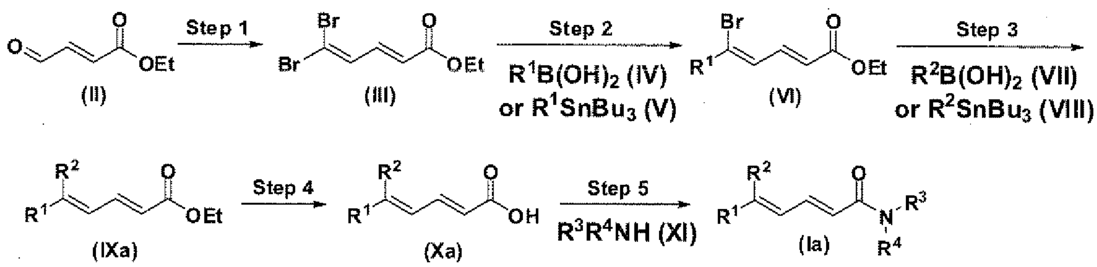


(II) (III) (VI) (IXa) (Xa) (Ia)

(wherein $R^1$, $R^2$, $R^2$, and $R^4$ have the same definitions as described above, respectively)

Step 1

Compound (III) can be prepared by treating Compound (II) in a solvent in the presence of preferably 2 to 10 equivalents of carbon tetrabromide and preferably 4 to 10 equivalents of triphenylphosphine at a temperature between 0˚C and the boiling point of the solvent used for 5 minutes to 72 hours.
Compound (II) can be obtained as a commercially available product.
Examples of the solvent include dichloromethane, chloroform, 1,2-dimethoxyethane (DME), dioxane, and the like, and these can be used alone or as a mixture thereof.

Step 2

Compound (VI) can be prepared by reacting Compound (III) with preferably 1 to 1.5 equivalents of Compound (IV) or (V) in a solvent in the presence of preferably 0.001 to 0.5 equivalent of a palladium catalyst, if necessary, in the presence of preferably 0.001 to 5 equivalents of a ligand and preferably 0.1 to 10 equivalents of a base at a temperature between 0˚C and the boiling point of the solvent used for 5 minutes to 72 hours.
Compounds (IV) and (V) are obtained as commercially available products or can be obtained by a known method [for example, "The Experimental Chemical Course 18 (Jikken Kagaku Koza 18), Synthesis of Organic Compounds VI, Organic Syntheses Using Metals", 5th. Ed., p. 97, Maruzen (2005)] or a modified method thereof.
Examples of the palladium catalyst include palladium acetate, tris(dibenzylideneacetone)dipalladium, tetrakis(triphenylphosphine)palladium, 1,1'-bis(diphenylphosphino)ferrocene-dichloropalladium/dichloromethane 1:1 adduct, and the like.
Examples of the ligand include tri(o-tolyl)phosphine, tri(2-furyl)phosphine, di-tert-butyldiphenylphosphine, and the like.
Examples of the base include potassium carbonate, potassium phosphate, potassium hydroxide, sodium hydroxide, potassium tert-butoxide, triethylamine, diisopropylethylamine, N-methylmorpholine, pyridine, 1,8-diazabicyclo[5.4.0]-7-undecene (DBU), and the like.
Examples of the solvent include methanol, ethanol, dichloromethane, chloroform, 1,2-dichloroethane, toluene, ethyl acetate, acetonitrile, diethyl ether, tetrahydrofuran (THF), DME, dioxane, N,N-dimethylformamide (DMF), N,N-dimethylacetamide (DMA), N-methylpyrrolidone (NMP), water, and the like. These are used alone or as a mixture thereof.

Step 3

Compound (IXa) can be prepared by subjecting Compound (VI) and Compound (VII) or (VIII) to a reaction in a similar manner to Step 2.

Step 4

Compound (Xa) can be prepared by treating Compound (IXa) in a solvent in the presence of preferably 1 equivalent to a large excess amount of a base at a temperature between 0˚C and the boiling point of the solvent used for 5 minutes to 72 hours.
Examples of the base include potassium carbonate, lithium hydroxide, potassium hydroxide, sodium hydroxide, sodium methoxide, and the like.
Examples of the solvent include solvents containing water, and for example, a solvent obtained by adding water to methanol, ethanol, dichloromethane, chloroform, 1,2-dichloroethane, toluene, ethyl acetate, acetonitrile, diethyl ether, THF, DME, dioxane, DMF, DMA, NMP, pyridine, or a mixed solvent thereof, or the like is used.

Step 5

Compound (Ia) can be prepared by (Step 5-1) treating Compound (Xa) without solvent or in a solvent, if necessary, in the presence of preferably 0.1 to 10 equivalents of a suitable additive with preferably 1 equivalent to a large excess amount of a chlorinating agent or a brominating agent at a temperature between - 20˚C and 150˚C for 5 minutes to 72 hours, and then (Step 5-2) reacting the resulting compound with preferably 1 to 10 equivalents of Compound (XI) without solvent or in a solvent, if necessary, in the presence of preferably 1 to 10 equivalents of a base at a temperature between -20˚C and 150˚C for 5 minutes to 72 hours.
Examples of the chlorinating agent used in (Step 5-1) include thionyl chloride, oxalyl chloride, phosphorous oxychloride, and the like. Examples of the brominating agent include thionyl bromide, phosphorous oxybromide, and the like.
Examples of the additive used in (Step 5-1) include DMF, pyridine, and the like.
Examples of the solvent used in (Step 5-1) include dichloromethane, chloroform, 1,2-dichloroethane, toluene, ethyl acetate, acetonitrile, diethyl ether, THF, DME, dioxane, DMF, DMA, NMP, pyridine, and the like. These are used alone or as a mixture thereof.
Examples of the base used in (Step 5-2) include potassium carbonate, potassium hydroxide, sodium hydroxide, potassium tert-butoxide, triethylamine, diisopropylethylamine, N-methylmorpholine, pyridine, DBU, 4-dimethylaminopyridine (DMAP), and the like.
Examples of the solvent used in (Step 5-2) include methanol, ethanol, dichloromethane, chloroform, 1,2-dichloroethane, toluene, ethyl acetate, acetonitrile, diethyl ether, THF, DME, dioxane, DMF, DMA, NMP, pyridine, water, and the like. These are used alone or as a mixture thereof.
Further, as another method, Compound (Ia) can also be prepared by reacting Compound (Xa) with preferably 0.5 to 5 equivalents of Compound (XI) in a solvent in the presence of preferably 1 to 5 equivalents of a condensing agent, if necessary, in the presence of preferably 1 to 5 equivalents of an additive at a temperature between -20˚C and the boiling point of the solvent used for 5 minutes to 72 hours.
Examples of the condensing agent include 1,3-dicyclohexanecarbodiimide (DCC), 1-ethyl-3-(3-dimethylaminopropyl) carbodiimide hydrochloride (EDC), carbonyldiimidazole (CDI), 2-chloro-1-methylpyridinium iodide, and the like.
Examples of the additive include 1-hydroxybenzotriazole monohydrate (HOBt), and the like.
Examples of the solvent include methanol, ethanol, dichloromethane, chloroform, 1,2-dichloroethane, toluene, ethyl acetate, acetonitrile, diethyl ether, THF, DME, dioxane, DMF, DMA, NMP, pyridine, water, and the like. These are used alone or as a mixture thereof.

Production method 2

Among Compounds (I), Compound (Ib) in which $R^5$, $R^6$, and $R^7$ are hydrogen atoms and $R^2$ and $R^1$ are the same group can be obtained by, for example, the following Production method 2.

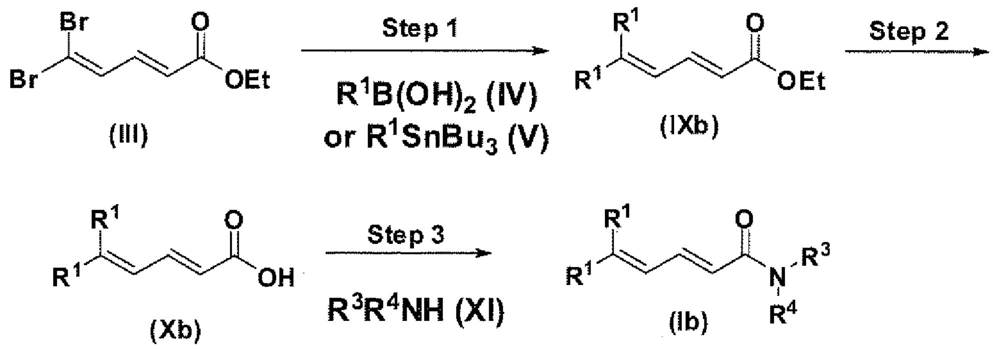


(wherein $R^1$, $R^3$, and $R^4$ have the same definitions as described above, respectively)

Step 1

Compound (IXb) can be prepared by subjecting Compound (III) and preferably 2 to 10 equivalents of Compound (IV) or (V) to a reaction in a similar manner to Step 2 of Production method 1.

Step 2

Compound (Xb) can be prepared by subjecting Compound (IXb) to a reaction in a similar manner to Step 4 of Production method 1.

Step 3

Compound (Ib) can be prepared by subjecting Compound (Xb) and Compound (XI) to a reaction in a similar manner to Step 5 of Production method 1.

Production method 3

Compound (Xa) can also be obtained by, for example, the following Production method 3.

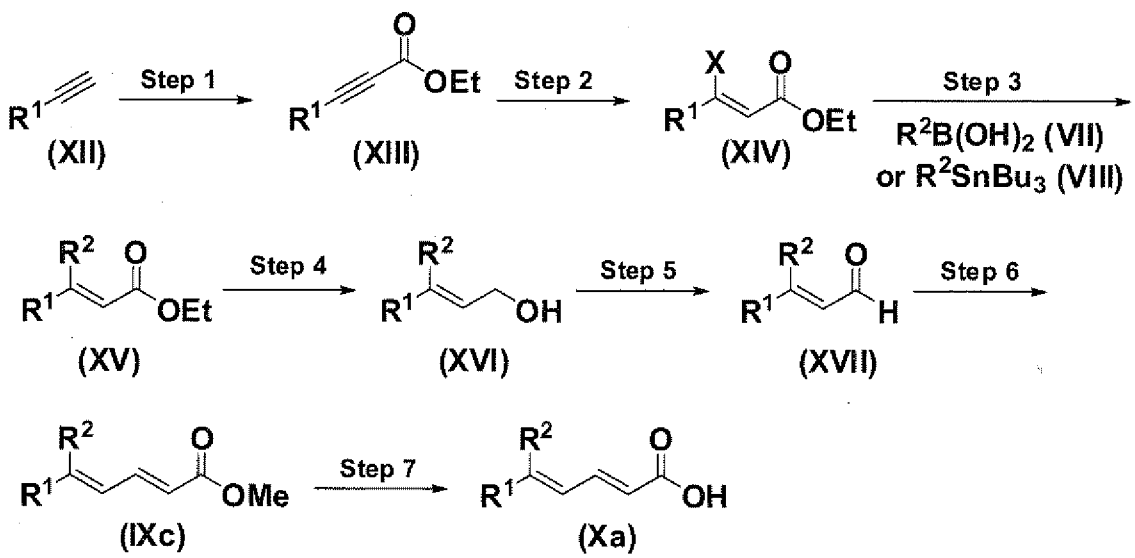


(wherein $R^1$ and $R^2$ have the same definitions as described above, respectively, and X represents halogen)

Step 1

Compound (XIII) can be prepared by treating Compound (XII) in a solvent in the presence of preferably 1 to 10 equivalents of a base with preferably 1 to 20 equivalents of ethyl chloroformate at a temperature between -78˚C and room temperature for 5 minutes to 72 hours.

Compound (XII) is obtained as a commercially available product or can be obtained by a known method [for example, "The Experimental Chemical Course 13 (Jikken Kagaku Koza 13), Synthesis of Organic Compounds I, Hydrocarbons/Halides", 5th. Ed. p. 283, Maruzen (2005)] or a modified method thereof. Examples of the base include lithium diisopropylamide, lithium bis(trimethylsilyl)amide, methyl lithium, n-butyl lithium, lithium hydride, sodium hydride, potassium hydride, methyl magnesium bromide, ethyl magnesium bromide, isopropyl magnesium chloride, and the like. These are used alone or in combination of two or more.

Examples of the solvent include THF, diethyl ether, DME, hexane, and the like. These are used alone or as a mixture thereof.

Step 2

Compound (XIV) can be prepared by treating Compound (XIII) in a solvent with preferably 1 to 10 equivalents of a halogenating agent at a temperature between -20˚C and the boiling point of the solvent used for 5 minutes to 72 hours.

Examples of the halogenating agent include sodium iodide, potassium iodide, potassium bromide, and the like. Examples of the solvent include acetone, 1,4-dioxane, acetonitrile, chloroform, dichloromethane, THF, ethyl acetate, DMF, acetic acid, water, and the like. These are used alone or as a mixture thereof.

Step 3

Compound (XV) can be prepared by subjecting Compound (XIV) and preferably 1 to 5 equivalents of Compound (VII) or (VIII) to a reaction in a similar manner to Step 2 of Production method 1.

Step 4

Compound (XVI) can be prepared by treating Compound (XV) in a solvent with preferably 1 to 10 equivalents of a reducing agent at a temperature between -78˚C and the boiling point of the solvent used for 5 minutes to 72 hours.

Examples of the reducing agent include lithium aluminum hydride, diisobutyl aluminum hydride, sodium bis(2-methoxyethoxy)aluminum hydride, lithium borohydride, sodium borohydride, and the like.

Examples of the solvent include methanol, ethanol, dichloromethane, chloroform, 1,2-dichloroethane, toluene, acetonitrile, diethyl ether, THF, DME, dioxane, DMF, DMA, NMP, and the like. These are used alone or as a mixture thereof.

Step 5

Compound (XVII) can be prepared by treating Compound (XVI) in a solvent with preferably 1 to 10 equivalents of an oxidizing agent at a temperature between -20˚C and the boiling point of the solvent used for 5 minutes to 2 hours.

Examples of the oxidizing agent include manganese dioxide, chromic acid, pyridinium chlorochromate, pyridinium dichromate, potassium permanganate, sulfur trioxide-pyridine, oxone, dimethyl sulfoxide (DMSO)/oxalyl chloride, and the like.

Examples of the solvent include dichloromethane, chloroform, 1,2-dichloroethane, toluene, ethyl acetate, acetonitrile, diethyl ether, THF, DME, dioxane, DMF, DMA, NMP, DMSO, pyridine, hydrochloric acid, acetic acid, propionic acid, acetic anhydride, sulfuric acid, water, and the like. These are used alone or as a mixture thereof.

Step 6

Compound (Ixc) can be prepared by reacting Compound (XVII) with preferably 1 to 10 equivalents of methyl triphenylphosphoranylidene acetate in a solvent in the presence of preferably 0.1 to 10 equivalents of a base at a temperature between -78˚C and the boiling point of the solvent used for 5 minutes to 72 hours.

Examples of the base include potassium acetate, sodium hydrogen carbonate, potassium carbonate, potassium hydroxide, sodium hydroxide, sodium methoxide, potassium tert-butoxide, triethylamine, diisopropylethylamine, N-methylmorpholine, pyridine, DBU, and the like.

Examples of the solvent include methanol, ethanol, dichloromethane, chloroform, 1,2-dichloroethane, toluene, ethyl acetate, acetonitrile, diethyl ether, THF, DME, dioxane, DMF, DMA, NMP, and the like. These are used alone or as a mixture thereof.

Step 7

Compound (Xa) can be prepared by subjecting Compound (Ixc) to a reaction in a similar manner to Step 4 of Production method 1.

Transformation of functional groups contained in Compound (I) and the intermediates can also be carried out by a known method [for example, Comprehensive Organic Transformations, R. C. Larock (1989)], other than the above-mentioned steps.

By suitably combining and performing the above-mentioned methods, Compound (I) having a desired functional group at a desired position can be obtained.

The intermediates and the desired compounds in the above-mentioned respective production methods can be isolated and purified through a separation or purification method generally employed in synthetic organic chemistry, for example, filtration, extraction, washing, drying, concentration, recrystallization, various types of chromatography, and the like. Further, the intermediate can be subjected to the subsequent reaction without particularly undergoing purification.

To obtain a salt of Compound (I), when Compound (I) is obtained in the form of a salt, it may be purified as it is. Further, when Compound (I) is obtained in a free form, Compound (I) may be dissolved or suspended in a suitable solvent, followed by addition of an acid or a base, and then, the resulting salt may be isolated and purified.

Further, Compound (I) and a pharmaceutically acceptable salt thereof may be present in the form of adducts with water or any of various solvents in some cases, and these adducts are also included in the present invention.

Specific examples of Compound (I) obtained by the above-mentioned production methods are shown in Tables 1 to 6.

[Table 1]

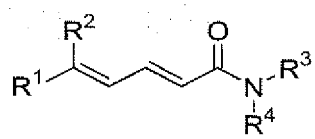

| Compound Number | $R^1$ | $R^2$ | $NR^3R^4$ |
|---|---|---|---|
| 1 | 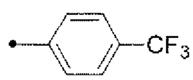 | 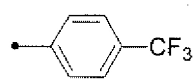 | 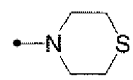 |
| 2 | 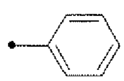 | 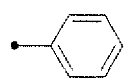 | 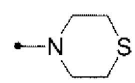 |
| 3 | 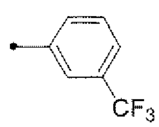 | 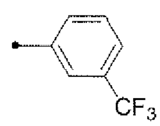 | 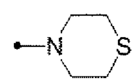 |
| 4 | 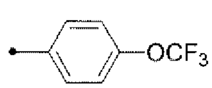 | 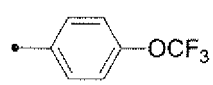 | 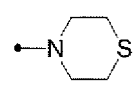 |
| 5 | 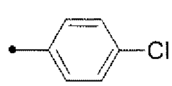 | 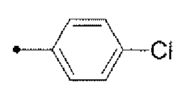 | 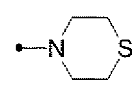 |
| 6 | 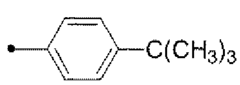 | 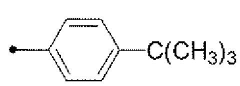 | 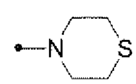 |

(continued)

| Compound Number | $R^1$ | $R^2$ | $NR^3R^4$ |
|---|---|---|---|
| 7 | 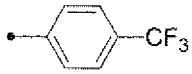 | 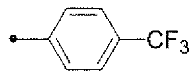 | 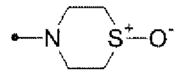 |
| 8 | 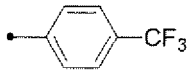 | 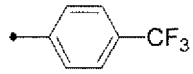 | 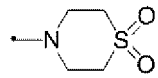 |
| 9 | 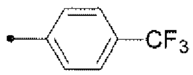 | 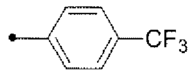 | 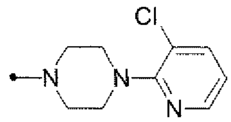 |

[Table 2-1]

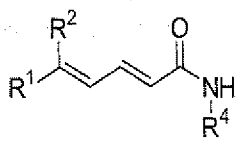

| Compound Number | $R^1$ | $R^2$ | $R^4$ |
|---|---|---|---|
| 10 | 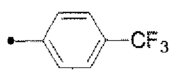 | 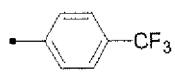 | 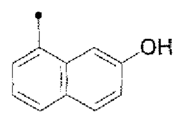 |
| 11 | 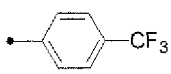 | 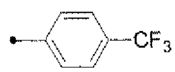 | 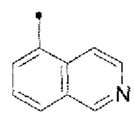 |
| 12 | 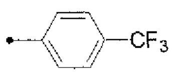 | 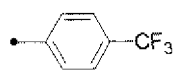 | 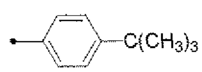 |
| 13 | 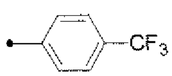 | 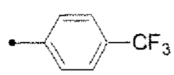 | 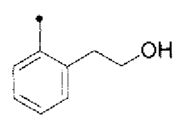 |
| 14 | 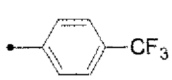 | 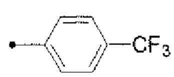 | 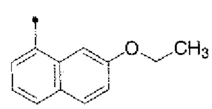 |

(continued)

| Compound Number | $R^1$ | $R^2$ | $R^4$ |
|---|---|---|---|
| 15 | 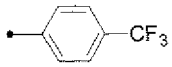 | 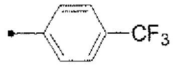 | 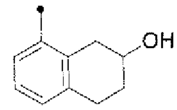 |
| 16 | 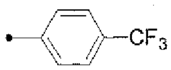 | 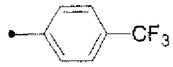 | 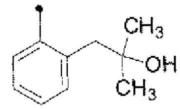 |
| 17 | 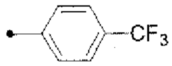 | 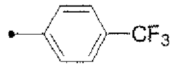 | 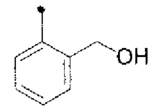 |
| 18 | 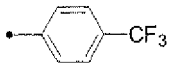 | 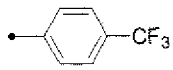 | 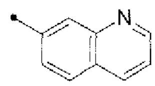 |
| 19 | 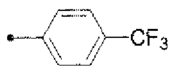 | 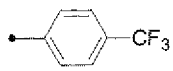 | 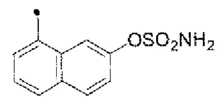 |
| 20 | 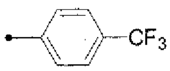 | 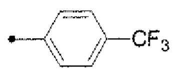 | 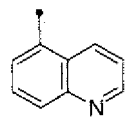 |

[Table 2-2]

| | | | |
|---|---|---|---|
| 21 | 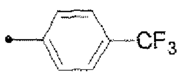 | 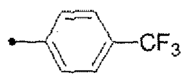 | 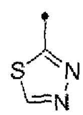 |
| 22 | 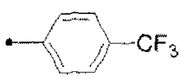 | 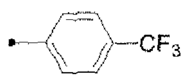 | 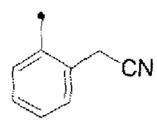 |
| 23 | 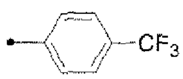 | 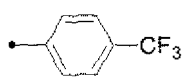 | 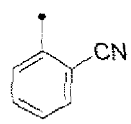 |
| 24 | 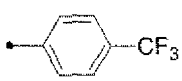 | 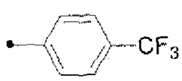 | 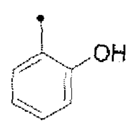 |
| 25 | 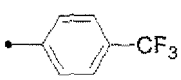 | 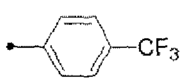 | 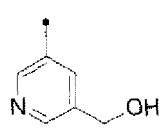 |

(continued)

| | | | |
|---|---|---|---|
| 26 | 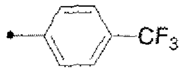 | 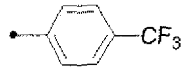 | 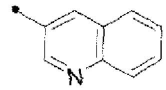 |
| 27 | 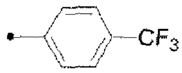 | 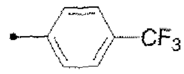 | 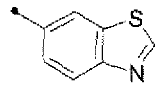 |
| 28 | 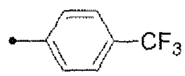 | 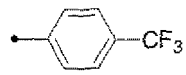 | 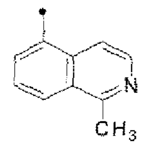 |
| 29 | 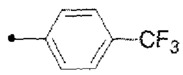 | 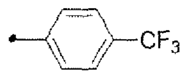 | 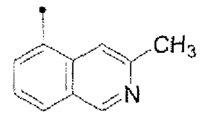 |
| 30 | 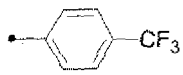 | 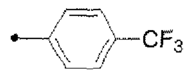 | 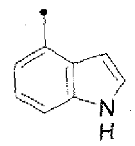 |
| 31 | 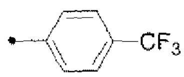 | 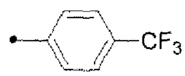 | 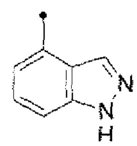 |

[Table 2-3]

| | | | |
|---|---|---|---|
| 32 | 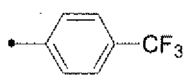 | 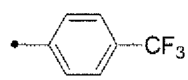 | 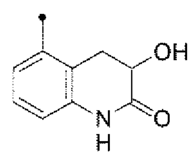 |
| 33 | 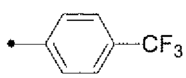 | 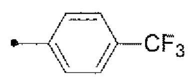 | 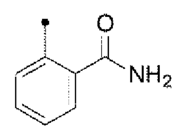 |
| 34 | 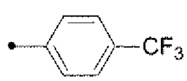 | 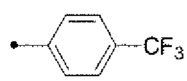 | 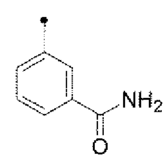 |
| 35 | 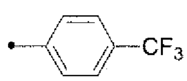 | 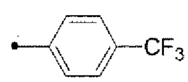 | 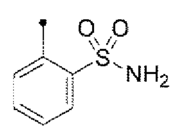 |
| 36 | 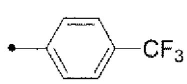 | 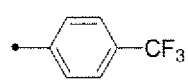 | 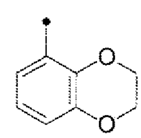 |

(continued)

| | | | |
|---|---|---|---|
| 37 | 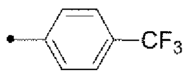 | 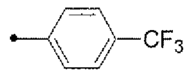 | 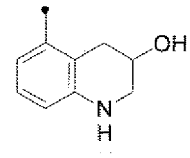 |
| 38 | 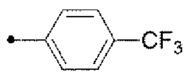 | 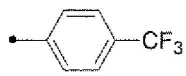 | 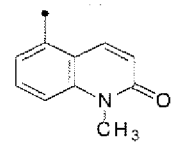 |
| 39 | 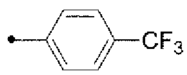 | 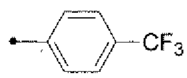 | 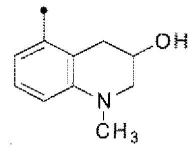 |
| 40 | 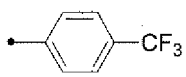 | 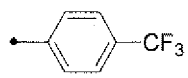 | 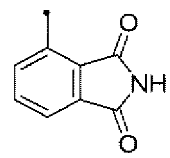 |
| 41 | 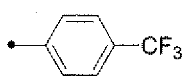 | 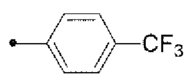 | 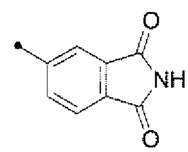 |

[Table 2-4]

| | | | |
|---|---|---|---|
| 42 | 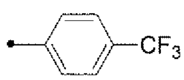 | 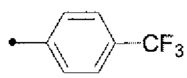 | 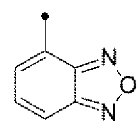 |
| 43 | 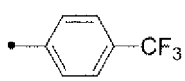 | 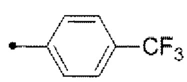 | 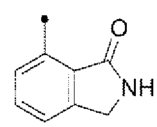 |
| 44 | 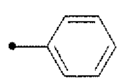 | 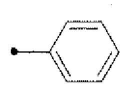 | 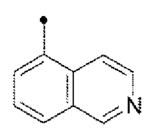 |
| 45 | 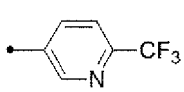 | 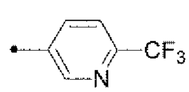 | 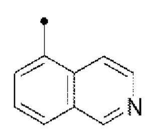 |
| 46 | 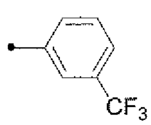 | 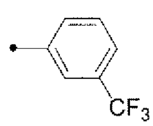 | 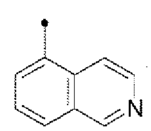 |

(continued)

| | | | |
|---|---|---|---|
| 47 | 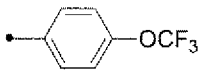 | 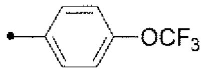 | 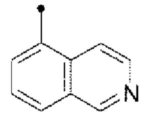 |
| 48 | 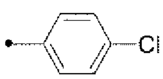 | 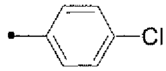 | 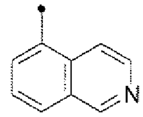 |
| 49 | 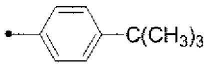 | 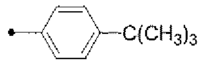 | 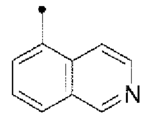 |
| 50 | 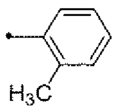 | 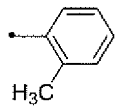 | 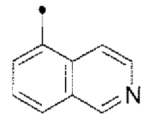 |
| 51 | 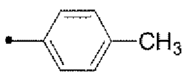 | 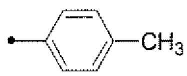 | 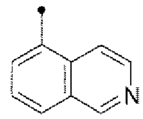 |
| 52 | 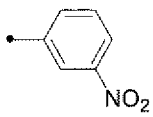 | 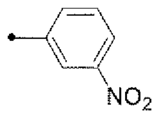 | 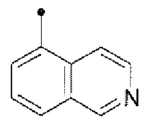 |

[Table 2-5]

| | | | |
|---|---|---|---|
| 53 | 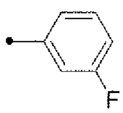 | 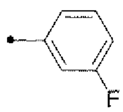 | 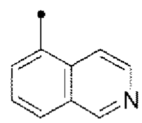 |
| 54 | 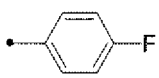 | 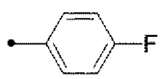 | 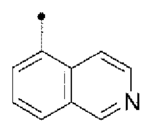 |
| 55 | 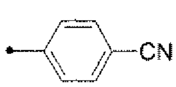 | 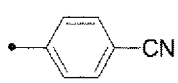 | 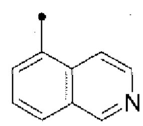 |
| 56 | 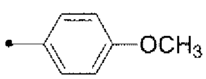 | 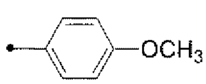 | 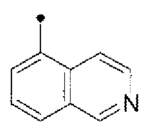 |

(continued)

| | | | |
|---|---|---|---|
| 57 | 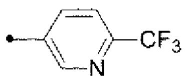 | 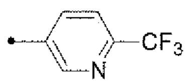 | 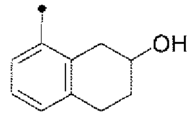 |
| 58 | 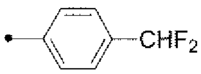 | 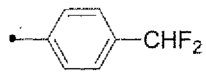 | 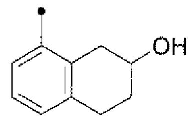 |
| 59 | 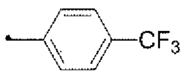 | 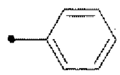 | 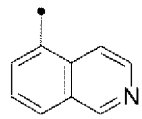 |
| 60 | 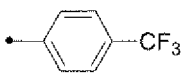 | 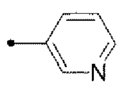 | 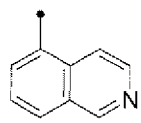 |
| 61 | 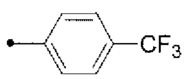 | 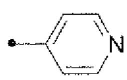 | 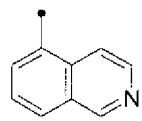 |
| 62 | 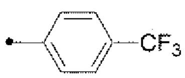 | 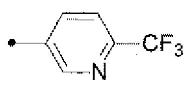 | 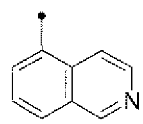 |
| 63 | 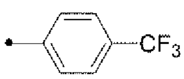 | 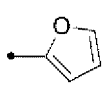 | 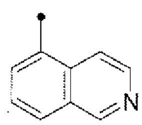 |

[Table 2-6]

| | | | |
|---|---|---|---|
| 64 | 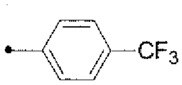 | 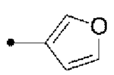 | 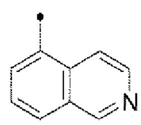 |
| 65 | 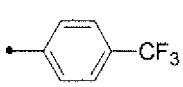 | 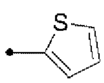 | 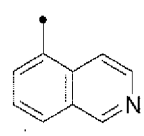 |
| 66 | 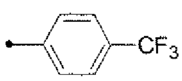 | 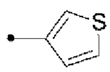 | 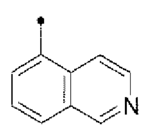 |

(continued)

| | | | |
|---|---|---|---|
| 67 | 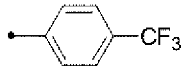 | 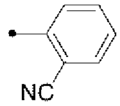 | 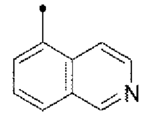 |
| 68 | 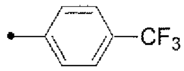 | 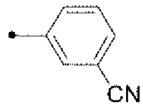 | 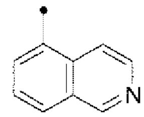 |
| 69 | 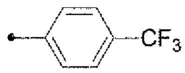 | 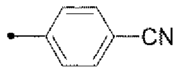 | 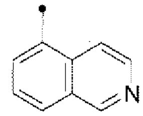 |
| 70 | 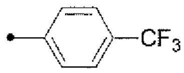 | 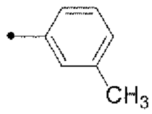 | 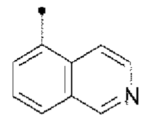 |
| 71 | 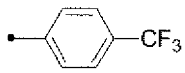 | 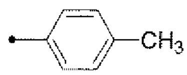 | 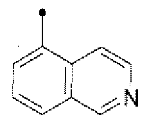 |
| 72 | 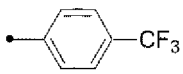 | 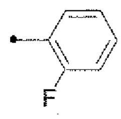 | 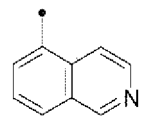 |
| 73 | 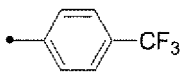 | 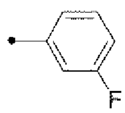 | 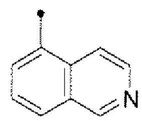 |
| 74 | 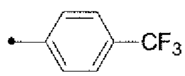 | 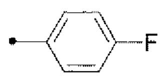 | 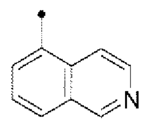 |

[Table 2-7]

| | | | |
|---|---|---|---|
| 75 | 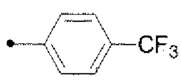 | 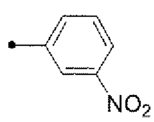 | 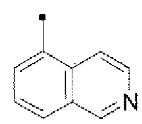 |
| 76 | 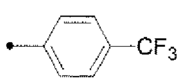 | 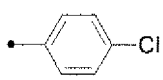 | 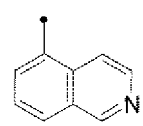 |
| 77 | 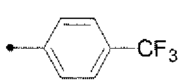 | 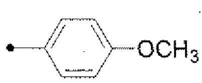 | 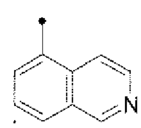 |

(continued)

| | | | |
|---|---|---|---|
| 78 | 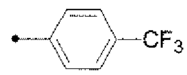 | 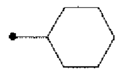 | 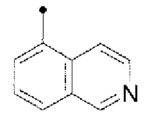 |
| 79 | 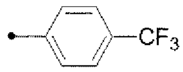 | 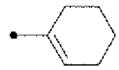 | 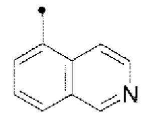 |
| 80 | 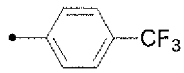 | 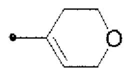 | 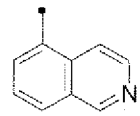 |
| 81 | 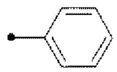 | 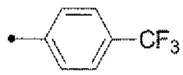 | 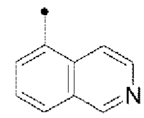 |
| 82 | 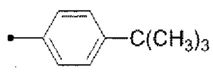 | 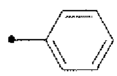 | 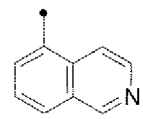 |
| 83 | 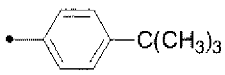 | 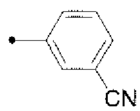 | 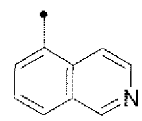 |
| 84 | 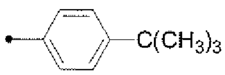 | 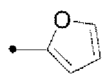 | 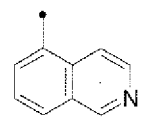 |
| 85 | 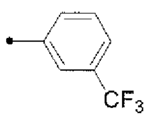 | 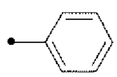 | 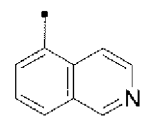 |

[Table 2-8]

| | | | |
|---|---|---|---|
| 86 | 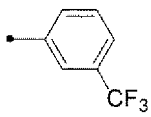 | 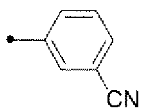 | 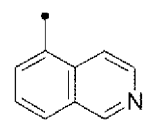 |
| 87 | 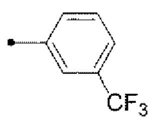 | 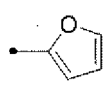 | 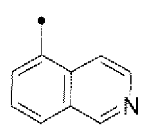 |

(continued)

| | | | |
|---|---|---|---|
| 88 | 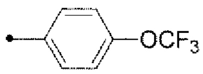 | 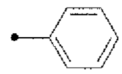 | 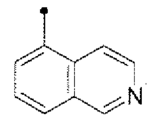 |
| 89 | 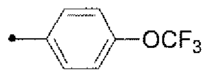 | 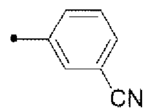 | 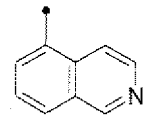 |
| 90 | 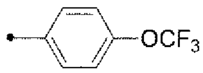 | 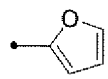 | 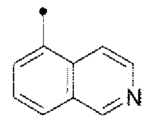 |
| 91 | 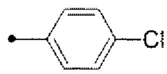 | 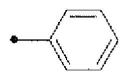 | 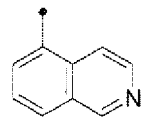 |
| 92 | 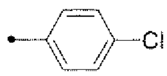 | 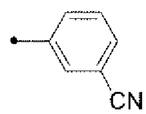 | 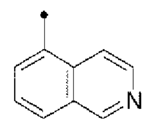 |
| 93 | 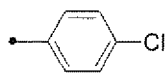 | 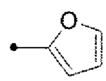 | 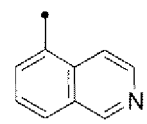 |
| 94 | 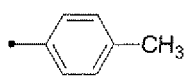 | 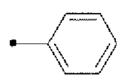 | 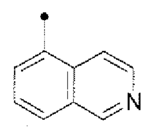 |
| 95 | 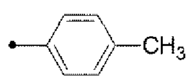 | 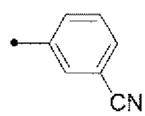 | 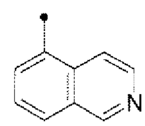 |
| 96 | 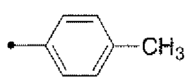 | 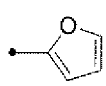 | 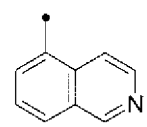 |

[Table 3]

| Compound Number | Structure |
|---|---|
| 97 | 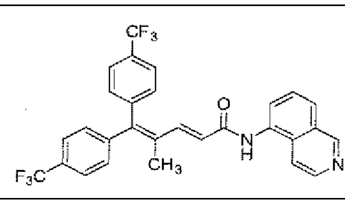 |

(continued)

| Compound Number | Structure |
|---|---|
| 98 | 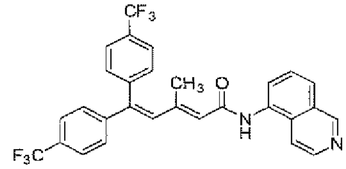 |
| 99 | 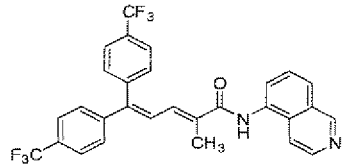 |

[Table 4-1]

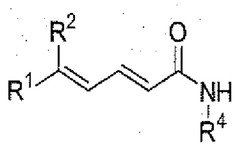

| Compound Number | $R^1$ | $R^2$ | $R^4$ | Salt |
|---|---|---|---|---|
| 100 | 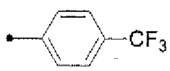 | 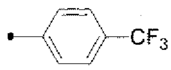 | 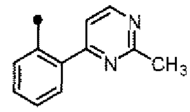 | |
| 101 | 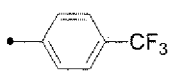 | 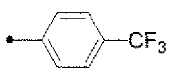 | 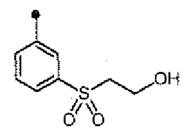 | |
| 102 | 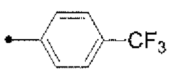 | 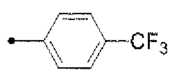 | 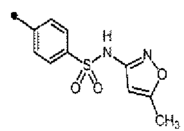 | |
| 103 | 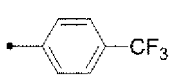 | 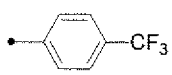 | 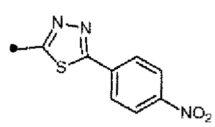 | |
| 104 | 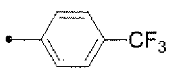 | 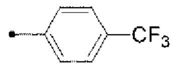 | 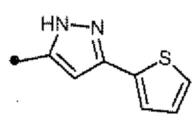 | |

(continued)

| Compound Number | $R^1$ | $R^2$ | $R^4$ | Salt |
|---|---|---|---|---|
| 105 | 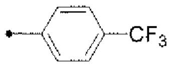 | 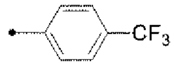 | 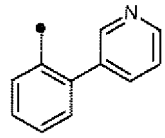 | |
| 106 | 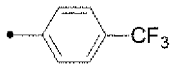 | 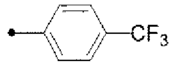 | 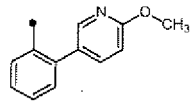 | |
| 107 | 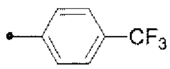 | 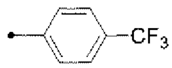 | 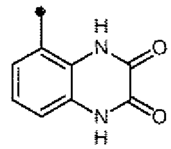 | |
| 108 | 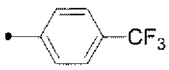 | 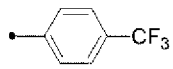 | 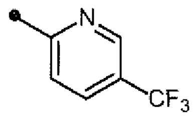 | |
| 109 | 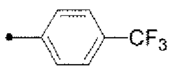 | 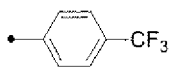 | 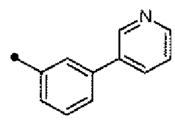 | |
| 110 | 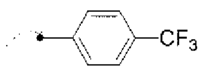 | 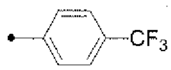 | 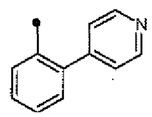 | |

[Table 4-2]

| | | | |
|---|---|---|---|
| 111 | 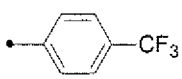 | 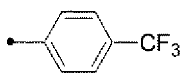 | 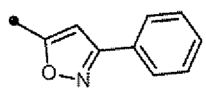 |
| 112 | 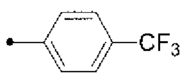 | 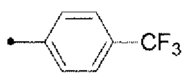 | 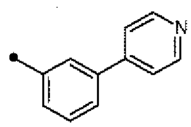 |
| 113 | 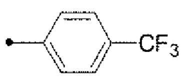 | 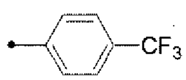 | 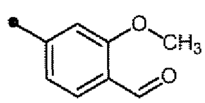 |
| 114 | 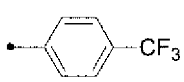 | 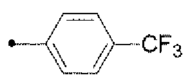 | 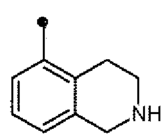 |

(continued)

| | | | |
|---|---|---|---|
| 115 | 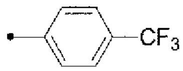 | 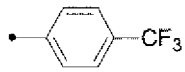 | 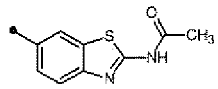 |
| 116 | 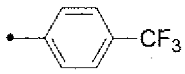 | 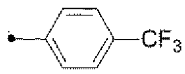 | 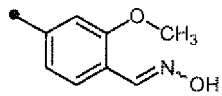 |
| 117 | 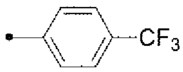 | 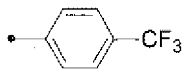 | 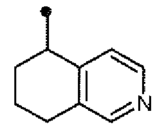 |
| 118 | 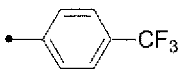 | 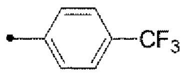 | 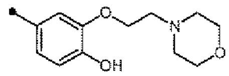 |
| 119 | 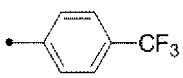 | 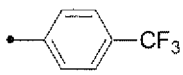 | 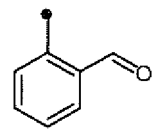 |
| 120 | 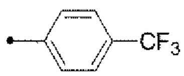 | 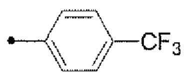 | 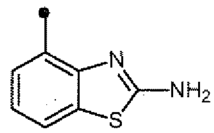 |
| 121 | 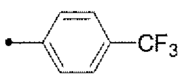 | 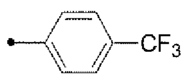 | 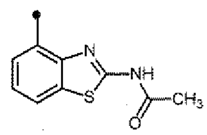 |

[Table 4-3]

| | | | |
|---|---|---|---|
| 122 | 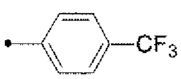 | 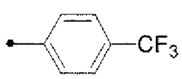 | 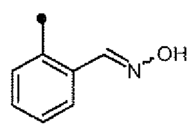 |
| 123 | 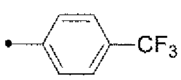 | 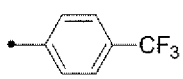 | 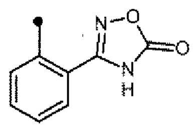 |
| 124 | 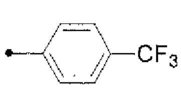 | 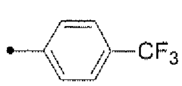 | 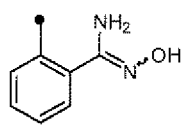 |

(continued)

| | | | |
|---|---|---|---|
| 125 | 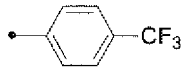 | 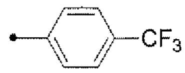 | 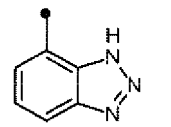 |
| 126 | 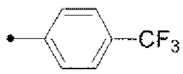 | 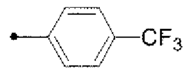 | 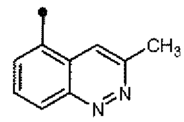 |
| 127 | 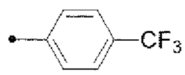 | 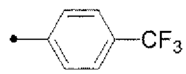 | 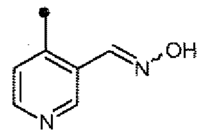 |
| 128 | 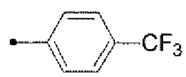 | 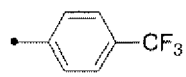 | 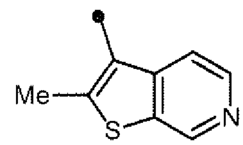 |
| 129 | 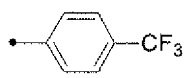 | 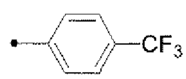 | 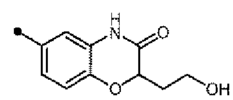 |
| 130 | 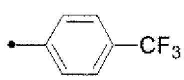 | 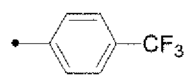 | 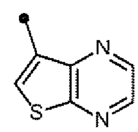 |
| 131 | 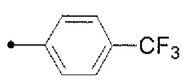 | 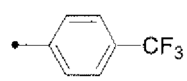 | 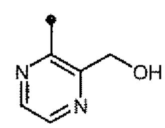 |

[Table 4-4]

| | | | |
|---|---|---|---|
| 132 | 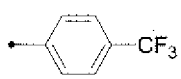 | 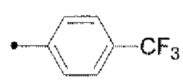 | 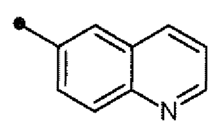 |
| 133 | 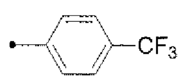 | 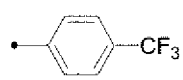 | 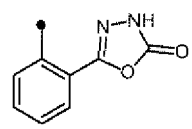 |
| 134 | 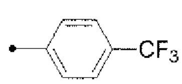 | 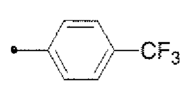 | 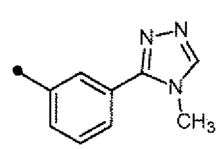 |

(continued)

| | | | |
|---|---|---|---|
| 135 | 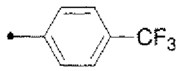 | 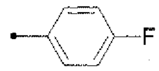 | 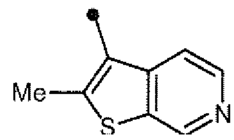 |
| 136 | 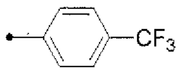 | 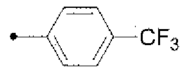 | 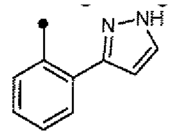 |
| 137 | 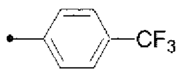 | 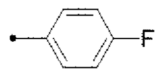 | 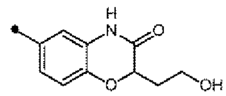 |
| 138 | 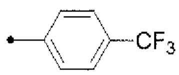 | 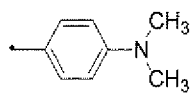 | 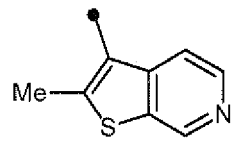 |
| 139 | 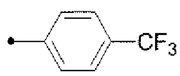 | 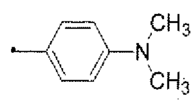 | 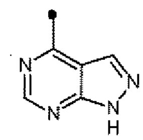 |
| 140 | 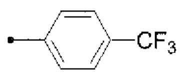 | 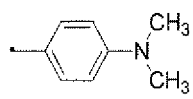 | 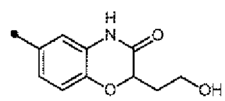 |
| 141 | 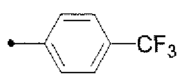 | 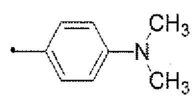 | 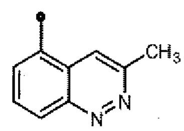 |
| 142 | 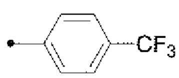 | 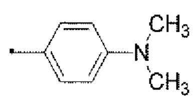 | 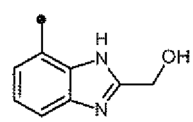 |

[Table 4-5]

| | | | |
|---|---|---|---|
| 143 | 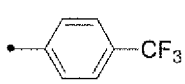 | 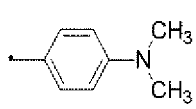 | 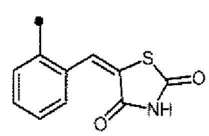 |
| 144 | 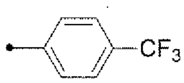 | 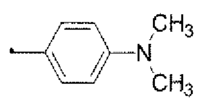 | 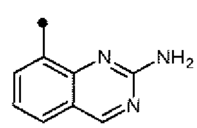 |

(continued)

| | | | |
|---|---|---|---|
| 145 | 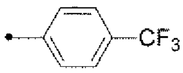 | 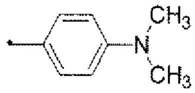 | 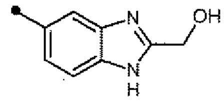 |
| 146 | 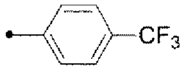 | 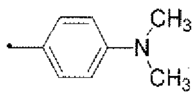 | 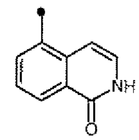 |
| 147 | 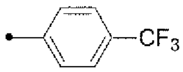 | 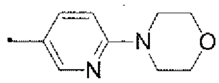 | 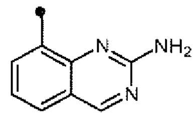 |
| 148 | 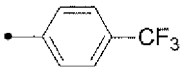 | 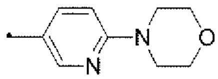 | 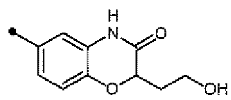 |
| 149 | 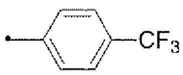 | 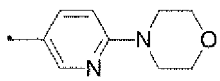 | 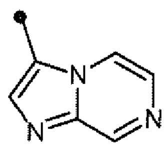 |
| 150 | 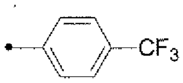 | 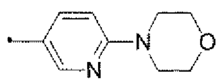 | 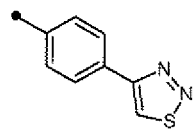 |
| 151 | 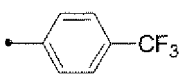 | 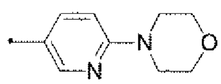 | 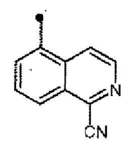 |
| 152 | 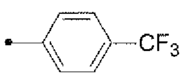 | 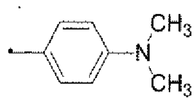 | 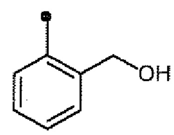 |
| 153 | 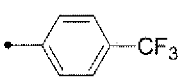 | 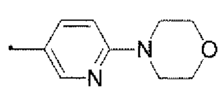 | 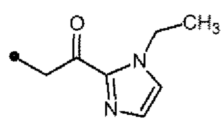 |

[Table 4-6]

| | | | |
|---|---|---|---|
| 154 | 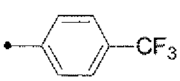 | 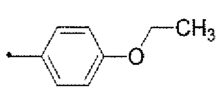 | 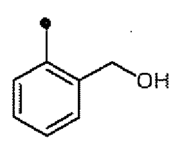 |

(continued)

| | | | |
|---|---|---|---|
| 155 | 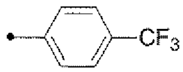 | 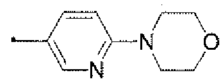 | 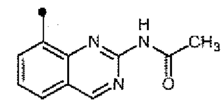 |
| 156 | 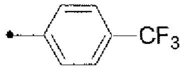 | 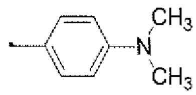 | 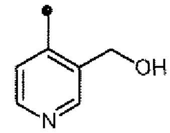 |
| 157 | 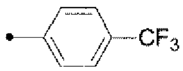 | 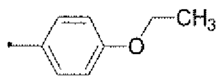 | 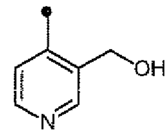 |
| 158 | 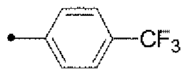 | 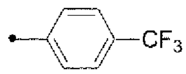 | 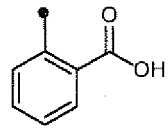 |
| 159 | 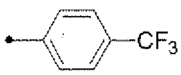 | 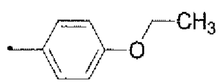 | 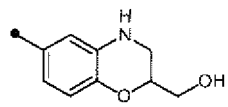 |
| 160 | 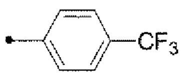 | 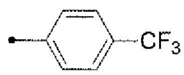 | 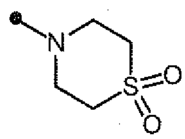 |
| 161 | 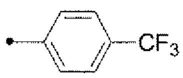 | 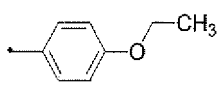 | 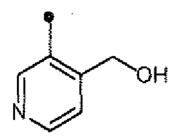 |
| 162 | 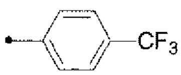 | 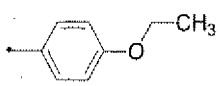 | 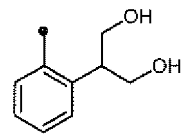 |
| 163 | 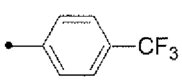 | 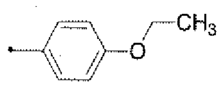 | 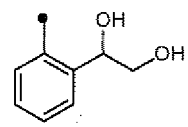 |
| 164 | 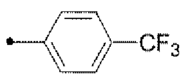 | 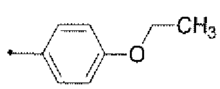 | 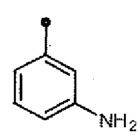 |

[Table 4-7]

| | | | |
|---|---|---|---|
| 165 | 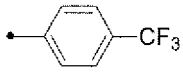 | 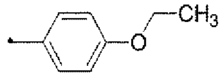 | 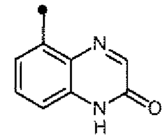 |
| 166 | 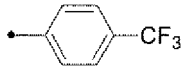 | 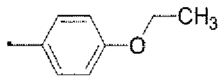 | 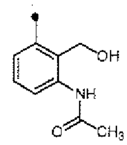 |
| 167 | 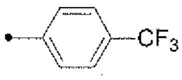 | 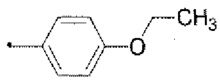 | 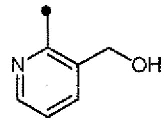 |
| 168 | 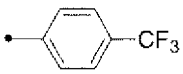 | 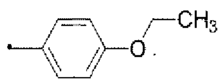 | 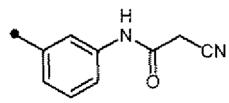 |
| 169 | 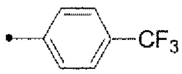 | 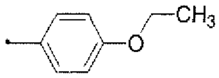 | 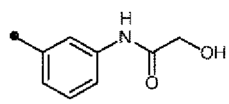 |
| 170 | 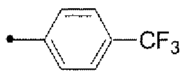 | 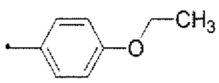 | 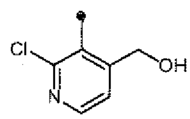 |
| 171 | 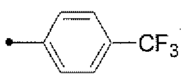 | 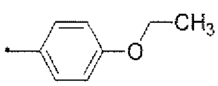 | 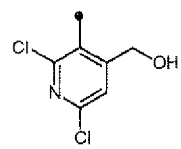 |
| 172 | 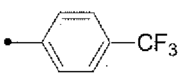 | 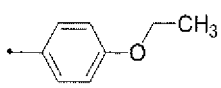 | 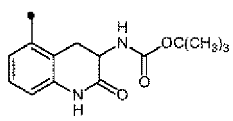 |
| 173 | 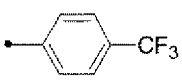 | 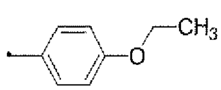 | 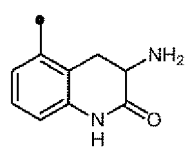 |
| 174 | 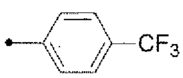 | 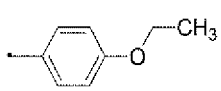 | 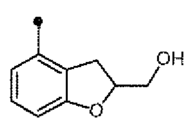 |

(continued)

| | | | |
|---|---|---|---|
| 175 | 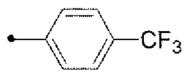 | 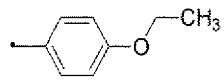 | 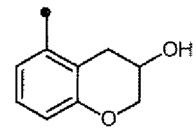 |

[Table 4-8]

| | | | |
|---|---|---|---|
| 176 | 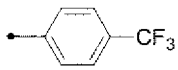 | 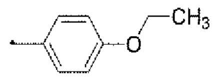 | 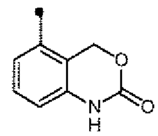 |
| 177 | 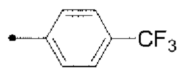 | 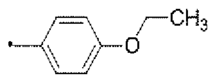 | 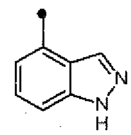 |
| 178 | 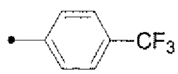 | 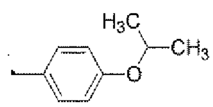 | 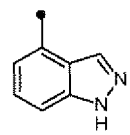 |
| 179 | 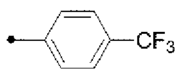 | 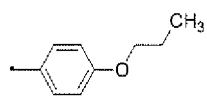 | 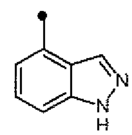 |

[Table 4-9]

| | | | | |
|---|---|---|---|---|
| 180 | 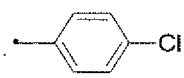 | 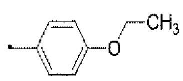 | 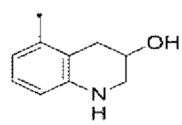 | HCl |
| 181 | 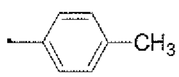 | 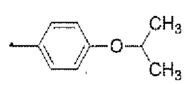 | 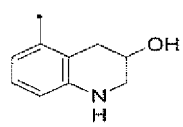 | HCl |
| 182 | 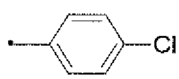 | 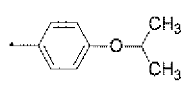 | 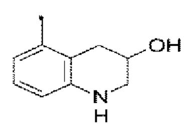 | HCl |
| 183 | 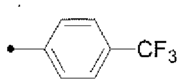 | 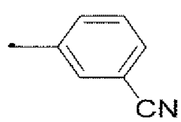 | 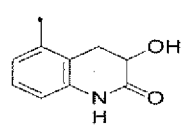 | |

(continued)

| | | | | |
|---|---|---|---|---|
| 184 | 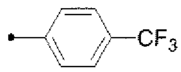 | 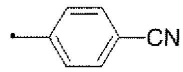 | 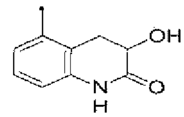 | |
| 185 | 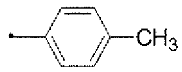 | 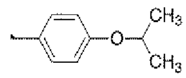 | 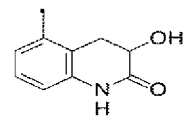 | |
| 186 | 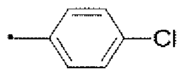 | 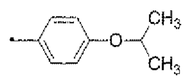 | 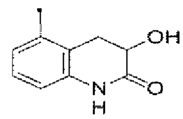 | |
| 187 | 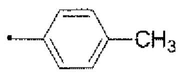 | 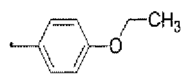 | 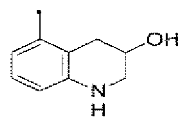 | HCl |
| 188 | 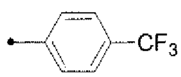 | 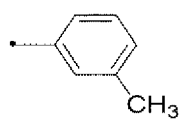 | 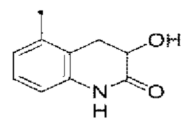 | |
| 189 | 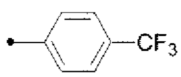 | 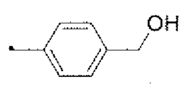 | 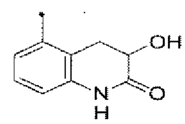 | |
| 190 | 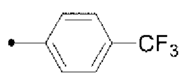 | 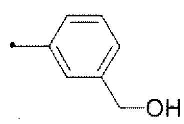 | 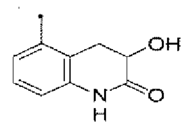 | |

[Table 4-10]

| | | | |
|---|---|---|---|
| 191 | 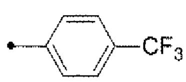 | 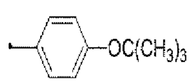 | 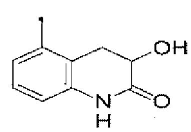 |
| 192 | 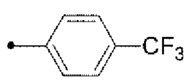 | 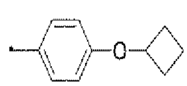 | 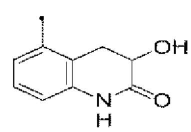 |
| 193 | 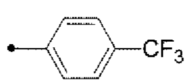 | 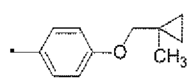 | 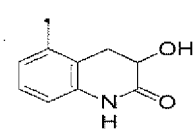 |

(continued)

| | | | |
|---|---|---|---|
| 194 | 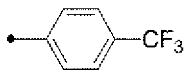 | 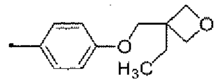 | 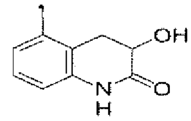 |
| 195 | 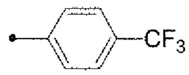 | 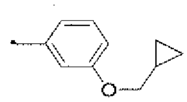 | 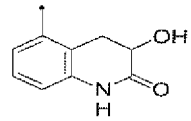 |
| 196 | 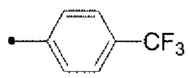 | 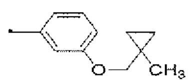 | 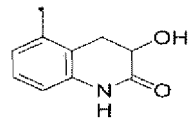 |
| 197 | 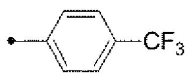 | 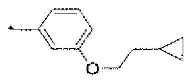 | 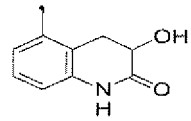 |
| 198 | 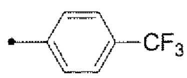 | 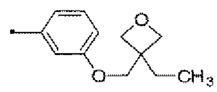 | 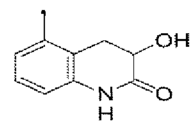 |
| 199 | 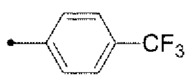 | 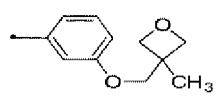 | 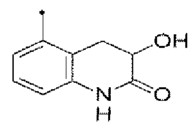 |
| 200 | 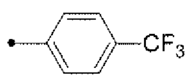 | 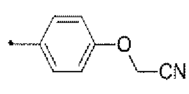 | 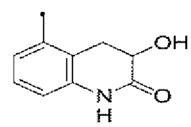 |
| 201 | 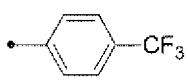 | 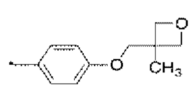 | 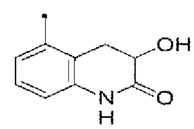 |

[Table 4-11]

| | | | |
|---|---|---|---|
| 202 | 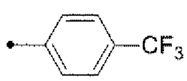 | 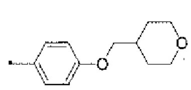 | 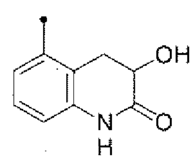 |
| 203 | 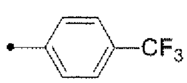 | 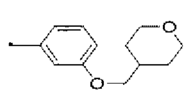 | 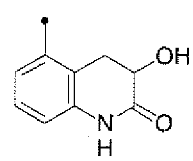 |

(continued)

| | | | | |
|---|---|---|---|---|
| 204 | 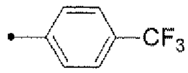 | 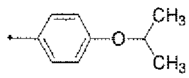 | 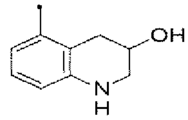 | HCl |
| 205 | 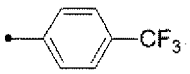 | 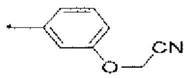 | 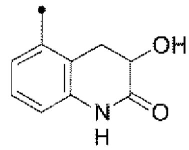 | |
| 206 | 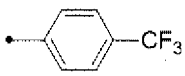 | 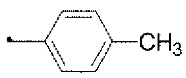 | 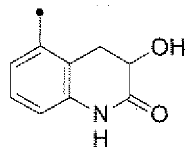 | |
| 207 | 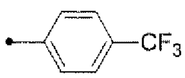 | 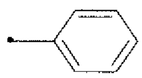 | 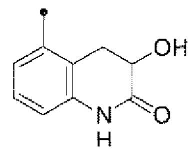 | |
| 208 | 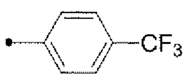 | 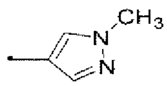 | 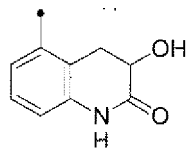 | |
| 209 | 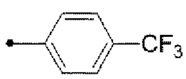 | 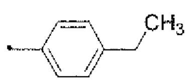 | 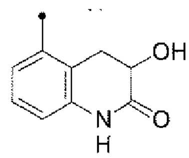 | |
| 210 | 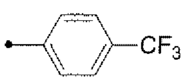 | 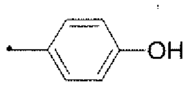 | 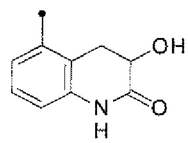 | |
| 211 | 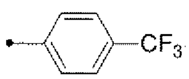 | 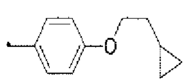 | 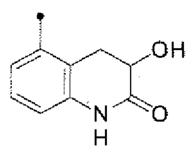 | |

[Table 4-12]

| | | | |
|---|---|---|---|
| 212 | 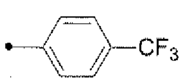 | 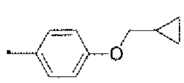 | 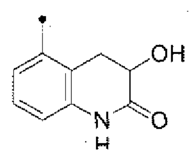 |
| 213 | 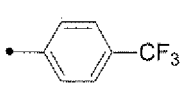 | 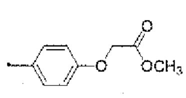 | 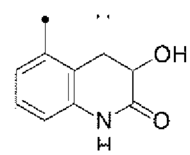 |

(continued)

| | | | | |
|---|---|---|---|---|
| 214 | 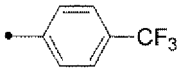 | 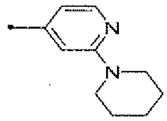 | 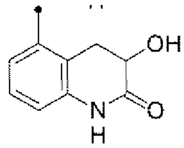 | |
| 215 | 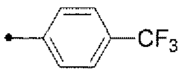 | 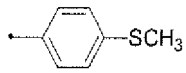 | 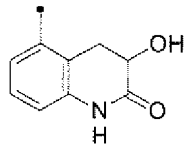 | |
| 216 | 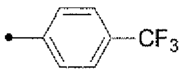 | 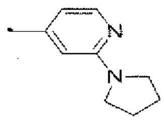 | 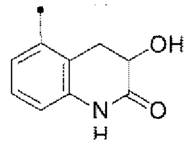 | |
| 217 | 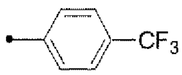 | 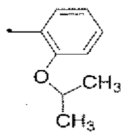 | 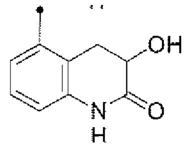 | |
| 218 | 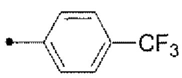 | 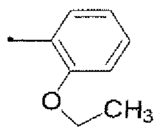 | 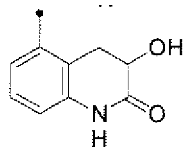 | |
| 219 | 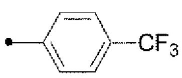 | 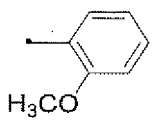 | 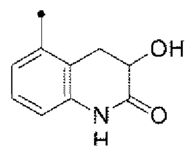 | |
| 220 | 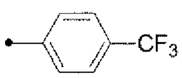 | 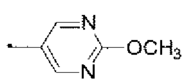 | 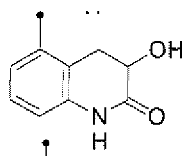 | |
| 221 | 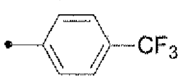 | 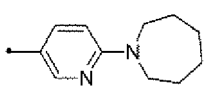 | 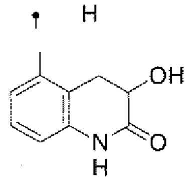 | HCl |
| 222 | 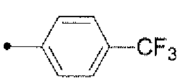 | 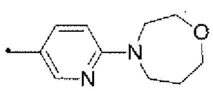 | 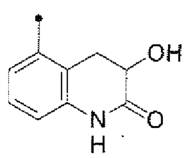 | HCl |

[Table 4-13]

| | | | | |
|---|---|---|---|---|
| 223 | 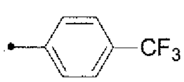 | 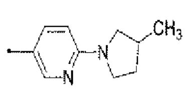 | 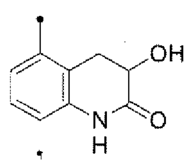 | HCl |

(continued)

| | | | | |
|---|---|---|---|---|
| 224 | 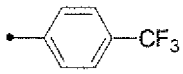 | 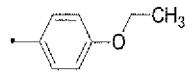 | 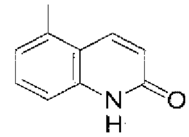 | |
| 225 | 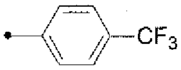 | 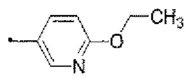 | 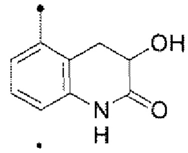 | |
| 226 | 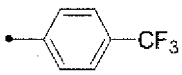 | 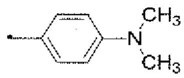 | 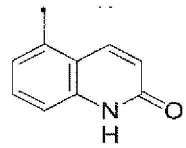 | HCl |
| 227 | 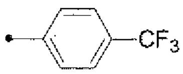 | 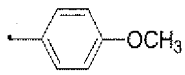 | 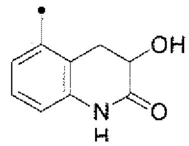 | |
| 228 | 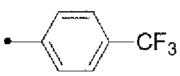 | 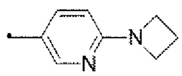 | 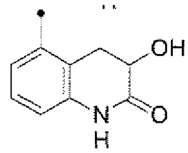 | |
| 229 | 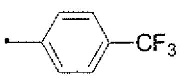 | 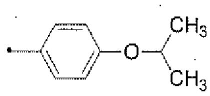 | 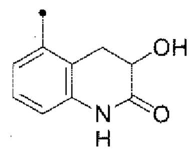 | |
| 230 | 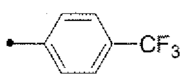 | 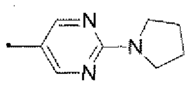 | 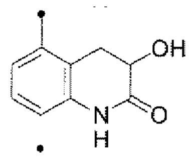 | HCl |
| 231 | 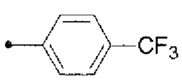 | 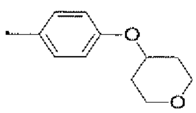 | 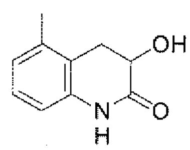 | |
| 232 | 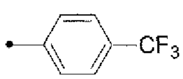 | 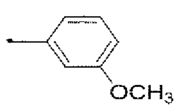 | 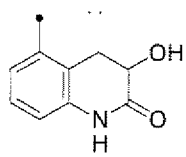 | |
| 233 | 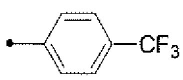 | 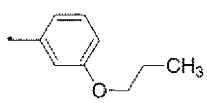 | 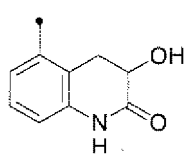 | |

[Table 4-14]

| | | | | |
|---|---|---|---|---|
| 234 | 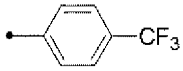 | 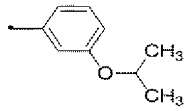 | 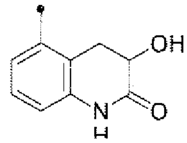 | |
| 235 | 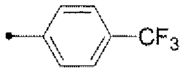 | 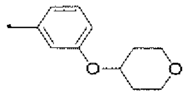 | 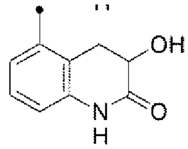 | |
| 236 | 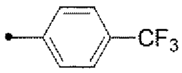 | 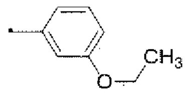 | 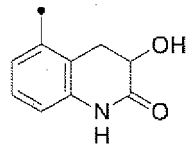 | |
| 237 | 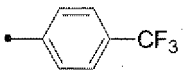 | 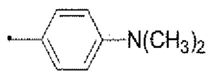 | 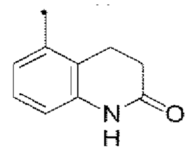 | HCl |
| 238 | 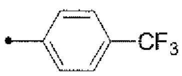 | 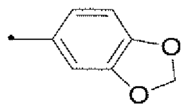 | 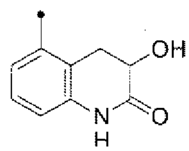 | |
| 239 | 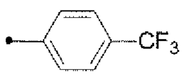 | 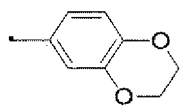 | 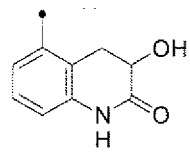 | |
| 240 | 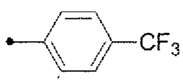 | 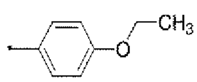 | 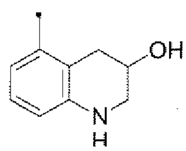 | HCl |
| 241 | 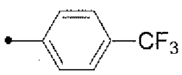 | 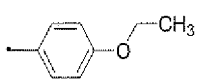 | 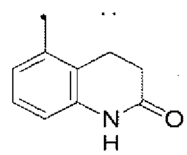 | |
| 242 | 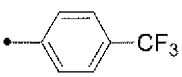 | 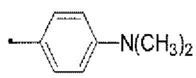 | 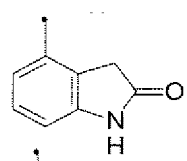 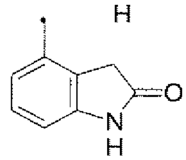 | |
| 244 | 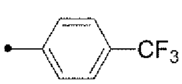 | 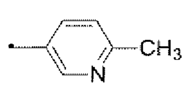 | 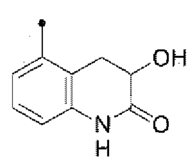 | |

[Table 4-15]

| | | | | |
|---|---|---|---|---|
| 245 | 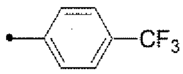 | 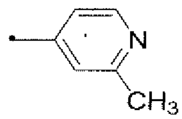 | 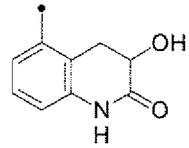 | |
| 246 | 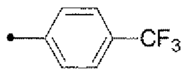 | 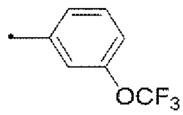 | 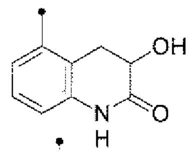 | |
| 247 | 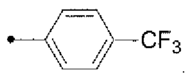 | 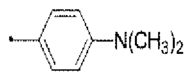 | 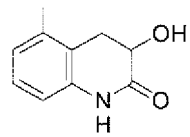 | HCl |
| 248 | 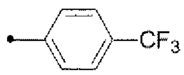 | 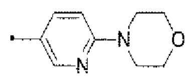 | 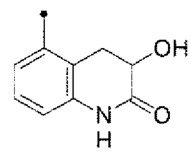 | HCl |
| 249 | 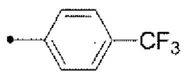 | 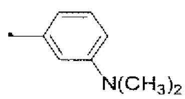 | 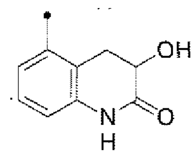 | HCl |
| 250 | 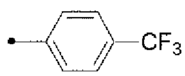 | 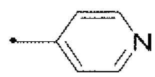 | 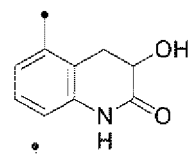 | HCl |
| 251 | 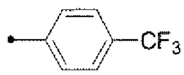 | 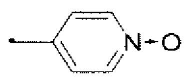 | 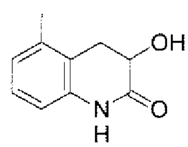 | HCl |
| 252 | 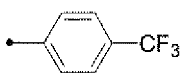 | 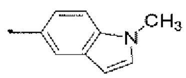 | 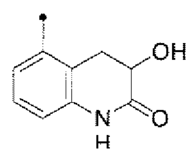 | |
| 253 | 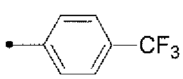 | 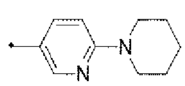 | 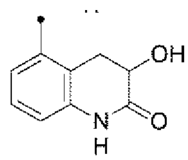 | HCl |
| 254 | 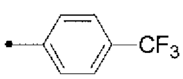 | 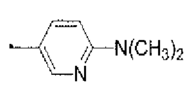 | 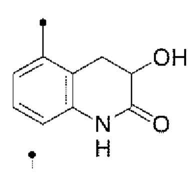 | HCl |

(continued)

| | | | | |
|---|---|---|---|---|
| 255 | 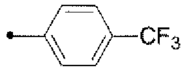 | 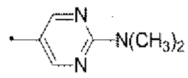 | 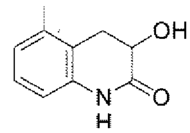 | |

[Table 4-16]

| | | | | |
|---|---|---|---|---|
| 256 | 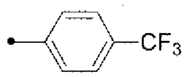 | 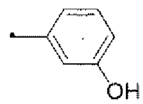 | 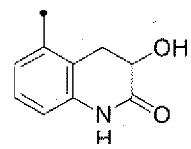 | |
| 257 | 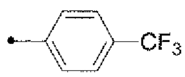 | 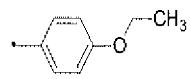 | 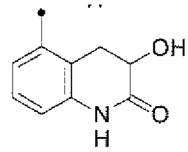 | |
| 258 | 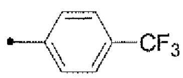 | 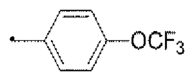 | 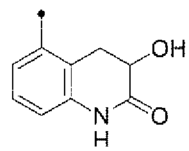 | |
| 259 | 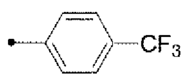 | 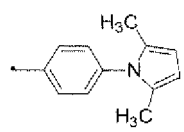 | 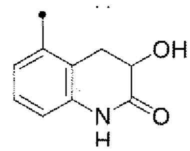 | |
| 260 | 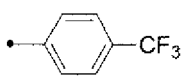 | 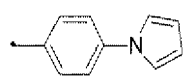 | 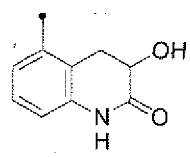 | |
| 261 | 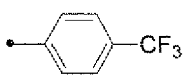 | 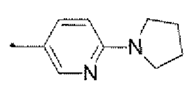 | 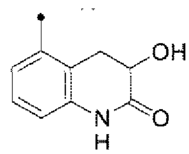 | HCl |
| 262 | 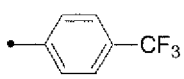 | 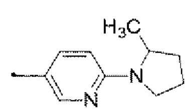 | 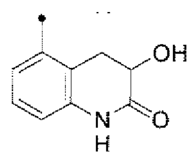 | HCl |
| 263 | 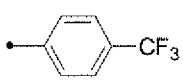 | 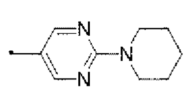 | 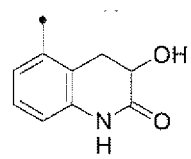 | HCl |
| 264 | 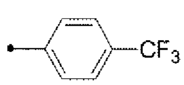 | 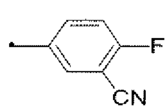 | 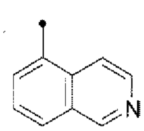 | HCl |

(continued)

| | | | | |
|---|---|---|---|---|
| 265 | 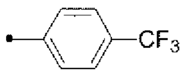 | 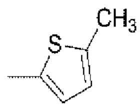 | 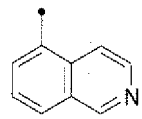 | HCl |
| 266 | 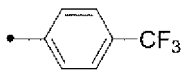 | 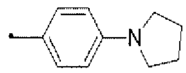 | 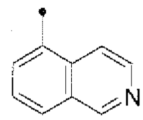 | |

[Table 4-17]

| | | | | |
|---|---|---|---|---|
| 267 | 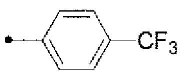 | 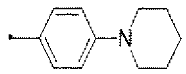 | 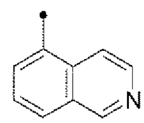 | 2HCl |
| 268 | 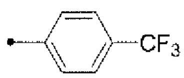 | 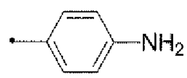 | 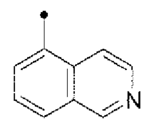 | |
| 269 | 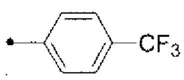 | 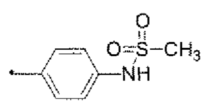 | 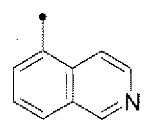 | |
| 270 | 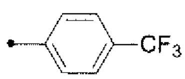 | 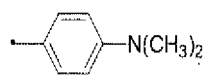 | 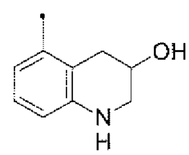 | HCl |
| 271 | 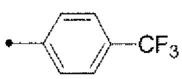 | 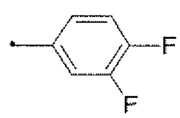 | 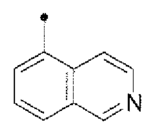 | |
| 272 | 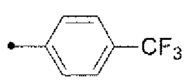 | 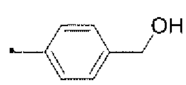 | 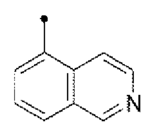 | |
| 273 | 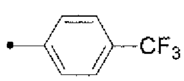 | 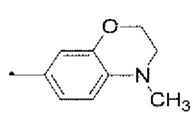 | 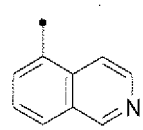 | |
| 274 | 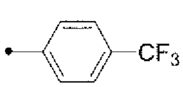 | 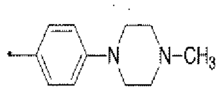 | 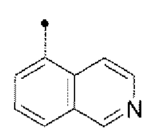 | |

(continued)

| | | | | |
|---|---|---|---|---|
| 275 | 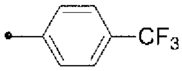 | 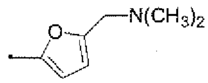 | 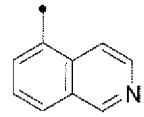 | |
| 276 | 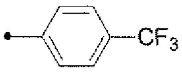 | 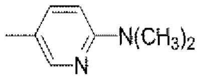 | 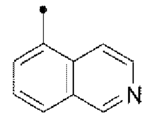 | |
| 277 | 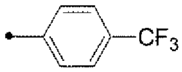 | 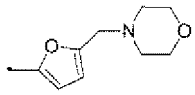 | 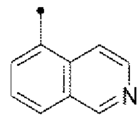 | |

[Table 4-18]

| | | | | |
|---|---|---|---|---|
| 278 | 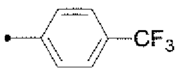 | 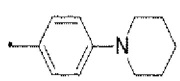 | 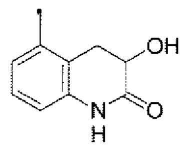 | HCl |
| 279 | 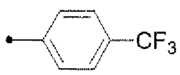 | 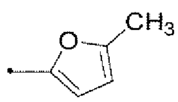 | 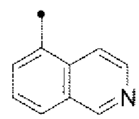 | |
| 280 | 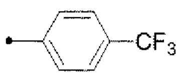 | 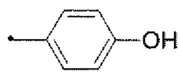 | 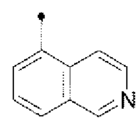 | |
| 281 | 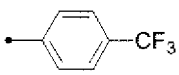 | 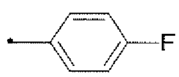 | 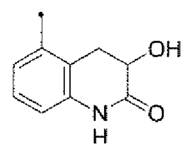 | |
| 282 | 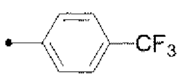 | 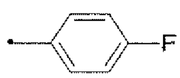 | 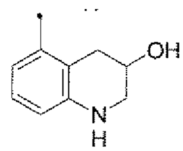 | |
| 283 | 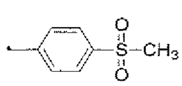 | 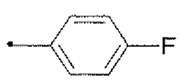 | 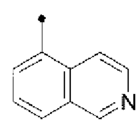 | |
| 284 | 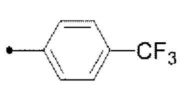 | 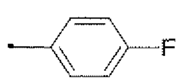 | 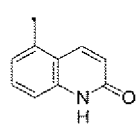 | |

(continued)

| | | | |
|---|---|---|---|
| 285 | 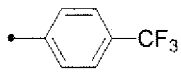 | 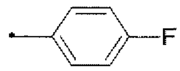 | 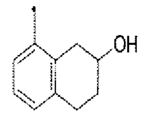 |
| 286 | 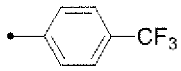 | 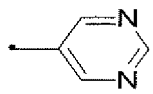 | 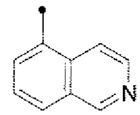 |
| 287 | 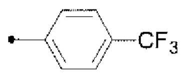 | 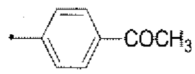 | 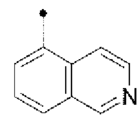 |
| 288 | 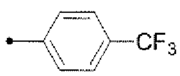 | 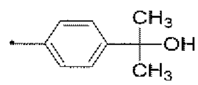 | 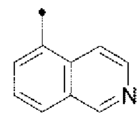 |

[Table 4-19]

| | | | |
|---|---|---|---|
| 289 | 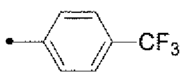 | 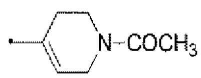 | 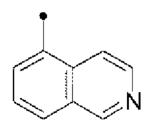 |
| 290 | 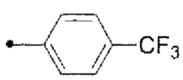 | 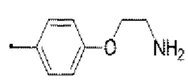 | 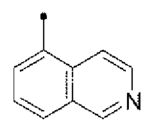 |
| 291 | 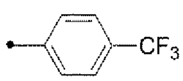 | 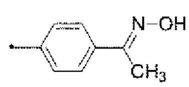 | 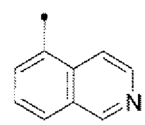 |
| 292 | 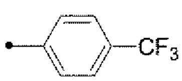 | 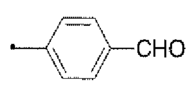 | 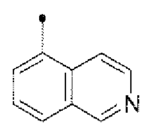 |
| 293 | 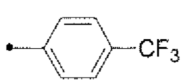 | 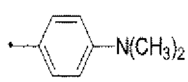 | 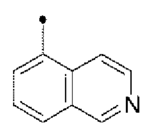 |
| 294 | 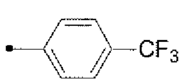 | 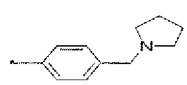 | 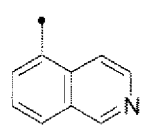 |

(continued)

| | | | | |
|---|---|---|---|---|
| 295 | 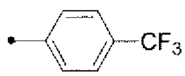 | 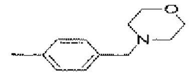 | 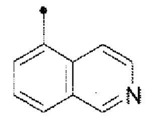 | |
| 296 | 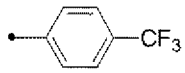 | 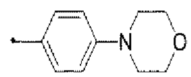 | 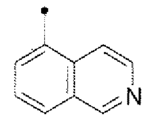 | |
| 297 | 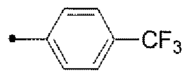 | 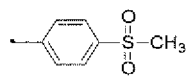 | 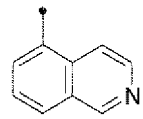 | |
| 298 | 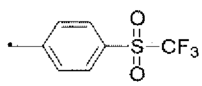 | 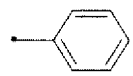 | 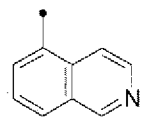 | |
| 299 | 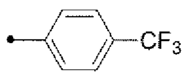 | 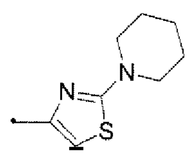 | 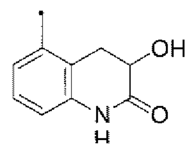 | |

[Table 4-20]

| | | | | |
|---|---|---|---|---|
| 300 | 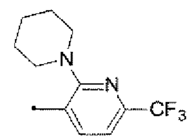 | 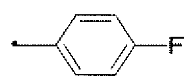 | 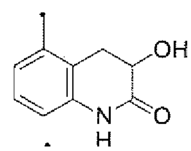 | |
| 301 | 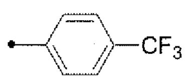 | 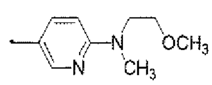 | 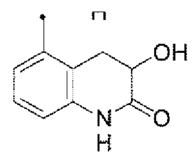 | HCl |
| 302 | 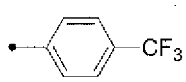 | 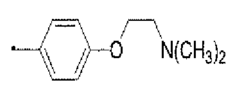 | 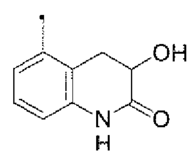 | |
| 303 | 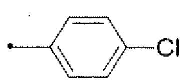 | 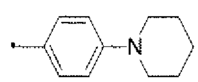 | 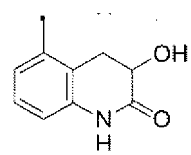 | HCl |
| 304 | 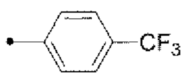 | 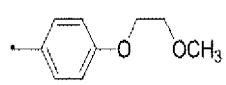 | 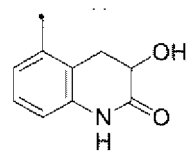 | |

(continued)

| | | | |
|---|---|---|---|
| 305 | 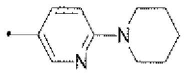 | 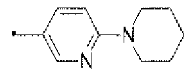 | 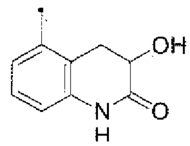 |
| 306 | 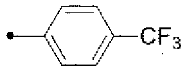 | 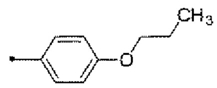 | 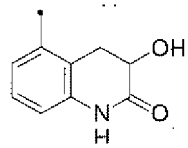 |
| 307 | 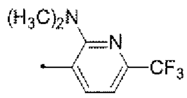 | 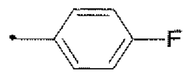 | 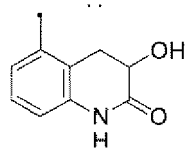 |
| 308 | 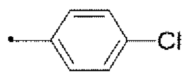 | 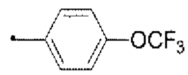 | 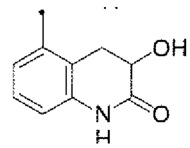 |
| 309 | 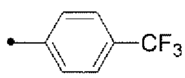 | 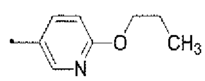 | 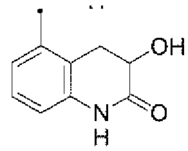 |
| 310 | 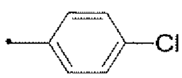 | 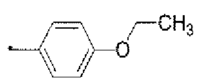 | 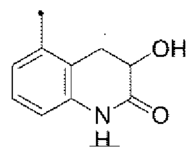 |

[Tables 4-21]

| | | | |
|---|---|---|---|
| 311 | 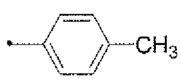 | 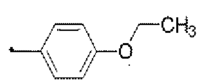 | 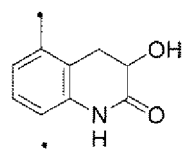 |
| 312 | 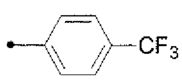 | 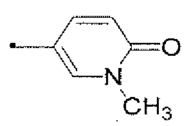 | 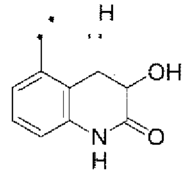 |
| 313 | 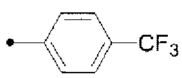 | 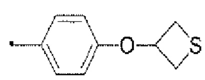 | 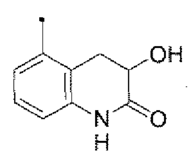 |
| 314 | 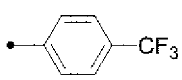 | 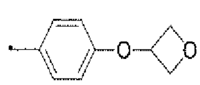 | 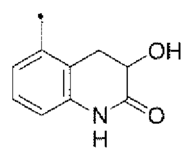 |

[Table 5-1]

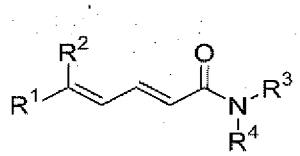

| Compound Number | $R^1$ | $R^2$ | $NR^3R^4$ |
|---|---|---|---|
| 315 | 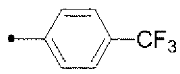 | 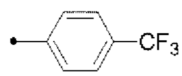 | 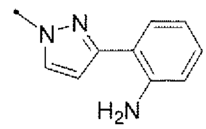 |
| 316 | 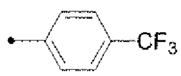 | 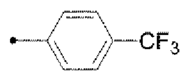 | 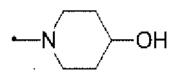 |
| 317 | 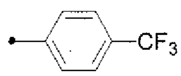 | 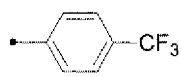 | 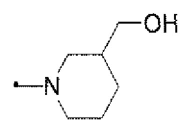 |
| 318 | 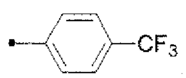 | 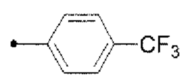 | 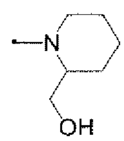 |
| 319 | 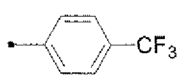 | 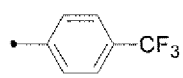 | 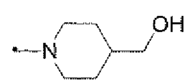 |
| 320 | 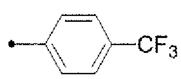 | 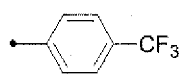 | 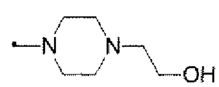 |
| 321 | 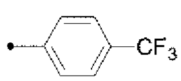 | 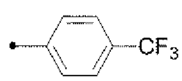 | 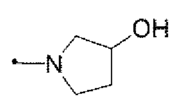 |
| 322 | 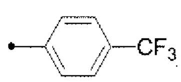 | 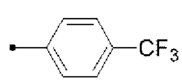 | 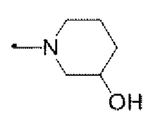 |

[Tables 5-2]

| | | | |
|---|---|---|---|
| 323 | 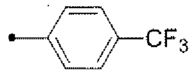 | 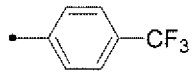 | 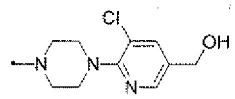 |
| 324 | 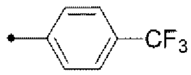 | 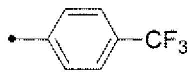 | 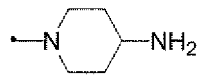 |
| 325 | 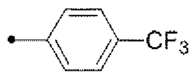 | 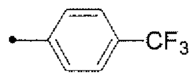 | 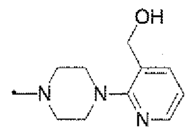 |

[Table 6]

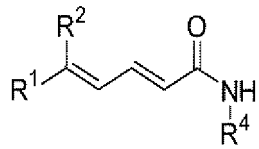

| Compound Number | $R^1$ | $R^2$ | $R^4$ |
|---|---|---|---|
| 326 | 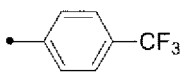 | 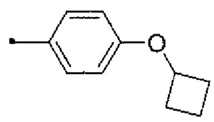 | 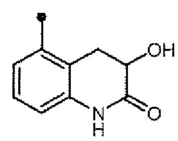 |
| 327 | 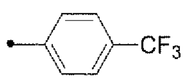 | 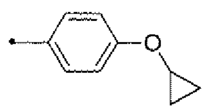 | 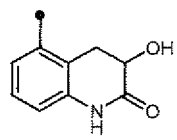 |
| 328 | 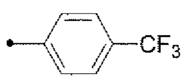 | 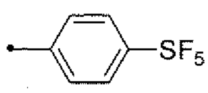 | 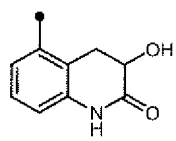 |
| 329 | 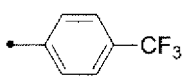 | 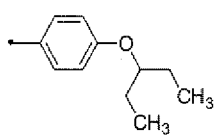 | 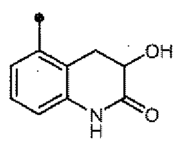 |
| 330 | 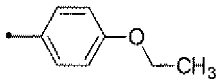 | 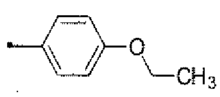 | 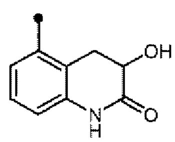 |

(continued)

| Compound Number | $R^1$ | $R^2$ | $R^4$ |
|---|---|---|---|
| 331 | 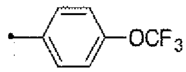 | 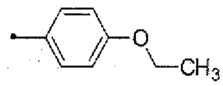 | 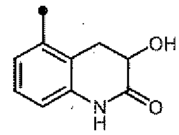 |

Specific examples of the intermediates in the above-mentioned Production methods are shown in Tables 7 to 10.

[Table 7-1]

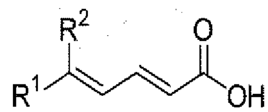

| Compound Number | $R^1$ | $R^2$ |
|---|---|---|
| b | 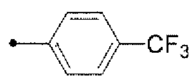 | 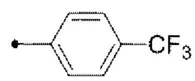 |
| c | 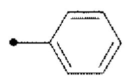 | 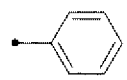 |
| d | 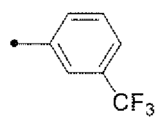 | 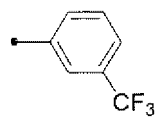 |
| e | 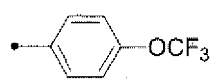 | 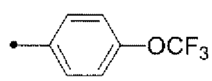 |
| f | 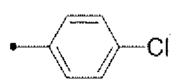 | 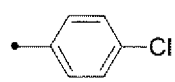 |
| g | 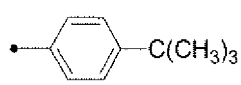 | 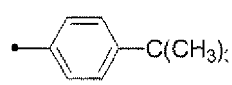 |
| h | 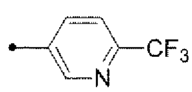 | 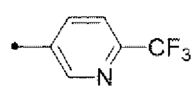 |
| i | 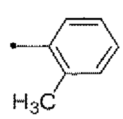 | 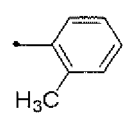 |

(continued)

| Compound Number | $R^1$ | $R^2$ |
|---|---|---|
| j | 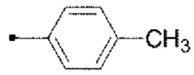 | 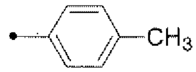 |
| k | 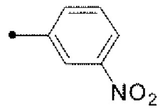 | 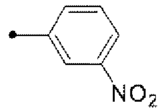 |
| l | 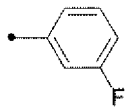 | 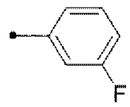 |

[Table 7-2]

| | | |
|---|---|---|
| m | 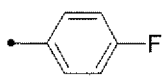 | 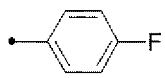 |
| n | 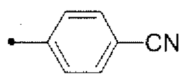 | 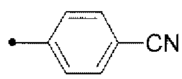 |
| o | 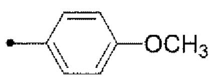 | 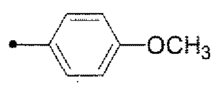 |
| p | 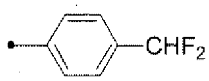 | 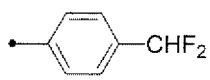 |
| r | 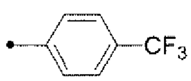 | 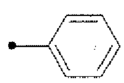 |
| s | 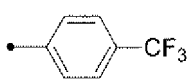 | 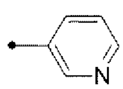 |
| t | 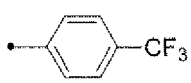 | 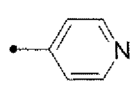 |
| u | 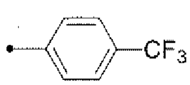 | 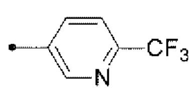 |

(continued)

| | | |
|---|---|---|
| v | 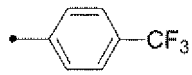 | 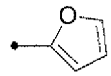 |
| w | 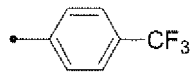 | 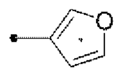 |
| x | 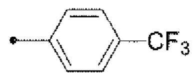 | 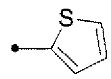 |

[Table 7-3]

| | | |
|---|---|---|
| y | 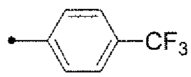 | 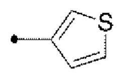 |
| z | 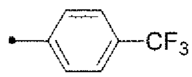 | 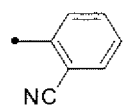 |
| aa | 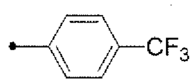 | 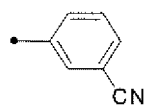 |
| ab | 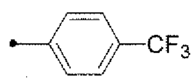 | 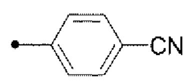 |
| ac | 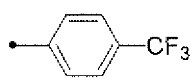 | 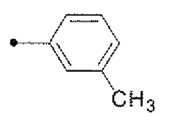 |
| ad | 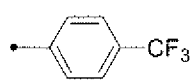 | 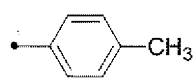 |
| ae | 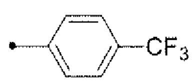 | 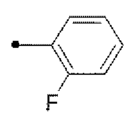 |
| af | 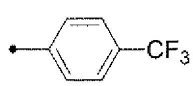 | 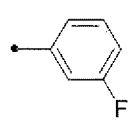 |

(continued)

| | | |
|---|---|---|
| ag | 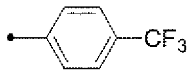 | 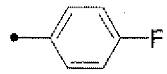 |
| ah | 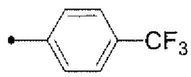 | 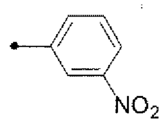 |
| ai | 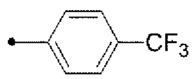 | 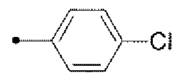 |

[Table 7-4]

| | | |
|---|---|---|
| aj | 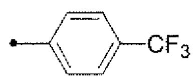 | 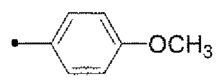 |
| ak | 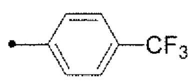 | 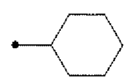 |
| al | 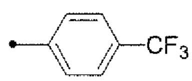 | 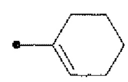 |
| am | 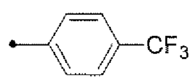 | 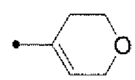 |
| ao | 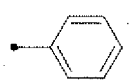 | 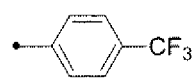 |
| aq | 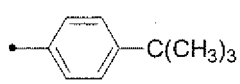 | 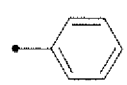 |
| ar | 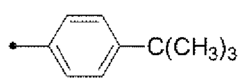 | 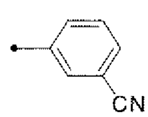 |
| as | 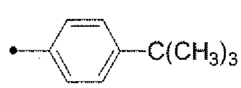 | 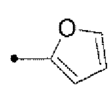 |

(continued)

| | | |
|---|---|---|
| au | 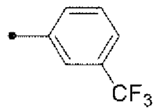 | 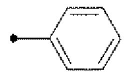 |
| av | 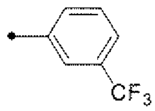 | 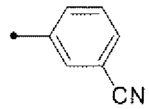 |
| aw | 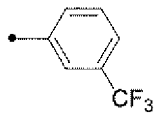 | 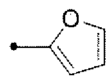 |

[Table 7-5]

| | | |
|---|---|---|
| ay | 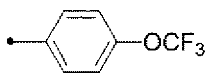 | 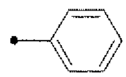 |
| az | 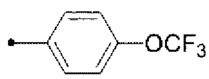 | 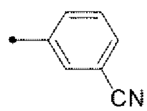 |
| ba | 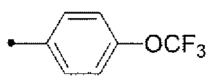 | 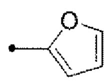 |
| bc | 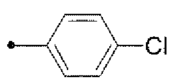 | 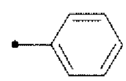 |
| bd | 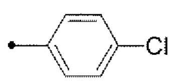 | 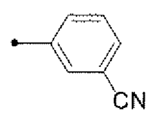 |
| be | 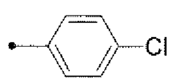 | 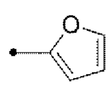 |
| bg | 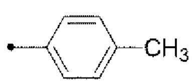 | 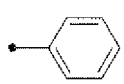 |
| bh | 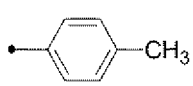 | 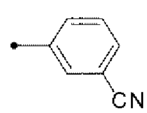 |

(continued)

| | | |
|---|---|---|
| bi | 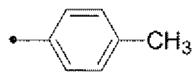 | 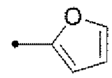 |

[Table 8-1]

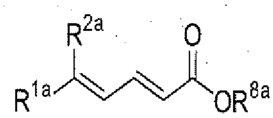

| Compound Number | $R^{1a}$ | $R^{2a}$ | $R^{8a}$ |
|---|---|---|---|
| a | Br | Br | $-CH_2CH_3$ |
| q | 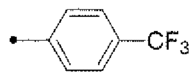 | Br | $-CH_2CH_3$ |
| an | 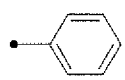 | 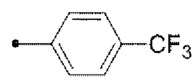 | $-CH_3$ |
| ap | 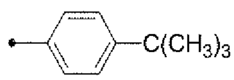 | Br | $-CH_2CH_3$ |
| at | 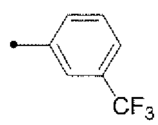 | Br | $-CH_2CH_3$ |
| ax | 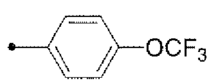 | Br | $-CH_2CH_3$ |
| bb | 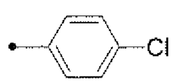 | Br | $-CH_2CH_3$ |
| bf | 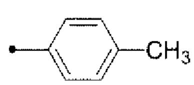 | Br | $-CH_2CH_3$ |

[Table 8-2]

| | | | |
|---|---|---|---|
| ch | 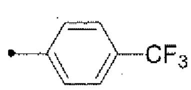 | 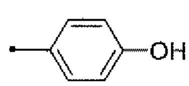 | $-CH_2CH_3$ |

(continued)

| | | | |
|---|---|---|---|
| cl | 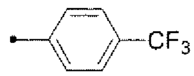 | 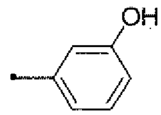 | $-CH_2CH_3$ |
| db | 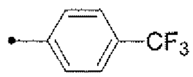 | 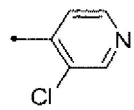 | $-CH_2CH_3$ |
| df | 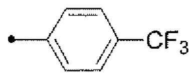 | 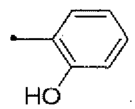 | $-CH_2CH_3$ |
| dk | 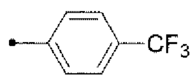 | 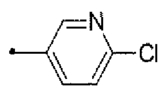 | $-CH_2CH_3$ |
| em | 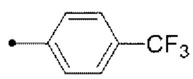 | 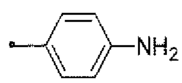 | $-CH_2CH_3$ |
| ez | 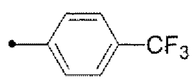 | 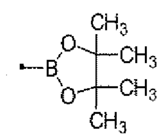 | $-CH_2CH_3$ |

[Table 9]

| Compound Number | Structure |
|---|---|
| bj | 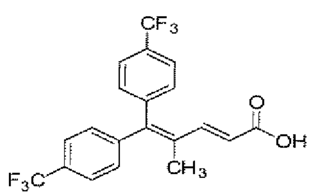 |
| bk | 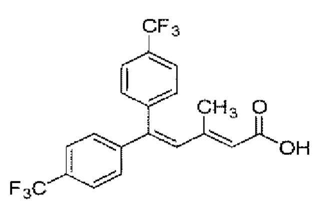 |
| bl | 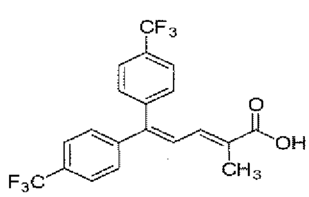 |

[Table 10-1]

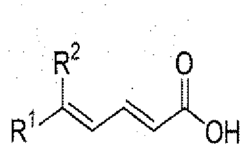

| Compound Number | $R^1$ | $R^2$ |
|---|---|---|
| ca | 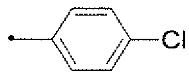 | 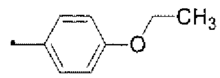 |
| cb | 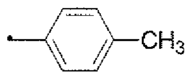 | 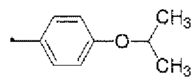 |
| cc | 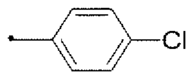 | 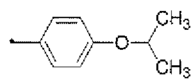 |
| cd | 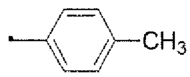 | 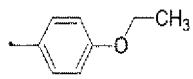 |
| ce | 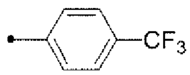 | 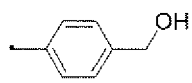 |
| cf | 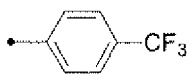 | 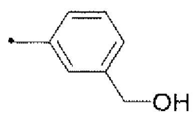 |
| cg | 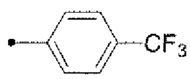 | 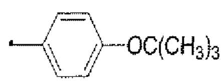 |
| ci | 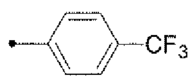 | 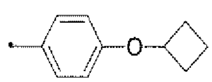 |
| cj | 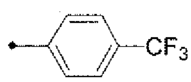 | 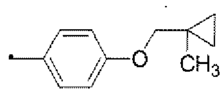 |
| ck | 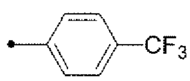 | 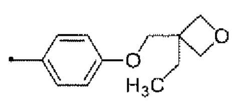 |

[Tables 10-2]

| | | |
|---|---|---|
| cm | 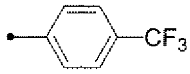 | 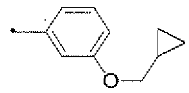 |
| cn | 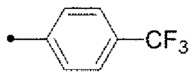 | 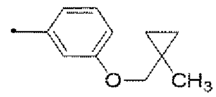 |
| co | 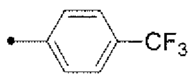 | 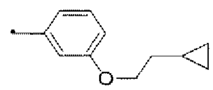 |
| cp | 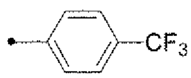 | 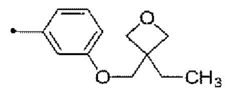 |
| cq | 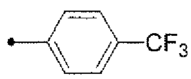 | 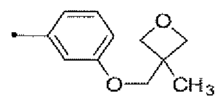 |
| cr | 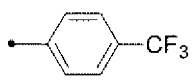 | 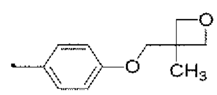 |
| cs | 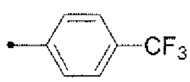 | 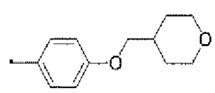 |
| ct | 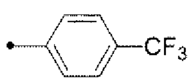 | 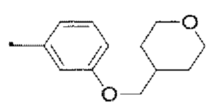 |
| cu | 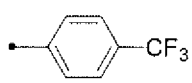 | 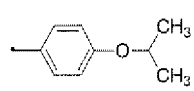 |
| cv | 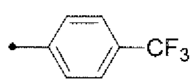 | 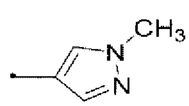 |

[Table 10-3]

| | | |
|---|---|---|
| cw | 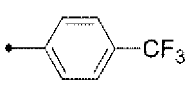 | 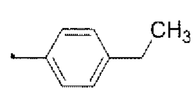 |

(continued)

| | | |
|---|---|---|
| cx | 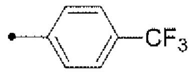 | 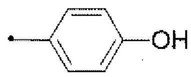 |
| cy | 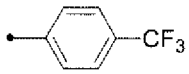 | 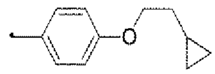 |
| cz | 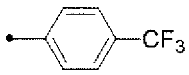 | 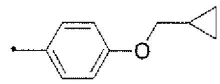 |
| da | 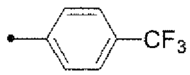 | 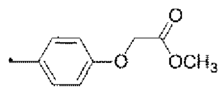 |
| dc | 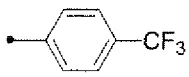 | 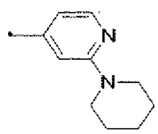 |
| dd | 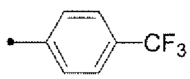 | 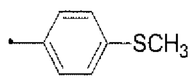 |
| de | 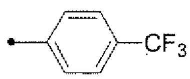 | 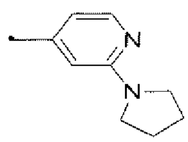 |
| dg | 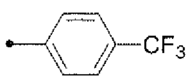 | 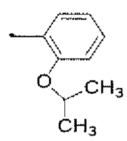 |
| dh | 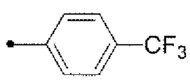 | 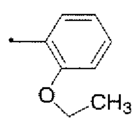 |

[Table 10-4]

| | | |
|---|---|---|
| di | 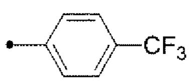 | 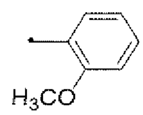 |

(continued)

| | | |
|---|---|---|
| dj | 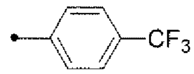 | 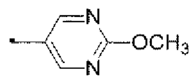 |
| dl | 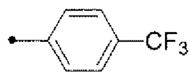 | 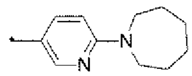 |
| dm | 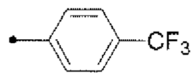 | 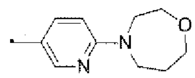 |
| dn | 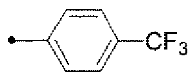 | 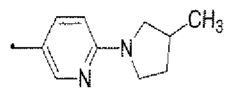 |
| do | 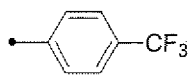 | 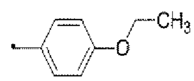 |
| dp | 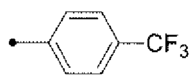 | 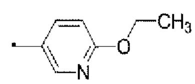 |
| dq | 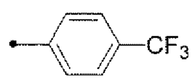 | 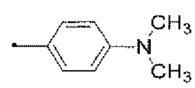 |
| dr | 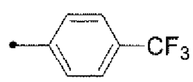 | 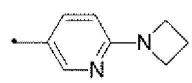 |
| ds | 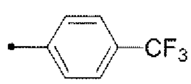 | 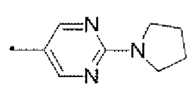 |

[Table 10-5]

| | | |
|---|---|---|
| dt | 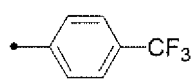 | 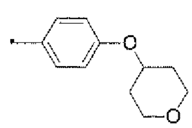 |
| du | 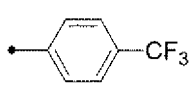 | 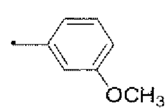 |

(continued)

| | | |
|---|---|---|
| dv | 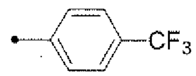 | 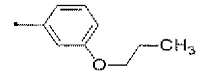 |
| dw | 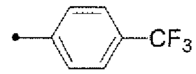 | 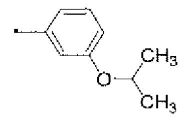 |
| dx | 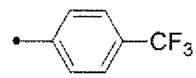 | 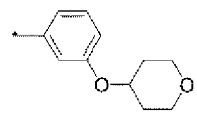 |
| dy | 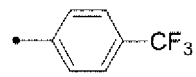 | 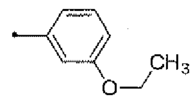 |
| dz | 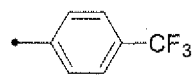 | 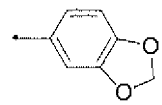 |
| ea | 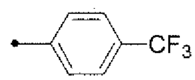 | 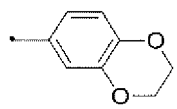 |
| eb | 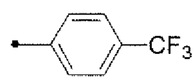 | 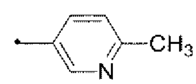 |

[Table 10-6]

| | | |
|---|---|---|
| ec | 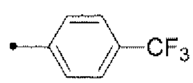 | 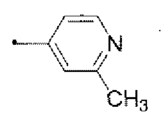 |
| ed | 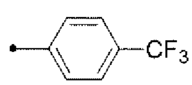 | 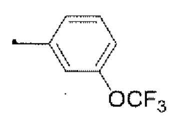 |
| ee | 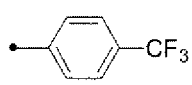 | 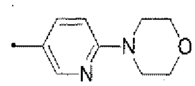 |
| ef | 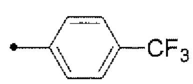 | 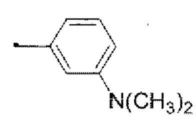 |

(continued)

| | | |
|---|---|---|
| eg | 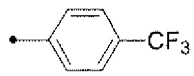 | 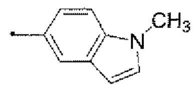 |
| eh | 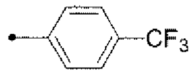 | 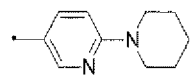 |
| ei | 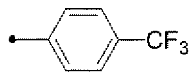 | 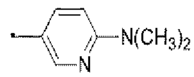 |
| ej | 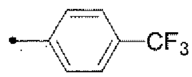 | 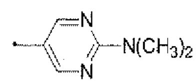 |
| ek | 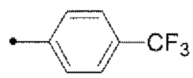 | 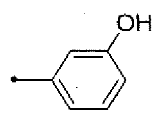 |
| el | 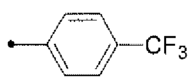 | 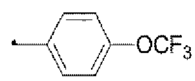 |

[Table 10-7]

| | | |
|---|---|---|
| en | 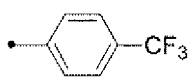 | 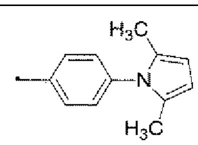 |
| eo | 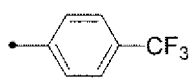 | 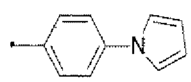 |
| ep | 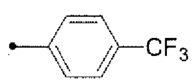 | 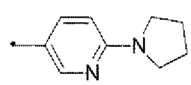 |
| eq | 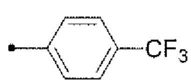 | 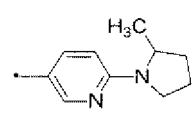 |
| er | 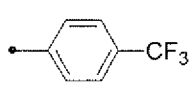 | 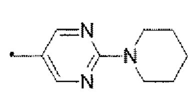 |

(continued)

| | | |
|---|---|---|
| es | 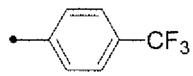 | 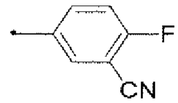 |
| et | 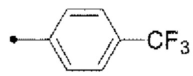 | 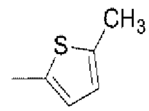 |
| eu | 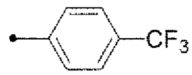 | 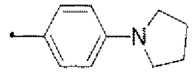 |
| ev | 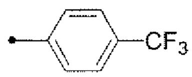 | 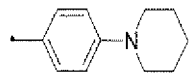 |
| ew | 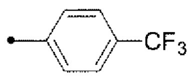 | 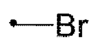 |

[Table 10-8]

| | | |
|---|---|---|
| ex | 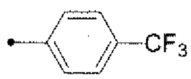 | 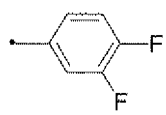 |
| ey | 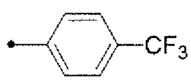 | 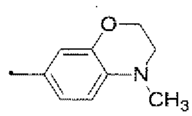 |
| fa | 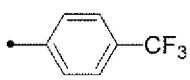 | 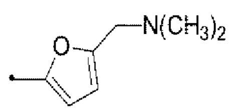 |
| fb | 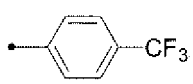 | 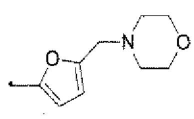 |
| fc | 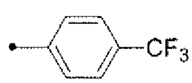 | 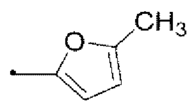 |
| fd | 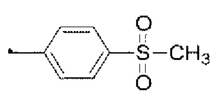 | -Br |

(continued)

| | | |
|---|---|---|
| fe | 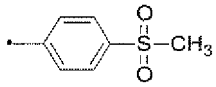 | 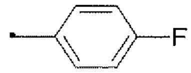 |
| ff | 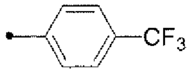 | 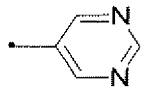 |
| fg | 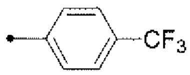 | 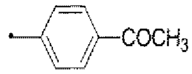 |

[Table 10-9]

| | | |
|---|---|---|
| fh | 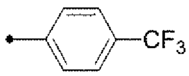 | 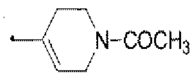 |
| fi | 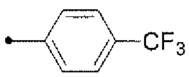 | 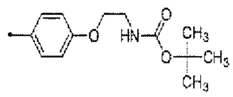 |
| fj | 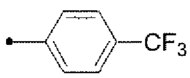 | 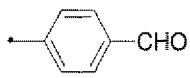 |
| fk | 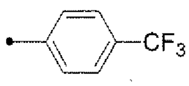 | 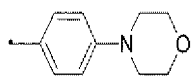 |
| fl | 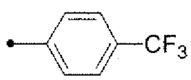 | 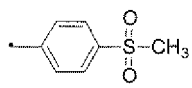 |
| fm | 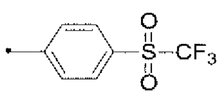 | 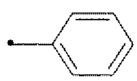 |
| fn | 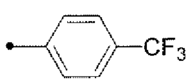 | 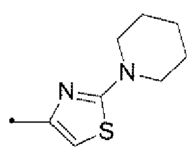 |
| fo | 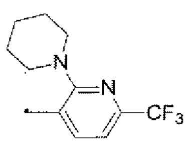 | 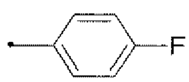 |

(continued)

| | | |
|---|---|---|
| fp | 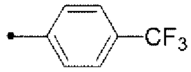 | 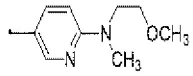 |
| fq | 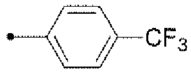 | 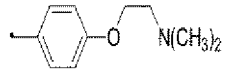 |

[Table 10-10]

| | | |
|---|---|---|
| fr | 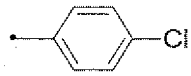 | 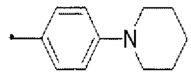 |
| fs | 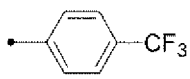 | 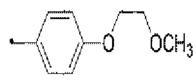 |
| ft | 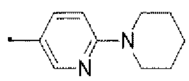 | 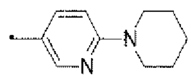 |
| fu | 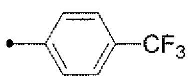 | 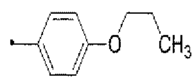 |
| fv | 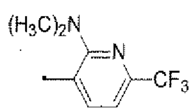 | 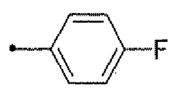 |
| fw | 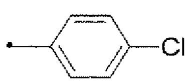 | 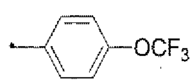 |
| fx | 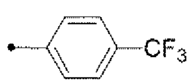 | 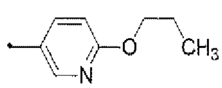 |
| fy | 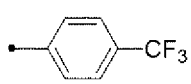 | 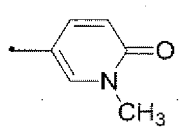 |
| fz | 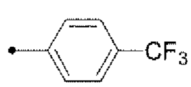 | 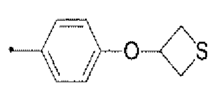 |

[Table 10-11]

| | | |
|---|---|---|
| ga | 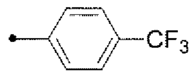 | 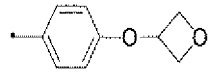 |
| ha | 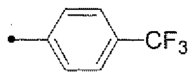 | 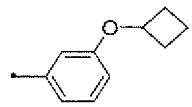 |
| hb | 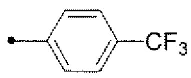 | 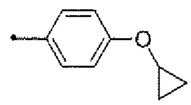 |
| hc | 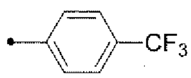 | 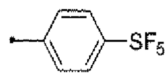 |
| hd | 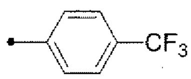 | 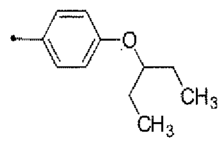 |
| he | 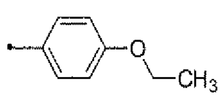 | 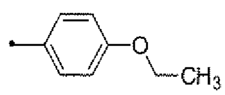 |
| hf | 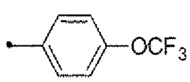 | 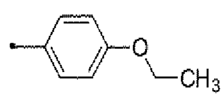 |

Subsequently, pharmacological activities of some typical Compounds (I) will be specifically described with reference to Test examples.

Test example 1: [$^3$H]resiniferatoxin ([$^3$H] RTX) binding experiment using rat spinal cord

1) Preparation of tissue membrane specimen

The spinal cord was extirpated from an SPF/VAF rat (Charles River Laboratories Japan Inc.) and homogenized in an ice-cooled homogenization buffer (10 mmol/L HEPES/NaOH, 5 mmol/L KCl, 5.8 mmol/L NaCl, 2 mmol/L $MgCl_2$, 0.75 mmol/L $CaCl_2$, 12 mmol/L glucose, 137 mmol/L sucrose, pH 7.4). The resulting homogenate was centrifuged at 1000 g for 10 minutes at 4˚C. The supernatant was centrifuged at 35000 g for 30 minutes at 4˚C. The obtained precipitate was suspended in the homogenization buffer. The suspension was used as a tissue membrane specimen, and the protein concentration was determined using a protein assay staining solution (Bio-Rad Laboratories, US). The thus prepared specimen was stored at -80˚C.

2) [$^3$H] RTX binding experiment

The known method [The Journal of Pharmacology and Experimental Therapeutics, 267: 728-733 (1993)] was modified and the modified method was performed. 400 μL of a membrane specimen solution (protein amount: about 50 μg) obtained by diluting the prepared specimen with an assay buffer (10 mmol/L HEPES/NaOH, 5 mmol/L KCl, 5.8

mmol/L NaCl, 2 mmol/L $MgCl_2$, 0.75 mmol/L $CaCl_2$, 12 mmol/L glucose, 137 mmol/L sucrose, 0.25 mg/mL bovine serum albumin, pH 7.4), 50 μL of a test compound solution, and 50 μL of [$^3$H]RTX (PerkinElmer Life Sciences Inc., US) were mixed and the mixture was incubated at 37°C for 60 minutes. The reaction mixture was transferred on ice, and 50 μL of previously ice-cooled 2 mg/mL $\alpha_1$ acid glycoprotein (Sigma) was added thereto, and then, the mixture was centrifuged at 12000 g for 15 minutes at 4°C. A procedure in which 0.4 mL of the assay buffer was added to the obtained precipitate and the mixture was centrifuged at 12000 g for 15 minutes at 4°C was performed twice. The precipitate obtained by the centrifugation was suspended in 0.5 mL of a 0.1 mol/L aqueous NaOH solution. 7 mL of Ultima Gold (Packard, US) was added to the suspension, and the radioactivity of the solution was measured using a liquid scintillation counter (LS6500: Beckman Coulter, Inc., US or TRI-CARB 2700TR: Packard, US). The [$^3$H] RTX binding amount in the presence of 0.1 μmol/L RTX was considered to be non-specific binding, and the specific binding was calculated by subtracting this value from the total amount of [$^3$H]RTX binding to the membrane specimen. Then, the replacement amount of the [$^3$H] RTX specific binding in the presence of the compound to the [$^3$H]RTX specific binding amount in the absence of the compound was calculated in percent. The $IC_{50}$ value of the compound in this experiment was obtained from a logistic equation.

The logistic equation was fit to the following equation (equation 205 in the program) in the curve regression program XLfit (IDBS Inc.).

$$Y = A + \frac{B - A}{1 + \left(\frac{C}{X}\right)^D}$$

Y: Inhibition ratio
X: Concentration of test compound

In this test, Compounds 10, 11, 25, 59, 68, 74, 163, 178, 188, 191, 229, 240, 257, 270, 310, and 314 showed an activity of $IC_{50}$ < 10 nmol/L.

Test example 2: Neuropathic pain inhibitory activity of compound in entrapment nerve injury rat

The method of Mosconi, Kruger et al. (Pain, vol. 64, pp. 37-57 (1996)) was modified partially and an entrapment nerve injury rat was produced.

By using a male Crl:CD(SD) rat, the sciatic nerve of the left hind limb was exfoliated under pentobarbital anesthesia, and the exfoliated region was covered with a polyethylene tube (trade name: Intramedic, size: PE-60, manufactured by Becton Dickinson and Company) with a length of 2 mm. On days 14 to 21 after the surgery, the rat was placed in a stainless-steel connected cage (750 mm (length) × 210 mm (depth) × 170 mm (height)) consisting of 5 cages connected in a row and having a mesh floor and was acclimated to the environment for at least 20 minutes, and then, the pain was evaluated.

The pain was evaluated using von Frey filaments (trade name: touch test sensory evaluator, Model number: model 58011, manufactured by Muromachi Kikai), and the results were calculated as a pain threshold. That is, by using a von Frey filament of different stimulation intensity, stimulation was given to the paw at the injured side of the entrapment nerve injury rat, and the stimulation intensity at which the paw was withdrawn was obtained. Then, the pain threshold (paw withdrawal threshold) (g) was calculated by the up down method of Dixon [Annual Review of Pharmacology and Toxicology, vol. 20, pp. 441-462 (1980)]. Incidentally, the pain threshold of a normal rat was from 10 to 12 g on an average.

In the evaluation of the test compound, a rat having a 50% pain threshold of less than 4 g was used, and the test compound was dissolved in a 0.5% aqueous methyl cellulose solution and orally administered at a dose of 5 mL/kg. After 1 hour, by using von Frey filaments, the pain threshold was measured.
As a result, Compounds 63, 74, 163, 178, 188, 191, 229, 240, 257, 270, 310 and 314 showed an activity which significantly increases the pain threshold at a dose of 20 mg/kg or less. That is, it was found that Compound (I) has a neuropathic pain inhibitory activity.

Compound (I) or a pharmaceutically acceptable salt thereof can be administered alone as it is. However, usually, Compound (I) or a pharmaceutically acceptable salt thereof is preferably provided as various pharmaceutical formulations. Further, such pharmaceutical formulations are to be used in animals or humans.
The pharmaceutical formulations according to the present invention can contain Compound (I) or a pharmaceutically acceptable salt thereof alone as an active ingredient or a mixture thereof with an optional active ingredient for another treatment. Further, these pharmaceutical formulations are prepared by mixing the active ingredient with one or more pharmaceutically acceptable carriers and then subjecting the mixture to any method well known in the technical field of

pharmaceutics.

As for the administration route, it is preferred to select the most effective route of administration in the treatment. Examples of the administration route include oral administration and parenteral administration such as intravenous administration.

As for the dosage form, for example, tablets, injections, and the like are included.

The suitable oral administration, for example, the tablet can be prepared with a diluent such as lactose or mannitol, a disintegrator such as starch, a lubricant such as magnesium stearate, a binder such as hydroxypropyl cellulose, a surfactant such as a fatty acid ester, a plasticizer such as glycerin, an antiseptic such as benzoic acid or p-hydroxybenzoate, or the like.

The suitable parenteral administration, for example, the injection preferably comprises a sterilized aqueous preparation containing the active compound which is isotonic to the blood of a recipient. A solution for injection is prepared using, for example, a medium consisting of a brine solution, a glucose solution, or a mixture of brine and a glucose solution, or the like.

Further, also to such a parenteral preparation, one or more additives selected from the diluent, disintegrator, lubricant, binder, surfactant, plasticizer, antiseptic, and the like, which are exemplified in the oral administration, may be added.

The doses and the frequencies of administration of Compound (I) or a pharmaceutically acceptable salt thereof may vary depending on dosage form, age and body weight of a patient, nature or seriousness of the symptom to be treated, and the like. However, in the oral administration, in general, a dose of 0.01 mg to 1 g, preferably, 0.05 to 50 mg is administered to an adult patient once or several times a day. In the parenteral administration such as intravenous administration, a dose of 0.001 to 100 mg, preferably, 0.01 to 50 mg is administered to an adult patient once or several times a day. However, these doses and frequencies of administration vary depending on the various conditions described above.

Hereinafter, the invention will be described in detail with reference to Reference examples and Examples.

Unless otherwise stated, a proton nuclear magnetic resonance spectrum ($^1$H-NMR) used in Reference examples and Examples was measured at 270 MHz. Further, in proton nuclear magnetic resonance spectrum, exchangeable hydrogen may not be clearly observed depending on the compounds and measurement conditions, and the hydrogen on the quaternary nitrogen atom may be observed in the case of hydrochloride. Further, the symbol "br" means a broad signal.

Reference example 1: Ethyl (E)-5,5-dibromo-2,4-pentadienoate (Compound a)

To a solution of carbon tetrabromide (50.7 g, 153 mmol) in dichloromethane (400 mL), triphenylphosphine (88.6 g, 338 mmol) was added at 0˚C, and further a solution of ethyl (E)-4-oxo-2-butenoate (10.0 g, 76.8 mmol) in dichloromethane (65 mL) was carefully added thereto, and then, the mixture was stirred for 30 minutes. Water (300 mL) was added to the reaction mixture, and then, the mixture was extracted with dichloromethane. The organic layer was washed with saturated brine and dried over anhydrous magnesium sulfate. Then, the solvent was evaporated under reduced pressure, and acetone-hexane (1:10, 300 mL) was added to the residue. The mixture was stirred and then filtered. Further, the residue was washed with acetone-hexane (1:10, 200 mL) and ethyl acetate-hexane (1:3, 200 mL), and all the filtrates were combined. The resulting mixture was concentrated under reduced pressure to give Compound a (21.1 g, 97%) as a colorless crystal.

$^1$H NMR ($CDCl_2$, δ ppm): 1.31 (t, J = 7.1 HZ, 3H), 4.23 (q, J = 7.1 Hz, 2H), 6.04 (d, J = 15.2 Hz, 1H), 7.08 (d, J = 10.7 Hz, 1H), 7.29 (dd, J = 10.7, 15.2 Hz, 1H).

Reference example 2: (E)-5,5-Bis[4-(trifluoromethyl)phenyl] -2,4-pentadienoic acid (Compound b)

Step 1

A mixture of Compound a (8.58 g, 30.0 mmol), 4-(trifluoromethyl)phenylboronic acid (14.3 g, 75.0 mmol), tetrakis(triphenylphosphine)palladium (1.73 g, 1.50 mmol), sodium carbonate (9.54 g, 90.0 mmol), dioxane (100 mL), and water (50 mL) was heated under reflux for 5 hours under an argon stream. After the reaction mixture was left to cool to room temperature, water and ethyl acetate were added to the mixture to separate an aqueous layer and an organic layer. Then, the organic layer was washed with water and saturated brine, and then dried over anhydrous magnesium sulfate. After the organic layer was filtered, the solvent was evaporated under reduced pressure, and then, the residue was purified by silica gel column chromatography (hexane/ethyl acetate = 9/1) to give ethyl (E)-5,5-bis [4-(trifluoromethyl)phenyl]-2,4- pentadienoate (10.12 g, 81%) as a pale yellow crystal.

$^1$H NMR ($CDCl_3$, δ ppm): 1.28 (t, J = 7.2 Hz, 3H), 4.19 (q, J = 7.2 Hz, 2H), 6.15 (d, J = 15.0 Hz, 1H), 6.89 (d, J = 11.6 Hz, 1H), 7.29 (dd, J = 11.6, 15.0 Hz, 1H), 7.33-7.39 (m, 4H), 7.59 (d, J = 8.1 Hz, 2H), 7.71 (d, J = 8.1 Hz, 2H).

Step 2

Ethyl (E)-5,5-bis[4-(trifluoromethyl)phenyl]-2,4-pentadienoate (10.1 g, 24.4 mmol) obtained in Step 1 was dissolved in THF (50 mL) and methanol (50 mL), and a 1 mol/L aqueous lithium hydroxide solution (50 mL) was added thereto, and then, the mixture was stirred at room temperature for 2 hours. After the reaction mixture was concentrated under reduced pressure, the residue was dissolved in water (1000 mL), and the pH of the mixture was adjusted to 5 with 6 mol/L hydrochloric acid. The precipitated crystal was filtered and washed with water to give Compound b (9.41 g, 100%)as a pale yellow crystal.
$^1$H NMR ($CDCl_3$, δ ppm): 6.14 (d, J = 15.2 Hz, 1H), 6.88 (d, J = 11.6 Hz, 1H), 7.30-7.39 (m, 4H), 7.35 (dd, J = 11.6, 15.2 Hz, 1H), 7.60 (d, J = 8.2 Hz, 2H), 7.72 (d, J = 8.2 Hz, 2H).

Reference example 3: (E)-5,5-Diphenyl-2,4-pentadienoic acid (Compound c)

Compound c was synthesized in a similar manner to Reference example 2 using Compound a and phenylboronic acid.
$^1$H NMR ($CDCl_3$, δ ppm) : 6.03 (d, J = 15.0 Hz, 1H), 6.81 (d, J = 11.6 Hz, 1H), 7.19-7.22 (m, 2H), 7.29-7.34 (m, 5H), 7.40-7.48 (m, 3H), 7.44 (dd, J = 11.6, 15.0 Hz, 1H).

Reference example 4: (E)-5,5-Bis[3-(trifluoromethyl)phenyl]-2,4-pentadienoic acid (Compound d)

Compound d was synthesized in a similar manner to Reference example 2 using Compound a and 3-(trifluoromethyl)phenylboronic acid.
$^1$H NMR ($CDCl_3$, δ ppm): 6.13 (d, J = 15.0 Hz, 1H), 6.88 (d, J = 11.4 Hz, 1H), 7.29 (dd, J = 11.4, 15.0 Hz, 1H), 7.37-7.49 (m, 4H), 7.56-7.71 (m, 4H).

Reference example 5: (E)-5,5-Bis(4-trifluoromethoxyphenyl)-2,4-pentadienoic acid (Compound e)

Compound e was synthesized in a similar manner to Reference example 2 using Compound a and 4-trifluoromethoxyphenylboronic acid.
$^1$H NMR ($CDCl_3$, δ ppm): 6.09 (d, J = 14.7 Hz, 1H), 6.81 (d, J = 11.6 Hz, 1H), 7.17-7.33 (m, 8H), 7.38 (dd, J = 11.6, 14.7 Hz, 1H).

Reference example 6: (E)-5,5-Bis(4-chlorophenyl)-2,4-pentadienoic acid (Compound f)

Compound f was synthesized in a similar manner to Reference example 2 using Compound a and 4-chlorophenylboronic acid.
$^1$H NMR ($CDCl_3$, δ ppm): 6.05 (d, J = 15.0 Hz, 1H), 6.77 (d, J = 11.6 Hz, 1H), 7.10-7.13 (m, 2H), 7.39-7.22 (m, 2H), 7.28-7.31 (m, 2H), 7.34 (dd, J = 11.6, 15.0 Hz, 1H), 7.37-7.40 (m, 2H).

Reference example 7: (E)-5,5-Bis(4-tert-butylphenyl)-2,4-pentadienoic acid (Compound g)

Compound g was synthesized in a similar manner to Reference example 2 using Compound a and 4-tert-butylphenylboronic acid.
$^1$H NMR ($CDCl_3$, δ ppm): 1.32 (s, 9H), 1.36 (s, 9H), 6.01 (d, J = 11.6 Hz, 1H), 6.77 (d, J = 15.0 Hz, 1H), 7.11-7.15 (m, 2H), 7.23-7.26 (m, 2H), 7.32-'7.35 (m, 2H), 7.38-7.42 (m, 2H), 7.50 (dd, J = 11.6, 15.0 Hz, 1H).

Reference example 8: (E)-5,5-Bis[6-(trifluoromethyl)pyridin-3-yl]-2,4-pentadienoic acid (Compound h)

Compound h was synthesized in a similar manner to Reference example 2 using Compound a and [6-(trifluoromethyl)pyridine-3-yl]boronic acid.
$^1$H NMR (DMSO-$d_6$, δ ppm): 6.34 (d, J = 14.9 Hz, 1H), 6.97 (dd, J = 11.5, 14.9 Hz, 1H), 7.51 (d, J = 11.5 Hz, 1H), 7.90-7.91 (m, 2H), 8.05-8.09 (m, 2H), 8.70 (s, 1H), 8.83 (s, 1H).

Reference example 9: (E)-5,5-Bis(2-methylphenyl)-2,4-pentadienoic acid (Compound i)

Compound i was synthesized in a similar manner to Reference example 2 using Compound a and 2-methylphenylboronic acid.
$^1$H NMR ($CDCl_3$, δ ppm): 2.05 (s, 3H), 2.22 (s, 3H), 5.98 (d, J = 15.2 Hz, 1H), 6.53 (d, J = 11.6 Hz, 1H), 7.08-7.24 (m,

8H), 7.29 (dd, J = 11.6, 15.2 Hz, 1H).

Reference example 10: (E)-5,5-Bis(4-methylphenyl)-2,4-pentadienaic acid (Compound j)

Compound j was synthesized in a similar manner to Reference example 2 using Compound a and 4-methylphenylboronic acid.
$^1$H NMR ($CDCl_3$, δ ppm): 2.36 (s, 3H), 2.41 (s, 3H), 6.00 (d, J = 15.2 Hz, 1H), 6.76 (d, J = 11.5 Hz, 1H), 7.09 (d, J = 8.2 Hz, 2H), 7.12 (d, J = 8.2 Hz, 2H), 7.21 (d, J = 8.2 Hz, 4H), 7.46 (dd, J = 11.5, 15.2 Hz, 1H).

Reference example 11: (E)-5,5-Bis(3-nitrophenyl)-2,4-pentadienoic acid (Compound k)

Compound k was synthesized in a similar manner to Reference example 2 using Compound a and 3-nitrophenylboronic acid.
$^1$H NMR (DMSO-$d_6$, δ ppm): 6.31 (d, J = 15.1 Hz, 1H), 6.97 (dd, J = 11.5, 15.1 Hz, 1H), 7.40 (d, J = 11.5 Hz, 1H), 7.64-7.79 (m, 3H), 7.85 (t, J = 8.0 Hz, 1H), 8.05 (t, J = 2.0 Hz, 1H), 8.12 (t, J = 2.0 Hz, 1H), 8.23 (dt, J = 2.0, 8.0 Hz, 1H), 8.36 (ddd, J = 1.2, 2.0, 8.0 Hz, 1H).

Reference example 12 : (E)-5,5-Bis(3-fluorophenyl)-2,4-pentadienoic acid (Compound 1)

Compound 1 was synthesized in a similar manner to Reference example 2 using Compound a and 3-fluorophenyl boronic acid.
$^1$H NMR ($CDCl_3$, δ ppm): 6.09 (d, J = 15.0 Hz, 1H), 6.83 (d, J = 11.7 Hz, 1H), 6.88-7.18 (m, 6H), 7.22-7.47 (m, 3H)

Reference example 13: (E)-5,5-Bis(4-fluorophenyl)-2,4-pentadienoic acid (Compound m)

Compound m was synthesized in a similar manner to Reference example 2 using Compound a and 4-fluorophenyl boronic acid.
$^1$H NMR (DMSO-$d_6$, δ ppm): 6.07-6.21 (m, 1H), 6.95-7.10 (m, 2H), 7.17-7.29 (m, 4H), 7.29-7.41 (m, 4H).

Reference example 14: (E)-5,5-Bis(4-cyanophenyl)-2,4-pentadienoic acid (Compound n)

Compound n was synthesized in a similar manner to Reference example 2 using Compound a and 4-cyanophenyl boronic acid.
$^1$H NMR (DMSO-$d_6$, δ ppm): 6.27 (d, J = 15.1 Hz, 1H), 6.94 (dd, J = 11.6, 15.1 Hz, 1H), 7.34 (d, J = 11.6 Hz, 1H), 7.43 (d, J = 8.5 Hz, 2H), 7.47 (d, J = 8.5 Hz, 2H), 7.85 (d, J = 8.5 Hz, 2H), 7.99 (d, J = 8.5 Hz, 2H).

Reference example 15: (E)-5,5-Bis(4-methoxyphenyl)-2,4-pentadienoic acid (Compound o)

Compound o was synthesized in a similar manner to Reference example 2 using Compound a and 4-methoxyphenyl boronic acid.
$^1$H NMR (DMSO-$d_6$, δ ppm): 3.77 (s, 3H), 3.82 (s, 3H), 6.04 (d, J = 15.1 Hz, 1H), 6.89 (d, J = 11.6 Hz, 1H), 6.93 (d, J = 8.6 Hz, 2H), 7.01-7.17 (m, 5H), 7.24 (d, J = 8.8 Hz, 2H).

Reference example 16: (E)-5,5-Bis(4-difluoromethylphenyl)-2,4-pentadienoic acid (Compound p)

Compound p was synthesized in a similar manner to Reference example 2 using Compound a and 4-difluoromethylphenylboronic acid.
$^1$H NMR ($CDCl_3$, δ ppm): 6.11 (d, J = 15.2 Hz, 1H), 6.65 (t, J = 56.3 Hz, 1H), 6.72 (t, J = 56.3 Hz, 1H), 6.88 (d, J = 11.7 Hz, 1H), 7.30 (d, J = 8.2 Hz, 2H), 7.30 (d, J = 8.2 Hz, 2H), 7.37 (dd, J = 11.7, 15.2 Hz, 1H), 7.48 (d, J = 8.2 Hz, 2H), 7.59 (d, J = 8.2 Hz, 2H).

Reference example 17: Ethyl (2E,4Z)--5-bromo-5-[4-(trifluoromethyl)phenyl]-2,4-pentadienoate (Compound q)

Compound a (3.00 g, 10.6 mmol), tri(2-furyl)phosphine (371 mg, 1.60 mmol), 4-(trifluoromethyl)phenylboronic acid (2.11 g, 11.1 mmol), tris(dibenzylideneacetone)dipalladium(0) (242 mg, 1.60 mmol), and sodium carbonate (2.24 g, 21.2 mmol) were dissolved in 1,4-dioxane (53 mL) and water (21 mL), and the mixture was stirred at 70°C for 6 hours. After the reaction mixture was left to cool, a saturated sodium hydrogen carbonate solution was added thereto, and then, the mixture was extracted three times with ethyl acetate. The organic layer was washed with saturated brine and

dried over anhydrous magnesium sulfate. Then, the solvent was evaporated under reduced pressure, and the residue was purified by silica gel column chromatography (hexane/ethyl acetate = 19/1) to give Compound q (1.98 g, 54%).
$^1H$ NMR ($CDCl_3$, δ ppm): 1.34 (t, J = 7.1 Hz, 3H), 4.27 (q, J = 7.1 Hz, 2H), 6.20 (d, J = 15.3 Hz, 1H), 7.00 (d, J = 10.8 Hz, 1H), 7.64 (d, J = 8.4 Hz, 2H), 7.70-7.78 (m, 3H).

Reference example 18: (2E,4E)-5-Phenyl-5-[4-(trifluoromethyl)phenyl]-2,4-pentadienoic acid (Compound r)

Step 1

Compound q (1.98 g, 5.68 mmol), tri(2-furyl)phosphine (203 mg, 0.875 mmol), phenylboronic acid (1.04 g, 8.53 mmol), tris(dibenzylideneacetone)dipalladium(0) (133 mg, 0.145 mmol), and sodium carbonate (1.21 g, 11.4 mmol) were dissolved in 1,4-dioxane (28 mL) and water (11 mL), and the mixture was stirred at 70°C for 4.5 hours. After the reaction mixture was left to cool, water was added thereto, and then, the mixture was extracted three times with ethyl acetate. The organic layer was washed with saturated brine and dried over anhydrous magnesium sulfate. Then, the solvent was evaporated under reduced pressure, and the residue was purified by silica gel column chromatography (hexane/ethyl acetate = 19/1) to give ethyl 5-phenol-5-[4-(trifluoromethyl)phenyl]-2,4- pentadienoate (1.79 g, 91%).
$^1H$ NMR ($CDCl_3$, δ ppm): 1.27 (t, J = 7.1 Hz, 3H), 4.18 (q, J = 7.1 Hz, 2H), 6.11 (d, J = 15.0 Hz, 1H), 6.82 (d, J = 11.4 Hz, 1H), 7.18-7.21 (m, 2H), 7.38-7.45 (m, 6H), 7.57 (d, J = 8.1 Hz, 2H).

Step 2

Ethyl 5-phenyl-5-[4-(trifluoromethyl)phenyl]-2,4-pentadienoate (1.79 g, 5.18 mmol) and lithium hydroxide monohydrate (761 mg, 18.1 mmol) were dissolved in THF (41 mL), methanol (16 mL) and water (16 mL), and the mixture was stirred at room temperature for 4 hours. After the reaction mixture was concentrated, water (80 mL) and 1 mol/L hydrochloric acid (20 mL) were added to the residue, and the mixture was stirred at 0°C for 30 minutes. The precipitated crystal was filtered and dried to give Compound r (1.63 g, 99%).
$^1H$ NMR (DMSO-$d_6$, δ ppm): 6.20 (d, J = 14.7 Hz, 1H), 7.06 (dd, J = 11.5, 14.7 Hz, 1H), 7.17-7.23 (m, 3H), 7.48-7.54 (m, 5H), 7.74 (d, J = 8.4 Hz, 2H).

Reference example 19: (2E,4Z)-5-(Pyridin-3-yl)-5-[4-(trifluoromethyl)phenyl]-2,4-pentadienoic acid (Compound s)

Compound s was synthesized in a similar manner to Reference example 18 using Compound q and 3-pyridylboronic acid.
$^1H$ NMR (DMSO-$d_6$, δ ppm): 6.27 (d, J = 15.1 Hz, 1H), 6.99 (dd, J = 11.6, 15.1 Hz, 1H), 7.32 (d, J = 11.6 Hz, 1H), 7.53 (d, J = 8.2 Hz, 2H), 7.57 (dd, J = 0.8, 4.8 Hz, 1H), 7.66-7.70 (m, 1H), 7.76 (d, J = 8.2 Hz, 2H), 8.44 (d, J = 1.6 Hz, 1H), 8.69 (dd, J = 1.6, 4.8 Hz, 1H), 12.46 (br s, 1H).

Reference example 20: (2E,4Z)-5-(Pyridin-4-yl)-5-[4-(trifluoromethyl)phenyl]-2,4-pentadienoic acid (Compound t)

Compound t was synthesized in a similar manner to Reference example 18 using Compound q and 4-pyridylboronic acid.
$^1H$ NMR (DMSO-$d_6$, δ ppm): 6.30 (d, J = 15.1 Hz, 1H), 6.97 (dd, J = 11.5, 15.1 Hz, 1H), 7.36 (d, J = 11.5 Hz, 1H), 7.41-7.45 (m, 2H), 7.53 (d, J = 8.1 Hz, 2H), 7.76 (d, J = 8.1 Hz, 2H), 8.77-8.81 (m, 2H).

Reference example 21: (2E,4Z)-5-[4-(Trifluoromethyl)phenyl]-5-[6-(trifluoromethyl)pyridin-3-yl]-2,4-pentadienoic acid (Compound u)

Compound u was synthesized in a similar manner to Reference example 18 using Compound q and 4-trifluoromethyl-3-pyridylboronic acid.
$^1H$ NMR (DMSO-$d_6$, δ ppm) : 6.32 (d, J = 14.8 Hz, 1H), 6.94 (dd, J = 11.5, 14.8 Hz, 1H), 7.41 (d, J = 11.5 Hz, 1H), 7.54 (d, J = 8.4 Hz, 2H), 7.76 (d, J = 8.4 Hz, 2H), 8.00 (d, J = 7.9 Hz, 1H), 8.07 (d, J = 7.9 Hz, 1H), 8.67 (s, 1H), 12. 54 (br s, 1H).

Reference example 22: (2E,4Z)-5-(Furan-2-yl)-5-[4-(trifluoromethyl)phenyl]-2,4-pentadienoic acid (Compound v)

Compound v was synthesized in a similar manner to Reference example 18 using Compound q and 2-furylboronic acid.
$^1H$ NMR (DMSO-$d_6$, δ ppm): 6.21 (d, J = 15.0 Hz, 1H), 6.51 (d, J = 3.3 Hz, 1H), 6.67-6.68 (m, 1H), 6.69 (d, J = 11.7 Hz, 1H), 7.60 (d, J = 8.0 Hz, 2H), 7.78 (d, J = 8.0 Hz, 2H), 7.87 (dd, J = 11.7, 15.0 Hz, 1H), 7.95-7.96 (m, 1H).

Reference example 23: (2E,4Z)-5-(Furan-3-yl)-5-[4-(trifluoromethyl)phenyl]-2,4-pentadienoic acid (Compound w)

Compound w was synthesized in a similar manner to Reference example 18 using Compound q and 3-furylboronic acid.
$^1$H NMR (DMSO-$d_6$, δ ppm): 6.21 (d, J = 15.1 Hz, 1H), 6.47-6.48 (m, 1H), 7.08 (d, J = 11.5 Hz, 1H), 7.40 (dd, J = 11.5, 15.1 Hz, 1H), 7.63 (d, J = 8.4 Hz, 2H), 7.76 (d, J = 8.4 Hz, 2H), 7.87-7.88 (m, 2H).

Reference example 24: (2E,4Z)-5-(Thiophen-2-yl)-5-[4-(trifluoromethyl)phenyl]-2,4-pentadienoic acid (Compound x)

Compound x was synthesized in a similar manner to Reference example 18 using Compound q and 2-thienylboronic acid.
$^1$H NMR (DMSO-$d_6$, δ ppm) : 6.26 (d, J = 15.1 Hz, 1H), 7.09 (d, J = 11.4 Hz, 1H), 7.15 (dd, J = 1.1, 3.5 Hz, 1H), 7.24 (dd, J = 3.5, 5.1 Hz, 1H), 7.45 (dd, J = 11.4, 15.1 Hz, 1H), 7.60 (d, J = 8.1 Hz, 2H), 7.77 (d, J = 8.1 Hz, 2H), 7.81 (dd, J = 1.1, 5.1 Hz, 1H), 12.44 (br s, 1H).

Reference example 25: (2E,4Z)-5-(Thiophen-3-yl)-5-[4-(trifluoromethyl)phenyl]-2,4-pentadienoic acid (Compound y)

Compound y was synthesized in a similar manner to Reference example 18 using Compound q and 3-thienylboronic acid.
$^1$H NMR (DMSO-$d_6$, δ ppm): 6.21 (d, J = 15.0 Hz, 1H), 6.99 (d, J = 4.8 Hz, 1H), 7.11 (d, J = 11.7 Hz, 1H), 7.26 (dd, J = 11.7, 15.0 Hz, 1H), 7.54 (d, J = 7.2 Hz, 2H), 7.55-7.58 (m, 1H), 7.73-7.76 (m, 3H).

Reference example 26: (2E,4Z)-5-(2-Cyanophenyl)-5-[4-(trifluoromethyl)phenyl]-2,4-pentadienoic acid (Compound z)

Compound z was synthesized in a similar manner to Reference example 18 using Compound q and 2-cyanophenylboronic acid.
$^1$H NMR (DMSO-$d_6$, δ ppm): 6.31 (d, J = 15.0 Hz, 1H), 6.76 (dd, J = 11.4, 15.0 Hz, 1H), 7.49-7.52 (m, 4H), 7.70 (d, J = 7.3 Hz, 1H), 7.77 (d, J = 8.4 Hz, 2H), 7.89 (t, J = 7.3 Hz, 1H), 8.05 (d, J = 7.3 Hz, 1H).

Reference example 27: (2E,4Z)-5-(3-Cyanophenyl)-5-[4-(trifluoromethyl)phenyl]-2,4-pentadienoic acid (Compound aa)

Compound aa was synthesized in a similar manner to Reference example 18 using Compound q and 3-cyanophenylboronic acid.
$^1$H NMR (DMSO-$d_6$, δ ppm): 6.25 (d, J = 15.1 Hz, 1H), 6.91 (dd, J = 11.7, 15.1 Hz, 1H), 7.28 (d, J = 11.7 Hz, 1H), 7.49 (d, J = 8.1 Hz, 2H), 7.57 (d, J = 7.8 Hz, 1H), 7.70-7.75 (m, 4H), 7.96 (d, J = 7.8 Hz, 1H).

Reference example 28: (2E,4E)-5-(4-Cyanophenyl)-5-[4-(trifluoromethyl)phenyl]-2,4-pentadienoic acid (Compound ab)

Compound ab was synthesized in a similar manner to Reference example 18 using Compound q and 4-cyanophenylboronic acid.
$^1$H NMR (DMSO-$d_6$, δ ppm): 6.27 (d, J = 15.0 Hz, 1H), 6.96 (dd, J = 11.6, 15.0 Hz, 1H), 7.30 (d, J = 11.6 Hz, 1H), 7.45 (d, J = 8.4 Hz, 2H), 7.50 (d, J = 8.4 Hz, 2H), 7.75 (d, J = 8.4 Hz, 2H), 8.00 (d, J = 8.4 Hz, 2H).

Reference example 29: (2E,4Z)-5-(3-Methylphenyl)-5-[4-(trifluoromethyl)phenyl]-2,4-pentadienoic acid (Compound ac)

Compound ac was synthesized in a similar manner to Reference example 18 using Compound q and 3-methylphenylboronic acid.
$^1$H NMR (DMSO-$d_6$, δ ppm): 2.35 (s, 3H), 6.19 (d, J = 14.7 Hz, 1H), 7.01-7.02 (m, 2H), 7.06 (dd, J = 11.6, 14.7 Hz, 1H), 7.18 (d, J = 11.6 Hz, 1H), 7.29 (d, J = 7.7 Hz, 1H), 7.41 (t, J = 7.7 Hz, 1H), 7.51 (d, J = 8.4 Hz, 2H), 7.73 (d, J = 8.4 Hz, 2H).

Reference example 30: (2E,4E)-5-(4-Methylphenyl)-5-[4-(trifluoromethyl)phenyl]-2,4-pentadienoic acid (Compound ad)

Compound ad was synthesized in a similar manner to Reference example 18 using Compound q and 4-methylphenylboronic acid.
$^1$H NMR (DMSO-$d_6$, δ ppm): 2.39 (s, 3H), 6.19 (d, J = 12.6 Hz, 1H), 7.06-7.19 (m, 4H), 7.32 (d, J = 7.5 Hz, 2H), 7.51 (d, J = 8.1 Hz, 2H), 7.73 (d, J = 7.8 Hz, 2H).

Reference example 31: (2E,4Z)-5-(2-Fluorophenyl)-5-[4-(trifluoromethyl)phenyl]-2,4-pentadienoic acid (Compound ae)

Compound ae was synthesized in a similar manner to Reference example 18 using Compound q and 2-fluorophenylboronic acid.
$^1$H NMR (DMSO-$d_6$, δ ppm): 6.26 (d, J = 15.1 Hz, 1H), 6.93 (ddd, J = 1.2, 11.5, 15.1 Hz, 1H), 7.28-7.48 (m, 5H), 7.54 (d, J = 8.7 Hz, 2H), 7.75 (d, J = 8.7 Hz, 2H), 12.46 (br s, 1H).

Reference example 32: (2E,4Z)-5-(3-Fluorophenyl)-5-[4-(trifluoromethyl)phenyl]-2,4-pentadienoic acid (Compound af)

Compound af was synthesized in a similar manner to Reference example 18 using Compound q and 3-fluorophenylboronic acid.
$^1$H NMR (DMSO-$d_6$, δ ppm): 6.24 (d, J = 15.0 Hz, 1H), 6.98-7.12 (m, 3H), 7.25 (d, J = 11.7 Hz, 1H), 7.34 (t, J = 7.9 Hz, 1H), 7.52 (d, J = 8.4 Hz, 2H), 7.57-7.61 (m, 1H), 7.75 (d, J = 8.4 Hz, 2H).

Reference example 33: (2E,4Z)-5-(4-Fluorophenyl)-5-[4-(triflunromethyl)phenyl]-2,4-pentadienoic acid (Compound ag)

Compound ag was synthesized in a similar manner to Reference example 18 using Compound q and 4-fluorophenylboronic acid.
$^1$H NMR (DMSO-$d_6$, δ ppm): 6.22 (d, J = 15.0 Hz, 1H), 7.05 (dd, J = 11.7, 15.0 Hz, 1H), 7.19-7.30 (m, 3H), 7.32-7.39 (m, 2H), 7.51 (d, J = 8.6 Hz, 2H), 7.74 (d, J = 8.6 Hz, 2H).

Reference example 34: (2E,4Z)-5-(3-Nitrophenyl)-5-[4-(trifluoromethyl)phenyl]-2,4-pentadienoic acid (Compound ah)

Compound ah was synthesized in a similar manner to Reference example 18 using Compound q and 3-nitrophenylboronic acid.
$^1$H NMR (DMSO-$d_6$, δ ppm): 6.29 (d, J = 15.0 Hz, 1H), 6.97 (dd, J = 11.5, 15.0 Hz, 1H), 7.33 (d, J = 11.5 Hz, 1H), 7.54 (d, J = 8.2 Hz, 2H), 7.71-7.77 (m, 3H), 7.84 (t, J = 8.1 Hz, 1H), 8.02 (s, 1H), 8.35 (d, J = 8.1 Hz, 1H).

Reference example 35: (2E,4Z)-5-(4-Chlorophenyl)-5-[4-(trifluoromethyl)phenyl]-2,4-pentadienoic acid (Compound ai)

Compound ai was synthesized in a similar manner to Reference example 18 using Compound q and 4-chlorophenylboronic acid.
$^1$H NMR (DMSO-$d_6$, δ ppm) : 6.22 (d, J = 14.7 Hz, 1H), 7.01 (dd, J = 12.6, 14.7 Hz, 1H), 7.21 (d, J = 12.6 Hz, 1H), 7.24 (d, J = 8.7 Hz, 2H), 7.51 (d, J = 8.1 Hz, 2H), 7.58 (d, J = 8.7 Hz, 2H), 7.74 (d, J = 8.4 Hz, 2H).

Reference example 36: (2E,4Z)-5-(4-Methoxyphenyl)-5-[4-(trifluoromethyl)phenyl]-2,4-pentadienoic acid (Compound aj)

Compound aj was synthesized in a similar manner to Reference example 18 using Compound q and 4-methoxyphenylboronic acid.
$^1$H NMR (DMSO-$d_6$, δ ppm): 3.83 (s, 3H), 6.19 (d, J = 14.1 Hz, 1H), 7.05-7.16 (m, 6H), 7.51 (d, J = 8.4 Hz, 2H), 7.73 (d, J = 8.4 Hz, 2H).

Reference example 37: (2E,4E)-5-Cyclohexyl-5-[4-(trifluaromethyl)phenyl]-2,4-pentadienaic acid (Compound ak)

Compound ak was synthesized in a similar manner to Reference example 18 using Compound q and cyclohexylboronic acid.
$^1$H NMR (DMSO-$d_6$, δ pom): 1.08-1.46 (m, 5H), 1.69-1.81 (m, 5H), 2.94-3.01 (m, 1H), 5.94 (d, J = 14.8 Hz, 1H), 6.07 (d, J = 11.8 Hz, 1H), 7.30 (d, J = 7.8 Hz, 2H), 7.59 (d, J = 7.8 Hz, 2H), 7.88 (dd, J = 11.8, 14.8 Hz, 1H).

Reference example 38: (2E,4E)-5-(Cyclohexen-1-yl)-5-[4-(trifluoromethyl)phenyl]-2,4-pentadienoic acid (Compound al)

Compound al was synthesized in a similar manner to Reference example 18 using Compound q and 1-cyclohexenylboronic acid.
$^1$H NMR (DMSO-$d_6$, δ ppm): 1.65-1.66 (m, 4H), 1.87-1.88 (m, 2H), 2.22-2.23 (m, 2H), 5.70-5.71 (m, 1H), 6.11 (d, J = 15.3 Hz, 1H), 6.82 (d, J = 11.4 Hz, 1H), 7.43 (dd, J = 11.4, 15.3 Hz, 1H), 7.65 (d, J = 8.3 Hz, 2H), 7.74 (d, J = 8.3 Hz, 2H).

Reference example 39: (2E,4E)-5-(3,6-Dihydro-2H-pyran-4-yl)-5-[4-(trifluoromethyl)phenyl]-2,4-pentadienoic acid (Compound am)

Compound am was synthesized in a similar manner to Reference example 18 using Compound q and 3,6-dihydro-*2H*-pyran-4-ylboronic acid.
$^1$H NMR (DMSO-$d_6$, δ ppm): 2.06-2.09 (m, 2H), 3.88 (t, J = 5.4 Hz, 2H), 4.36-4.38 (m, 2H), 5.86-5.87 (m, 1H), 6.11 (dd, J = 0.6, 15.3 Hz, 1H), 6.78 (d, J = 11.4 Hz, 1H), 7.68 (dd, J = 11.4, 15.0 Hz, 1H), 7.65-7.66 (m, 4H).

Reference example 40: Methyl (2E,4Z)-5-phenyl-5-[4-(trifluoromethyl)phenyl]-2,4-pentadienoate (Compound an)

Step 1

Commercially available ethyl 3-phenylpropionate (513 mg, 2.94 mmol) and sodium iodide (1.42 g, 9.48 mmol) were dissolved in acetic acid (2.2 mL), and the mixture was stirred at 110°C for 4.5 hours. After the reaction mixture was left to cool, water was added thereto, and then, the mixture was extracted twice with ethyl acetate. The organic layer was washed with a saturated sodium hydrogen carbonate solution, an aqueous sodium thiosulfate solution, and saturated brine, and then, dried over anhydrous magnesium sulfate. Then, the solvent was evaporated under reduced pressure to give ethyl (Z)-3-iodo-3- phenylacrylate (853 mg, 96%).
$^1$H NMR ($CDCl_3$, δ ppm): 1.34 (t, J = 7.1 Hz, 3H), 4.29 (q, J = 7.1 Hz, 2H), 6.63 (s, 1H), 7.34-7.37 (m, 3H), 7.52-7.55 (m, 2H).

Step 2

Ethyl (Z)-3-iodo-3-phenylacrylate (406 mg, 1.34 mmol), tri(2-furyl)phosphine (47.7 mg, 0.205 mmol), 4-(trifluoromethyl)phenylboronic acid (384 mg, 2.02 mmol), tris(dibenzylideneacetone)dipalladium(0) (31.7 mg, 0.0346 mmol), and sodium carbonate (286 mg, 2.70 mmol) were dissolved in 1,4-dioxane (6.8 mL) and water (2.7 mL), and the mixture was stirred at 70°C for 17 hours. After the reaction mixture was left to cool, water was added thereto, and then, the mixture was extracted twice with ethyl acetate. The organic layer was washed with saturated brine and dried over anhydrous magnesium sulfate. Then, the solvent was evaporated under reduced pressure, and the residue was purified by silica gel column chromatography (hexane/ethyl acetate = 19/1) to give ethyl (z)-3-phenyl-3-[4-(trifluoromethyl)phenyl] acrylate (240 mg, 56%).
$^1$H NMR ($CDCl_3$, δ ppm): 1.12 (t, J = 6.8 Hz, 3H), 4.06 (q, J = 6.8 Hz, 2H), 6.44 (s, 1H), 7.25-7.41 (m, 7H), 7.65 (d, J = 6.0 Hz, 2H).

Step 3

Ethyl (Z)-3-phenyl-3-[4-(trifluoromethyl)phenyl] acrylate (240 mg, 0.748 mmol) was dissolved in THF (4.0 mL), and a 1.01 mol/L diisobutylaluminum hydride-toluene solution (2.75 mL, 2.78 mmol) was added thereto, and then, the mixture was stirred at -78°C for 5 hours. After the temperature of the mixture was raised, a saturated aqueous ammonium chloride solution was added to the reaction mixture, and then, the mixture was extracted three times with ethyl acetate. The organic layer was washed with saturated brine and dried over anhydrous magnesium sulfate. Then, the solvent was evaporated under reduced pressure to give (Z)-3-phenyl-3-(trifluoromethyl)phenyl-2-propen-1-ol (201 mg, 97%).
$^1$H NMR ($EDCl_3$, δ ppm): 4.20 (d, J = 6.7 Hz, 2H), 6.32 (t, J = 6.7 Hz, 1H), 7.16-7.30 (m, 7H), 7.64 (d, J = 8.0 Hz, 2H).

Step 4

(Z)-3-Phenyl-3-(4-trifluoromethylphenyl)-2-propen-1-ol (201 mg, 0.723 mmol) was dissolved in dichloromethane (4.0 mL), and manganese dioxide (630 mg, 7.25 mmol) was added thereto, and then, the mixture was stirred at room temperature for 4 hours. After the reaction mixture was filtered, the solvent was evaporated under reduced pressure to give (Z)-3-phenyl-3-[4-(trifluoromethyl)phenyl]propenal (194 mg, 97%).
$^1$H NMR ($CDCl_3$, δ ppm): 6.67 (d, J = 7.9 Hz, 1H), 7.31-7.47 (m, 7H), 7.74 (d, J = 8.0 Hz, 2H), 9.50 (d, J = 8.0 Hz, 1H).

Step 5

(Z)-3-Phenyl-3-[4-(trifluoromethyl)phenyl]propenal (194 mg, 0.702 mmol) was dissolved in dichloromethane (2.0 mL), and methyl (triphenylphosphoranylidene)acetate (290 mg, 0.868 mmol) was added thereto, and then, the mixture was stirred at room temperature for 24 hours. After water was added to the reaction mixture, the mixture was extracted twice with ethyl acetate. The organic layer was washed with saturated brine and dried over anhydrous magnesium sulfate. Then, the solvent was evaporated under reduced pressure, and the residue was purified by silica gel

column chromatography (hexane/ethyl acetate = 19/1) to give Compound an (191 mg, 82%).
$^1$H NMR ($CDCl_3$, $\delta$ ppm): 3.72 (s, 3H), 6.10 (dd, J = 0.7, 15.2 Hz, 1H), 6.86 (d, J = 11.7 Hz, 1H), 7.23-7.35 (m, 8H), 7.69 (d, J = 8.1 Hz, 2H).

Reference example 41: (2E,4Z)-5-Phenyl-5-[4-(trifluoromethyl)phenyl]-2,4-pentadienoic acid (Compound ao)

Compound ao was synthesized in a similar manner to step 2 of Reference example 18 using Compound an.
$^1$H NMR (DMSO-$d_6$, $\delta$ ppm): 6.20 (d, J = 14.9 Hz, 1H) 6.98 (dd, J = 11.7, 14.9 Hz, 1H), 7.17 (d, J = 11.7 Hz, 1H), 7.28-7.31 (m, 2H), 7.38-7.40 (m, 3H), 7.44 (d, J = 8.0 Hz, 2H), 7.87 (d, J = 8.0 Hz, 2H), 12.35 (br s, 1H).

Reference example 42: Ethyl (2E,4Z)-5-bromo-5-(4-tert-butylphenyl)-2,4-pentadienoate (Compound ap)

Compound ap was synthesized in a similar manner to Reference example 17 using Compound a and 4-tert-buthylphenylboronic acid.
$^1$H NMR (DMSO-$d_6$, $\delta$ ppm): 1.31-1.35 (m, 12H), 4.25 (q, J = 7.1 Hz, 2H), 6.12 (d, J = 15.3 Hz, 1H), 6.93 (d, J = 10.7 Hz, 1H), 7.40 (d, J = 8.6 Hz, 2H), 7.58 (d, J = 8.6 Hz, 2H), 7.76 (dd, J = 10.7, 15.3 Hz, 1H).

Reference example 43: (2E,4E)-5-(4-tert-Butylphenyl)-5-phenyl-2,4-pentadienoic acid (Compound aq)

Compound aq was synthesized in a similar manner to Reference example 18 using Compound ap and phenylboronic acid.
$^1$H NMR (DMSO-$d_6$, $\delta$ ppm): 1.27 (s, 9H), 6.05-6.16 (m, 1H), 7.01-7.11 (m, 2H), 7.16-7.20 (m, 2H), 7.23 (d, J = 8.4 Hz, 2H), 7.39 (d, J = 8.4 Hz, 2H), 7.45-7.53 (m, 3H).

Reference example 44: (2E,4Z)-5-(4-tert-Butylphenyl)-5-(3-cyanophenyl)-2,4-pentadienoic acid (Compound ar)

Compound ar was synthesized in a similar manner to Reference example 18 using Compound ap and 3-cyanophenylboronic acid.
$^1$H NMR (DMSO-$d_6$, $\delta$ ppm): 1.27 (s, 9H), 6.17 (d, J = 15.0 Hz, 1H), 6.91 (dd, J = 11.6, 15.0 Hz, 1H), 7.14 (d, J = 11.6 Hz, 1H), 7.22 (d, J = 8.4 Hz, 2H), 7.41 (d, J = 8.4 Hz, 2H), 7.54 (d, J = 8.1 Hz, 1H), 7.70-7.74 (m, 2H), 7.95 (d, J = 7.5 Hz, 1H).

Reference example 45: (2E,4Z)-5-(4-tert-Butylphenyl)-5-(furan-2-yl)-2,4-pentadienoic acid (Compound as)

Compound as was synthesized in a similar manner to Reference example 18 using Compound ap and 2-furylboronic acid.
$^1$H NMR (DMSO-$d_6$, $\delta$ ppm): 1.30 (s, 9H), 6.13 (d, J = 15.2 Hz, 1H), 6.49 (d, J = 3.3 Hz, 1H), 6.61 (d, J = 12.0 Hz, 1H), 6.65 (dd, J = 1.8, 3.3 Hz, 1H), 7.31 (d, J = 8.4 Hz, 2H), 7.44 (d, J = 8.4 Hz, 2H), 7.85 (dd, J = 12.0, 15.2 Hz, 1H), 7.91-7.92 (m, 1H), 12.32 (br s, 1H).

Reference example 46: Ethyl (2E,4Z)-5-bromo-5-[3-(trifluoromethyl)phenyl]-2,4-pentadiene (Compound at)

Compound at was synthesized in a similar manner to Reference example 17 using Compound a and 3-(trifluoromethyl)phenylboronic acid.
$^1$H NMR ($CDCl_3$, $\delta$ ppm): 1.34 (t, J = 7.1 Hz, 3H), 4.27 (q, J = 7.1 Hz, 2H), 6.20 (dd, J = 0.7, 15.4 Hz, 1H), 7.00 (dd, J = 0.7, 10.6 Hz, 1H), 7.52 (t, J = 7.9 Hz, 1H), 7.63 (d, J = 7.9 Hz, 1H), 7.74 (dd, J = 10.6, 15.4 Hz, 1H), 7.83 (d, J = 7.9 Hz, 1H), 7.88 (s, 1H).

Reference example 47: (2E,4E)-5-Phenyl-5-[3-(trifluoromethyl)phenyl]-2,4-pentadienoic acid (Compound au)

Compound au was synthesized in a similar manner to Reference example 18 using Compound at and phenylboronic acid.
$^1$H NMR ($EDCl_3$, $\delta$ ppm): 6.10 (d, J = 15.3 Hz, 1H), 6.83 (d, J = 11.7 Hz, 1H), 7.16-7.24 (m, 2H), 7.39-7.50 (m, 6H), 7.56-7.62 (m, 2H).

Reference example 48: (2E,4E)-5-(3-Cyanophenyl)-5-[3-(trifluoromethyl)phenyl]-2,4-pentadienoic acid (Compound av)

Compound av was synthesized in a similar manner to Reference example 18 using Compound at and 3-cyanophenylboronic acid.

$^1H$ NMR ($CDCl_3$, δ ppm): 6.16 (d, J = 15.0 Hz, 1H), 6.91 (d, J = 11.5 Hz, 1H), 7.21-7.44 (m, 2H), 7.44-7.68 (m, 6H), 7.76 (dt, J = 1.4, 7.7 Hz, 1H).

Reference example 49: (2E,4Z)-5-(Furan-2-yl)-5-[3-(trifluoromethyl)phenyl]-2,4-pentadienoic acid (Compound aw)

Compound aw was synthesized in a similar manner to Reference example 18 using Compound at and 2-furylboronic acid.
$^1H$ NMR ($CDCl_3$, δ ppm): 6.09 (d, J = 15.2 Hz, 1H), 6.37 (d, J = 3.4 Hz, 1H), 6.39 (d, J = 11.9 Hz, 1H), 6.51 (dd, J = 1.8, 3.4 Hz, 1H), 7.46-7.61 (m, 2H), 7.61-7.69 (m, 3H), 8.29 (dd, J = 11.9 Hz, 15.2 Hz, 1H).

Reference example 50: Ethyl (2E,4Z)-5-bromo-5-[4-(trifluoromethoxy)phenyl]-2,4-pentadienoate (Compound ax)

Compound ax was synthesized in a similar manner to Reference example 17 using Compound a and 4-(trifluoromethoxy)phenylboronic acid.
$^1H$ NMR ($CDCl_3$, δ ppm): 1.33 (t, J = 7.1 Hz, 3H), 4.26 (q, J = 7.1 Hz, 2H), 6.16 (dd, J = 0.8 Hz, 15.4 Hz, 1H), 6.92 (dd, J = 0.8 Hz, 10.6 Hz, 1H), 7.23 (d, J = 8.6 Hz, 2H), 7.67 (d, J = 8.6 Hz, 2H), 7.72 (dd, J = 10.6 Hz, 15.4 Hz, 1H).

Reference example 51: (2E,4E)-5-Phenyl-5-[4-(trifluoromethoxy)phenyl]-2,4-pentadienoic acid (Compound ay)

Compound ay was synthesized in a similar manner to Reference example 18 using Compound ax and phenylboronic acid.
$^1H$ NMR ($CDCl_3$, δ ppm): 6.05 (d, J = 15.1 Hz, 1H), 6.79 (d, J = 11.6 Hz, 1H), 7.11-7.50 (m, 10H).

Reference example 52: (2E,4Z)-5- (3-Cyanophenyl)-5- [4-(trifluoromethoxy)phenyl]-2,4-pentadienoic acid (Compound az)

Compound az was synthesized in a similar manner to Reference example 18 using Compound ax and 3-cyanophenylboronic acid.
$^1H$ NMR ($CDCl_3$, δ ppm) : 6.12 (d, J = 15.0 Hs, 1H), 6.86 (d, J = 11.7 Hz, 1H), 7.14-7.35 (m, 5H), 7.43-7.67 (m, 3H), 7.73 (d, J = 7.6 Hz, 1H).

Reference example 53: (2E,4Z)-5-(Furan-2-yl)-5-[4-(trifluoromethoxy)phenyl]-2,4-pentadienoic acid (Compound ba)

Compound ba was synthesized in a similar manner to Reference example 18 using Compound ax and 2-furylboronic acid.
$^1H$ NMR ($EDCl_3$, δ ppm): 6.07 (d, J = 15.1 Hz, 1H), 6.38 (d, J = 12.2 Hz, 1H), 6.40 (d, J = 3.2 Hz, 1H), 6.51 (dd, J = 1.8, 3.2 Hz, 1H), 7.23 (d, J = 8.6 Hz, 2H), 7.42 (d, J = 8.6 Hz, 2H), 7.62 (d, J = 1.8 Hz, 1H), 8.27 (dd, J = 12.2, 15.1 Hz, 1H).

Reference example 54: Ethyl (2H,4Z)-5-bromo-5-(4-chlorophenyl)-2,4-pentadienoate (Compound bb)

Compound bb was synthesized in a similar manner to Reference example 17 using Compound a and 4-chlorophenylboronic acid.
$^1H$ NMR ($CDCl_3$, δ ppm): 1.33 (t, J = 7.1 Hz, 3H), 4.26 (q, J = 7.1 Hz, 2H), 6.15 (d, J = 15.4 Hz, 1H), 6.92 (d, J = 10.6 Hz, 1H), 7.35 (d, J = 8.5 Hz, 2H), 7.58 (d, J = 8.5 Hz, 2H), 7.72 (dd, J = 10.6, 15.4 Hz, 1H).

Reference example 55: (2H,4H)-5-(4-Chlorophenyl)-5-phenyl-2,4-pentadienoic acid (Compound bc)

Compound bc was synthesized in a similar manner to Reference example 18 using Compound bb and phenylboronic acid.
$^1H$ NMR ($CDCl_3$, δ ppm): 6.05 (d, J = 15.1 Hz, 1H), 6.79 (d, J = 11.3 Hz, 1H), 7.14-7.49 (m, 10H).

Reference example 56: (2E,4Z)-5-(4-Chlorophenyl)-5-(3-cyanophenyl)-2,4-pentadienoic acid (Compound bd)

Compound bd was synthesized in a similar manner to Reference example 18 using Compound bb and 3-cyanophenylboronic acid.
$^1H$ NMR ($CDCl_3$, δ ppm): 6.11 (d, J = 15.0 Hz, 1H), 6.86 (d, J = 11.7 Hz, 1H), 7.17 (d, J = 8.6 Hz, 2H), 7.22-7.37 (m, 3H), 7.43-7.51 (m, 2H), 7.58 (t, J = 7.7 Hz, 1H), 7.73 (d, J = 7.7 Hz, 1H).

Reference example 57: (2E,4Z)-5-(4-Chlorophenyl)-5-(furan-2-yl)-2,4-pentadienoic acid (Compound be)

Compound be was synthesized in a similar manner to Reference example 18 using Compound bb and 2-furylboronic acid.
$^1$H NMR (DMSO-$d_6$, δ ppm): 6.17 (d, J = 15.1 Hz, 1H), 6.49 (d, J = 3.4 Hz, 1H), 6.63 (d, J = 11.5 Hz, 1H), 6.66 (dd, J = 1.9, 3.4 Hz, 1H), 7.39 (d, J = 8.8 Hz, 2H), 7.48 (d, J = 8.8 Hz, 2H), 7.85 (dd, J = 11.5, 15.1 Hz, 1H), 7.94 (dd, J = 0.7, 1.9 Hz, 1H).

Reference example 58: Ethyl (2E,4Z)-5-bromo-5-(4-methylphenyl)-2,4-pentadienoate (Compound bf)

Compound bf was synthesized in a similar manner to Reference example 17 using Compound a and 4-methylphenylboronic acid.
$^1$H NMR ($CDCl_3$, δ ppm): 1.33 (t, J = 7.1 Hz, 3H), 2.38 (s, 3H), 4.25 (q, J = 7.1 Hz, 2H), 6.11 (d, J = 15.4 Hz, 1H), 6.91 (d, J = 10.6 Hz, 1H), 7.18 (d, J = 8.4 Hz, 2H), 7.54 (d, J = 8.4 Hz, 2H), 7.75 (dd, J = 10.6, 15.4 Hz, 1H).

Reference example 59: (2E,4E)-5-(4-Methylphenyl)-5-phenyl-2,4-pentadienoic acid (Compound bg)

Compound bg was synthesized in a similar manner to Reference example 18 using Compound bf and phenylboronic acid.
$^1$H NMR ($CDCl_3$, δ ppm): 2.36 (s, 3H), 6.01 (d, J = 15.0 Hz, 1H), 6.80 (d, J = 11.7 Hz, 1H), 7.09-7.16 (m, 2H), 7.17-7.24 (m, 4H), 7.37-7.48 (m, 4H).

Reference example 60: (2E,4Z)-5-(3-Cyanophenyl)-5-(4-methylphenyl)-2,4-pentadienoic acid (Compound bh)

Compound bh was synthesized in a similar manner to Reference example 18 using Compound bf and 3-cyanophenylboronic acid.
$^1$H NMR ($CDCl_3$, δ ppm): 2.37 (s, 3H), 8.07 (d, J = 15.0 Hz, 1H), 6.86 (d, J = 11.6 Hz, 1H), 7.12 (d, J = 9.0 Hz, 2H), 7.16 (d, J = 9.0 Hz, 2H), 7.25 (dd, J = 11.6, 15.0 Hz, 1H), 7.44-7.52 (m, 2H), 7.56 (td, J = 0.8, 7.6 Hz, 1H), 7.71 (dt, J = 1.5, 7.6 Hz, 1H).

Reference example 61: (2E,4Z)-5-(Furan-2-yl)-5-(4-methylphenyl)-2,4-pentadienoic acid (Compound bi)

Compound bi was synthesized in a similar manner to Reference example 18 using Compound bf and 2-furylboronic acid.
$^1$H NMR ($EDCl_3$, δ ppm): 2.39 (s, 3H), 6.03 (d, J = 15.2 Hz, 1H), 6.40 (d, J = 11.8 Hz, 1H), 6.41 (d, J = 3.4 Hz, 1H), 6.49 (dd, J = 1.8, 3.4 Hz, 1H), 7.19 (d, J = 8.2 Hz, 2H), 7.29 (d, J = 8.2 Hz, 2H), 7.60 (d, J = 1.8 Hz, 1H), 8.25 (dd, J = 11.8, 15.2 Hz, 1H).

Reference example 62: (E)-5,5-Bis[4-(trifluoromethyl)phenyl]-4-methyl-2,4-pentadienoic acid (Compound bj)

Step 1

4-(Trifluoromethyl)phenylboronic acid (2.17 g, 11.4 mmol), potassium phosphate (3.63 g, 17.1 mmol), bis (triphenylphosphine)palladium dichloride (400 mg, 0.570 mmol), and water (0.308 mL, 17.1 mmol) were suspended in toluene (20 mL), and 4-(trifluoromethyl)benzoyl chloride (1.69 mL, 11.4 mmol) was added thereto. Then, the temperature of the mixture was raised to 110˚C, and the mixture was stirred for 1 hour. A saturated sodium hydrogen carbonate solution was added to the reaction mixture, and the mixture was extracted with ethyl acetate. The organic layer was washed with saturated brine and dried over anhydrous magnesium sulfate. Then, the solvent was evaporated under reduced pressure, and the residue was purified by silica gel column chromatography (hexane/ethyl acetate = 9/1) to give 4,4'-bis(trifluoromethyl)benzophenone (2.95 g, 81%) as a colorless crystal.
$^1$H NMR ($CDCl_3$, δ ppm): 7.79 (d, J = 8.3 Hz, 4H), 7.91 (d, J = 8.3 Hz, 4H).

Step 2

Potassium tert-butoxide (752 mg, 6.70 mmol) was dissolved in THF (8 mL), and triethyl 2-phosphonopropionate (1.44 mL, 6.70 mmol) was added thereto, and then, the mixture was stirred at room temperature for 5 minutes. Further, a solution obtained by dissolving 4,4'-bis(trifluoromethyl)benzophenone (426 mg, 1.34 mmol) obtained in Step 1 in THF (7 mL) was added thereto, and the mixture was stirred at 60˚C for 2 hours. After the reaction mixture was cooled, a

saturated aqueous ammonium chloride solution was added to the reaction mixture, and then, the mixture was extracted with ethyl acetate. The organic layer was washed with saturated brine and dried over anhydrous magnesium sulfate. Then, the solvent was evaporated under reduced pressure, and the residue was purified by silica gel column chromatography (hexane/ethyl acetate = 19/1) to give ethyl 3,3-bis[4-(trifluoromethyl)phenyl]-2-methylacrylate (357 mg, 66%) as a colorless crystal.
$^1$H NMR ($CDCl_3$, δ ppm): 0.91 (t, J = 7.2 Hz, 3H), 2.05 (s, 3H), 3.97 (q, J = 7.2 Hz, 2H), 7.24 (d, J = 8.1 Hz, 2H), 7.29 (d, J = 8.1 Hz, 2H), 7.55 (d, J = 8.1 Hz, 2H), 7.62 (d, J = 8.1 Hz, 2H).

Step 3

Ethyl 3,3-bis[4-(trifluoromethyl)phenyl]-2-methylacrylate (410 mg, 1.02 mmol) obtained in Step 2 was dissolved in dichloromethane (5 mL), and a 1.01 mol/L diisobutylaluminum hydride-toluene solution (2.20 mL, 2.18 mmol) was added thereto at -78°C. Then, the temperature of the mixture was raised to 0°C, and the mixture was stirred for 1 hour. Methanol was added to the reaction mixture, and the mixture was stirred for an additional 30 minutes. To the reaction mixture, a saturated aqueous (+)-sodium potassium tartrate tetrahydrate solution was added, and the mixture was extracted with ethyl acetate. The organic layer was washed with saturated brine and dried over anhydrous magnesium sulfate. Then, the solvent was evaporated under reduced pressure, and the residue (372 mg) was used in the subsequent reaction as it is. The residue (372 mg) and manganese dioxide (440 mg, 5.06 mmol) were suspended in dichloromethane (5 mL), and the mixture was stirred at room temperature for 24 hours. The reaction mixture was filtered using Celite, and the solvent was evaporated under reduced pressure. The residue was purified by silica gel column chromatography (hexane/ethyl acetate = 9/1) to give 3,3-bis[4-(trifluoromethyl)phenyl]-2- methylpropenal (348 mg, 95%) as a pale yellow oily substance.
$^1$H NMR ($CDCl_3$, δ ppm): 1.98 (s, 3H), 7.31 (d, J = 8.3 Hz, pH), 7.66 (d, J = 8.3 Hz, 2H), 7.67 (d, J = 8.3 Hz, 2H), 9.60 (s, 1H).

Step 4

Triethyl phosphonoacetate (0.970 mL, 4.89 mmol) was dissolved in THF (10 mL), and potassium tert-butoxide (540 mg, 4.81 mmol) was added thereto at 0°C, and then, the mixture was stirred for 5 minutes. Further, a solution obtained by dissolving 3,3-bis[4-(trifluoromethyl)phenyl]-2- methylpropenal (347 mg, 0.969 mmol) obtained in Step 3 in THF (5 mL) was added thereto, and the temperature of the mixture was raised to 60°C, and the mixture was stirred for 4 hours. After the reaction mixture was cooled, a saturated aqueous ammonium chloride solution was added to the reaction mixture, and then, the mixture was extracted with ethyl acetate. The organic layer was washed with saturated brine and dried over anhydrous magnesium sulfate. Then, the solvent was evaporated under reduced pressure, and the residue was purified by silica gel column chromatography (hexane/ethyl acetate = 9/1) to give ethyl (E)-5,5-bis [4-(trifluoromethyl)phenyl]-4-methyl-2,4- pentadienoate (403 mg, 97%) as a colorless oily substance.
$^1$H NMR ($CDCl_3$, δ ppm): 1.27 (t, J = 7.1 Hz, 3H), 1.99 (s, 3H), 4.19 (q, J = 7.1 Hz, 2H), 6.11 (d, J = 15.7 Hz, 1H), 7.22 (d, J = 8.3 Hz, 4H), 7.50 (d, J = 15.7 Hz, 1H), 7.60 (d, J = 8.3 Hz, 2H), 7.61 (d, J = 8.3 Hz, 2H).

Step 5

Ethyl (E)-5,5-bis[4-(trifluoromethyl)phenyl]-4-methyl- 2,4-pentadienoate (402 mg, 0.938 mmol) obtained in Step 4 was dissolved in methanol (4 mL), and a 2 mol/L aqueous sodium hydroxide solution (0.940 mL, 1.88 mmol) was added thereto, and then, the mixture was stirred at 60°C for 1 hour. After the reaction mixture was cooled, the solvent was evaporated under reduced pressure. To the residue, water and 1 mol/L hydrochloric acid were added, and the mixture was extracted with chloroform. The organic layer was washed with saturated brine and dried over anhydrous magnesium sulfate. Then, the solvent was evaporated under reduced pressure, and the residue was purified by silica gel column chromatography (chloroform/methanol = 9/1) to give Compound bj (241 mg, 64%) as a colorless crystal.
$^1$H NMR (DMSO-$d_6$, δ ppm): 1.95 (s, 3H), 6.11 (d, J = 15.6 Hz, 1H), 7.24 (d, J = 15.6 Hz, 1H), 7.38 (d, J = 8.0 Hz, 2H), 7.43 (d, J = 8.0 Hz, 2H), 7.77 (d, J = 8.0 Hz, 2H), 7.79 (d, J = 8.0 Hz, 2H).

Reference example 63: (E)-5,5-Bis[4-(trifluoromethyl)phenyl]-3-methyl-2,4-pentadienoic acid (Compound bk)

Step 1

Potassium tert-butoxide (546 mg, 4.88 mmol) was suspended in THF (6 mL), and a solution of diethyl(2-oxopropyl) phosphonate (900 mg, 4.88 mmol) in THF (3 mL) was added thereto, and then, the mixture was stirred at 0°C for 15 minutes. Further, a solution obtained by dissolving 4,4'-bis(trifluoromethyl) benzophenone (300 mg, 0.945 mmol) in THF (4 mL) was added thereto, and the mixture was stirred at room temperature for 5 hours. After the reaction

mixture was cooled, a saturated aqueous ammonium chloride solution was added to the reaction mixture, and then, the mixture was extracted with hexane. The organic layer was washed with saturated brine and dried over anhydrous magnesium sulfate. Then, the solvent was evaporated under reduced pressure to give 4,4-bis[4-(trifluoromethyl)phenyl]-3-buten-2-one (274 mg, 81%) as a colorless oily substance.
$^1$H NMR ($CDCl_3$, δ ppm): 2.08 (s, 3H), 6.70 (s, 1H), 7.33 (d, J = 8.1 Hz, 2H), 7.37 (d, J = 8.1 Hz, 2H), 7.61 (d, J = 8.1 Hz, 2H), 7.69 (d, J = 8.1 Hz, 2H).

Step 2

Potassium tert-butoxide (428 mg, 3.83 mmol) was suspended in THF (6 mL), and a solution of triethyl phosphonoacetate (0.758 mL, 3.83 mmol) in THF (4 mL) was added thereto, and then, the mixture was stirred at 0˚C for 15 minutes. Further, a solution obtained by dissolving 4,4-bis[4-(trifluoromethyl) phenyl]-3-buten-2-one (274 mg, 0.765 mmol) obtained in Step 1 in THF (4 mL) was added thereto, and the temperature of the mixture was raised to 50˚C, and the mixture was stirred for 6 hours. After the reaction mixture was cooled, a saturated aqueous ammonium chloride solution was added to the reaction mixture, and then, the mixture was extracted with ethyl acetate. The organic layer was washed with saturated brine and dried over anhydrous magnesium sulfate. Then, the solvent was evaporated under reduced pressure, and the residue was purified by silica gel column chromatography (hexane/ethyl acetate = 10/1) to give ethyl (E)-5,5-bis[4-(trifluoromethyl)phenyl]-3-methyl-2,4- pentadienoate (319 mg, 73%) as a colorless oily substance.
$^1$H NMR ($CDCl_3$, δ ppm): 1.27 (t, J = 7.1 Hz, 3H), 2.01 (s, 3H), 4.20 (q, J = 7.1 Hz, 2H), 6.18 (s, 1H), 6.84 (s, 1H), 7.32 (d, J = 8.2 Hz, 2H), 7.36 (d, J = 8.2 Hz, 2H), 7.60 (d, J = 8.2 Hz, 2H), 7.67 (d, J = 8.2 Hz, 2H).

Step 3

Ethyl (E)-5,5-bis[4-(trifluoromethyl)phenyl]-3-methyl -2,4-pentadienoate (319 mg, 0.745 mmol) obtained in Step 2 and lithium hydroxide monohydrate (94 mg, 2.24 mmol) were dissolved in THF (3 mL), methanol (1 mL) and water (1 mL), and the mixture was stirred at room temperature for 5 hours. After the reaction mixture was concentrated, water was added to the residue, and then, the pH of the mixture was adjusted to 4 with 2 mol/L hydrochloric acid. The precipitated crystal was filtered and dried to give Compound bk (299 mg, quantitative).
$^1$H NMR (DMSO-$d_6$, δ ppm): 2.03 (s, 3H), 6.19 (s, 1H), 6.71 (s, 1H), 7.33 (d, J = 8.0 Hz, 2H), 7.39 (d, J = 8.0 Hz, 2H), 7.60 (d, J = 8.0 Hz, 2H), 7.72 (d, J = 8.0 Hz, 2H).

Reference example 64: (E)-5,5-Bis[4-(trifluoromethyl)phenyl]-2-methyl-2,4-pentadienoic acid (Compound bl)

Step 1

Potassium tert-butoxide (546 mg, 4.88 mmol) was suspended in THF (6 mL), and a solution of triethyl phosphonoacetate (0.966 mL, 4.88 mmol) in THF (4 mL) was added thereto, and then, the mixture was stirred at 0˚C for 15 minutes. Further, a solution obtained by dissolving 4,4'-bis(trifluoromethyl) benzophenone (300 mg, 0.945 mmol) in THF (3 mL) was added thereto, and the mixture was stirred at room temperature for 3 hours. After the reaction mixture was cooled, a saturated aqueous ammonium chloride solution was added to the reaction mixture, and then, the mixture was extracted with hexane. The organic layer was washed with saturated brine and dried over anhydrous magnesium sulfate. Then, the solvent was evaporated under reduced pressure to give ethyl 3,3-bis[4-(trifluoromethyl) phenyl]acrylate (323 mg, 88%) as a colorless oily substance.
$^1$H NMR ($EDCl_3$, δ ppm): 1.13 (t, J = 7.2 Hz, 3H), 4.08 (q, J = 7.2 Hz, 2H), 6.45 (s, 1H), 7.33 (d, J = 8.0 Hz, 2H), 7.38 (d, J = 8.0 Hz, 2H), 7.60 (d, J = 8.0 Hz, 2H), 7.67 (d, J = 8.0 Hz, 2H).

Step 2

Ethyl 3,3-bis[4-(trifluoromethyl)phenyl] acrylate (323 mg, 0.832 mmol) obtained in Step 1 was dissolved in dichloromethane (4.5 mL), and a 1.01 mol/L diisobutylaluminum hydride-toluene solution (1.81 mL, 1.83 mmol) was added thereto at -78˚C. Then, the temperature of the mixture was gradually raised to 0˚C, and the mixture was stirred for 1.5 hours. Acetic acid was added to the reaction mixture, and the mixture was stirred for an additional 15 minutes. To the reaction mixture, a saturated aqueous (+)-sodium potassium tartrate tetrahydrate solution was added, and the mixture was extracted with ethyl acetate. The organic layer was washed with a saturated sodium hydrogen carbonate solution and saturated brine and dried over anhydrous magnesium sulfate. Then, the solvent was evaporated under reduced pressure, and the residue was used in the subsequent reaction as it is. The residue was dissolved in dichloromethane (8 mL), and manganese dioxide (723 mg, 8.32 mmol) was added thereto, and then, the mixture was stirred at room temperature for 24 hours. The reaction mixture was filtered using Celite, and the solvent was evaporated under

reduced pressure. The residue (148 mg) was used in the subsequent reaction as it is. Potassium tert-butoxide (241 mg, 2.15 mmol) was dissolved in THF (4 mL), and a solution of triethyl 2-phosphonopropionate (0.46 mL, 2.15 mmol) in THF (2 mL) was added thereto, and then, the mixture was stirred at 0˚C for 15 minutes. Further, a solution obtained by dissolving the above residue (148 mg, 0.430 mmol) in THF (3 mL) was added thereto, and the mixture was stirred at room temperature for 8 hours. To the reaction mixture, a saturated aqueous ammonium chloride solution was added, and the mixture was extracted with ethyl acetate. The organic layer was washed with saturated brine and dried over anhydrous magnesium sulfate. Then, the solvent was evaporated under reduced pressure, and the residue was purified by silica gel column chromatography (hexane/ethyl acetate = 20/1) to give ethyl (E)-5,5-bis[4-(trifluoromethyl)phenyl]-2-methyl-2,4-pentadienoate (111 mg, 31%) as a colorless crystal.
$^1$H NMR ($CDCl_3$, δ ppm): 1.11 (t, J = 7.2 Hz, 3H), 2.08 (s, 1H), 4.08 (q, J = 7.2 Hz, 2H), 6.91 (d, J = 11.5 Hz, 1H), 7.38 (d, J = 8.0 Hz, 2H), 7.41 (d, J = 11.5 Hz, 1H), 7.60 (d, J = 8.0 Hz, 2H), 7.67 (d, J = 8.0 Hz, 2H).

Step 3

Ethyl (E)-5,5-bis[4-(trifluoromethyl)phenyl]-2-methyl-2,4-pentadienoate (111 mg, 0.259 mmol) obtained in Step 2 and lithium hydroxide monohydrate (33 mg, 0.778 mmol) were dissolved in THF (2 mL), methanol (1 mL), and water (1 mL), and the mixture was stirred at room temperature for 5 hours. After the reaction mixture was concentrated, water was added to the residue, and then, the pH of the mixture was adjusted to 4 with 2 mol/L hydrochloric acid. The precipitated crystal was filtered and dried to give Compound bl (94 mg, 91%).
$^1$H NMR ($EDCl_3$, δ pom): 2.11 (s, 1H), 6.90 (d, J = 11.5 Hz, 1H), 6.35 (d, J = 11.5 Hz, 1H), 7.38 (d, J = 8.0 Hz, 2H), 7.43 (d, J = 8.0 Hz, 2H), 7.60 (d, J = 8.0 Hz, 2H), 7.67 (d, J = 8.0 Hz, 2H).

Reference example 65: (2E,4E)-5-(4-Chlorophenyl)-5-(4-ethoxyphenyl)-2,4-pentadienoic acid (Compound ca)

Compound ca was synthesized in a similar manner to Reference example 18 using Compound bb and 4-ethoxyphenylboronic acid.
$^1$H NMR (DMSO-$d_6$, δ pom): 1.36 (t, J = 7.0 Hz, 3H), 4.09 (q, J = 7.0 Hz, 2H), 6.12 (d, J = 14.6 Hz, 1H), 6.97-7.18 (m, 6H), 7.31 (d, J = 8.8 Hz, 2H), 7.43 (d, J = 8.8 Hz, 2H).

Reference example 66: (2E,4Z)-5- (4-Isopropoxyphehyl) -5-(4-methylphexayl3-2,4-pentadienoic acid (Compound cb)

Compound cb was synthesized in a similar manner to Reference example 18 using Compound bf and 4-isopropoxyphenylboronic acid.
$^1$H NMR (DMSO- $d_6$, 5 ppm): 1.31 (d, J = 6.1 Hz, 6H), 2.31 (s, 3H), 4.68 (sept, J = 6.1 Hz, 1H), 6.07 (d, J = 14.8 Hz, 1H), 6.87-7.22 (m, 10H).

Reference example 67: (2E,4E)-5-(4-Chlorophenyl)-5-(4-isopropoxyphenyl)-2,4-pentadienoic acid (Compound cc)

Compound cc was synthesized in a similar manner to Reference example 18 using Compound bb and 4-isopropoxyphenylboronic acid.
$^1$H NMR (DMSO-$d_6$, δ ppm): 1.31 (d, J = 6.1 Hz, 6H), 4.68 (set, J = 6.1 Hz, 1H), 6.12 (d, J = 14.9 Hz, 1H), 6.96-7.09 (m, 5H), 7.14 (dd, J = 11.6, 14.9 Hz, 1H), 7.31 (d, J = 8.7 Hz, 2H), 7.43 (d, J = 8.7 Hz, 2H).

Reference example 68: (2E,4Z)-5-(4-Ethoxyphenyl)-5-(4-methylphenyl)-2,4-pentadienoic acid (Compound cd)

Compound cd was synthesized in a similar manner to Reference example 18 using Compound bf and 4-ethoxyphenylboronic acid.
$^1$H NMR (DMSO-$d_6$, δ ppm) : 1.36 (t, J = 6.9 Hz, 3H), 2.31 (s, 3H), 4.09 (q, J = 6.9 Hz, 2H), 6.07 (d, J = 15.0 Hz, 1H), 6.93 (d, J = 11.7 Hz, 1H), 7.00-7.21 (m, 9H).

Reference example 69: (2E,4E)-5-[4-(Hydroxymethyl)phenyl]-5-[4-(trifluoromethyl)phenyl]-2,4-pentadienoic acid (Compound ce)

Compound ce was synthesized in a similar manner to Reference example 18 using Compound q and 4-(hydroxymethyl)phenylboronic acid.
$^1$H NMR (DMSO-$d_6$, δ ppm) : 4.55 (s, 2H), 5.27 (br s, 1H), 6.20 (d, J = 14.8 Hz, 1H), 7.02-7.21 (m, 4H), 7.40-7.53 (m, 4H), 7.74 (d, J = 8.4 Hz, 2H).

Reference example 70: (2E,4Z)-5-[3-(Hydroxymethyl)phenyl]-5- [4- (trifluoromethyl)phenyl] -2,4-pentadienamide (Compound cf)

Compound cf was synthesized in a similar manner to Reference example 18 using Compound q and 3-(hydroxymethyl)phenylboronic acid.
$^1$H NMR (DMSO-$d_6$, δ ppm): 4.58 (d, J = 5.9 Hz, 2H), 5.29 (t, J = 5.9 Hz, 1H), 6.20 (d, J = 13.8 Hz, 1H), 7.05-7.20 (m, 4H), 7.44-7.53 (m, 4H), 7.73 (d, J = 8.4 Hz, 2H).

Reference example 71: (2E,4Z)-5-(4-tert-Butoxyphenyl)-5-[4-(trifluoromethyl)phenyl]-2,4-pentadienoic acid (Compound cg)

Compound cg was synthesized in a similar manner to Reference example 18 using Compound q and 4-tert-butoxyphenylboronic acid.
$^1$H NMR (DMSO-$d_6$, δ ppm): 1.37 (s, 9H), 6.19 (d, J = 12.7 Hz, 1H), 7.08-7.19 (m, 6H), 7.51 (d, J = 8.3 Hz, 2H), 7.74 (d, J = 8.3 Hz, 2H).

Reference example 72: Ethyl (2E,4Z)-5-(4-hydroxyphenyl)-5-[4-(trifluoromethyl)phenyl]-2,4-pentadienoate (Compound ch)

Compound ch was synthesized in a similar manner to step 1 of Reference example 18 using Compound q and 4-hydroxyphenylboronic acid.
$^1$H NMR ($CDCl_3$, δ ppm): 1.29 (t, J = 7.1 Hz, 3H), 4.21 (q, J = 7.1 Hz, 2H), 5.70 (br s, 1H), 6.10 (d, J = 15.4 Hz, 1H), 6.74 (d, J = 11.5 Hz, 1H), 6.87 (d, J = 8.4 Hz, 2H), 7.07 (d, J = 8.4 Hz, 2H), 7.41 (d, J = 8.4 Hz, 2H), 7.48 (dd, J = 11.5, 15.4 Hz, 1H), 7.57 (d, J = 8.4 Hz, 2H).

Reference example 73: (2E,4Z)-5-(4-Cyclobutoxyphenyl)-5-[4-(trifluo-romethyl)phenyl]-2,4-pentadienoic acid (Compound ci)

Step 1

Compound ch (200 mg, 0.552 mmol), cyclobutanol (87.5 mg, 1.21 mmol), triphenylphosphine (304 mg, 1.16 mmol), and diethyl azodicarboxylate (0.504 mL, 1.11 mmol) were dissolved in toluene (4 mL), and the mixture was stirred at room temperature for 48 hours. Then, the solvent was evaporated under reduced pressure, and the residue was purified by silica gel column chromatography (hexane/ethyl acetate = 5/1) to give ethyl (2E,4Z)-5-(4-cyclobutoxyphenyl)-5-[4-(trifluoromethyl)phenyl]-2,4-pentadienoate (151 mg, 66%).
$^1$H NMR ($EDCl_3$, δ ppm): 1.28 (t, J = 7.2 Hz, 3H), 1.63-1.95 (m, 2H), 2.19-2.26 (m, 2H), 2.47-2.51 (m, 2H), 4.19 (q, J = 7.2 Hz, 2H), 4.69 (quint, J = 7.0 Hz, 1H), 6.08 (d, J = 15.1 Hz, 1H), 6.72 (d, J = 11.6 Hz, 1H), 6.85 (d, J = 8.5 Hz, 2H), 7.09 (d, J = 8.5 Hz, 2H), 7.40 (d, J = 8.6 Hz, 2H), 7.45 (dd, J = 11.6, 15.1 Hz, 1H), 7.56 (d, J = 8.6 Hz, 2H) .

Step 2

Compound ci was obtained from ethyl (2E,4Z)-5-(4-cyclobutoxyphenyl)-5-[4-(trifluoromethyl)phenyl]-2,4-pentadienoate in a similar manner to Step 2 of Reference example 18.
$^1$H NMR (DMSO-$d_6$, δ ppm): 1.61-1.86 (m, 2H), 2.06-2.13 (m, 2H), 2.42-2.49 (m, 2H), 4.76 (quint, J = 7.2 Hz, 1H), 6.18 (d, J = 14.0 Hz, 1H), 6.96 (d, J = 8.6 Hz, 2H), 7.05-7.20 (m, 4H), 7.50 (d, J = 8.4 Hz, 2H), 7.73 (d, J = 8.4 Hz, 2H).

Reference example 74: (2E,4Z)-5-[4-(1-Methylcyclopropylmethoxy)phenyl]-5-[4-(trifluoromethyl)phenyl]-2,4-pentadienoic acid (Compound cj)

Compound cj was obtained from Compound ch in a similar manner to Reference example 73 using 1-methylcyclopropanemethanol.
$^1$H NMR (DMSO-$d_6$, δ ppm): 0.42 (t, J = 4.0 Hz, 2H), 0.55 (t, J = 4.0 Hz, 2H), 1.21 (s, 3H), 3.82 (s, 2H), 6.18 (d, J = 14.0 Hz, 1H), 7.02-7.22 (m, 6H), 7.51 (d, J = 8.3 Hz, 2H), 7.73 (d, J = 8.3 Hz, 2H), 12.30 (br s, 1H).

Reference example 75: (2E,4Z)-5-{4-[(3-Ethyloxetan-3-yl)methoxy]phenyl}-5-[4-(trifluoromethyl)phenyl]-2,4-pentadienoic acid (Compounds ck)

Compound ck was obtained from Compound ch in a similar manner to Reference example 73 using 3-ethyl-

3-oxetanemethanol.
$^1$H NMR (DMSO-$d_6$, δ ppm) : 0.93 (t, J = 7.4 Hz, 3H), 1.82 (q, J = 7.4 Hz, 2H), 4.17 (s, 2H), 4.36 (d, J = 5.8 Hz, 2H), 4.48 (d, J = 5.8 Hz, 2H), 6.19 (d, J = 14.0 Hz, 1H), 7.06-7.22 (m, 6H), 7.51 (d, J = 8.4 Hz, 2H), 7.73 (d, J = 8.4 Hz, 2H), 12.31 (br s, 1H).

Reference example 76: Ethyl (2E,4Z)-5-(3-hydroxyphenyl)-5-[4-(trifluoromethyl)phenyl]-2,4-pentadienoate (Compound cl)

Compound cl was synthesized in a similar manner to step 1 of Reference example 18 using Compound q and 3-hydroxyphenylboronic acid.
$^1$H NMR (DMSO-$d_6$, δ ppm) : 1.27 (t, J = 7.3 Hz, 3H), 4.19 (q, J = 7.3 Hz, 2H), 5.42 (br s, 1H), 6.10 (d, J = 15.4 Hz, 1H), 6.66-6.67 (m, 1H), 6.74-6.81 (m, 2H), 6.88 (dd, J = 1.9, 8.1 Hz, 1H), 7.28-7.31 (m, 1H), 7.38-7.43 (m, 3H), 7.56 (d, J = 8.4 Hz, 2H).

Reference example 77: (2E,4Z)-5-[3-(Cyclopropylmethoxy)phenyl]-5-[4-(trifluoromethyl)phenyl]-2,4-pentadienoic acid (Compound cm)

Compound cm was obtained from Compound cl in a similar manner to Reference example 73 using cyclopropanemethanol.
$^1$H NMR (DMSO-$d_6$, δ ppm): 0.28-0.34 (m, 2H), 0.52-0.59 (m, 2H), 1.18-1.23 (m, 1H), 3.82 (d, J = 7.0 Hz, 2H), 6.19 (d, J = 14.3 Hz, 1H), 6.72-6.76 (m, 2H), 7.01-7.20 (m, 3H), 7.41 (t, J = 7.8 Hz, 1H), 7.52 (d, J = 8.3 Hz, 2H), 7.73 (d, J = 8.3 Hz, 2H), 12.36 (br s, 1H).

Reference example 78: (2E,4Z)-5-[3-(1-Methylcyclopropylmethoxy)phenyl]-5-[4-(trifluoromethyl)phenyl]-2,4-pentadienoic acid (Compound cn)

Compound cn was obtained from Compound cl in a similar manner to Reference example 73 using 1-methylcyclopropanemethanol.
$^1$H NMR (DMSO-$d_6$, δ ppm): 0.35-0.38 (m, 2H), 0.49-0.52 (m, 2H), 1.16 (s, 3H), 3.76 (s, 2H), 6.19 (d, J = 14.3 Hz, 1H), 6.71-6.72 (m, 1H), 6.76 (d, J = 7.6 Hz, 1H), 7.01-7.21 (m, 3H), 7.41 (t, J = 8.1 Hz, 1H), 7.52 (d, J = 8.1 Hz, 2H), 7.73 (d, J = 8.1 Hz, 2H), 12.35 (br s, 1H).

Reference example 79: (2E,4Z)-5-[3-(2-Cyclopropylethoxy)phenyl]-5-[4-(trifluoromethyl)phenyl]-2,4-pentadienoic acid (Compound co)

Compound co was obtained from Compound cl in a similar manner to Reference example 73 using 2-cyclopropylethanol.
$^1$H NMR (DMSO-$d_6$, δ ppm) : 0.08-0.13 (m, 2H), 0.40-0.44 (m, 2H), 0.78-0.85 (m, 1H), 1.62 (q, J = 6.6 Hz, 2H), 4.04 (t, J = 6.6 Hz, 2H), 6.20 (d, J = 14.3 Hz, 1H), 6.73-6.78 (m, 2H), 7.04-7.21 (m, 3H), 7.43 (t, J = 8.1 Hz, 1H), 7.53 (d, J = 8.1 Hz, 2H), 7.74 (d, J = 8.1 Hz, 2H).

Reference example 80: (2E,4Z)-5-{3-[(3-Ethyloxetan-3-yl)methoxy]phenyl}-5-[4-(trifluoromethyl)phenyl]-2,4-pentadienoic acid (Compound cp)

Compound cp was obtained from Compound cl in a similar manner to Reference example 73 using 3-ethyl-3-oxetanemethanol.
$^1$H NMR (DMSO-$d_6$, δ ppm): 0.88 (t, J = 7.4 Hz, 3H), 1.77 (q, J = 7.4 Hz, 2H), 4.11 (s, 2H), 4.32 (d, J = 6.0 Hz, 2H), 4.44 (d, J = 6.0 Hz, 2H), 6.20 (d, J = 14.7 Hz, 1H), 6.80-6.82 (m, 2H), 7.04-7.21 (m, 3H), 7.45 (t, J = 7.8 Hz, 1H), 7.53 (d, J = 8.1 Hz, 2H), 7.74 (d, J = 8.1 Hz, 2H).

Reference example 81: (2E,4Z)-5-{3-[(3-Methyloxetan-3-yl)methoxy]phenyl}-5-[4-(trifluoromethyl)phenyl]-2,4-pentadienoic acid (Compound cq)

Compound cq was obtained from Compound cl in a similar manner to Reference example 73 using 3-methyl-3oxetanemethanol.
$^1$H NMR (DMSO-$d_6$, δ ppm): 1.35 (s, 3H), 4.06 (s, 2H), 4.28 (d, J = 5.7 Hz, 2H), 4.48 (d, J = 5.7 Hz, 2H), 6.20 (d, J = 14.6 Hz, 1H), 6.77-6.82 (m, 2H), 7.02-7.21 (m, 3H), 7.45 (t, J = 7.8 Hz, 1H), 7.53 (d, J = 8.4 Hz, 2H), 7.73 (d, J = 8.4 Hz, 2H).

Reference example 82: (2E,4Z)-5-{4-[(3-Methyloxetan-3-yl)methoxy]phenyl}-5-[4-(trifluoromethyl)phenyl]-2,4-pentadienoic acid (Compound cr)

Compound cr was obtained from Compound ch in a similar manner to Reference example 73 using 3-methyl-3-oxetanemethanol.
$^1$H NMR (DMSO-$d_6$, δ ppm): 1.40 (s, 3H), 4.13 (s, 2H), 4.33 (d, J = 5.8 Hz, 2H), 4.53 (d, J = 5.8 Hz, 2H), 6.19 (d, J = 13.5 Hz, 1H), 7.07-7.21 (m, 6H), 7.52 (d, J = 8.4 Hz, 2H), 7.73 (d, J = 8.4 Hz, 2H) .

Reference example 83: (2E,4Z)-5-[4-(Tetrahydropyran-4-ylmethoxy)phenyl]-5-[4-(trifluoromethyl)phenyl]-2,4-pentadienoic acid (Compound cs)

Compound cs was obtained from Compound ch in a similar manner to Reference example 73 using (tetrahydro-2H-pyran-4-yl)methanol.
$^1$H NMR (DMSO-$d_6$, δ ppm): 1.29-1.44 (m, 2H), 1.69-1.74 (m, 2H), 1.96-2.10 (m, 1H), 3.33-3.39 (m, 2H), 3.87-3.92 (m, 4H), 6.18 (d, J = 14.0 Hz, 1H), 7.04-7.21 (m, 6H), 7.51 (d, J = 8.5 Hz, 2H), 7.73 (d, J = 8.5 Hz, 2H).

Reference example 84: (2E,4Z)-5-[3-(Tetrahydropyran-4-ylmethoxy)phenyl]-5-[4-(trifluoromethyl)phenyl]-2,4-pentadienoic acid (Compound ct)

Compound ct was obtained from Compound cl in a similar manner to Reference example 73 using (tetrahydro-2H-pyran-4-yl)methanol.
$^1$H NMR (DMSO-$d_6$, δ ppm): 124-1.39 (m, 2H), 1.64-1.68 (m, 2H), 1.90-2.04 (m, 1H), 3.27-3.35 (m, 2H), 3.83-3.88 (m, 4H), 6.20 (d, J = 14.0 Hz, 1H), 6.73-6.78 (m, 2H), 7.03-7.20 (m, 3H), 7.42 (t, J = 8.0 Hz, 1H), 7.52 (d, J = 8.3 Hz, 2H), 7.73 (d, J = 8.3 Hz, 2H).

Reference example 85: (2E,4Z)-5-(4-Isopropoxyphenyl)-5-[4-(trifluoromethyl)phenyl]-2,4-pentadienoic acid (Compound cu)

Compound cu was synthesized in a similar manner to Reference example 18 using Compound q and 4-isopropoxyphenylboronic acid.
$^1$H NMR (DMSO-$d_6$, δ ppm): 1.32 (d, J = 5.9 Hz, 6H), 4.69 (sept, J = 5.9 Hz, 1H), 6.18 (d, J = 14.6 Hz, 1H), 7.01-7.22 (m, 6H), 7.51 (d, J = 8.3 Hz, 2H), 7.73 (d, J = 8.3 Hz, 2H).

Reference example 86: (2E,4Z)-5-(1-Methyl-1H-pyrazol-4-yl)-5-[4-(trifluoromethyl)phenyl]-2,4-pentadienamide (Compound cv)

Compound cv was synthesized in a similar manner to Reference example 18 using Compound q and 1-methyl-4-(4,4,5,5-tetramethyl-1,3,2-dioxaborolan-2-yl)-1H-pyrazole.
$^1$H NMR (DMSO-$d_6$, δ ppm): 3.91 (s, 3H), 6.17 (d, J = 15.4 Hz, 1H), 6.87 (d, J = 11.6 Hz, 1H), 7.41-7.51 (m, 2H), 7.60 (d, J = 8.3 Hz, 2H), 7.74 (d, J = 8.3 Hz, 2H), 7.83 (s, 1H), 12.30 (br s, 1H).

Reference example 87: (2E,4E)-5-(4-Ethylphenyl)-5-[4-(trifluoromethyl)phenyl]-2,4-pentadienoic acid (Compound cw)

Compound cw was synthesized in a similar manner to Reference example 18 using Compound q and 4-ethylphenylboronic acid.
$^1$H NMR (DMSO-$d_6$, δ ppm): 1.25 (t, J = 7.6 Hz, 3H), 2.70 (q, J = 7.6 Hz, 2H), 6.17-6.24 (m, 1H), 7.11-7.14 (m, 4H), 7.35 (d, J = 7.8 Hz, 2H), 7.51 (d, J = 8.5 Hz, 2H), 7.73 (d, J = 8.5 Hz, 2H), 12.35 (br s, 1H).

Reference example 88: (2E,4Z)-5-(4-Hydroxyphenyl)-5-[4-(trifluoromethyl)phenyl]-2,4-pentadienoic acid (Compound cx)

Compound cx was synthesized from Compound ch in a similar manner to step 2 of Reference example 18.
$^1$H NMR (DMSO-$d_6$, δ ppm): 6.16 (d, J = 15.0 Hz, 1H), 6.87 (d, J = 8.6 Hz, 2H), 6.99-7.04 (m, 3H), 7.19 (dd, J = 11.6, 15.0 Hz, 1H), 7.51 (d, J = 8.3 Hz, 2H), 7.72 (d, J = 8.3 Hz, 2H), 9.78 (br s, 1H), 12.27 (br s, 1H).

Reference example 89: (2E,4Z)-5-[4-(2-Cyclopropylethoxy)phenyl]-5-[4-(trifluoromethyl)phenyl]-2,4-pentadienoic acid (Compound cy)

Compound cy was synthesized from Compound ch in a similar manner to Reference example 73 using 2-cyclopropylethanol.
$^1$H NMR (DMSO-$d_6$, δ ppm): 0.12-0.18 (m, 2H), 0.43-0.49 (m, 2H), 0.80-0.92 (m, 1H), 1.67 (q, J = 6.6 Hz, 2H), 4.10 (t, J = 6.6 Hz, 2H), 6.19 (d, J = 14.4 Hz, 1H) 7.05-7.22 (m, 6H), 7.52 (d, J = 8.4 Hz, 2H), 7.73 (d, J = 8.4 Hz, 2H), 12.29 (br s, 1H) .

Reference example 90: (2E,4Z)-5-[4-(Cyclopropylmethoxy)phenyl]-5-[4-(trifluoromethyl)phenyl]-2,4-pentadienoic acid (Compound cz)

Compound cz was obtained from Compound ch in a similar manner to Reference example 73 using cyclopropanemethanol.
$^1$H NMR (DMSO-$d_6$, δ ppm): 0.33-0.38 (m, 2H), 0.56-0.63 (m, 2H), 1.23-1.27 (m, 1H), 3.89 (d, J = 7.2 Hz, 2H), 6.18 (d, J = 14.1 Hz, 1H), 7.02-7.16 (m, 6H), 7.51 (d, J = 8.1 Hz, 2H), 7.73 (d, J = 8.1 Hz, 2H), 12.30 (br s, 1H).

Reference example 91: (2E,4Z)-5-[4-(Cyanomethoxy)phenyl]-5-[4-(trifluoromethyl)phenyl]-2,4-pentadienoic acid (Compound da)

Ethyl (2E,4Z)-5-[4-(cyanomethoxy)phenyl]-5-[4-(trifluoromethyl)phenyl]-2,4-pentadienoate (300 mg, 0.747 mmol) synthesized in a similar manner to Example 117 from Compound ch and lithium hydroxide monohydrate (112 mg, 2.66 mmol) were dissolved in THF (9 mL), methanol (4 mL), and water (4 mL), and the mixture was stirred at room temperature for 5 hours. After the reaction mixture was concentrated, water (20 mL) and 1 mol/L hydrochloric acid (5 mL) were added to the residue, and then, the mixture was extracted three times with chloroform. The organic layer was washed with saturated brine and dried over anhydrous magnesium sulfate. Then, the solvent was evaporated under reduced pressure to give Compound da (304 mg, 100%)
$^1$H NMR (DMSO-$d_6$, δ ppm): 3.73 (s, 3H), 4.88 (s, 2H), 6.19 (d, J = 14.3 Hz, 1H), 7.02-7.21 (m, 5H), 7.51 (d, J = 8.5 Hz, 2H), 7.73 (d, J = 8.5 Hz, 2H), 12.36 (br s, 1H).

Reference example 92: Ethyl (2E,4Z)-5-(2-chloropyridin-4-yl)-5-[4-(trifluoromethyl)phenyl]-2,4-pentadienoate (Compound db)

Compound db was synthesized in a similar manner to step 1 of Reference example 18 using Compound q and 3-chloropyridylboronic acid.
$^1$H NMR ($CDCl_3$, δ ppm): 1.29 (t, J = 7.2 Hz, 3H), 4.21 (q, J = 7.2 Hz, 2H), 6.17 (dd, J = 0.8, 15.3 Hz, 1H), 6.91 (d, J = 11.6 Hz, 1H), 7.10 (dd, J = 1.1, 4.9 Hz, 1H), 7.16-7.22 (m, 2H), 7.36 (d, J = 8.1 Hz, 2H), 7.61 (d, J = 8.1 Hz, 2H), 8.50 (dd, J = 0.8, 4.9 Hz, 1H).

Reference example 93: (2E,4Z)-5--[2-(Piperidin-1-yl) pyridin-4-yl]-5-[4-(trifluoromethyl)phenyl]-2,4-pentadienoic acid (Compound dc)

Step 1

Compound db (200 mg, 0.524 mmol), N,N-diisopropylethylamine (0.200 mL, 1.15 mmol), and piperidine (0.250 mL, 2.53 mmol) were dissolved in DMF (2 mL), and the mixture was stirred at 110˚C for 2 hours. Water was added to the reaction mixture, and the mixture was extracted three times with ethyl acetate. The organic layer was washed with saturated brine and dried over anhydrous magnesium sulfate. Then, the solvent was evaporated under reduced pressure to give ethyl (2E,4Z)-5-[2-(piperidin-1-yl)pyridin-4-yl]-5-[4-(trifluoromethyl)phenyl]-2,4-pentadienoate (98.0 mg, 43%).
$^1$H NMR ($CDCl_3$, δ ppm) : 1.28 (t, J = 7.1 Hz, 3H), 1.65-1.66 (m, 6H), 3.53-3.55 (m, 4H), 4.19 (q, J = 7.1 Hz, 2H), 6.10 (dd, J = 0.7, 15.2 Hz, 1H), 6.38 (dd, J = 1.2, 5.0 Hz, 1H), 6.43 (s, 1H), 6.83 (d, J = 11.7 Hz, 1H), 7.33-7.42 (m, 1H), 7.43 (d, J = 8.1 Hz, 2H), 7.58 (d, J = 8.1 Hz, 2H), 8.23 (dd, J = 0.7, 5.0 Hz, 1H) .

Step 2

Compound dc was obtained from ethyl (2E,4Z)-5-[2-(piperidin-1-yl)pyridin-4-yl]-5-[4-(trifluoromethyl)phenyl]-2,4-pentadienoate in a similar manner to Step 2 of Reference example 18.
$^1$H NMR (DMSO-$d_6$, δ ppm): 1.54-1.58 (m, 6H), 3.52-3.56 (m, 4H), 6.22 (d, J = 15.0 Hz, 1H), 6.42 (d, J = 5.0 Hz, 1H), 6.67 (s, 1H), 7.06 (dd, J = 11.6, 15.0 Hz, 1H), 7.24 (d, J = 11.6 Hz, 1H), 7.56 (d, J = 8.3 Hz, 2H), 7.74 (d, J = 8.3 Hz,

2H), 8.18 (d, J = 5.0 Hz, 1H).

Reference example 94: (2E, 4Z) -5- (4-Methylthiophenyl) -5- [4-(trifluoromethyl)phenyl]-2,4-pentadienoic acid (Compound dd)

Compound dd was synthesized in a similar manner to Reference example 18 using Compound q and 4-methylthiophenylboronic acid.
$^1$H NMR (DMSO-$d_6$, δ ppm) : 2.54 (s, 3H), 6.16-6.25 (m, 1H), 7.11-7.16 (m, 4H), 7.38 (d, J = 8.4 Hz, 2H), 7.51 (d, J = 8.4 Hz, 2H), 7.73 (d, J = 8.4 Hz, 2H), 12.35 (br s, 1H). Reference example 95: (2E,4Z)-5-[2-(1-Pyrrolidinyl)pyridin-4-yl]-5-[4-(trifluoromethyl)phenyl]-2,4-pentadienoic acid (Compound de)
Compound de was obtained from Compound db in a similar manner to Reference example 93 using pyrrolidine.
$^1$H NMR (DMSO-$d_6$, δ ppm): 1.92-1.96 (m, 4H), 3.36-3.41 (m, 4H), 6.22 (d, J = 14.9 Hz, 1H), 6.26 (s, 1H), 6.37 (d, J = 5.1 Hz, 1H), 7.09 (dd, J = 11.6, 14.9 Hz, 1H), 7.24 (d, J = 11.6 Hz, 1H), 7.57 (d, J = 8.3 Hz, 2H), 7.74 (d, J = 8.3 Hz, 2H), 8.16 (d, J = 5.1 Hz, 1H), 12.40 (br s, 1H) .

Reference example 96: Ethyl (2E,4Z)-5-(2-hydroxyphenyl)-5-[4-(trifluoromethyl)phenyl]-2,4-pentadienoate (Compound df)

Compound df was synthesized in a similar manner to step 1 of Reference example 18 using Compound q and 2-hydroxyphenylboronic acid.
$^1$H NMR ($CDCl_3$, δ ppm): 1.26 (t, J = 7.2 Hz, 3H), 4.17 (q, J = 7.2 Hz, 2H), 4.78 (br s, 1H), 6.14 (d, J = 15.1 Hz, 1H), 6.95-7.04 (m, 4H), 7.22 (dd, J = 11.5, 15.1 Hz, 1H), 7.31-7.37 (m, 1H), 7.45 (d, J = 8.1 Hz, 2H), 7.58 (d, J = 8.1 Hz, 2H).

Reference example 97: (2E,4Z)-5-(2-Isopropoxyphenyl)-5-[4-(trifluoromethyl)phenyl]-2,4-pentadienoic acid (Compound dg)

Compound dg was obtained from Compound df in a similar manner to Reference example 73 using 2-propanol.
$^1$H NMR (DMSO-$d_6$, δ ppm): 0.86-0.94 (m, 6H), 4.48 (sept, J = 6.1 Hz, 1H), 6.15 (d, J = 14.6 Hz, 1H), 6.95-7.15 (m, 5H), 7.40-7.47 (m, 3H), 7.69 (d, J = 8.4 Hz, 2H).

Reference example 98: (2E,4Z)-5-(2-Ethoxyphenyl)-5-[4-(trifluoromethyl)phenyl]-2,4-pentadienoic acid (Compound dh)

Compound dh was obtained from Compound df in a similar manner to Reference example 73 using ethanol.
$^1$H NMR (DMSO-$d_6$, δ ppm) : 0.91 (t, J = 6.9 Hz, 3H), 3.89-3.91 (m, 2H), 6.16 (d, J = 14.7 Hz, 1H), 6.98-7.20 (m, 5H), 7.46-7.49 (m, 3H), 7.69 (d, J = 8.4 Hz, 2H), 12.30 (br s, 1H).

Reference example 99: (2E,4Z)-5-(2-Methoxyphenyl)-5-[4-(trifluoromethyl)phenyl]-2,4-pentadienoic acid (Compound di)

Compound di was obtained from Compound df in a similar manner to Reference example 73 using methanol.
$^1$H NMR (DMSO-$d_6$, δ ppm): 3.62 (s, 3H), 6.16 (d, J = 15.1 Hz, 1H), 6.91 (dd, J = 11.3, 15.1 Hz, 1H), 7.09-7.27 (m, 4H), 7.45-7.50 (m, 3H), 7.69 (d, J = 8.6 Hz, 2H), 12.30 (br s, 1H).

Reference example 100: (2E,4Z)-5-(2-Methoxypyrimidin-5-yl)-5-[4-(trifluoromethyl)phenyl]-2,4-pentadienoic acid (Compound dj)

Compound dj was synthesized in a similar manner to Reference example 18 using Compound q and 5-(2-methoxypyrimidinyl)boronic acid.
$^1$H NMR (DMSO-$d_6$, δ ppm): 4.01 (s, 3H), 6.29 (d, J = 14.8 Hz, 1H), 7.05 (dd, J = 11.6, 14.8 Hz, 1H), 7.32 (d, J = 11.6 Hz, 1H), 7.59 (d, J = 8.3 Hz, 2H), 7.76 (d, J = 8.3 Hz, 2H), 8.50 (s, 2H), 12.46 (br s, 1H).

Reference example 101: Ethyl (2E,4Z)-5-(6-chloropyridin-3-yl)-5-[4-(trifluoromethyl)phenyl]-2,4-pentadienoate (Compound dk)

Compound dk was synthesized in a similar manner to step 1 of Reference example 18 using Compound q and 4-chloro-3-pyridylboronic acid.
$^1$H NMR ($CDCl_3$, δ ppm): 1.29 (t, J = 7.2 Hz, 3H), 4.21 (q, J = 7.2 Hz, 2H), 6.17 (d, J = 15.1 Hz, 1H), 6.92 (d, J = 11.5 Hz, 1H), 7.25 (dd, J = 11.5, 15.1 Hz, 1H), 7.36-7.52 (m, 4H), 7.61 (d, J = 8.1 Hz, 2H), 8.26 (d, J = 2.4 Hz, 1H).

Reference example 102: (2E,4Z)-5-[6-(Azepan-1-yl)pyridin-3-yl]-5-[4-(trifluoromethyl)phenyl]-2,4-pentadienoic acid (Compound dl)

Compound dl was obtained from Compound dk in a similar manner to Reference example 93 using azepane.
$^1$H NMR (DMSO-$d_6$, δ ppm): 1.51-1.53 (m, 4H), 1.72-1.75 (m, 4H), 3.64 (t, J = 5.7 Hz, 4H), 6.17 (d, J = 14.8 Hz, 1H), 6.70 (d, J = 8.9 Hz, 1H), 6.97 (d, J = 11.3 Hz, 1H), 7.20-7.30 (m, 2H), 7.53 (d, J = 8.3 Hz, 2H), 7.73 (d, J = 8.3 Hz, 2H), 7.89 (d, J = 2.2 Hz, 1H), 12.29 (br s, 1H).

Reference example 103: (2E,4Z)-5-{6-([1,4]Oxazepan-4-yl)pyridin-3-yl]-5-[4-(trifluoromethyl)phenyl]-2,4-pentadienoic acid (Compound dm)

Compound dm was obtained from Compound dk in a similar manner to Reference example 93 using [1,4] oxazepane.
$^1$H NMR ($CDCl_3$, δ ppm): 2.06 (quint, J = 5.9 Hz, 2H), 3.77-3.83 (m, 4H), 3.88-3.89 (m, 4H), 6.09 (d, J = 14.8 Hz, 1H), 6.55 (d, J = 8.9 Hz, 1H), 6.71 (d, J = 11.3 Hz, 1H), 7.22-7.25 (m, 1H), 7.45 (d, J = 7.8 Hz, 2H), 7.55-7.65 (m, 3H), 8.02 (d, J = 1.9 Hz, 1H).

Reference example 104: (2E,4Z)-5-[6-(3-Methylpyrrolidin-1-yl)pyridin-3-yl]-5-[4-(trifluoromethyl)phenyl]-2,4-pentadienoic acid (Compound dn)

Compound dn was obtained from Compound dk in a similar manner to Reference example 93 using 3-methylpyrrolidine.
$^1$H NMR ($CDCl_3$, δ ppm): 1.16 (d, J = 6.7 Hz, 3H), 1.82-1.88 (m, 1H), 2.12-2.23 (m, 1H), 2.37-2.51 (m, 1H), 3.06-3.12 (m, 1H), 3.48-3.57 (m, 1H), 3.73-3.78 (m, 2H), 6.11 (d, J = 14.8 Hz, 1H), 6.42 (d, J = 8.6 Hz, 1H), 6.72 (d, J = 11.3 Hz, 1H), 7.21 (dd, J = 2.4, 8.6 Hz, 1H), 7.43 (d, J = 8.1 Hz, 2H), 7.53-7.63 (m, 3H), 8.08 (d, J = 1.9 Hz, 1H).

Reference example 105: (2E,4Z)-5-(4-Ethoxyphenyl)-5-[4-(trifluoromethyl)phenyl]-2,4-pentadienoic acid (Compound do)

Compound do was synthesized in a similar manner to Reference example 18 using Compound q and 4-ethoxyphenylboronic acid.
$^1$H NMR (DMSO-$d_6$, δ ppm): 1.37 (t, J = 7.0 Hz, 3H), 4.10 (q, J = 7.0 Hz, 2H), 6.18 (d, J = 14.0 Hz, 1H), 7.02-7.22 (m, 6H), 7.51 (d, J = 8.4 Hz, 2H), 7.73 (d, J = 8.4 Hz, 2H), 12.32 (br s, 1H).

Reference example 106: (2E, 4Z) -5- (6-Ethoxypyridin-3-yl) -5-[4-(trifluoromethyl)phenyl]-2,4-pentadienoic acid (Compound dp)

Compound dp was obtained from Compound dk in a similar manner to Reference example 93 using ethanol in place of piperidine.
$^1$H NMR (DMSO-$d_6$, δ ppm) : 1.39 (t, J = 7.0 Hz, 3H), 4.37 (q, J = 7.0 Hz, 2H), 6.07 (d, J = 14.9 Hz, 1H), 6.70 (d, J = 8.7 Hz, 1H) 6.77-6.85 (m, 1H), 7.31-7.37 (m, 3H), 7.49 (dd, J = 2.4, 8.7 Hz, 1H), 7.70 (d, J = 8.1 Hz, 2H), 8.03 (d, J = 2.4 Hz, 1H).

Reference example 107: (2E,4Z)-5-[4-(N,N-Dimethylamino)phenyl]-5-[4-(trifluoromethyl)phenyl]-2,4-pentadienoic acid (Compound dq)

Compound dq was synthesized in a similar manner to Reference example 18 using Compound q and 4-(N,N-dimethylamino)phenylboronic acid.
$^1$H NMR (DMSO-$d_6$, δ ppm): 2.98 (s, 6H), 6.14 (d, J = 15.2 Hz, 1H), 6.80 (d, J = 8.8 Hz, 2H), 6.93 (d, J = 11.6 Hz, 1H), 7.01 (d, J = 8.8 Hz, 2H), 7.29 (dd, J = 11.6, 15.2 Hz, 1H), 7.51 (d, J = 8.3 Hz, 2H), 7.72 (d, J = 8.3 Hz, 2H), 12.25 (br s, 1H).

Reference example 108: (2E,4Z)-5-[6-(Azetidin-1-yl)pyridin-3-yl]-5-[4-(trifluoromethyl)phenyl]-2,4-pentadienoic acid (Compound dr)

Compound dr was obtained from Compound dk in a similar manner to Reference example 93 using azetidine in place of piperidine.
$^1$H NMR (DMSO-$d_6$, δ ppm): 2.39 (quint, J = 7.4 Hz, 2H), 4.03 (t, J = 7.4 Hz, 4H), 6.19 (d, J = 14.8 Hz, 1H), 6.44 (d, J = 8.6 Hz, 1H), 7.04 (d, J = 11.5 Hz, 1H), 7.21 (dd, J = 11.5, 14.8 Hz, 1H), 7.29 (dd, J = 2.4, 8.6 Hz, 1H), 7.53 (d, J = 8.1

Hz, 2H), 7.74 (d, J = 8.1 Hz, 2H), 7.90 (d, J = 2.4 Hz, 1H), 12.33 (br s, 1H) .

Reference example 109: (2E,4Z)-5-[2-(Pyrrolidin-1-yl)pyrimidin-5-yl]-5-[4-(trifluoromethyl)phenyl]-2,4-pentadienoic acid (Compound ds)

Compound ds was synthesized in a similar manner to Reference example 18 using Compound q and 2-(pyrrolidin-1-yl)pyrimidin-5-ylboronic acid.
$^1$H NMR (DMSO-$d_6$, δ ppm): 1.94-1.99 (m, 4H), 3.53-3.58 (m, 4H), 6.22 (d, J = 14.0 Hz, 1H), 7.08-7.23 (m, 2H), 7.59 (d, J = 8.6 Hz, 2H), 7.75 (d, J = 8.6 Hz, 2H), 8.17 (d, J = 0.5 Hz, 2H), 12.39 (br s, 1H).

Reference example 110: (2E,4Z)-5-[4-(Tetrahydropyran-4-yloxy)phenyl]-5-[4-(trifluoromethyl)phenyl]-2,4-pentadienoic acid (Compound dt)

Compound dt was obtained from Compound ch in a similar manner to Reference example 73 using tetrahydro-2H-pyran-4-ol.
$^1$H NMR (DMSO-$d_6$, δ ppm): 1.57-1.70 (m, 2H), 1.99-2.05 (m, 2H), 3.47-3.60 (m, 2H), 3.84-3.91 (m, 2H), 4.61-4.68 (m, 1H), 6.18 (d, J = 14.0 Hz, 1H), 7.05-7.21 (m, 6H), 7.51 (d, J = 8.3 Hz, 2H), 7.73 (d, J = 8.3 Hz, 2H), 12.32 (br s, 1H).

Reference example 111: (2E,4Z)-5-(3-Methoxyphenyl)-5-[4-(trifluoromethyl)phenyl]-2,4-pentadienoic acid (Compound du)

Compound du was synthesized in a similar manner to Reference example 18 using Compound q and 3-methoxyphenylboronic acid.
$^1$H NMR (DMSO-$d_6$, δ ppm) : 3.77 (s, 3H), 6.20 (d, J = 14.3 Hz, 1H), 6.73-6.79 (m, 2H), 7.03-7.22 (m, 3H), 7.44 (t, J = 8.0 Hz, 1H), 7.53 (d, J = 8.5 Hz, 2H), 7.73 (d, J = 8.5 Hz, 2H).

Reference example 112: (2E,4Z)-5-(3-Propoxyphenyl)-5-[4-(trifluoromethyl)phenyl]-2,4-pentadienoic acid (Compound dv)

Compound dv was synthesized in a similar manner to Reference example 18 using Compound q and 3-propoxyphenylboronic acid.
$^1$H NMR (DMSO-$d_6$, δ ppm): 0.96 (t, J = 6.9 Hz, 3H), 1.72 (sext, J = 6.9 Hz, 2H), 3.93 (t, J = 6.9 Hz, 2H), 6.19 (d, J = 14.6 Hz, 1H), 6.72-6.77 (m, 2H), 7.02-7.20 (m, 3H), 7.42 (t, J = 7.8 Hz, pH), 7.52 (d, J = 8.4 Hz, 2H), 7.73 (d, J = 8.4 Hz, 2H).

Reference example 113: (2E,4Z)-5-(3-Isopropoxyphenyl)-5-[4-(trifluoromethyl)phenyl]-2,4-pentadienoic acid (Compound dw)

Compound dw was synthesized in a similar manner to Reference example 18 using Compound q and 3-isopropoxyphenylboronic acid.
$^1$H NMR (DMSO-$d_6$, δ ppm): 1.26 (d, J = 6.0 Hz, 6H), 4.63 (t, J = 6.0 Hz, 1H), 6.19 (d, J = 14.0 Hz, 1H), 6.70-6.74 (m, 2H), 7.00-7.19 (m, 3H), 7.40 (t, J = 8.0 Hz, 1H), 7.52 (d, J = 8.4 Hz, 2H), 7.73 (d, J = 8.4 Hz, 2H).

Reference example 114: (2E,4Z)-5-[3-(Tetrahydropyran-4-yloxy)phenyl]-5-[4-(trifluoromethyl)phenyl]-2,4-pentadienoic acid (Compound dx)

Compound dx was obtained from Compound cl in a similar manner to Reference example 73 using tetrahydro-2H-pyran-4-ol.
$^1$H NMR (DMSO-$d_6$, δ ppm): 1.51-1.64 (m, 2H), 1.94-2.00 (m, 2H), 3.41-3.50 (m, 2H), 3.83 (td, J = 4.3, 11.9 Hz, 2H), 4.58-4.64 (m, 1H), 6.19 (d, J = 14.0 Hz, 1H), 6.74-6.80 (m, 2H), 7.05-7.20 (m, 3H), 7.41 (t, J = 7.6 Hz, 1H), 7.52 (d, J = 8.1 Hz, 2H), 7.74 (d, J = 8.1 Hz, 2H), 12.34 (br s, 1H).

Reference example 115 : (2E,4Z)-5-(3-Ethoxy-phenyl)-5-[4-(trifluoromethyl)phenyl]-2,4-pentadienoic acid (Compound dy)

Compound dy was synthesized in a similar manner to Reference example 18 using Compound q and 3-ethoxyphenylboronic acid.
$^1$H NMR (DMSO-$d_6$, δ ppm) : 1.32 (t, J = 7.1 Hz, 3H), 4.03 (q, J = 7.1 Hz, 2H), 6.20 (d, J = 14.3 Hz, 1H), 6.71-6.77 (m, 2H), 7.02-7.26 (m, 2H), 7.40 (d, J = 7.3 Hz, 2H), 7.52 (d, J = 8.4 Hz, 2H), 7.73 (d, J = 8.4 Hz, 2H).

Reference example 116 : (2E,4Z)-5-(Benzo[1,3]dioxol-5-yl)-5-[4-(trifluoromethyl)phenyl]-2,4-pentadienoic acid (Compound dz)

Compound dz was synthesized in a similar manner to Reference example 18 using Compound q and 3,4-methylenedioxyphenylboronic acid.
$^1$H NMR (DMSO-$d_6$, δ ppm): 6.12 (s, 2H), 6.19 (dd, J = 1.2, 13.0 Hz, 1H), 6.69 (d, J = 7.8 Hz, 1H), 6.76 (d, J = 1.2 Hz, 1H), 7.03-7.21 (m, 3H), 7.53 (d, J = 8.4 Hz, 2H), 7.73 (d, J = 8.4 Hz, 2H), 12.35 (br s, 1H).

Reference example 117: (2E,4Z)-5-(2,3-Dihydrobenzo[1,4]dioxin-6-yl)-5-[4-(trifluoromethyl)phenyl]-2,4-pentadienoic acid (Compound ea)

Compound ea was synthesized in a similar manner to Reference example 18 using Compound q and 3,4-ethylenedioxyphenylboronic acid.
$^1$H NMR (DMSO-$d_6$, δ ppm): 4.30-4.31 (m, 4H), 6.18 (d, J = 14.3 Hz, 1H), 6.62-6.68 (m, 2H), 6.97-7.22 (m, 3H), 7.51 (d, J = 8.4 Hz, 2H), 7.73 (d, J = 8.4 Hz, 2H), 12.36 (br s, 1H).

Reference example 118: (2E,4Z)-5-(6-Methylpyridin-3-yl)-5-[4-(trifluoromethyl)phenyl]-2,4-pentadienoic acid (Compound eb)

Compound eb was synthesized in a similar manner to Reference example 18 using Compound q and 4-methyl-3-pyridylboronic acid.
$^1$H NMR (DMSO-$d_6$, δ ppm): 2.57 (s, 3H), 6.25 (d, J = 15.0 Hz, 1H), 7.04 (dd, J = 11.6, 15.0 Hz, 1H), 7.28 (d, J = 11.6 Hz, 1H), 7.41 (d, J = 8.1 Hz, 1H), 7.51-7.54 (m, 3H), 7.75 (d, J = 8.7 Hz, 2H), 8.30 (d, J = 2.1 Hz, 1H), 12.42 (br s, 1H).

Reference example 119: (2E,4Z)-5-(2-Methylpyridin-4-yl)-5-[4-(trifluoromethyl)phenyl]-2,4-pentadienoic acid (Compound ec)

Compound ec was synthesized in a similar manner to Reference example 18 using Compound q and 3-methyl-4-pyridylboronic acid.
$^1$H NMR (DMSO-$d_6$, δ ppm): 2.53 (s, 3H), 6.26 (d, J = 15.0 Hz, 1H), 6.97 (dd, J = 11.7, 15.0 Hz, 1H), 7.09 (d, J = 5.1 Hz, 1H), 7.14 (s, 1H), 7.29 (d, J = 11.7 Hz, 1H), 7.52 (d, J = 8.3 Hz, 2H), 7.75 (d, J = 8.3 Hz, 2H), 8.60 (d, J = 5.1 Hz, 1H), 12.45 (br s, 1H).

Reference example 120: (2E,4Z)-5-[3-(Trifluoromethoxy)phenyl]-5-[4-(trifluoromethyl)phenyl]-2,4-pentadienoic acid (Compound ed)

Compound ed was synthesized in a similar manner to Reference example 18 using Compound q and 3-(trifluoromethoxy)phenylboronic acid.
$^1$H NMR (DMSO-$d_6$, δ ppm): 6.25 (d, J = 14.9 Hz, 1H), 7.02 (dd, J = 11.5, 14.9 Hz, 1H), 7.23-7.29 (m, 3H), 7.50-7.53 (m, 3H), 7.67 (t, J = 8.0 Hz, 1H), 7.75 (d, J = 8.4 Hz, 2H) .

Reference example 121: (2E,4Z)-5-[6-(Morpholin-4-yl)pyridin-3-yl]-5-[4-(trifluoromethyl)phenyl]-2,4-pentadienoic acid (Compound ee)

Compound ee was obtained from Compound dk in a similar manner to Reference example 93 using morpholine in place of piperidine.
$^1$H NMR (DMSO-$d_6$, δ ppm): 3.54-3.57 (m, 4H), 3.71-3.74 (m, 4H), 6.20 (d, J = 14.6 Hz, 1H), 6.94 (d, J = 8.9 Hz, 1H), 7.07 (d, J = 11.7 Hz, 1H), 7.21 (dd, J = 11.7, 14.6 Hz, 1H), 7.35 (dd, J = 2.4, 8.9 Hz, 1H), 7.54 (d, J = 8.4 Hz, 2H), 7.74 (d, J = 8.4 Hz, 2H), 7.97 (d, J = 2.4 Hz, 1H), 12.37 (br s, 1H).

Reference example 122: (2E,4Z)-5-[3-(N,N-Dimethylamino)phenyl]-5-[4-(trifluoromethyl)phenyl]-2,4-pentadienoic acid (Compound ef)

Compound ef was synthesized in a similar manner to Reference example 18 using Compound q and 3-(N,N-dimethylamino)phenylboronic acid.
$^1$H NMR (DMSO-$d_6$, δ ppm): 2.90 (s, 6H), 6.12-6.22 (m, 1H), 6.44-6.47 (m, 2H), 6.79-0.83 (m, 1H), 7.12-7.22 (m, 2H), 7.30 (t, J = 7.9 Hz, 1H), 7.55 (d, J = 8.3 Hz, 2H), 7.72 (d, J = 8.3 Hz, 2H), 12.32 (br s, 1H) .

Reference example 123: (2E,4Z)-5-(1-Metyl-1H-indol-5-yl)-5-[4-(trifluoromethyl)phenyl]-2,4-pentadienoic acid (Compound eg)

Compound eg was synthesized in a similar manner to Reference example 18 using Compound q and N-methyl-5-indolylboronic acid.
$^1$H NMR (DMSO-$d_6$, δ ppm): 3.85 (s, 3H), 6.17 (d, J = 14.0 Hz, 1H), 6.49 (d, J = 3.0 Hz, 1H), 6.94 (d, J = 8.4 Hz, 1H), 7.09-7.24 (m, 2H), 7.39 (s, 1H), 7.42 (d, J = 3.0 Hz, 1H), 7.51-7.56 (m, 3H), 7.71 (d, J = 8.4 Hz, 2H), 12.24 (br s, 1H).

Reference example 124: (2E,4Z)-5-[6-(Piperidin-1-yl)pyridin-3-yl]-5-[4-(trifluoromethyl)phenyl]-2,4-pentadienoic acid (Compound eh)

Compound eh was obtained from Compound dk in a similar manner to Reference example 93.
$^1$H NMR (DMSO-$d_6$, δ ppm): 1.59-1.61 (m, 6H), 3.60-3.62 (m, 4H), 6.19 (d, J = 14.8 Hz, 1H), 6.92 (d, J = 9.2 Hz, 1H), 7.02 (d, J = 11.6 Hz, 1H), 7.19-7.30 (m, 2H), 7.54 (d, J = 7.8 Hz, 2H), 7.74 (d, J = 8.4 Hz, 2H), 7.92 (d, J = 2.2 Hz, 1H), 12.33 (br s, 1H).

Reference example 125: (2E,4Z)-5-[6-(N,N-Dimethylamino)pyridin-3-yl]-5-[4-(trifluoromethyl)phenyl]-2,4-pentadienoic acid (Compound ei)

Compound ei was obtained from Compound dk in a similar manner to Reference example 93 using dimethylamine. $^1$H NMR (DMSO-$d_6$, δ ppm): 3.15 (s, 6H), 6.22 (d, J = 14.7 Hz, 1H), 6.90 (d, J = 8.9 Hz, 1H), 7.09 (d, J = 11.4 Hz, 1H), 7.22 (dd, J = 11.4, 14.7 Hz, 1H), 7.41 (dd, J = 2.3, 8.9 Hz, 1H), 7.55 (d, J = 8.4 Hz, 2H), 7.75 (d, J = 8.4 Hz, 2H), 7.92 (d, J = 2.3 Hz, 1H) .

Reference example 126: (2E,4Z)-5-[2-(N,N-Dimethylamino)pyrimidin-5-yl]-5-[4-(trifluoromethyl)phenyl]-2,4-pentadienoic acid (Compound ej)

Compound ej was synthesized in a similar manner to Reference example 18 using Compound q and 4-dimethylamino-3-pyrimidylboronic acid pinacol ester.
$^1$H NMR (DMSO-$d_6$, δ ppm): 3.20 (s, 6H), 6.22 (d, J = 13.8 Hz, 1H), 7.09-7.23 (m, 2H), 7.59 (d, J = 8.3 Hz, 2H), 7.75 (d, J = 8.3 Hz, 2H), 8.19 (br s, 2H).
Reference example 127: (2E,4Z)-5-(3-Hydroxyphenyl)-5-[4-(trifluoromethyl)phenyl]-2,4-pentadienoic acid (Compound ek)
Compound ek was obtained from Compound cl in a similar manner to step 2 of Reference example 18.
$^1$H NMR (DMSO-$d_6$, δ ppm): 6.19 (td, J = 6.8, 14.3 Hz, 1H), 6.57-6.63 (m, 2H), 6.84-6.88 (m, 1H), 7.12 (d, J = 1.3 Hz, 1H), 7.15 (d, J = 3.8 Hz, 1H), 7.30 (t, J = 7.7 Hz, 1H), 7.52 (d, J = 8.5 Hz, 2H), 7.73 (d, J. = 8.5 Hz, 2H), 9.65 (br s, 1H), 12.36 (br s, 1H) .

Reference example 128: (2E,4Z)-5-[4-(Trifluoromethoxy)phenyl]-5-[4-(trifluoromethyl)phenyl]-2,4-pentadienoic acid (Compound el)

Compound el was synthesized in a similar manner to Reference example 18 using Compound q and 4-(trifluoromethoxy)phenylboronic acid.
$^1$H NMR (DMSO-$d_6$, δ ppm): 6.25 (d, J = 14.9 Hz, 1H), 7.03 (dd, J = 11.7, 14.9 Hz, 1H), 7.24 (d, J = 11.7 Hz, 1H), 7.36 (d, J = 8.6 Hz, 2H), 7.50-7.53 (m, 4H), 7.75 (d, J = 8.4 Hz, 2H), 12.42 (br s, 1H).

Reference example 129: Ethyl (2E,4Z)-5-(4-aminophenyl)-5-[4-(trifluoromethyl)phenyl]-2,4-pentadienoate (Compound em)

Compound em was synthesized in a similar manner to step 1 of Reference example 18 using Compound q and 4-aminophenylboronio acid.
$^1$H NMR ($CDCl_3$, δ ppm) : 1.28 (t, J = 7.0 Hz, 3H), 3.85 (br s, 2H), 4.19 (q, J = 7.0 Hz, 2H), 6.06 (d, J = 15.1 Hz, 1H), 6.65-6.73 (m, 3H), 6.99 (d, J = 8.4 Hz, 2H), 7.42 (d, J = 8.3 Hz, 2H), 7.46-7.56 (m, 1H), 7.56 (d, J = 8.3 Hz, 2H).

Reference example 130: (2E,4Z)-5-[4-(2,5-Dimethylpyrrol-1-yl)phenyl]-5-[4-(trifluoromethyl)phenyl]-2,4-pentadienoic acid (Compound en)

Step 1

Compound em (220 mg, 0.608 mmol) was dissolved in toluene (5.0 mL), and acetonylacetone (0.111 mL, 0.950 mmol) and tosic acid monohydrate (5.00 mg, 0.0263 mmol) were added thereto, and then, the mixture was stirred at 70˚C for 2 hours. After the reaction mixture was left to cool, water was added thereto, and the mixture was extracted with ethyl acetate. The organic layer was washed with saturated brine and dried over anhydrous magnesium sulfate. After the organic layer was filtered, the solvent was evaporated under reduced pressure, and then, the residue was purified by silica gel column chromatography (ethyl acetate/hexane = 5/1) to give ethyl (2E,4Z)-5-[4-(2,5-dimethylpyrrol-1-yl)phenyl]-5-[4-(trifluoromethyl)phenyl]-2,4-pentadienoate (230 mg, 86 %).

Step 2

Compound en was synthesized in a similar manner to Step 2 of Reference example 18 using ethyl (2E,4Z)-5-[4-(2,5-dimethylpyrrol-1-yl)phenyl]-5-[4-(trifluoromethyl)phenyl]-2,4-pentadienoate.
$^1$H NMR (DMSO-$d_6$, δ ppm) : 2.04 (s, 6H), 5.84 (s, 2H), 6.25 (d, J = 14.3 Hz, 1H), 7.09-7.25 (m, 2H), 7.33-7.42 (m, 4H), 7.55 (d, J = 8.1 Hz, 2H), 7.78 (d, J = 8.1 Hz, 2H).

Reference example 131: (2E,4Z)-5-[4-(Pyrrol-1-yl)phenyl]-5-[4-(trifluoromethyl)phenyl]-2,4-pentadienoic acid (Compound eo)

Compound eo was obtained from Compound en in a similar manner to Reference example 130 using 2,5-dimethoxytetrahydrofuran in place of acetonylacetone.
$^1$H NMR (DMSO-$d_6$, δ ppm): 6.23 (dd, J = 3.0, 11.3 Hz, 1H), 6.31 (t, J = 2.1 Hz, 2H), 7.14-7.19 (m, 2H), 7.29 (d, J = 8.6 Hz, 2H), 7.48 (t, J = 2.1 Hz, 2H), 7.55 (d, J = 8.4 Hz, 2H), 7.71-7.77 (m, 4H).

Reference example 132: (2E,4Z)-5-[6-(Pyrrolidin-1-yl)pyridin-3-yl]-5-[4-(trifluoromethyl)phenyl]-2,4-pentadienoic acid (Compound ep)

Compound ep was obtained from Compound dk in a similar manner to Reference example 93 using pyrrolidine in place of piperidine.
$^1$H NMR (DMSO-$d_6$, δ ppm): 1.95-1.99 (m, 4H), 3.43-3.48 (m, 4H), 6.17 (d, J = 14.6 Hz, 1H), 6.55 (d, J = 8.9 Hz, 1H), 7.01 (d, J = 11.6 Hz, 1H), 7.20-7.23 (m, 2H), 7.54 (d, J = 8.3 Hz, 2H), 7.74 (d, J = 8.3 Hz, 2H), 7.90 (d, J = 1.9 Hz, 1H), 12.27 (br s, 1H) .

Reference example 133: (2E,4Z)-5-[6-(2-Methylpyrrolidin-1-yl)pyridin-3-yl]-5-[4-(trifluoromethyl)phenyl]-2,4-pentadienoic acid (Compound eq)

Compound eq was obtained from Compound dk in a similar manner to Reference example 93 using 2-methylpyrrolidine in place of piperidine.
$^1$H NMR (DMSO-$d_6$, δ ppm): 1.19 (d, J = 6.5 Hz, 3H), 1.74-1.78 (m, 2H), 1.91-2.09 (m, 2H), 3.58-3.63 (m, 2H), 4.16-4.21 (m, 1H), 6.17 (d, J = 14.8 Hz, 1H), 6.55 (d, J = 8.9 Hz, 1H), 7.00 (d, J = 11.6 Hz, 1H), 7.20-7.30 (m, 2H), 7.55 (d, J = 8.1 Hz, 2H), 7.74 (d, J = 8.1 Hz, 2H), 7.91 (d, J = 2.4 Hz, 1H).

Reference example 134: (2E,4Z)-5-[2-(Piperidin-1-yl)pyrimidin-5-yl]-5-[4-trifluoromethyl)phenyl]-2,4-pentadienoic acid (Compound er)

Compound er was synthesized in a similar manner to Reference example 18 using Compound q and (2-piperidin-1-ylpyrimidin-5-yl)boronic acid.
$^1$H NMR (DMSO-$d_6$, δ ppm): 1.57-1.66 (m, 6H), 3.80-3.84 (m, 4H), 6.23 (d, J = 14.3 Hz, 1H), 7.07-7.25 (m, 2H), 7.59 (d, J = 8.3 Hz, 2H), 7.75 (d, J = 8.3 Hz, 2H), 8.17 (s, 2H).

Reference example 135: (2E,4Z)-5-(3-Cyano-4-fluorophenyl)-5-[4-(trifluoromethyl)phenyl]-2,4-pentadienoic acid (Compound es)

Compound es was synthesized in a similar manner to Reference example 18 using Compound q and 3-cyano-

4-fluorophenylboronic acid.
$^1$H NMR (DMSO-$d_6$, δ ppm): 6.21-6.30 (m, 1H), 6.90-7.07 (m, 1H), 7.19-7.41 (m, 1H), 7.90-7.55 (m, 7H).

Reference example 136: (2E,4Z)-5-(5-Methylthiophen-2-yl)-5-[4-(trifluoromethyl)phenyl]-2,4-pentadienoic acid (Compound et)

Compound et was synthesized in a similar manner to Reference example 18 using Compound q and 3-methyl-2-thienylboronic acid.
$^1$H NMR (DMSO-$d_6$, δ ppm): 3.79 (s, 3H), 6.28 (d, J = 15.1 Hz, 1H), 6.67 (d, J = 3.4 Hz, 1H), 6.90 (d, J = 11.9 Hz, 1H), 7.17 (d, J = 3.4 Hz, 1H), 7.62 (d, J = 8.1 Hz, 2H), 7.78-7.90 (m, 3H). 8.53 (br s, 1H).

Reference example 137: (2E,4Z)-5-[4-(Pyrrolidin-1-yl)phenyl]-5-[4-(trifluoromethyl)phenyl]-2,4-pentadienoic acid (Compound eu)

Compound eu was synthesized in a similar manner to Reference example 18 using Compound q and 4-(pyrrolidin-1-yl)phenylboronio acid.
$^1$H NMR (DMSO-$d_6$, δ ppm): 1.96-2.00 (m, 4H), 3.28-3.30 (m, 4H), 6.12 (d, J = 15.2 Hz, 1H), 6.62 (d, J = 8.4 Hz, 2H), 6.90 (d, J = 11.5 Hz, 1H), 6.98 (d, J = 8.6 Hz, 2H), 7.30 (dd, J = 11.5, 15.2 Hz, 1H), 7.51 (d, J = 8.4 Hz, 2H), 7.72 (d, J = 8.6 Hz, 2H).

Reference example 138: (2E,4Z)-5-[4-(Piperidin-1-yl)phenyl]-5-[4-(trifluoromethyl)phenyl]-2,4-pentadienoic acid (Compound ev)

Compound eu was synthesized in a similar manner to Reference example 18 using Compound q and 4-(piperidine-1-yl)phenylboronic acid.
$^1$H NMR (DMSO-$d_6$, δ ppm) : 1.60-1.64 (m, 6H), 3.24-3.26 (m, 4H), 6.15 (d, J = 14.9 Hz, 1H), 6.94-7.02 (m, 5H), 7.26 (dd, J = 11.5, 14.9 Hz, 1H), 7.51 (d, J = 8.2 Hz, 2H), 7.73 (d, J = 8.2 Hz, 2H), 12.26 (br s, 1H).

Reference example 139: (2E,4Z)-5-Bromo-N-(isoquinolin-5-yl)-5-[4-(trifluoromethyl)phenyl]-2,4-pentadienoic acid (Compound ew)

Compound ew was obtained from Compound q in a similar manner to step 2 of Reference example 18.
$^1$H NMR (DMSO-$d_6$, δ ppm): 6.38 (d, J = 14.3 Hz, 1H), 7.44-7.60 (m, 2H), 7.81-7.84 (m, 2H), 7.93-7.96 (m, 2H).

Reference example 140: (2E,4Z)-5-(3,4,-Difluorophenyl)-5-[4-(trifluoromethyl)phenyl]-2,4-pentadienoic acid (Compound ex)

Compound ex was synthesized in a similar manner to Reference example 18 using Compound q and 3,4-difluorophenylboronic acid.
$^1$H NMR (DMSO-$d_6$, δ ppm): 6.25 (d, J = 14.7 Hz, 1H), 7.03 (dd, J = 11.4, 15.0 Hz, 1H), 7.07-7.12 (m, 1H), 7.25 (d, J = 11.4 Hz, 1H), 7.34-7.41 (m, 1H), 7.46-7.64 (m, 3H), 7.75 (d, J = 8.4 Hz, 2H), 12.46 (br s, 1H).

Reference example 141: (2E,4Z)-5-(4-Methyl-3,4-dihydro-2H-benzo[1,4]oxazin-7-yl)-5-[4-(trifluoromethyl)phenyl]-2,4-pentadienoic acid (Compound ey)

Compound ey was synthesized in a similar manner to Reference example 18 using Compound q and 4-methyl-7-(4,4,5,5,-tetramethyl-1,3,2-dioxaborolan-2-yl)-3,4-dihydro-2H-1,4-benzoxazine.
$^1$H NMR (DMSO-$d_6$, δ ppm): 2.91 (s, 3H), 3.32-3.33 (m, 2H), 4.25-4.27 (m, 2H), 6.14 (d, J = 15.3 Hz, 1H), 6.45 (d, J = 2.0 Hz, 1H), 6.62 (dd, J = 2. 0, 8.2 Hz, 1H), 6.79 (d, J = 8.2 Hz, 1H), 6.94 (d, J = 11.7 Hz, 1H), 7.27 (dd, J = 11.7, 15.3 Hz, 1H), 7.51 (d, J = 8.3 Hz, 2H), 7.72 (d, J = 8.3 Hz, 2H).

Reference example 142: Ethyl (2E,4Z)-5-(4,4,5,5-tetramethyl-[1,3,2]di-oxaborolan-2-yl)-5-[4-(trifluoromethyl)phenyl]-2,4-pentadienoate (Compound ez)

Compound q (4.00 g, 11.5 mmol), bis (pinacolato) cliboron (4.36 g, 17.2 mmol), [1,1'-bis(diphenylphosphino) ferrocene] dichloropalladium(II) (730 mg, 0.894 mmol), and potassium acetate (3.37 g, 34.3 mmol) were dissolved in 1,4-dioxane (80 mL), and the mixture was stirred at 100°C for 6.5 hours. After the reaction mixture was left to cool, water was added thereto, and the mixture was extracted three times with ethyl acetate. The organic layer was washed with

saturated brine and dried over anhydrous magnesium sulfate. Then, the solvent was evaporated under reduced pressure, and the residue was purified by silica gel column chromatography (hexane/ethyl acetate = 9/1) to give Compound ez (2.20 g, 49%) .

$^1$H NMR ($CDCl_3$, δ ppm): 1.32 (t, J = 7.4 Hz, 2H), 1.39 (s, 12H), 4.24 (q, J = 7.4 Hz, 3H), 6.06 (d, J = 15.1 Hz, 1H), 7.02 (d, J = 11.7 Hz, 1H), 7.51-7.60 (m, 4H), 8.07 (dd, J = 11.7, 15.1 Hz, 1H).

Reference example 143: (2E,4Z)-5-[5-(Dimethylaminomethyl) furan-2-yl]-5-[4-(trifluoromethyl)phenyl]-2,4-pentadienoic acid (Compound fa)

Step 1

5-Bromo-2-furfural (500 mg, 2.86 mmol) was dissolved in dichloromethane (50 mL), and dimethylamine monohydrochloride (1.12 g, 13.7 mmol), sodium triacetoxyborohydride (1.27 g, 5.99 mmol), and triethylamine (1.90 mL, 13.6 mmol) were added thereto, and then, the mixture was stirred at room temperature for 2 hours. Water was added to the reaction mixture, and the mixture was extracted with ethyl acetate. The organic layer was washed with saturated brine and dried over anhydrous magnesium sulfate. After the organic layer was filtered, the solvent was evaporated under reduced pressure, and then, the residue was purified by silica gel column chromatography (ethyl acetate/hexane = 1/9) to give 2-bromo-5-(dimethylaminomethyl)furan (441 mg, 76%).

$^1$H NMR ($CDCl_3$, δ ppm): 2.27 (s, 6H), 3.44 (s, 2H), 6.18 (d, J = 3.7 Hz, 1H), 6.23 (d, J = 3.7 Hz, 1H).

Step 2

Compound fa was synthesized in a similar manner to Reference example 18 using Compound ez and 2-bromo-5-(dimethylaminomethyl)furan.

$^1$H NMR (DMSO-$d_6$, δ ppm): 2.21 (s, 6H), 3.54 (s, 2H), 6.19 (d, J = 15.0 Hz, 1H), 6.46 (d, J = 3.3 Hz, 1H), 6.49 (d, J = 3.3 Hz, 1H), 6.67 (d, J = 11.9 Hz, 1H), 7.60 (d, J = 8.1 Hz, 2H), 7.78 (d, J = 8.1 Hz, 2H), 7.93 (dd, J = 11.9, 15.0 Hz, 1H).

Reference example 144: (2E,4Z)-5-[5-(Morpholin-4-ylmethyl)furan-2-yl]-5-[4-(trifluoromethyl)phenyl]-2,4-pentadienoic acid (Compound fb)

Compound fb was obtained in a similar manner to Reference example 143 using morpholine.

$^1$H NMR (DMSO-$d_6$, ppm): 2.42-2.45 (m, 4H), 3.56-3.60 (m, 6H), 6.18 (d, J = 15.1 Hz, 1H), 6.43 (d, J = 3.5 Hz, 1H), 6.49 (d, J = 3.5 Hz, 1H), 6.65 (d, J = 11.9 Hz, 1H), 7.61 (d, J = 8.1 Hz, 2H), 7.78 (d, J = 8.1 Hz, 2H), 7.94 (dd, J = 11.9, 15.1 He, 1H) .

Reference example 145: (2H,4Z)-5-(5-Methylfuran-2-yl)-5-[4-(trifluoromethyl)phenyl]-2,4-pentadienamide (Compound fc)

Compound fc was synthesized in a similar manner to Reference example 18 using Compound q and 3-methyl-2-furylboronic acid.

$^1$H NMR (DMSO-$d_6$, δ ppm): 2.36 (s, 3H), 6.17 (d, J = 15.1 Hz, 1H), 6.30 (dd, J = 0.9, 3.3 Hz, 1H), 6.39 (d, J = 3.3 Hz, 1H), 6.58 (d, J = 11.8 Hz, 1H), 7.60 (d, J = 8.1 Hz, 2H), 7.78 (d, J = 8.1 Hz, 2H), 7.93 (dd, J = 11.8, 15.1 Hz, 1H).

Reference example 146: Ethyl (2E,4Z)-5-bromo-5-[4-(methansulfonyl)phenyl]-2,4-pentadienoate (Compound fd)

Compound fd was synthesized in a similar manner to Reference example 17 using Compound a and 4-(methansulfonyl)phenylboronic acid.

$^1$H NMR ($CDCl_3$, δ ppm): 1.34 (t, J = 7.1 Hz, 3H), 3.07 (s, 3H), 4.27 (q, J = 7.1 Hz, 2H), 6.22 (d, J = 15.4 Hz, 1H), 7.04 (d, J = 10.6 Hz, 1H), 7.73 (dd, J = 10.6, 15.4 Hz, 1H), 7.82 (dd, J = 1.8, 6.7 Hz, 2H), 7.96 (dd, J = 1.8, 6.7 Hz, 2H).

Reference example 147: (2E,4E)-5-(4-Fluorophenyl)-5-[4-(methansulfonyl)phenyl]-2,4-pentadienoic acid (Compound fe)

Compound fe was synthesized in a similar manner to Reference example 18 using Compound fd and 4-fluorophenylboronic acid.

$^1$H NMR (DMSO-$d_6$, δ ppm): 3.23 (s, 3H), 6.23 (d, J = 15.0 Hz, 1H), 7.04 (dd, J = 12.4, 15.0 Hz, 1H), 7.21-7.29 (m, 3H), 7.33-7.39 (m, 2H), 7.55 (d, J = 8.6 Hz, 2H), 7.91 (d, J = 8.6 Hz, 2H).

Reference example 148: (2E,42)-5-(Pyrimidin-5-yl)-5-[4-(trifluoromethyl)phenyl]-2,4-pentadienoic acid (Compound ff)

Compound ff was synthesized in a similar manner to Reference example 18 using Compound ez and 5-bromopyrimidine.
$^1$H NMR (DMSO-$d_6$, δ ppm) : 6.32 (d, J = 15.0 Hz, 1H), 6.96 (dd, J = 11.6, 15.0 Hz, 1H), 7.42 (d, J = 11.6 Hz, 1H), 7.57 (d, J = 8.3 Hz, 2H), 7.77 (d, J = 8.3 Hz, 2H), 8.74 (s, 2H), 9.31 (s, 1H), 12.47 (br s, 1H).

Reference example 149: (2E,4E)-5-(4-Acetylphenyl)-5-[4-(trifluoromethyl)phenyl]-2,4-pentadienoic acid (Compound fg)

Compound fg was synthesized in a similar manner to Reference example 18 using Compound q and 4-acethylphenylboronic acid.
$^1$H NMR (DMSO-$d_6$, δ ppm): 2.65 (s, 3H), 6.25 (d, J = 15.0 Hz, 1H), 7.03 (dd, J = 11.5, 15.0 Hz, 1H), 7.27 (d, J = 11.5 Hz, 1H), 7.38 (d, J = 8.2 Hz, 2H), 7.51 (d, J = 8.2 Hz, 2H), 7.75 (d, J = 8.4 Hz, 2H), 8.09 (d, J = 8.2 Hz, 2H).

Reference example 150: (2E,4Z)-5-(N-Acetyl-1,2,3,6-tetrahydropyridin-4-yl)-5-[4-(trifluoromethyl)phenyl]-2,4-pentadienoic acid (Compound fh)

Step 1

Ethyl (2E,4Z)-5-(N-tert-butoxycarbonyl-1,2,3,6-tetrahydropyridin-4-yl)-5-[4-(trifluoromethyl)phenyl]-2,4-pentadienoate was synthesized in a similar manner to Step 1 of Reference example 18 using Compound q and N-tert-butoxyoarbonyl-1,2,3,6-tetrahydropyridin-4-yl boronic acid pinacol ester.

Step 2

To ethyl (2E,4Z)-5-(N-tert-butoxycarbonyl-1,2,3,6-tetrahydropyridin-4-yl)-5-[4-(trifluoromethyl)phenyl]-2,4-pentadienoate (200 mg, 0.444 mmol), a 4.0 mol/L hydrochloric acid -ethyl acetate solution (4.0 mL) was added, and the mixture was stirred at room temperature for 3 hours. To the reaction mixture, a saturated aqueous sodium hydrogen carbonate solution was added, and the mixture was extracted with ethyl acetate. The organic layer was washed with saturated brine and dried over anhydrous magnesium sulfate. After the organic layer was filtered, the solvent was evaporated under reduced pressure to give ethyl (2E,4Z)-5-(1,2,3,6-tetrahydropyridin-4-yl)-5-[4-(trifluoromethyl)phenyl]-2,4-pentadienoate (106 mg, 68%).

Step 3

Ethyl (2E,42)-5-(1,2,3,6-tetrahydropyridin-4-yl)-5-[4-(trifluoromethyl)phenyl]-2,4-pentadienoate (106 mg, 0.301 mmol) was dissolved in dichloromethane (3.2 mL), and triethylamine (0.0840 mL, 0.603 mmol) and acetyl chloride (0.0320 mL, 0.450 mmol) were added thereto, and then, the mixture was stirred at room temperature for 4 hours. To the reaction mixture, a saturated aqueous sodium hydrogen carbonate solution was added, and the mixture was extracted with ethyl acetate. The organic layer was washed with saturated brine and dried over anhydrous magnesium sulfate. After the organic layer was filtered, the solvent was evaporated under reduced pressure, and then, the residue was purified by silica gel column chromatography (ethyl acetate) to give ethyl (2E,4Z)-5-(N-acetyl-1,2,3,6-tetrahydropyridin-4-yl)-5-[4-(trifluoromethyl)phenyl]-2,4-pentadienoate (68.7 mg, 58%).

Step 4

Compound fh was synthesized in a similar manner to Step 2 of Reference example 18 using ethyl (2E,4Z)-5-(N-acetyl-1,2,3,6-tetrahydropyridin-4-yl)-5-[4-(trifluoromethyl)phenyl]-2,4-pentadienoate.
$^1$H NMR (DMSO-$d_6$, δ ppm) : 2.11 (s, 3H), 3.69 (td, J = 5.6, 15.2 Hz, 2H), 4.23-4.27 (m, 2H), 5.79-5.80 (m, 1H), 6.08 (dd, J = 3.5, 15.2 Hz, 1H), 6.75 (dd, J = 3.5, 11.4 Hz, 1H), 7.52-7.65 (m, 5H).

Reference example 151: (2E,4Z)-5-[4-(2-tert-Butoxycarbonylaminoethoxy)phenyl]-5-[4-(trifluoromethyl)phenyl]-2,4-pentadienoic acid (Compound fi)

Compound fi was obtained from Compound ch in a similar manner to Reference example 73 using hydroxyethylcarbamic acid tert-butyl ester.
$^1$H NMR ($CDCl_3$, δ ppm): 1.47 (s, 9H), 3.57-3.59 (m, 2H), 4.08 (t, J = 5.0 Hz, 2H), 5.07 (br s, 1H), 6.08 (d, J = 14.8 Hz, 1H), 6.77 (d, J = 11.4 Hz, 1H), 6.95 (d, J = 8.6 Hz, 2H), 7.12 (d, J = 8.6 Hz, 2H), 7.39-7.59 (m, 5H).

Reference example 152: (2E,4E)-5-(4-Formylphenyl)-5-[4-(trifluoromethyl)phenyl]-2,4-pentadienoic acid (Compound fj)

Compound fj was synthesized in a similar manner to Reference example 18 using Compound q and 4-formylphenylboronic acid.
$^1$H NMR (DMSO-$d_6$, δ ppm): 1.27 (t, J = 7.1 Hz, 3H), 4.19 (q, J = 7.1 Hz, 2H), 6.16 (d, J = 15.2 Hz, 1H), 6.90 (d, J = 11.5 Hz, 1H), 7.28 (dd, J = 3.7, 11.5 Hz, 1H), 7.34-7.41 (m, 4H), 7.59 (d, J = 8.2 Hz, 2H), 7.97 (d, J = 8.0 Hz, 2H), 10.09 (s, 1H).

Reference example 153: (2E,4Z)-5-[4-(Morpholin-4-yl)phenyl]-5-[4-(trifluoromethyl)phenyl]-2,4-pentadienoic acid (Compound fk)

Compound fk was synthesized in a similar manner to Reference example 18 using Compound q and 4-(morpholin-4-yl)phenylboronic acid.
$^1$H NMR (DMSO-$d_6$, δ pm): 3.22-3.23 (m, 4H), 3.76-3.77 (m, 4H), 6.17 (d, J = 15.0 Hz, 1H), 6.92-7.06 (m, 5H), 7.15-7.29 (m, 1H), 7.52 (d, J = 8.1 Hz, 2H), 7.74 (d, J = 8.1 Hz, 2H), 12.28 (br s, 1H).

Reference example 154: (2E,4Z)-5-[4-(Methansulfonyl)phenyl]-5-[4-(trifluoromethyl)phenyl]-2,4-pentadienoic acid (Compound fl)

Compound f1 was synthesized in a similar manner to Reference example 18 using Compound q and 4-(methansulfonyl)phenylboronic acid.
$^1$H NMR (DMSO-$d_6$, δ ppm): 3.21 (s, 3H), 6.22 (d, J = 13.4 Hz, 1H), 7.13-7.26 (m, 2H), 7.48-7.52 (m, 4H), 7.66 (d, J = 8.4 Hz, 2H), 8.08 (d, J = 8.4 Hz, 2H).

Reference example 155: (2E,4E)-5-Phenyl-5-[4-(trifluoromethanesulfonyl)phenyl]-2,4-pentadienoic acid (Compound fm)

Step 1

Methyl (E)-3-[4-(trifluoromethanesulfonyl)phenyl] acrylate was obtained from commercially available 4-trifluoromethanesulfonylbenzaldehyde in a similar manner to Step 5 of Reference example 40.

Step 2

(E)-3-[4-(Trifluoromethanesulfonyl)phenyl]-2-propen-1-ol was obtained from methyl (E)-3-[4-(trifluoromethanesulfonyl)phenyl]acrylate in a similar manner to Step 3 of Reference example 40.

Step 3

(E)-3-[4-(Trifluoromethanesulfonyl)phenyl]propenal was obtained from (E)-3-[4-(trifluoromethanesulfonyl)phenyl]-2-propen-1-ol in a similar manner to Step 4 of Reference example 40.
$^1$H NMR ($CDCl_3$, δ ppm): 4.41-4.45 (m, 2H), 6.61 (td, J = 4.8, 15.9 Hz, 1H), 6. 75 (d, J = 15. 9 Hz, 1H), 7.63 (d, J = 8.5 Hz, 2H), 7.98 (d, J = 8.5 Hz, 2H).

Step 4

(E)-3-[4-(Trifluoromethanesulfonyl)phenyl]propenal (99.8 mg, 0.378 mmol), iodobenzene (123 mg, 0.601 mmol), palladium acetate (10.8 mg, 0.0481 mmol), tetrabutylammonium bromide (125 mg, 0.387 mmol), and sodium acetate (49.5 mg, 0.603 mmol) were dissolved in DMF (3.0 mL), and the mixture was stirred at 70˚C for 8 hours. After the reaction mixture was left to cool, a saturated aqueous sodium hydrogen carbonate solution was added thereto, and the mixture was extracted with ethyl acetate. The organic layer was washed with saturated brine and dried over anhydrous magnesium sulfate. Then, the solvent was evaporated under reduced pressure, and the residue was purified by silica gel column chromatography (hexane/ethyl acetate = 8/2) to give (E)-3-phenyl-3-[4-(trifluoromethanesulfonyl)phenyl] propenal (75.1 mg, 75%). $^1$H NMR ($CDCl_3$, δ ppm): 6.72 (d, J = 8.1 Hz, 1H), 7.26-7.53 (m, 5H), 7.63-7.66 (m, 2H), 8.16 (d, J = 8.2 Hz, 2H), 9.47 (d, J = 8.1 Hz, 1H).

Step 5

Methyl (E)-5-phenyl-5-[4-(trifluoromethanesulfonyl)phenyl]-2,4-pentadienoate was obtained from (E)-3-phenyl-3-[4-(trifluoromethanesulfonyl)phenyl]propenal in a similar manner to Step 5 of Reference example 40.

Step 6

Compound fm was obtained from methyl (E)-5-phenyl-5-[4-(trifluoromethanesulfonyl)phenyl]-2,4-pentadienoate in a similar manner to Step 2 of Reference example 18. $^1$H NMR ($CDCl_3$, δ ppm): 6.25 (d, J = 15.0 Hz, 1H), 6.94 (dd, J = 11.6, 15.0 Hz, 1H), 7.22-7.33 (m, 3H), 7.40-7.42 (m, 3H), 7.69 (d, J = 8.4 Hz, 2H), 8.27 (d, J = 8.4 Hz, 2H).

Reference example 156: (2E,4Z)-5-[2-(Piperidin-1-yl)thiazol-4-yl]phenyl-5-[4-(trifluoromethyl)phenyl]-2,4-pentadienoic acid (Compound fn)

Step 1

Compound q (1.55 g, 4.43 mmol), tetrakis(triphenylphosphine)palladium (513 mg, 0.443 mmol), and tributyl (1-ethoxyvinyl)tin (2.05 g, 5.68 mmol) were dissolved in toluene (46 mL), and the mixture was heated under reflux for 2 hours. After the reaction mixture was left to cool, a saturated aqueous sodium hydrogen carbonate solution was added thereto, and the mixture was extracted with ethyl acetate. The organic layer was washed with saturated brine and dried over anhydrous magnesium sulfate. Then, the solvent was evaporated under reduced pressure, and the residue was purified by silica gel column chromatography (hexane/ethyl acetate = 19/1) to give ethyl (Z)-6-ethoxy-5-[4-(trifluoromethyl) phenyl]-2,4,6-heptatrienoate (1.28 g, 85%).

Step 2

Ethyl (Z)-6-ethoxy-5-[4-(trifluoromethyl)phenyl]-2,4,6-heptatrienoate (1.28 g, 3.77 mmol) was dissolved in THF (64 mL), and 2 mol/L hydrochloric acid (38 mL) was added thereto, and then, the mixture was stirred at room temperature for 30 minutes. Water was added to the reaction mixture, and the mixture was extracted with ethyl acetate. The organic layer was washed with saturated brine and dried over anhydrous magnesium sulfate. Then, the solvent was evaporated under reduced pressure, and the residue was purified by silica gel column chromatography (hexazae/ethyl acetate = 9/1) to give ethyl (2E,4Z)-5-acetyl-5-[4-(trifluoromethyl)phenyl]-2,4-pentadienoate (883 mg, 75%).
$^1$H NMR ($CDCl_3$, δ ppm) : 1.32 (t, J = 7.1 Hz, 3H), 2.33 (s, 3H), 4.24 (q, J = 7.1 Hz, 2H), 6.14 (dd, J = 0.7, 15.3 Hz, 1H), 6.58 (dd, J = 0.7, 11.8 Hz, 1H), 7.45 (dd, J = 0.7, 8.1 Hz, 2H), 7.54 (dd, J = 11.8, 15.3 Hz, 1H), 7.67 (dd, J = 0.7, 8.7 Hz, 2H).

Step 3

Ethyl (2E,4Z)-5-acetyl-5-[4-(trifluoromethyl)phenyl]-2,4-pentadienoate (221 mg, 0.707 mmol) was dissolved in THF (6.6 mL), and pyrrolidone hydrotribromide (428 mg, 0.863 mmol) was added thereto, and then, the mixture was stirred at room temperature for 24 hours. Water was added to the reaction mixture, and the mixture was extracted with ethyl acetate. The organic layer was washed with saturated brine and dried over anhydrous magnesium sulfate. Then, the solvent was evaporated under reduced pressure, and the residue was purified by silica gel column chromatography (hexane/ethyl acetate = 9/1) to give ethyl (2E,4Z)-5-(bromoacetyl)-5-[4-(trifluoromethyl)phenyl]-2,4-pentadienoate (165 mg, 60%).

Step 4

Ethyl (2E,4Z)-5-(bromoacetyl)-5-[4-(trifluoromethyl)phenyl]-2,4-pentadienoate (80.4 mg, 0.206 mmol) was dissolved in ethanol (2.4 mL), and 1-piperidinethiocarboxamide (36.7 mg, 0.254 mmol) was added thereto, and then, the mixture was stirred at 60˚C for 1 hour. To the reaction mixture, a saturated aqueous sodium hydrogen carbonate solution was added, and the mixture was extracted with chloroform. The organic layer was washed with saturated brine and dried over anhydrous magnesium sulfate. Then, the solvent was evaporated under reduced pressure, and the residue was purified by silica gel column chromatography (hexane/ethyl acetate = 9/1) to give ethyl (2E,4Z)-5-[2-(piperidin-1-yl)thiazol-4-yl]phenyl-5-[4-(trifluoromethyl)phenyl]-2,4-pentadienoate (54.2 mg, 60%).

Step 5

Compound fn was obtained from ethyl (2E,4Z)-5-[2-(piperidin-1-yl)thiazol-4-yl]phenyl-5-[4-(trifluoromethyl)phenyl]-2,4-pentadienoate in a similar manner to Step 2 of Reference example 18.
$^1$H NMR (DMSO-$d_6$, δ ppm): 1.61-1.62 (m, 6H), 3.47-3.48 (m, 4H), 6.12 (d, J = 15.1 Hz, 1H), 6.22 (s, 1H), 6.87 (dd, J = 12.1, 15.1 Hz, 1H), 7.34 (d, J = 12.1 Hz, 1H), 7.50 (d, J = 8.1 Hz, 2H), 7.86 (d, J = 8.1 Hz, 2H).

Reference example 157: (2E,4E)-5-(4-Fluorophenyl)-5-[2-(piperidin-1-yl)-6-(trifluoromethyl)pyridin-3-yl]-2,4-pentadienoic acid (Compound fo)

Step 1

Commercially available 2-chloro-6-(trifluoromethyl) nicotinic acid (2.02 g, 8.97 mmol) was dissolved in piperidine (5.7 mL), and the mixture was stirred at room temperature for 30 minutes. To the reaction mixture, 1.0 mol/L hydrochloric acid was added, and the mixture was extracted with ethyl acetate. The organic layer was washed with saturated brine and dried over anhydrous magnesium sulfate. Then, the solvent was evaporated under reduced pressure to give 2-(piperidin-1-yl)-6-(trifluoromethyl) nicotinic acid (2.41 g, 98%).

Step 2

N-Methoxy-N-methyl-3-[2-(piperidin-1-yl)-6-(trifluoromethyl)pyridine]carboxamide was obtained in a similar manner to Example 10 using N,O-dimethylhydroxylamine hydrochloride in place of 5-aminoisoquinoline.
$^1$H NMR (DMSO-$d_6$, $\delta$ ppm): 1.62-1.63 (m, 6H), 3.29 (s, 3H), 3.46-3.47 (m, 7H), 6.99 (d, J = 7.4 Hz, 1H), 7.59 (d, J = 7.4 Hz, 1H).

Step 3

N-Methoxy-N-methyl-3-[2-(piperidin-1-yl)-6-(trifluoromethyl)pyridine]carboxamide (535 mg, 1.69 mmol) was dissolved in THF (10 mL), and a 1.00 mol/L solution of 4-fluorophenyl magnesium bromide in THF (5.00 mL, 5.00 mmol) was added thereto, and then, the mixture was stirred at room temperature for 24 hours. To the reaction mixture, a saturated aqueous ammonium chloride solution was added, and the mixture was extracted with chloroform. The organic layer was washed with saturated brine and dried over anhydrous magnesium sulfate. After the organic layer was filtered, the solvent was evaporated under reduced pressure, and then, the residue was purified by silica gel column chromatography (hexane/ethyl acetate = 9/1) to give 3-(4-fluorobenzoyl)-2-(piperidin-1-yl)-6-(trifluoromethyl)pyridine (330 mg, 55%).

Step 4

3-(4-Fluorobenzoyl)-2-(piperidin-1-yl)-6-(trifluoromethyl)pyridine (400 mg, 1.14 mmol) and triethyl phosphonoacetate (2.30 mL, 11.6 mmol) were dissolved in toluene (20 mL), and sodium hydride (276 mg, 11.5 mmol) was added thereto, and then, the mixture was stirred at 100°C for 5 days. After the reaction mixture was left to cool, water was added thereto, and the mixture, was extracted with ethyl acetate. The organic layer was washed with saturated brine and dried over anhydrous magnesium sulfate. After the organic layer was filtered, the solvent was evaporated under reduced pressure, and then, the residue was purified by silica gel column chromatography (hexaxae/ethyl acetate = 19/1) to give ethyl (E)-3-(4-fluorophenyl)-3-[2-(piperidin-1-yl)-6-(trifluoromethyl)pyridin-3-yl]acrylate (205 mg, 43%).
$^1$H NMR ($CDCl_3$, $\delta$ ppm): 1.24-1.35 (m, 3H), 1.55-1.56 (m, 6H), 4.01-4.32 (m, 6H), 6.20 (s, 1H), 6.38 (dd, J = 0.8, 15.3 Hz, 1H), 7.08 (dd, J = 11.6, 15.3 Hz, 1H), 7.37-7.56 (m, 2H), 7.74 (dd, J = 0.8, 8.6 Hz, 2H).

Step 5

(E)-3-(4-Fluorophenyl)-3-[2-(piperidin-1-yl)-6-(trifluoromethyl)pyridin-3-yl]-2-propen-1-ol was obtained from ethyl (E)-3-(4-fluorophenyl)-3-[2-(piperidin-1-yl)-6-(trifluoromethyl)pyridin-3-yl]acrylate in a similar manner to Step 3 of Reference example 40.

Step 6

(E)-3-(4-Fluorophenyl)-3-[2-(piperidin-1-yl)-6-(trifluoromethyl)pyridin-3-yl]propenal was obtained from (E)-3-(4-fluorophenyl)-3-[2-(piperidin-1-yl)-6-(trifluoromethyl)pyridin-3-yl]-2-propen-1-ol in a similar manner to Step 4 of Reference example 40.
$^1$H NMR ($CDCl_3$, $\delta$ ppm): 1.21-1.50 (m, 6H), 3.26-3.29 (m, 4H), 6.53 (d, J = 7.7 Hz, 1H), 7.07-7.38 (m, 5H), 7.46 (d, J = 7.9 Hz, 1H), 9.61 (d, J = 8.0 Hz, 1H).

Step 7

Methyl (E)-5-(4-fluorophenyl)-5-[2-(piperidin-1-yl)-6-(trifluoromethyl)pyridin-3-yl]-2,4-pentadienoate was obtained from (E)-3-(4-fluorophenyl)-3-[2-(piperidin-1-yl)-6-(trifluoromethyl)pyridin-3-yl]propenal in a similar manner to Step 5 of Reference example 40.

Step 8

Compound fo was obtained from methyl (E)-5-(4-fluorophenyl)-5-[2-(piperidin-1-yl)-6-(trifluoromethyl)pyridin-3-yl]-2,4-pentadienoate in a similar manner to Step 2 of Reference example 18.
$^1$H NMR (DMSO-$d_6$, δ ppm): 1.18-1.38 (m, 6H), 3.14-3.22 (m, 4H), 6.20 (d, J = 14.1 Hz, 1H), 7.06-7.38 (m, 7H), 7.55 (d, J = 7.5 Hz, 1H).

Reference example 158: (2E,4Z)-5-[6-(3-Methoxy-1-methylpropyl)pyridin-3-yl]-5-[4-(trifluoromethyl)phenyl]-2,4-pentadienoic acid (Compound fp)

Compound fp was obtained from Compound dk in a similar manner to Reference example 93 using N-(2-methoxyethyl)methylamine in place of piperidine.
$^1$H NMR ($CD_3OD$, δ ppm): 3.22 (s, 3H), 3.37 (s, 3H), 3.66 (t, J = 5.3 Hz, 2H), 3.84 (t, J = 5.3 Hz, 2H), 6.19 (d, J = 15.2 Hz, 1H), 6.98-7.02 (m, 2H), 7.38-7.48 (m, 2H), 7.56 (d, J = 8.4 Hz, 2H), 7.68 (d, J = 8.4 Hz, 2H), 7.88 (d, J = 2.0 Hz, 1H).

Reference example 159: (2E,4Z)-5- [4- (2-Dimethylaminoethoxy)phenyl]-5-[4-(trifluoromethyl)phenyl]-2,4-pentadienoic acid (Compound fq)

Compound fq was obtained from Compound ch in a similar manner to Reference example 73 using 2-dimethylaminoethanol.
$^1$H NMR (DMSO-$d_6$, δ ppm): 2.85 (s, 6H), 3.52 (t, J = 4.8 Hz, 2H), 4.42 (t, J = 4.8 Hz, 2H), 6.14-6.24 (m, 1H), 7.11-7.18 (m, 6H), 7.50 (d, J = 8.3 Hz, 2H), 7.73 (d, J = 8.3 Hz, 2H).

Reference example 160: (2E,4E)-5-(4-Chloraphenyl)-5-[4-(piperidin-1-yl)phenyl]-2,4-pentadienoic acid (Compound fr)

Compound fr was synthesized in a similar manner to Reference example 18 using Compound bb and 4-(piperidine-1-yl)phenylboronic acid.
$^1$H NMR (DMSO-$d_6$, δ ppm): 1.64-1.84 (m, 6H), 3.45-3.46 (m, 4H), 6.16 (d, J = 14.3 Hz, 1H), 7.05-7.28 (m, 4H), 7.30 (d, J = 8.6 Hz, 2H), 7.45 (d, J = 8.6 Hz, 2H), 7.43-7.60 (m, 2H).

Reference example 161: (2E,4Z)-5-[4-(2-Methoxyethoxy)phenyl]-5-[4-(trifluoromethyl)phenyl]-2,4-pentadienoic acid (Compound fs)

Compound fs was obtained from Compound ch in a similar manner to Reference example 73 using 2-methoxyethanol.
$^1$H NMR (DMSO-$d_6$, δ ppm): 3.33 (s, 3H), 3.68-3.71 (m, 2H), 4.15-4.18 (m, 2H), 6.18 (d, J = 13.9 Hz, 1H), 7.05-7.20 (m, 6H), 7. 51 (d, J = 8.1 Hz, 2H), 7.73 (d, J = 8.1 Hz, 2H), 12.33 (br s, 1H).

Reference example 162: (E)-5,5-Bis[6-(piperidin-1-yl)pyridin-3-yl]-2,4-pentadienoic acid (Compound ft)

Compound ft was synthesized in a similar manner to Reference example 2 using Compound a and 6-(1-piperidinyl)-3-pyridinylboronic acid.
$^1$H NMR (DMSO-$d_6$, δ ppm): 1.54-1.50 (m, 12H), 3.57-3.58 (m, 8H), 5.99 (d, J = 15.0 Hz, 1H), 6.78-6.90 (m, 3H), 7.15 (dd, J = 11.6, 15.0 Hz, 1H), 7.28 (dd, J = 2.4, 8.8 Hz, 1H), 7.45 (dd, J = 2.5, 9.1 Hz, 1H), 7.90 (d, J = 2.2 Hz, 1H), 8.01 (d, J = 2.4 Hz, 1H).

Reference example 163: (2E,4Z)-5-(4-Propoxyphenyl)-5-[4-(trifluoromethyl)phenyl]-2,4-pentadienamide (Compound fu)

Compound fu was obtained from Compound ch in a similar manner to Reference example 73 using propanol.
$^1$H NMR (DMSO-$d_6$, δ ppm): 1.01 (t, J = 7.3 Hz, 3H), 1.77 (sext, J = 7.3 Hz, 2H), 4.00 (t, J = 7.3 Hz, 2H), 6.18 (d, J = 14.3 Hz, 1H), 7.03-7.21 (m, 6H), 7.51 (d, J = 8.2 Hz, 2H), 7.73 (d, J = 8.2 Hz, 2H), 12.34 (br s, 1H).

Reference example 164: (2E,4E)-5-[2-(Dimethylamino)-6-(trifluoromethyl)pyridin-3-yl]-5-(4-fluorophenyl)-2,4-pentadienoic acid (Compound fv)

Compound fv was obtained in a similar manner to Reference example 157 using dimethylamine.
$^1$H NMR (DMSO-$d_6$, δ ppm): 2.80 (s, 6H), 6.19 (d, J = 14.6 Hz, 1H), 6.99-7.51 (m, 8H), 12.38 (br s, 1H).

Reference example 165: (2E,4E)-5-(4-Chlorophenyl)-5-[4-(trifluoromethoxy)phenyl]-2,4-pentadienoic acid (Compound fw)

Compound fw was synthesized in a similar manner to Reference example 18 using Compound bb and 4-(trifluoromethoxy)phenylboronic acid.
$^1$H NMR (DMSO-$d_6$, δ ppm) : 6.19 (d, J = 14.8 Hz, 1H), 6.99 (dd, J = 11.7, 14.8 Hz, 1H), 7.15 (d, J = 11.7 Hz, 1H), 7.30-7.36 (m, 4H), 7.44-7.52 (m, 4H), 12.36 (br s, 1H).

Reference example 166: (2E,4Z)-5-(6-Propoxypyridin-3-yl)-5-[4-(trifluoromethyl)phenyl] -2,4-pentadienoic acid (Compound fx)

Compound fx was obtained from Compound dk in a similar manner to Reference example 93 using propanol in place of piperidine.
$^1$H NMR (DMSO-$d_6$, δ ppm): 1.06 (t, J = 7.0 Hz, 3H), 1.82 (sext, J = 7.0 Hz, 2H), 4.29 (t, J = 7.0 Hz, 2H), 6.07 (dd, J = 1.0, 15.6 Hz, 1H), 6.68-6.86 (m, 2H), 7.26-7.52 (m, 4H), 7.60 (d, J = 9.1 Hz, 1H), 7.70 (d, J = 9.1 Hz, 1H) 8.03 (d, J = 2.8 Hz, 1H).

Reference example 167: (2E,42)-5-(1-Methyl-6-oxo-1,6-dihydropyridin-3-yl)-5-[4-(trifluoromethyl)phenyl]-2,4-pentadienoic acid (Compound fy)

Step 1

Commercially available 5-bromo-2(1H)-pyridone (1.01 g, 5.79 mmol), methyl iodide (1.80 mL, 28.9 mmol), and potassium carbonate (4.01 g, 29.0 mmol) were dissolved in acetonitrile (30 mL), and the mixture was stirred at room temperature for 3 hours. The solvent was evaporated under reduced pressure, and the residue was purified by silica gel column chromatography (chloroform/methanol = 9/1) to give 5-bromo-1-methyl-2(1H)-pyridone (1.05 g, 96%). $^1$H NMR ($CDCl_3$, δ ppm): 3.53 (s, 3H), 6.50 (d, J = 8.6 Hz, 1H), 7.35 (dd, J = 2.4, 8.6 Hz, 1H), 7.42 (d, J = 2.4 Hz, 1H).

Step 2

Compound fy was synthesized in a similar manner to Reference example 18 using Compound ez and 5-bromo-1-methyl-2(1H)-pyridone.
$^1$H NMR (DMSO-$d_6$, δ ppm): 3.48 (s, 3H), 6.22 (d, J = 14.7 Hz, 1H), 6.48 (d, J = 9.4 Hz, 1H), 7.07 (d, J = 11.5 Hz, 1H), 7.19-7.29 (m, 2H), 7.61 (d, J = 8.2 Hz, 2H), 7.74-7.77 (m, 3H), 12.39 (br s, 1H).

Reference example 168: (2E,4Z)-5-[4-(Thiethan-3-yloxy)phenyl]-5-[4-(trifluoromethyl)phenyl]-2,4-pentadienoic acid (Compound fz)

Compound fz was obtained from Compound ch in a similar manner to Reference example 73 using thiethan-3-ol prepared according to the method described in the article [Chemical Reviews, vol. 102, pp. 29-60 (2002)].
$^1$H NMR (DMSO-$d_6$, δ ppm) : 3.45-3.50 (m, 2H), 3.53-3.59 (m, 2H), 5.42-5.47 (m, 1H), 6.13 (d, J = 15.4 Hz, 1H), 6.93-6.99 (m, 3H), 7.14 (d, J = 8.8 Hz, 2H), 7.49 (d, J = 8.1 Hz, 2H), 7.55-7.73 (m, 1H), 7.62 (d, J = 8.2 Hz, 2H).

Reference example 169: (2E,4Z)-5-[4-(Oxetan-3-yloxy)phenyl]-5-[4-(trifluoromethyl)phenyl-2,4-pentadienoic acid (Compound ga)

Compound ga was obtained from Compound ch in a similar manner to Reference example 73 using oxetan-3-o1 prepared according to the method described in the article [Chemical Reviews, vol. 102, pp. 29-60 (2002)].
$^1$H NMR (DMSO-$d_6$, δ ppm): 4.59-4.63 (m, 2H), 4.93-4.98 (m, 2H), 5.37 (quint, J = 5.9 Hz, 1H), 6.19 (d, J = 14.2 Hz, 1H), 6.92 (d, J = 8.6 Hz, 2H), 7.10-7.15 (m, 4H), 7.50 (d, J = 8.2 Hz, 2H), 7.73 (d, J = 8.2 Hz, 2H), 12.33 (br s, 1H).

Reference example 170: (2E,4Z)-5-(Cyclobutoxyphenyl)-5-[4-(trifluoromethyl)phenyl]-2,4-pentadienoic acid (Compound ha)

Compound ha was obtained from Compound cl in a similar manner to Reference example 73.
ESIMS m/z: $[M+H]^-$ 387.

Reference example 171: (2E,4Z)-5-(4-Cyclopropoxyphenyl)-5-[4-(trifluoromethyl)phenyl]-2,4-pentadienoic acid (Compound hb)

Compound hb was synthesized in a similar manner to Reference example 18 using Compound q and 4-cyclopropoxyphenylboronic acid.
$^1$H NMR (DMSO-$d_6$, δ ppm): 0.69-0.76 (m, 2H), 0.78-0.85 (m, 2H), 3.89-3.95 (m, 1H), 6.19 (d, J = 13.9 Hz, 1H), 7.07-7.21 (m, 6H), 7.51 (d, J = 8.3 Hz, 2H), 7.74 (d, J = 8.3 Hz, 2H), 12.35 (br s, 1H).

Reference example 172: (2E,4Z)-5-[4-(Pentafluorosulfanyl)phenyl]-5-[4-(trifluoromethyl)phenyl]-2,4-pentadienoic acid (Compound hc)

Compound hc was synthesized in a similar manner to Reference example 18 using Compound ez and 1-promo-4-(pentafluorosulfanyl)benzene.
ESIMS m/z: $[M+H]^-$ 443.

Reference example 173: (2E,4Z)-5-[4-(1-Ethylpropoxy)phenyl]-5-[4-(trifluoromethyl)phenyl]-2,4-pentadienoic acid (Compound hd)

Compound hd was synthesized from Compound ch in a similar manner to Reference example 73 using 1-ethylpropanol.
$^1$H NMR (DMSO-$d_6$, δ ppm) : 0.94 (t, J = 6.6 Hz, 6H), 1.66 (quint, J = 6.6 Hz, 4H), 4.30 (quint, J = 6.6 Hz, 1H), 6.19 (d, J = 14.6 Hz, 1H), 7.02-7.24 (m, 6H), 7.51 (d, J = 8.2 Hz, 2H), 7.73 (d, J = 8.2 Hz, 2H), 12.32 (br s, 1H).

Reference example 174: (E)-5,5-Bis(4-ethoxyphenyl)-2,4-pentadienoic acid (Compound he)

Compound he was synthesized in a similar manner to Reference example 2 using Compound a and 4-ethoxyphenylboronic acid.
$^1$H NMR (DMSO-$d_6$, δ ppm): 1.32 (t, J = 6.9 Hz, 3H), 1.36 (t, J = 6.9 Hz, 3H), 4.03 (q, J = 6.9 Hz, 2H), 4. 09 (q, J = 6.9 Hz, 2H), 6.04 (d, J = 15.0 Hz, 1H), 6.86-6.92 (m, 3H), 7.00-7.16 (m, 5H), 7.23 (d, J = 8.9 Hz, 2H), 12.12 (br s, 1H).

Reference example 175: (2E,4E)-5-(4-Ethoxyphenyl)-5-[4-(trifluoromethoxy)phenyl]-2,4-pentadienoic acid (Compound hf)

Compound hf was synthesized in a similar manner to Reference example 18 using Compound ax and 4-ethoxyphenylboronic acid.
$^1$H NMR (DMSO-$d_6$, δ ppm): 1.36 (t, J = 6.9 Hz, 3H), 4.10 (q, J = 6.9 Hz, 2H), 6.13 (d, J = 14.8 Hz, 1H), 6.97-7.19 (m, 6H), 7.34-7.44 (m, 4H), 12.29 (br s, 1H).

Reference example 176: 4-Amino-2-[2-(morpholino-4-yl)ethoxy]phenol (for use in the synthesis of Compound 118)

Step 1

Commercially available 2-methoxy-5-nitrophenol (900 mg, 5.3 mmol) was dissolved in DMF (6.0 mL), and sodium hydride (640 mg) and N-(2-chloroethyl)morpholine hydrochloride (1.49 g) were added thereto under ice-cooling, and then, the mixture was stirred at 110°C for 4.5 hours. Water was added to the reaction mixture, and the mixture was extracted with ethyl acetate. After the organic layer was dried over anhydrous magnesium sulfate, the solvent was evaporated under reduced pressure, and then, the residue was purified by silica gel column chromatography to give 2-(2-morpholino-4-ylethoxy)-4-nitroanisole (1.43 g, 95%).

Step 2

4-Methoxy-3-(2-morpholino-4-ylethoxy)nitrobenzene (405 mg, 1.4 mmol) was dissolved in DMF (3.0 mL), and

1-dodecanthiol (2.92 g) and sodium methoxide (778 mg) were added thereto, and then, the mixture was stirred at 80˚C for 5 hours. Water was added to the reaction mixture, and the mixture was extracted with ethyl acetate. The organic layer was washed with saturated brine and dried over anhydrous magnesium sulfate. Then, the solvent was evaporated under reduced pressure, and the residue was purified by silica gel column chromatography (hexane/ethyl acetate = 19/1) to give 4-nitro-2-(2-morpholino-4-ylethoxy)phenol (275 mg, 71%).

Step 3

4-Nitro-2-(2-morpholino-4-ylethoxy)phenol (268 mg, 1.0 mmol) was dissolved in DMF (4.0 mL), and 10% palladium carbon (50 mg) was added thereto, and then, the mixture was stirred at 60˚C for 4 hours under hydrogen atmosphere. After the mixture was filtered through Celite, the solvent was evaporated to give 4-amino-2-[2-(morpholino-4-yl)ethoxy]phenol (207 mg, 87%).
ESIMS m/z: $[M+H]^+$ 239

Reference example 177: 2-Aminopyrazine-3-methanol (for use in the synthesis of Compound 131)

Step 1

Aluminum lithium hydride (760 mg) was suspended in THF (50 mL), and commercially available 2-aminopyrazine-3-carboxylic acid (1.5 g, 10 mmol) was added thereto under ice-cooling, and then, the mixture was heated at 60˚C for 4 hours. After the reaction mixture was cooled to 0˚C, sodium sulfate 10-hydrate (6.0 g) was added thereto, and the mixture was stirred at room temperature for 1 hour. After the mixture was filtered through Celite, the solvent was evaporated under reduced pressure, and then, the residue was purified by silica gel column chromatography to give 2-aminopyrazine-3-methanol (655 mg, 52%).
ESIMS m/z: $[M+H]^+$ 126

Reference example 178: 5-(2-Aminophenyl)-3H-[1,3,4] oxadiazol-2-one (for use in the synthesis of Compound 133)
Step 1

After˚ a mixture of chloroform (10 mL) and a saturated sodium hydrogen carbonate solution (10 mL) was cooled to 0˚C, hydrazine monohydrate (0.59 mL) and 2-nitrobenzoyl chloride (1.5 g, 10 mmol) were added thereto, and the mixture was stirred at room temperature for 30 minutes. After the organic layer was separated, the aqueous layer was washed with chloroform. After the organic layer was dried over anhydrous magnesium sulfate, the solvent was evaporated under reduced pressure to give 2-nitrobenzoyl hydrazide (800 mg, 55%).

Step 2

2-Nitrobenzoyl hydrazide (800 mg, 4.4 mmol) was dissolved in DMF (20 mL), and 1,1-carbonyldiimidazole (1.0 g) was added thereto, and then, the mixture was stirred overnight at 80˚C. After the solvent was evaporated, the residue was purified by silica gel column chromatography to give 5- (2-nitrophenyl)-3H-[1,3,4]oxadiazol-2-one (450 mg, 49%).

Step 3

5- (2-Nitrophenyl) -3H- [1,3,4]oxadiazol-2-one (450 mg, 2.2 mmol) was dissolved in DMF (20 mL), and 10% palladium carbon (45 mg) was added thereto, and then, the mixture was stirred at room temperature for 6 hours. The mixture was filtered through Celite, and the solvent was evaporated to give 5-(2-aminophenyl)-3H-[1,3,4]oxadiazol-2-one (315 mg, 82%).
ESIMS m/z: $[M+H]^+$ 178

Reference example 179: tert-Butyl 3-(2-aminophenyl) pyrazole-1-carboxylate (for use in the synthesis of Compound 136)

Commercially available 2-(1H-pyrazol-3-yl)aniline (537 mg, 3.4 mmol) was dissolved in dichloromethane (5.0 mL), and di-tert-butyl dicarboxylate (770 mg) and 4-dimethylaminopyridine (10 mg) were added thereto, and then, the mixture was stirred overnight at room temperature. The reaction mixture was extracted by adding water and ethyl acetate thereto. Then, the organic layer was dried over anhydrous magnesium sulfate, and the solvent was evaporated. Then, the residue was purified by silica gel column chromatography to give tert-butyl 3-(2-aminophenyl) pyrazole-1-carboxylate (247 mg, 27%).
ESIMS m/z: $[M+H]^+$ 260

Reference example 180: (4-Aminobenzimidazol-2-yl)methanol (for use in the synthesis of Compound 142)

(4-Nitrobenzimidazol-2-yl)methanol (206 mg, 1.1 mmol) prepared according to the method described in the article [Chemical & Pharmaceutical Bulltin, vol. 43, pp. 493-498 (1995)] was dissolved in ethanol (4.0 mL), and tin(II) chloride dihydrate (1.0 g) was added thereto, and then, the mixture was stirred at 70˚C for 3 hours. Ice water was poured into the mixture, and the mixture was neutralized with a saturated sodium hydrogen carbonate solution, and then, the mixture was extracted with ethyl acetate. The organic layer was dried over anhydrous magnesium sulfate, and the solvent was evaporated to give (4-aminobenzimidazol-2-yl)methanol (102 mg, 63%).
ESIMS m/z: $[M+H]^+$

Reference example 181: 5-(2-Aminobenzylidene)thiazolidine-2,4-dione (for use in the synthesis of Compound 143)

Step 1

2-Nitrobenzaldehyde (1.5 g, 10 mmol), thiazolidine-2,4-dione (1.2 g), and sodium acetate (2.6 g) were stirred overnight in acetic acid (6.0 mL) under reflux. Water was added to the reaction mixture, and the obtained crystal was collected by filtration, and recrystallized from pyridine-ethanol to give 5-(2-nitrobenzylidene)thiazolidine-2,4-dione (810 mg, 63%).

Step 2

5-(2-Nitrobenzylidene)thiazolidine-2,4-dione (344 mg, 1.4 mmol) was dissolved in ethyl acetate (10 mL), and 10% palladium carbon (70 mg) was mixed therein, and then, the mixture was stirred overnight at 50˚C under hydrogen atmosphere. After the reaction mixture was filtered through Celite, the solvent was evaporated to give 5-(2-aminobenzylidene)thiazolidine-2,4-dione (310 mg, quantitative yield).

Reference example 182: Quinazoline-2,8-diamine (for use in the synthesis of Compounds 144 and 147)

Step 1

Commercially available 2-chloro-3-nitrobenzoic acid (2.0 g, 10 mmol), oxalyl chloride (5.0 mL), and DMF (1.0 mL) were mixed, and the mixture was stirred at 60˚C for 2 hours. After the reaction mixture was concentrated under reduced pressure, the residue was cooled to 0˚C and dissolved in DMF (15 mL). Then, sodium borohydride (400 mg) was added thereto, and the mixture was stirred overnight at room temperature. After ice water was added to the reaction mixture, the mixture was extracted with ethyl acetate, and the organic layer was dried over anhydrous magnesium sulfate. Then, the solvent was evaporated, and the residue was purified by silica gel column chromatography to give 2-chloro-3-nitrobenzyl alcohol (1.5 g, 78%).

Step 2

2-Chloro-3-nitrobenzyl alcohol (1.5 g, 7.8 mmol) was dissolved in chloroform (30 mL), and manganese dioxide (9.0 g) was mixed therein, and then, the mixture was stirred overnight at 40˚C. After the reaction mixture was filtered through Celite, the solvent was evaporated to give 2-chloro-3-nitrobenzaldehyde (1.3 g, 89%).

Step 3

2-Chloro-3-nitrobenzaldehyde (1.1 g, 5.9 mmol) was dissolved in dimethylacetamide (5.0 mL), and guanidine carbonate (3.2 g) was added thereto at 160˚C. After 20 minutes, the mixture was cooled to room temperature and water was added thereto, and the precipitated crystal was collected by filtration to give 2-amino-8-nitroquinazoline (680 mg, 60%).

Step 4

2-Amino-8-nitroquinazoline (190 mg, 1.0 mmol) was dissolved in DMF (2.0 mL) and ethanol (2.0 mL), and tin (II) chloride dihydrate (1.0 g) was added thereto, and then, the mixture was stirred at 70˚C for 1 hour. Ice water was poured into the mixture, and the mixture was neutralized with a saturated sodium hydrogen carbonate solution, and then, the mixture was extracted with ethyl acetate. The organic layer was dried over anhydrous magnesium sulfate, and the solvent was evaporated to give quinazoline-2,8-diamine (157 mg, 98%).

ESIMS m/z: $[M+H]^+$ 161

Reference example 183: (5-Aminobenzimidazol-2-yl)methanol (for use in the synthesis of Compound 145)

(5-Aminobenzimidazol-2-yl)methanol was synthesized from (5-nitrobenzimidazol-2-yl)methanol obtained according to the method discribed in Reference example 180.
ESIMS m/z: $[M+H]^+$ 164

Reference example 184: 5-Amino-2H-isoquinolin-1-one (for use in the synthesis of Compound 146)

Step 1

Commercially available 5-nitroisoquinoline (5.6 g, 32 mmol) was dissolved in dichloromethane (80 mL), and methyltrioxorhenium (380 mg) and a hydrogen peroxide solution (30%, 40 mL) were added thereto, and then, the mixture was stirred at room temperature for 1.5 hours. The reaction mixture was extracted with chloroform, and the organic layer was dried over anhydrous magnesium sulfate. Then, the solvent was evaporated to give 5-nitroisoquinoline-N-oxide (5.9 g, 97%).

Step 2

5-Nitroisoquinoline-N-oxide (1.0 g, 5.3 mmol) was mixed with acetic anhydride (5.0 mL), and the mixture was stirred overnight under reflux. The reaction mixture was concentrated under reduced pressure, and a 10% aqueous potassium carbonate solution was added to the residue, and then, the mixture was stirred for 30 minutes. The formed crystal was collected by filtration to give 5-nitro-2H-isoquinolin-1-one (820 mg, 82%).

Step 3

5-Nitro-2H-isoquinolin-1-one (1.1 g, 5.5 mmol) was dissolved in DMF (15 mL), and 10% palladium carbon (100 mg) was added thereto, and then, the mixture was stirred overnight at 50˚C. After the reaction mixture was filtered through Celite, the solvent was evaporated, and the residue was purified by silica gel column chromatography to give 5-amino-2H-isoquinolin-1-one (620 mg, 70%).
ESIMS m/z: $[M+H]^+$ 161

Reference example 185: 5-Aminoisoquinoline-1-carbonitrile (for use in the synthesis of Compound 151)

Step 1

5-Nitroisoquinoline-N-oxide (500 mg, 2.6 mmol) obtained in Step 1 of Reference example 184 was dissolved in chloroform, and potassium cyanide (760 mg) was added thereto under ice-cooling. Then, benzoyl chloride was added thereto, and the mixture was stirred for 1 hour. To the reaction mixture, a saturated aqueous potassium carbonate solution was added, and the mixture was extracted with chloroform. Then, the organic layer was dried over anhydrous magnesium sulfate, and the solvent was evaporated. Then, the residue was purified by silica gel column chromatography to give 5-nitroisoquinoline-1-carbonitrile (315 mg, 60%).

Step 2

5-Nitroisoquinoline-1-carbonitrile (315 mg, 1.6 mmol) was mixed with DMF (4.0 mL) and ethanol (4.0 mL), and tin(II) chloride dihydrate (1.4 g) was added thereto, and then, the mixture was stirred at 70˚C for 1.5 hours. The reaction mixture was poured into ice water, and the mixture was extracted by adding a saturated sodium hydrogen carbonate solution and ethyl acetate thereto. After the organic layer was dried over anhydrous magnesium sulfate, the solvent was evaporated, and the residue was purified by silica gel column chromatography to give 5-aminoisoquinoline-1-carbonitrile (146 mg, 56%).
ESIMS m/z: $[M+H]^+$ 170

Reference example 186: (6-Amino-3,4-dihydro-2H-benzo[1,4]oxazin-2-yl)methanol (for use in the synthesis of Compound 159)

(6-Nitro-3,4-dihydro-2H-benzo[1,4]oxazin-2-yl)methanol (317 mg, 1.5 mmol) prepared according to the method

described in WO 2005/044802 was dissolved in ethanol (10 mL), and 10% palladium carbon (30 mg) was added thereto, and then, the mixture was stirred overnight at room temperature under hydrogen atmosphere. The reaction mixture was filtered through Celite, and the solvent was evaporated to give (6-amino-3,4-dihydro-2H-benzo[1,4]oxazin-2-yl)methanol (270 mg, quantitative yield).
ESIMS m/z: $[M+H]^+$ 181

Reference example 187: [5-Chloro-6-(piperazin-1-yl) pyridin-3-yl]methanol (for use in the synthesis of Compound 323)

Step 1

Ethyl 5,6-dichloronicotinate (660 mg, 3.0 mmol), 1-(tert-butoxycarbonyl)piperazine (671 mg), and N,N-diisopropylethylamine (388 mg) were dissolved in N-methylpyrrolidone (3.0 mL), and the mixture was stirred at 130˚C for 10 minutes under microwave irradiation. Water was added to the reaction mixture, and the mixture was extracted with ethyl acetate. After the organic layer was dried over anhydrous magnesium sulfate, the solvent was evaporated, and the residue was purified by silica gel column chromatography to give ethyl 6-[1-(test-butoxycarbonyl)piperazin-4-yl]-5-chloronicotinate (1.4 g, 95%).

Step 2

Ethyl 6-[1-(tert-butoxycarbonyl)piperazin-4-yl]-5-chloronicotinate (1.4 g, 3.8 mmol) was dissolved in THF (20 mL), and lithium borohydride (100 mg) was added thereto, and then, the mixture was stirred at room temperature for 2 days. The reaction mixture was poured into ice water, and the mixture was extracted with ethyl acetate. Then, the organic layer was dried over anhydrous magnesium sulfate, and the solvent was evaporated to give 2-[1-(tert-butoxycarbonyl) piperazin-4-yl]-3-chloropyridine-5-methanol (745 mg, 60%).

Step 3

2-[1-(tert-Butoxycarbonyl)piperazin-4-yl]-3-chloropyridine-5-methanol (745 mg, 2.3 mmol) was dissolved in dichloromethane (6.0 mL), and trifluoroacetic acid (2.0 mL) was added thereto, and then, the mixture was stirred at room temperature for 3 hours. To the reaction mixture, a saturated sodium hydrogen carbonate solution was added and the pH of the mixture was adjusted to 6. Then, the mixture was subjected to solid-phase extraction using a column packed with Bondesil SCX (manufactured by Varian, 0.180 g), and the solvent was evaporated to give [5-chloro-5-(paperazin-1_yl)pyridin-3-yl]methanol (300 mg, 58%).
ESIMS m/z: $[M+H]^+$ 228

Reference example 188: [(2-Piperazin-1-yl)pyridin-3-yl]methanol (for use in the synthesis of Compound 325)

[(2-Piperazin-1-yl)pyridin-3-yl]methanol was obtained from ethyl 2-chloronicotinate in a similar manner to Reference example 187.
ESIMS m/z: $[M+H]^+$ 194

Reference example 189: 2-(2-Aminophenyl)propane-1,3-diol (for use in the synthesis of Compound 162)

2-(2-Nitrophenyl)propane-1,3-diol (2.3 g, 12 mmol) prepared according to the method described in the article [Journal of Organic Chemistry, vol. 51, pp. 3143-3147 (1986)] was dissolved in ethanol (5.0 mL), and 10% palladium carbon (100 mg) was added thereto, and then, the mixture was stirred at room temperature for 6 hours under hydrogen atmosphere. The reaction mixture was filtered through Celite, and the solvent was evaporated to give 2-(2-aminophenyl) propane-1,3-diol (2.0 g, quantitative yield).
ESIMS m/z: $[M+H]^+$ 168

Reference example 190: 1-(2-Aminophenyl)ethane-1,2-diol (for use in the synthesis of Compound 163)

Commercially available 1-(2-nitrophenyl)ethane-1,2-diol (500 mg, 2.7 mmol) was dissolved in ethyl acetate (15 mL), and 10% palladium hydrogen (50 mg) was added thereto, and then, the mixture was stirred at 40˚C for 6 hours under hydrogen atmosphere. The reaction mixture was filtered through Celite, and the solvent was evaporated to give 1-(2-aminophenyl)ethane-1,2-diol (402 mg, 96%).
ESIMS m/z: $[M+H]^+$ 154

Reference example 191: 5-Amino-1H-quinoxalin-2-one (for use in the synthesis of Compound 165)

Step 1

Glycine (1.8 g) and sodium hydrogen carbonate (3.7 g) were dissolved in water (12 mL), and a methanol solution (40 mL) of 2-chloro-1,3-dinitrobenzene (4.0 g, 20 mmol) was added dropwise thereto, and then, the mixture was heated under reflux for 4 hours. After the reaction mixture was concentrated under reduced pressure, the pH of the mixture was adjusted to 3 with 1.0 mol/L hydrochloric acid, and the precipitated crystal was collected by filtration. Then, the crystal was dissolved in ethanol (100 mL), and concentrated sulfuric acid (1.1 mL) was added thereto, and then, the mixture was heated under reflux overnight. After the reaction mixture was concentrated under reduced pressure, water was added to the residue, and the precipitated crystal was collected by filtration to give ethyl (2,6-dinitrophenylamino) acetate (3.3 g, 70%).

Step 2

Ethyl (2,6-dinitrophenylamino)acetate (1.0 g, 4.2 mmol) was dissolved in DMF (10 mL) and ethanol (20 mL), and 10% palladium carbon (200 mg) was added thereto, and then, the mixture was stirred overnight at room temperature under hydrogen atmosphere. After the reaction mixture was filtered through Celite, the solvent was evaporated, and chloroform (40 mL), DMF (3.0 mL), and manganese dioxide (7.0 g) were added thereto. Then, the mixture was stirred overnight at room temperature. After the reaction mixture was filtered through Celite, the solvent was evaporated to give 5-amino-1H-quinoxalin-2-one (386 mg, 58%).
ESIMS m/z: $[M+H]^+$ 162

Reference example 192: 3-Amino-2-chloropyridine-4-methanol (for use in the synthesis of Compound 170) and 3-amino-2,6-dichloropyridine-4-methanol (for use in the synthesis of Compound 171)

Methyl 3-amino-2-chloroisonicotinate (822 mg, 4.4 mmol) prepared according to the method described in the article [Journal of Heterocyclic Chemistry, vol. 23, pp. 1253-1255 (1986)] was dissolved in THF (20 mL), and lithium borohydride (378 mg) was added thereto, and then, the mixture was stirred overnight at room temperature. The reaction mixture was poured into ice water, and the mixture was extracted with ethyl acetate, and then, the organic layer was dried over anhydrous magnesium sulfate. The solvent was evaporated to give 3-amino-2-chloropyridine-4-methanol (492 mg, 71%). Also, methyl 3-amino-2,6-dichloroisonicotinate simultaneously prepared by the method described in the above article was converted into 3-amino-2,6-dichloropyridine-4-methanol in the same manner as above.
ESIMS m/z: $[M+H]^+$ 159 (3-amino-2-chloropyridine-4-methanol), 193 (3-amino-2,6-dichloropyridine-4-methanol)

Reference example 193: tert-Butyl (5-amino-2-oxo-1,2,3,4-tetrahydroquinolin-3-yl)carbamate (for use in the synthesis of Compound 172)

Step 1

Methyl 3-(2,6-dinitrophenyl)-2-hydroxypropionate (6.2 g, 23 mmol) prepared according to the method described in the article [Bioorganic and Medicinal Chemistry Letters, vol. 10, pp. 1459-1462 (2000)] and triethylamine (3.8 mL) were dissolved in THF (100 mL), and methanesulfonyl chloride (3.2 g) was added thereto under ice-cooling, and then, the mixture was stirred at room temperature for 1 hour. To the reaction mixture, a saturated sodium hydrogen carbonate solution was added, and the mixture was extracted with ethyl acetate. After the organic layer was dried over anhydrous magnesium sulfate, the solvent was evaporated to give methyl 3-(2,6-dinitrophenyl)-2-methanesulfonyloxypropionate (8.0 quantitative yield).

Step 2

Methyl 3-(2,6-dinitrophenyl)-2-methanesulfonyloxy propionate (8.0 g, 23 mmol) was mixed with DMF (20 mL) and sodium azide (1.7 g), and the mixture was stirred at 60˚C for 7 hours. To the reaction mixture, saturated brine was added, and the mixture was extracted with ethyl acetate. After the organic layer was dried over anhydrous magnesium sulfate, the solvent was evaporated, and the residue was purified by silica gel column chromatography to give methyl 2-azido-3-(2,6-dinitrophenyl)propionate (3.2 g, 48%).

Step 3

Methyl 2-azido-3-(2,6-dinitrophenyl)propionate (3.2 g, 11 mmol) was mixed with THF (40 mL), water (3.0 mL), and triphenylphosphine (2.6 g), and the mixture was stirred at room temperature for 1 hour. Then, di-tert-butyldicarbonate (2.2 g) was added thereto, and the mixture was further stirred overnight. The reaction mixture was extracted by adding ethyl acetate and a saturated sodium hydrogen carbonate solution thereto. After the organic layer was dried over anhydrous magnesium sulfate, the solvent was evaporated, and the residue was purified by silica gel column chromatography. Further, DMF (10 mL) and ethanol (10 mL) were added to the resulting matter, and, 10% palladium carbon (100 mg) was added thereto, and the mixture was stirred at room temperature for 3 days under hydrogen atmosphere. After the reaction mixture was filtered through Celite, the filtrate was concentrated under reduced pressure, and the residue was purified by silica gel column chromatography to give tert-butyl (5-amino-2-oxo-1,2,3,4-tetrahydroquinolin-3-yl)carbamate (887 mg, 29%).
ESIMS m/z: $[M+H]^+$ 278

Reference example 194: (4-Amino-2,3-dihydrobenzofuran-2-yl)methanol (for use in the synthesis of Compound 174)

Step 1

3-Nitrophenol (20 g, 0.15 mol), allyl bromide (19 g), and potassium carbonate (22 g) were mixed, and the mixture was heated under reflux for 2 days in acetone (300 mL). After the precipitate was filtered, the filtrate was concentrated, and a 10% aqueous potassium carbonate solution was added thereto, and then, the mixture was extracted with ethyl acetate. After the organic layer was dried over anhydrous magnesium sulfate, the solvent was evaporated to give 3-(allyloxy)nitrobenzene (26 g, 99%).

Step 2

3-(Allyloxy)nitrobenzene (26 g, 0.15 mol) was heated under stirring in 1,3-dichlorobenzene (100 mL) at 150˚C for 3 days. After the precipitate was filtered, the solvent was evaporated, and the residue was purified by silica gel column chromatography to give 2-allyl-3-nitrophenol (1.3 g, 5%).

Step 3

2-Allyl-3-nitrophenol (179 mg, 1.0 mmol) and 3-chloroperbenzoic acid (282 mg) were heated under reflux for 9 hours in chloroform (4.0 mL). After the reaction mixture was washed with a saturated sodium hydrogen carbonate solution, the organic layer was dried over anhydrous magnesium sulfate. Then, the solvent was evaporated, and the residue was purified by silica gel column chromatography to give (4-nitro-2,3-dihydrobenzofuran-2-yl)methanol (148 mg, 76%).

Step 4

(4-Nitro-2,3-dihydrobenzofuran-2-yl)methanol (129 mg, 0.66 mmol) was dissolved in ethyl acetate (2.5 mL) and ethanol (2.5 mL), and 10% palladium carbon (50 mg) was added thereto, and then, the mixture was stirred overnight at room temperature under hydrogen atmosphere. After the reaction mixture was filtered through Celite, the solvent was evaporated to give (4-amino-2,3-dihydrobenzofuran-2-yl)methanol (110 mg, quantitative yield).
ESIMS m/z: $[M+H]^+$ 166

Reference example 195: 5-Aminochroman-3-ol (for use in the synthesis of Compound 175)

Step 1

2-Allyl-3-nitrophenol (1.7 g, 9.7 mmol) obtained in Step 2 of Reference example 194 and acetic anhydride (1.1 mL) were stirred in pyridine (10 mL) at room temperature for 30 minutes. After the reaction mixture was concentrated under reduced pressure, the residue was diluted with ethyl acetate, and the mixture was sequentially washed with dilute hydrochloric acid, a saturated sodium hydrogen carbonate solution, and brine. After the organic layer was dried over anhydrous magnesium sulfate, the solvent was evaporated to give 3-acetoxy-2-allylnitrobenzene (2.1 g, 99%).

Step 2

3-Acetoxy-2-allylnitrobenzene (2.1 g, 9.6 mmol) and 3-chloroperbenzoic acid (3.6 g) were stirred overnight in chloroform (22 mL) at room temperature. The reaction mixture was sequentially washed with an aqueous sodium sulfite solution, a saturated sodium hydrogen carbonate solution, and saturated brine, and the organic layer was dried over anhydrous magnesium sulfate. The solvent was evaporated, and the residue was purified by silica gel column chromatography to give 3-nitro-2-(oxiranylmethyl)phenol (2.0 g, 87%).

Step 3

3-Nitro-2-(oxiranylmethyl)phenol (2.0 g, 8.4 mmol) and sodium iodide (1.3 g) were heated in acetone (10 mL) at 50°C for 3 hours. After the reaction mixture was concentrated under reduced pressure, the residue was diluted with ethyl acetate, and the mixture was washed with water. The organic layer was dried over anhydrous magnesium sulfate, and the solvent was evaporated to give 3-acetoxy-5-nitrochroman (1.6 g, 81%).

Step 4

3-Acetoxy-5-nitrochroman (1.6 g, 6.8 mmol) was dissolved in ethanol (10 mL), and a 2.0 mol/L aqueous sodium hydroxide solution (4.0 mL) was added thereto, and the mixture was stirred at room temperature for 40 minutes. After the mixture was neutralized with dilute hydrochloric acid, the mixture was extracted with ethyl acetate, and the organic layer was dried over anhydrous magnesium sulfate. Then, the solvent was evaporated to give 5-nitrochroman-3-ol (1.4 g, quantitative yield).

Step 5

5-Nitrochroman-3-ol (1.4 g, 6.8 mmol) was dissolved in ethyl acetate (20 mL), and 10% palladium carbon (300 mg) was added thereto, and the mixture was stirred at room temperature for 5 hours under hydrogen atmosphere. After the reaction mixture was filtered through Celite, the solvent was evaporated to give 5-aminochroman-3-ol (1.0 g, 92%).
ESIMS m/z: $[M+H]^+$ 166

Reference example 196: 5-Amino-1,4-dihydrobenzo[d][1,3] oxazin-2-one (for use in the synthesis of Compound 176)

Step 1

Methyl 2-amino-6-nitrobenzoate (2.7 g, 14 mmol) prepared according to the method described in the article [Journal of Heterocyclic Chemistry, vol. 23, pp. 1253-1255 (1986)] and lithium borohydride (1.0 g) were stirred overnight in THF (20 mL) at room temperature. The reaction mixture was poured into ice water, and the mixture was extracted with ethyl acetate. After the organic layer was dried over anhydrous magnesium sulfate, the solvent was evaporated, and the residue was purified by silica gel column chromatography to give 2-amino-6-nitrobenzyl alcohol (1.5 g, 65%).

Step 2

2-Amino-6-nitrobenzyl alcohol (400 mg, 2.4 mmol) and carbonyldiimidazole (500 mg) were stirred overnight in THF (6.0 mL) at 60°C. After the reaction mixture was diluted with ethyl acetate, the mixture was washed with dilute hydrochloric acid. After the organic layer was dried over anhydrous magnesium sulfate, the solvent was evaporated to give 5-nitro-1,4-dihydrobenzo[d] [1,3]oxazin-2-one (295 mg, 64%) .

Step 3

5-Nitro-1,4-dihydrobenzo [d] [1,3]oxazin-2-one (295 mg, 1.5 mmol) was dissolved in DMF (10 mL) and ethanol (5.0 mL), and 10% palladium carbon (80 mg) was added thereto, and then, the mixture was stirred at room temperature for 2 days under hydrogen atmosphere. After the reaction mixture was filtered through Celite, the solvent was evapoarated to give 5-amino-1,4-dihydrobenzo[d][1,3]oxazin-2-one (204 mg, 83%).
ESIMS m/z: $[M+H]^+$ 165.

Example 1

(E)-1-(Thiomorpholino)-5,5-bis[4-(trifluoromethyl)phenyl]penta-2,4-dien-1-one (Compound 1)

Compound b (97 mg, 0.25 mmol) was dissolved in thionyl chloride (2 mL), and the mixture was heated under reflux for 2 hours. The reaction mixture was concentrated under reduced pressure, and the residue was dissolved in dichloromethane (2 mL). Then, thiomorpholine (0.030 mL, 0.30 mmol) and triethylamine (0.052 mL, 0.38 mmol) were added thereto, and the mixture was stirred at room temperature for 4 hours. Water was added to the reaction mixture, and the mixture was extracted with chloroform. The organic layer was washed with 1 mol/L hydrochloric acid, a saturated sodium hydrogen carbonate solution, and saturated brine, and dried over anhydrous magnesium sulfate. After the organic layer was filtered, the solvent was evaporated under reduced pressure, and the residue was recrystallized from diethyl ether/hexane to give Compound 1 (84 mg, 71%).
$^1$H NMR ($CDCl_3$, δ m): 2.63-2.66 (m, 4H), 3.88-3.91 (m, 4H), 6.58 (d, J = 14.7 Hz, 1H), 6.91 (d, J = 11.6 Hz, 1H), 7.30 (dd, J = 11.6, 14.7 Hz, 1H), 7.32 (d, J = 8.1 Hz, 2H), 7.37 (d, J = 8.1 Hz, 2H), 7.58 (d, J = 8.1 Hz, 2H), 7.69 (d, J = 8.1 Hz, 2H); APCIMS m/z: $[M+H]^+$ 472.

Example 2

(E)-5,5-Diphenyl-1-(thiomorpholino)penta-2,4-dien-1-one (Compound 2)

Compound 2 was obtained from Compound c in a similar manner to Example 1.
$^1$H NMR ($CDCl_3$, δ ppm): 2.61-2.65 (m, 4H), 3.89 (brs, 4H), 6.46 (d, J = 14.7 Hz, 1H), 6.81 (d, J = 11.6 Hz, 1H), 7.19-7.22 (m, 2H), 7.26-7.34 (m, 5H), 7.34-7.42 (m, 4H); APCIMS m/z: $[M+H]^+$ 336.

Example 3

(E)-1-(Thiomorpholino)-5,5-bis[3-(trifluoromethyl)phenyl]penta-2,4-dien-1-one (Compound 3)

Compound 3 was obtained from Compound d in a similar manner to Example 1.
$^1$H NMR ($CDCl_3$, δ ppm): 2.63-2.66 (m, 4H), 3.90 (brs, 4H), 6.59 9 (d, J = 14.5 Hz, 1H), 6.91 (d, J = 11.6 Hz, 1H), 7.26 (dd, J = 11.6, 14.5 Hz, 1H), 7.32-7.35 (m, 1H), 7.43-7.46 (m, 3H), 7.56-7.60 (m, 3H), 7.67-7.69 (m, 1H); APCIMS m/z: $[M+H]^+$ 472:

Example 4

(E)-1-(Thiomorpholino)-5,5-bis[4-(trifluoromethoxy)phenyl]penta-2,4-dien-1-one (Compound 4)

Compound 4 was obtained from Compound e in a similar manner to Example 1.
$^1$H NMR ($CDCl_3$, δ ppm): 2.63-2.66 (m, 4H), 3.88-3.91 (m, 4H), 6.53 (d, J = 14.5 Hz, 1H), 6.80 (d, J = 11.6 Hz, 1H), 7.15-7.31 (m, 8H), 7.34 (dd, J = 11.6, 14.5 Hz, 1H); APCIMS m/z: $[M+H]^+$ 504.

Example 5

(E)-5,5-Bis(4-chlorophenyl)-1-(thiomorpholino)penta-2,4-dien-1-one (Compound 5)

Compound 5 was obtained from Compound f in a similar manner to Example 1.
$^1$H NMR ($CDCl_3$, δ ppm): 2.62-2.65 (m, 4H), 3.87-3.90 (m, 4H), 6.50 (d, J = 14.7 Hz, 1H), 6.78 (d, J = 11.6 Hz, 1H), 7.11-7.14 (m, 2H), 7.18-7.22 (m, 2H), 7.27-7.30 (m, 2H), 7.31 (dd, J = 11.6, 14.7 Hz, 1H), 7.36-7.39 (m, 2H); APCIMS m/z: $[M+H]^+$ 404.

Example 6

(E)-5,5-Bis(4-tert-butylphenyl)-1-(thiomorpholino)penta-2,4-dien-1-one (Compound 6)

Compound 6 was obtained from Compound g in a similar manner to Example 1.
$^1$H NMR ($CDCl_3$, δ ppm): 1.32 (s, 9H), 1.35 (s, 9H), 2.62-2.65 (m, 4H), 3.89 (brs, 4H), 6.43 (d, J = 14.7 Hz, 1H), 6.78 (d, J = 11.7 Hz, 1H), 7.12-7.15 (m, 2H), 7.22-7.26 (m, 2H), 7.32-7.34 (m, 2H), 7.37-7.40 (m, 2H), 7.45 (dd, J = 11.7, 14.7 Hz, 1H); APCIMS m/z: $[M+H]^+$ 448.

Example 7

(E)-1-(1-Oxothiomorpholin-4-yl)-5,5-bis[4-(trifluoromethyl)phenyl]penta-2,4-dien-1-one (Compound 7)

(E)-1-(1,1-Dioxothiomorpholin-4-yl)-5,5-bis[4-(trifluoromethyl)phenyl]penta-2,4-dien-1-one (Compound 8)

Compound 1 (124 mg, 0.263 mmol) was dissolved in dichloromethane (3 mL), and meta-chloroperbenzoic acid (92 mg, 0.526 mmol) was added thereto under ice-cooling, and then, the mixture was stirred at room temperature for 7 hours. To the reaction mixture, a 1 mol/L aqueous sodium hydroxide solution was added, and the mixture was extracted with chloroform. The organic layer was washed with water and saturated brine, and dried over anhydrous magnesium sulfate. After the organic layer was filtered, the solvent was evaporated under reduced pressure, and the reside was purified by silica gel column chromatography (ethyl acetate/methanol = 10/1) to give Compound 7 (58 mg, 44%) and Compound 8 (67 mg, 52%).

Compound 7

$^1$H NMR ($CDCl_3$, δ ppm): 2.63-2.73 (m, 2H), 2.83-2.87 (m, 2H), 3.99 (brs, 4H), 6.59 (d, J = 14.7 Hz, 1H), 6.92 (d, J = 11.6 Hz, 1H), 7.28-7.38 (m, 4H), 7.33 (dd, J = 11.6, 14.7 Hz, 1H), 7.58 (d, J = 8.7 Hz, 2H), 7.70 (d, J = 8.7 Hz, 2H); APCIMS m/z: $[M+H]^+$ 488.

Compound 8

$^1$H NMR ($CDCl_3$, δ ppm): 3.05-3.08 (m, 4H), 4.11 (brs, 4H), 6.60 (d, J = 14.5 Hz, 1H), 6.93 (d, J = 11.6 Hz, 1H), 7.32-7.41 (m, 4H), 7.37 (dd, J = 11.6, 14.7 Hz, 1H), 7.60 (d, J = 8.2 Hz, 2H), 7.71 (d, J = 8.2 Hz, 2H); APCIMS m/z: $[M-H]^-$ 502.

Example 8

(E)-1-[4-(3-Chloropyridin-2-yl)piperazin-1-yl]-5,5-bis[4-trifluoromethyl]phenyl]penta-2,4-dien-1-one (Compound 9)

In a similar manner to Example 1, Compound 9 was obtained from Compound b using 4-(3-chloropyridin-2-yl) piperazine in place of thiomorpholine.
$^1$H NMR ($CDCl_3$, δ ppm): 3.38 (brs, 4H), 3.76-3.83 (m, 4H), 6.66 (d, J = 14.5 Hz, 1H), 6.87-6.91 (m, 1H), 6.94 (d, J = 11.7 Hz, 1H), 7.33 (dd, J = 11.7, 14.5 Hz, 1H), 7.33-7.39 (m, 4H), 7.58 (d, J = 8.1 Hz, 2H), 7.60-7.64 (m, 1H), 7.69 (d, J = 8.1 Hz, 2H), 8.18-8.20 (m, 1H); APCIMS m/z: $[M+H]^+$ 566.

Example 9

(E)-N-(7-Hydroxynaphthalen-1-yl)-5,5-bis[4-(trifluoromethyl)phenyl]-2,4-pentadienamide (Compound 10)

In a similar manner to Example 1, Compound 10 was obtained from Compound b using 8-amino-2-naphthol in place of thiomorpholine.
mp : 249-251˚C
$^1$H NMR (DMSO - $d_6$, δ ppm): 6.76 (d, J = 14.7 Hz, 1H), 7.08-7.11 (m, 1H), 7.10 (dd, J = 11.6, 14.7 Hz, 1H), 7.18-7.26 (m, 2H), 7.23 (d, J = 11.6 Hz, 1H), 7.48 (d, J = 8.2 Hz, 2H), 7.53-7.56 (m, 1H), 7.54 (d, J = 8.2 Hz, 2H), 7.60-7.63 (m, 1H), 7.72 (d, J = 8.2 Hz, 2H), 7.73-7.73 (m, 1H), 7.85 (d, J = 8.2 Hz, 2H), 9.79 (s, 1H); APCIMS m/z: $[M-H]^-$ 526.

Example 10

(E)-N-(Isoquinolin-5-yl)-5,5-bis[4-(trifluoromethyl)phenyl]-2,4-pentadienamide (Compound 11)

Compound b (2.32 g, 6.00 mmol) was dissolved in DMF (30 mL), and 5-aminoisoquinoline (720 mg, 5.00 mmol), EDC hydrochloride (1.92 g, 10.0 mmol), and HOBt (1.15 g, 7.50 mmol) were added thereto, and then, the mixture was stirred at 60˚C for 7 hours. After the reaction mixture was left to cool, a saturated sodium hydrogen carbonate solution was added thereto, and the mixture was extracted with ethyl acetate. The organic layer was washed with saturated brine and dried over anhydrous magnesium sulfate. After the organic layer was filtered, the solvent was evaporated under reduced pressure, and the residue was purified by silica gel column chromatography (ethyl acetate/hexane = 1/1), and then recrystallized from isopropyl ether/hexane to give Compound 11 (1.34 g, 52%). mp : 164-166˚C; $^1$H NMR ($CDCl_3$,

δ ppm): 6.38 (d, J = 14.7 Hz, 1H), 6.93 (d, J = 11.6 Hz, 1H), 7.36 (d, J = 8.2 Hz, 2H), 7.39 (d, J = 8.2 Hz, 2H), 7.46 (dd, J = 11.6, 14.7 Hz, 1H), 7.58-7.64 (m, 3H), 7.60 (d, J = 8.2 Hz, 2H), 7.71 (d, J = 8.2 Hz, 2H), 7.83-7.85 (m, 1H), 8.20 (brs, 1H), 8.56-8.58 (m, 1H), 9.27 (s, 1H); APCIMS m/z: $[M+H]^+$ 513.

Example 11

(E)-N-(4-tert-Butylphenyl)-5,5-bis[4-(trifluoromethyl)phenyl]-2,4-pentadienamide (Compound 12)

In a similar manner to Example 10, Compound 12 was obtained from Compound b using 4-tert-butylaniline in place of 5-aminoisoquinoline.
$^1$H NMR ($CDCl_3$, δ ppm): 1.30 (s, 9H), 6.25 (d, J = 14.6 Hz, 1H), 6.91 (d, J = 11.6 Hz, 1H), 7.21 (s, 1H), 7.33-7.39 (m, 6H), 7.38 (dd, J = 11.6, 14.6 Hz, 1H), 7.47-7.50 (m, 2H), 7.58 (d, J = 8.2 Hz, 2H), 7.70 (d, J = 8.2 Hz, 2H); APCIMS m/z: $[M+H]^+$ 518.

Example 12

(E)-N-[2-(2-Hydroxyethyl)phenyl]-5,5-bis[4-(trifluoromethyl)phenyl]-2,4-pentadienamide (Compound 13)

In a similar manner to Example 10, Compound 13 was obtained from Compound b using 2-(2-hydroxyethyl) aniline in place of 5-aminoisoquinoline.
$^1$H NMR (DMSO-$d_6$, δ ppm): 2.72-2.76 (m, 2H), 3.55-3.61 (m, 2H), 4.98-5.01 (m, 1H), 6.62 (d, J = 14.7 Hz, 1H), 7.04 (dd, J = 11.6, 14.7 Hz, 1H), 7.08-7.25 (m, 3H), 7.31 (d, J = 11.6 Hz, 1H), 7.47-7.56 (m, 5H), 7.75 (d, J = 8.2 Hz, 2H), 7.89 (d, J = 8.2 Hz, 2H), 9.86 (s, 1H); APCIMS m/z: $[M+H]^+$ 506.

Example 13

(E)-N-(7-Ethoxynaphthalen-1-yl)-5,5-bis[4-(trifluoromethyl)phenyl]-2,4-pentadienamide (Compound 14)

In a similar manner to Example 10, Compound 14 was obtained from Compound b using 8-amino-2-ethoxynaphthalene in place of 5-aminoisoquinoline.
$^1$H NMR (DMSO-$d_6$, δ ppm): 1.41 (t, J = 6.8 Hz, 3H), 4.17 (q, J = 6.8 Hz, 2H), 6.86 (d, J = 14.7 Hz, 1H), 7.10 (dd, J = 11.6, 14.7 Hz, 1H), 7.19-7.22 (m, 1H), 7.28-7.39 (m, 2H), 7.33 (d, J = 11.6 Hz, 1H), 7.50 (d, J = 8.2 Hz, 2H), 7.56 (d, J = 8.2 Hz, 2H), 7.66-7.59 (m, 1H), 7.75-7.78 (m, 3H), 7.84-7.91 (m, 3H), 10.1 (s, 1H); APCIMS m/z: $[M+H]^+$ 556.

Example 14

(E)-N-(7-Hydroxy-5,6,7,8-tetrahydronaphthalen-1-yl)-5,5-bis[4-(trifluoromethyl)phenyl]-2,4-pentadienamide (Compound 15)

In a similar manner to Example 1, Compound 15 was obtained from Compound b using 8-amino-1,2,3,4-tetrahydro-2-naphthol in place of thiomorpholine.
$^1$H NMR (DMSO-$d_6$, δ ppm): 1.55-1.61 (m, 1H), 1.85-1.88 (m, 1H), 2.38-2.46 (m, 1H), 2.72-2.89 (m, 3H), 3.87-3.88 (m, 1H), 4.79-4.81 (m, 1H), 6.68 (d, J = 14.7 Hz, 1H), 6.90-7.07 (m, 2H), 7.03 (dd, J = 11.6, 14.7 Hz, 1H), 7.21-7.23 (m, 1H), 7.28 (d, J = 11.6 Hz, 1H), 7.48 (d, J = 8.2 Hz, 2H), 7.54 (d, J = 8.2 Hz, 2H), 7.75 (d, J = 8.2 Hz, 2H), 7.89 (d, J = 8.2 Hz, 2H), 9.47 (s, 1H); APCIMS m/z: $[M+H]^+$ 532.

Example 15

(E)-N-[2-(2-Hydroxy-2-methylpropyl)phenyl]-5,5-bis[4-(trifluoromethyl)phenyl]-2,4-pentadienamide (Compound 16)

In a similar manner to Example 10, Compound 16 was obtained from Compound b using 2-(2-hydroxy-2-methylpropyl)aniline in place of 5-aminoisoquinoline.
$^1$H NMR (DMSO-$d_6$, δ ppm): 1.13 (s, 6H), 2.72 (s, 2H), 5.46 (s, 1H), 6.48 (d, J = 14.7 Hz, 1H), 7.00-7.06 (m, 1H), 7.05 (dd, J = 11.6, 14.7 Hz, 1H), 7.16-7.21 (m, 2H), 7.35 (d, J = 11.6 Hz, 1H), 7.48 (d, J = 8.3 Hz, 2H), 7.54 (d, J = 8.3 Hz, 2H), 7.75 (d, J = 8.3 Hz, 2H), 7.80-7.83 (m, 1H), 7.90 (d, J = 8.3 Hz, 2H), 10.3 (s, 1H); APCIMS m/z: $[M+H]^+$ 534.

Example 16

(E)-N-[2-(2-Hydroxymethyl)phenyl]-5,5-bis[4-(trifluoromethyl)phenyl]-2,4-pentadienamide (Compound 17)

In a similar manner to Example 10, Compound 17 was obtained from Compound b using 2-(hydroxymethyl) aniline in place of 5-aminoisoquinoline.
$^1$H NMR (DMSO-$d_6$, δ ppm): 4.49-4.50 (m, 2H), 5.30-5.35 (m, 1H), 6.63 (d, J = 14.9 Hz, 1H), 7.04 (dd, J = 11.6, 14.9 Hz, 1H), 7.12-7.25 (m, 2H), 7.31 (d, J = 11.6 Hz, 1H), 7.40-7.42 (m, 1H), 7.48 (d, J = 8.2 Hz, 2H), 7.54 (d, J = 8.2 Hz, 2H), 7.55-7.61 (m, 1H), 7.75 (d, J = 8.2 Hz, 2H), 7.89 (d, J = 8.2 Hz, 2H), 9.60 (s, 1H); APCIMS m/z: $[M+H]^+$ 492.

Example 17

(E)-N-(Quinolin-7-yl)-5,5-bis[4-(trifluoromethyl)phenyl]-2,4-pentadienamide (Compound 18)

In a similar manner to Example 10, Compound 18 was obtained from Compound b using 7-aminoquinoline in place of 5-aminoisoquinoline.
$^1$H NMR (DMSO-$d_6$, δ ppm): 6.65 (d, J = 14.7 Hz, 1H), 7.13 (dd, J = 11.7, 14.7 Hz, 1H), 7.34 (d, J = 11.7 Hz, 1H). 7.39-7.43 (m, 1H), 7.51 (d, J = 8.1 Hz, 2H), 7.56 (d, J = 8.1 Hz, 2H). 7.74-7.78 (m, 3H), 7.91-7.93 (m, 3H), 8.25-8.28 (m, 1H), 8.47-8.48 (m, 1H), 8.82-8.84 (m, 1H), 10.6 (s, 1H); APCIMS m/z: $[M+H]^+$ 513.

Example 18

(E)-N-[7-(Sulfamoyloxy)naphthalen-1-yl]-5,5-bis[4-(trifluoromethyl)phenyl]-2,4-pentadienamide (Compound 19)

Compound 10 (80 mg, 0.15 mmol) was dissolved in DMA (3 mL), and sulfamoyl chloride (70 mg, 0.61 mmol) was added thereto under ice-cooling, and then, the mixture was stirred at room temperature for 18 hours. Water was added to the reaction mixture, and the precipitated crystal was washed with water to give Compound 19 (90 mg, 98%).
$^1$H NMR (DMSO-$d_6$, δ ppm): 6.84 (d, J = 14.7 Hz, 1H), 7.12 (dd, J = 11.6, 14.7 Hz, 1H), 7.34 (d, J = 11.6 Hz, 1H), 7.47-7.58 (m, 7H), 7.75-7.95 (m, 3H), 7.77 (d, J = 8.2 Hz, 2H), 7.90 (d, J = 8.2 Hz, 2H), 8.07 (s, 1H), 10.3 (s, 1H); APCIMS m/z: $[M+H]^+$ 607.

Example 19

(E)-N-(Quinolin-5-yl)-5,5-his[4-(trifluoromethyl)phenyl]-2,4-pentadienamide (Compound 20)

In a similar manner to Example 10, Compound 20 was obtained from Compound b using 5-aminoquinoline in place of 5-aminoisoquinoline.
$^1$H NMR (DMSO-$d_6$, δ ppm): 6.80 (d, J = 14.7 Hz, 1H), 7.07 (d, J = 11.4 Hz, 1H), 7.31 (d, J = 8.0 Hz, 2H), 7.39 (d, J = 8.0 Hz, 2H), 7.60 (d, J = 8.0 Hz, 2H), 7.72 (d, J = 8.0 Hz, 2H), 7.93 (d, J = 7.8 Hz, 1H), 8.02 (d, J = 6.1 Hz, 1H), 8.18 (d, J = 7.8 Hz, 1H), 8.55 (d, J = 6.1 Hz, 1H), 9.31 (s, 1H), 10.35 (brs, 1H); APCIMS m/z: $[M+H]^+$ 513.

Example 20

(E)-N-([1,3,4]Thiadiazol-2-yl)-5,5-bis[4-(tritluoromethyl)phenyl]-2,4-pentadienamide (Compound 21)

In a similar manner to Example 10, Compound 21 was obtained from Compound b using 2-amino [1,3,4] thiadiazole in place of 5-aminoisoquinoline.
$^1$H NMR ($CDCl_3$, δ ppm): 6.80 (d, J = 14.7 Hz, 1H), 7.07 (d, J = 11.4 Hz, 1H), 7.34 (d, J = 8.0 Hz, 2H), 7.43 (d, J = 8.0 Hz, 2H), 7.56 (dd, J = 11.4, 14.7 Hz, 1H), 7.61 (s, 1H), 7.62 (d, J = 8.0 Hz, 2H), 7.73 (d, J = 8.0 Hz, 2H), 8.86 (s, 1H); APCIMS m/z: $[M+H]^+$ 470.

Example 21

(E)-N-[2-(Cyanomethyl)phenyl]-5,5-bis[4-(trifluoromethyl)phenyl]-2,4-pentadienamide (Compound 22)

In a similar manner to Example 10, Compound 22 was obtained from Compound b using 2- (cyanomethyl) aniline in place of 5-aminoisoquinoline.
$^1$H NMR ($EDCl_3$, δ ppm): 3.72 (s, 2H), 6.30 (d, J = 14.4 Hz, 1H), 6.92 (d, J = 11.4 Hz, 1H), 7.19 (s, 1H), 7.29-7.43 (m,

8H), 7.38 (dd, J = 11.4, 14.4 Hz, 1H), 7.60 (d, J = 8.7 Hz, 2H), 7.70 (d, J = 8.7 Hz, 2H); APCIMS m/z: $[M+H]^+$ 501.

Example 22

(E)-N-(2-Cyanophenyl)-5,5-bis[4-(trifluoromethyl)phenyl]-2,4-pentadienamide (Compound 23)

In a similar manner to Example 10, Compound 23 was obtained from Compound b using 2-cyanoaniline in place of 5-aminoisoquinoline.
$^1$H NMR (DMSO-$d_6$, δ pom): 6.22 (d, J = 14.8 Hz, 1H), 7.01 (dd, J = 11.4, 14.8 Hz, 1H), 7.19 (d, J = 11.4 Hz, 1H), 7.35-7.40 (m, 1H), 7.44-7.53 (m, 5H), 7.64-7.67 (m, 1H), 7.70 (d, J = 8.5 Hz, 2H), 7.84 (d, J = 8.5 Hz, 2H), 7.90-7.93 (m, 1H); ESIMS m/z: $[M-H]^-$ 444.

Example 23

(E)-N-(2-Hydroxyphenyl)-5,5-bis[4-(trifluoromethyl)phenyl]-2,4-pentadienamide (Compound 24)

In a similar manner to Example 10, Compound 24 was obtained from Compound b using 2-aminophenol in place of 5-aminoisoquinoline.
$^1$H NMR ($CDCl_3$, δ ppm): 6.34 (d, J = 14.5 Hz, 1H), 6.84-6.90 (m, 1H), 6.92 (d, J = 11.7 Hz, 1H), 7.00-7.07 (m, 2H), 7.11-7.17 (m, 1H), 7.34-7.40 (m, 4H), 7.43 (dd, J = 11.7, 14.5 Hz, 1H), 7.54 (s, 1H), 7.61 (d, J = 8.1 Hz, 2H), 7.72 (d, J = 8.1 Hz, 2H), 8.86 (s, 1H); ESIMS m/z: $[M+H]^+$ 478.

Example 24

(E)-N-[(5-Hydroxymethyl)pyridin-3-yl]-5,5-bis[4-(trifluoromethyl)phenyl]-2,4-pentadienamide (Compound 25)

In a similar manner to Example 10, Compound 25 was obtained from Compound b using 3-amino-5-(hydroxymethyl)pyridine in place of 5-aminoisoquinoline. mp : 255-257°C; $^1$H NMR (DMSO-$d_6$, δ ppm): 4.49-4.52 (m, 2H), 5.07-5.10 (m, 1H), 6.56 (d, J = 14.7 Hz, 1H), 7.09 (dd, J = 11.7, 14.7 Hz, 1H), 7.23 (d, J = 11.7 Hz, 1H), 7.46 (d, J = 8.2 Hz, 2H), 7.54 (d, J = 8.2 Hz, 2H), 7.72 (d, J = 8.2 Hz, 2H), 7.86 (d, J = 8.2 Hz, 2H), 8.00-8.01 (m, 1H), 8.19-8.20 (m, 1H), 8.65-8.66 (m, 1H), 10.2 (s, 1H); ESIMS m/z: $[M+H]^+$ 493.

Example 25

(E)-N-(Quinolin-3-yl)-5,5-bis[4-(trifluoromethyl)phenyl]-2,4-pentadienamide (Compound 26)

In a similar manner to Example 10, Compound 26 was obtained from Compound b using 3-aminoquinoline in place of 5-aminoisoquinoline.
$^1$H NMR (DMSO-$d_6$, δ ppm) : 6.63 (d, J = 14.5 Hz, 1H), 7.15 (dd, J = 11.5, 14.5 Hz, 1H), 7.27 (d, J = 11.5 Hz, 1H), 7.49 (d, J = 8.1 Hz, 2H), 7.51-7.63 (m, 2H), 7.57 (d, J = 8.1 Hz, 2H), 7.73 (d, J = 8.1 Hz, 2H), 7.84-7.95 (m, 2H), 7.88 (d, J = 8.1 Hz, 2H), 8.67-8.68 (m, 1H), 8.97-8.98 (m, 1H), 10.5 (s, 1H); ESIMS m/z: $[M+H]^+$ 513.

Example 26

(E)-N-(Benzothiazol-6-yl)-5,5-bis[4-(trifluoromethyl)phenyl]-2,4-pentadienamide (Compound 27)

In a similar manner to Example 10, Compound 27 was obtained from Compound b using 6-aminobenzothiazole in place of 5-aminoisoquinoline.
$^1$H NMR ($CDCl_3$, δ ppm) : 6.28 (d, J = 14.7 Hz, 1H), 6.92 (d, J = 11.5 Hz, 1H), 7.33-7.47 (m, 6H), 7.42 (dd, J = 11.5, 14.7 Hz, 1H), 7.60 (d, J = 8.3 Hz, 2H), 7.71 (d, J = 8.3 Hz, 2H), 8.03-8.06 (m, 1H), 8.61 (s, 1H), 8.91 (s, 1H); APCIMS m/z: $[M+H]^+$ 519.

Example 27

(E)-N-(1-Methylisoquinolin-5-yl)-5,5-bis[4-(trifluoromethyl)phenyl]-2,4-pentadienamide (Compound 28)

In a similar manner to Example 10, Compound 28 was obtained from Compound b using 5-amino-1-methylisoquinoline prepared according to the method described in the article [Journal of Medicinal Chemistry, vol. 11, pp.

700-703 (1968)] in place of 5-aminoisoquinoline.
$^1$H NMR (NMSO-$d_6$,δppm): 2.91 (s, 3H), 6.84 (d, J = 14.7 Hz, 1H), 7.12 (dd, J = 11.5 Hz, 14.7 Hz, 1H), 7.34 (d, J = 11.5 Hz, 1H), 7.50 (d, J = 8.1 Hz, 2H), 7.57 (d, J = 8.4 Hz, 2H), 7.66 (t, J = 8.2 Hz, 1H), 7.76 (d, J = 8.4 Hz, 2H), 7.83 (d, J = 6.2 Hz, 1H), 7.90 (d, J = 8.1 Hz, 2H), 8.06 (d, J = 8.2 Hz, 2H), 8.39 (d, J = 6.2 Hz, 1H), 10.31 (s, 1H); APCIMS m/z: $[M+H]^+$ 527.

Example 28

(E)-N-(3-Methylisoquinolin-5-yl)-5,5-bis[4-(trifluoromethyl)phenyl]-2,4-pentadienamide (Compound 29)

In a similar manner to Example 10, Compound 29 was obtained from Compound b using 5-amino-3-methylisoquinoline prepared according to the method described in WO2004/07874 in place of 5-aminoisoquinoline. $^1$H NMR (DMSO-$d_6$,δppm): 2.64 (s, 3H), 6.85 (d, J = 14.8 Hz, 1H), 7.11 (dd, J = 11.6 Hz, 14.8 Hz, 1H), 7.32 (d, J = 11.6 Hz, 1H), 7.46-7.62 (m, 5H), 7.75-7.81 (m, 3H), 7.87-7.92 (m, 3H), 8.05 (d, J = 6.9 Hz, 1H), 9.22 (s, 1H), 10.25 (s, 1H); APCIMS m/z: $[M+H]^+$ 527.

Example 29

(E)-N-(Indol-4-yl)-5,5-bis[4-(trifluoromethyl)phenyl]-2,4-pentadienamide (Compound 30)

In a similar manner to Example 10, Compound 30 was obtained from Compound b using 4-aminoindole in place of 5-aminoisoquinoline.
$^1$H NMR (DMSO-$d_6$,δ ppm): 6.74 (s, 1H), 6.87 (d, J = 14.5 Hz, 1H), 6.95-7.10 (m, 2H), 7.14 (d, J = 8.1 Hz, 1H), 7.23-7.34 (m, 2H), 7.50 (d, J = 8.2 Hz, 2H), 7.55 (d, J = 8.2 Hz, 2H), 7.70 (d, J = 7.1 Hz, 1H), 7.75 (d, J = 8.2 Hz, 2H), 7.91 (d, J = 8.2 Hz, 2H), 9.88 (s, 1H), 11.14 (s, 1H); APCIMS m/z: $[M+H]^+$ 501.

Example 30

(E)-N-(1H-Indazol-4-yl)-5,5-bis[4-(trifluoromethyl)phenyl]-2,4-pentadienamide (Compound 31)

In a similar manner to Example 10, Compound 31 was obtained from Compound b using 4-amino-1H-indazole in place of 5-aminoisoquinoline.
$^1$H NMR (DMSO-$d_6$, δ ppm): 6.82 (d, J = 14.6 Hz, 1H), 7.11 (dd, J = 11.5, 14.6 Hz, 1H), 7.21-7.28 (m, 2H), 7.31 (d, J = 11.5 Hz, 1H), 7.50 (d, J = 8.0 Hz, 2H), 7.56 (d, J = 8.0 Hz, 2H), 7.70-7.83 (m, 3H), 7.91 (d, J = 8.0 Hz, 2H), 8.31 (s, 1H), 10.29 (s, 1H), 13.09 (s, 1H); APCIMS m/z: $[M+H]^+$ 502.

Example 31

(E)-N-(3-Hydroxy-2-oxo-1,2,3,4-tetrahydroquinolin-5-yl)-5,5-bis[4-(trifluoromethyl)phenyl]-2,4-pentadienamide (Compound 32)

In a similar manner to Example 10, Compound 32 was obtained from Compound b using 5-amino-3-hydroxy-2-oxo-1,2,3,4-tetrahydroquinoline prepared according to the method described in the article [Bioorganic and Medicinal Chemistry Letters, vol. 10, pp. 1459-1462 (2000)] in place of 5-aminoisoquinoline.
$^1$H NMR (DMSO-$d_6$, δ ppm): 2.61 (dd, J = 11.9, 15.8 Hz, 1H), 3.00 (dd, J = 6.0 Hz, 15.8 Hz, 1H), 4.01-4.11 (m, 1H), 5.44 (d, J = 4.6 Hz, 1H), 6.63-6.73 (m, 2H), 6.98-7.16 (m, 3H), 7.29 (d, J = 11.7 Hz, 1H), 7.48 (d, J = 8.2 Hz, 2H), 7.54 (d, J = 8.2 Hz, 2H), 7.75 (d, J = 8.2 Hz, 2H), 7.89 (d, J = 8.2 Hz, 2H), 9.78 (s, 1H), 10.17 (s, 1H); APCIMS m/z: $[M+H]^+$ 547.

Example 32

(E)-N-(2-Carbamoylphenyl)-5,5-bis[4-(trifluoromethyl)phenyl]-2,4-pentadienamide (Compound 33)

In a similar manner to Example 10, Compound 33 was obtained from Compound b using 2-aminobenzamide in place of 5-aminoisoquinoline.
$^1$H NMR (DMSO-$d_6$,δ ppm): 6.56 (d, J = 14.6 Hz, 1H), 7.01-7.16 (m, 2H), 7.40 (d, J = 11.7 Hz, 1H), 7.44-7.59 (m, 6H), 7.76 (d, J = 8.3 Hz, 2H), 7.80-7.92 (m, 3H), 8.32 (s, 1H), 8.49 (d, J = 8.0 Hz, 1H), 12.04 (s, 1H); APCIMS m/z: $[M+H]^+$ 505.

Example 33

(E)-N-(3-Carbamoylphenyl)-5,5-bis[4-(trifluoromethyl)phenyl]-2,4-pentadienamide (Compound 34)

In a similar manner to Example 10, Compound 34 was obtained from Compound b using 3-aminobenzamide in place of 5-aminoisoquinoline.
$^{1}$H NMR (DMSO-$d_6$,δ ppm): 6.58 (d, J = 14.7 Hz, 1H), 7.07 (dd, J = 11.6, 14.7 Hz, 1H), 7.28-7.43 (m, 3H), 7.45-7.64 (m, 5H), 7.76 (d, J = 8.2 Hz, 2H), 7.84 (d, J = 8.4 Hz, 1H). 7.91 (d, J = 8.2 Hz, 2H), 7.92 (s, 1H), 8.07 (s, 1H), 10.41 (s, 1H); APCIMS m/z: $[M+H]^+$ 505.

Example 34

(E)-N-(2-Sulfamoylphenyl)-5,5-bis[4-(trifluoromethyl)phenyl]-2,4-pentadienamide (Compound 35)

In a similar manner to Example 10, Compound 35 was obtained from Compound b using 2-aminobenzenesulfonamide in place of 5-aminoisoquinoline.
$^{1}$H NMR (DMSO-$d_6$,δ ppm): 6.60 (d, J = 14.7 Hz, 1H), 7.06 (dd, J = 11.5, 14.7 Hz, 1H), 7.28 (t, J = 7.9 Hz, 1H), 7.39 (d, J = 11.5 Hz, 1H), 7.46-7.60 (m, 5H), 7.76 (d, J = 8.4 HZ, 2H), 7.85 (dd, J = 1.5, 7.9 Hz, 1H), 7.90 (d, J = 8.3 Hz, 2H), 8.23 (d, J = 7.9 Hz, 1H); APCIMS m/z: $[M+H]^+$ 541.

Example 35

(E)-N-(1,4-Benzodioxan-5-yl)-5,5-bis[4-(trifluoromethyl)phenyl]-2,4-pentadienamide (Compound 36)

In a similar manner to Example 10, Compound 36 was obtained from Compound b using 5-amino-1,4-benzodioxane in place of 5-aminoisoquinoline.
$^{1}$H NMR (DMSO-$d_6$,δ ppm): 4.26 (d, J = 4.9 Hz, 2H), 4.31 (d, J = 4.9 Hz, 2H), 6.62 (dd, J = 1.5, 8.2 Hz, 1H), 6.74 (t, J = 8.2 Hz, 1H), 6.82 (d, J = 14.7 Hz, 1H), 7.02 (dd, J = 11.4 Hz, 14.7 Hz, 1H), 7.20 (d, J = 11.4 Hz, 1H), 7.44-7.60 (m, 5H), 7.75 (d, J = 8.1 Hz, 2H), 7.90 (d, J = 8.1 Hz, 2H), 9.49 (s, 1H); APCIMS m/z: $[M+H]^+$ 520.

Example 36

(E)-N-(3-Hydroxy-1,2,3,4-tetrahydroquinolin-5-yl)-5,5-bis[4-(trifluoromethyl)phenyl]-2,4-pentadienamide (Compound 37)

In a similar manner to Example 10, Compound 37 was obtained from Compound b using 5-amino-3-hydroxy-1,2,3,4-tetrahydroquinoline prepared according to the method described in WO2005/04480 in place of 5-aminoisoquinoline. $^{1}$H NMR (DMSO-$d_6$, δ ppm): 2.35 (dd, J = 8.4, 15.9 Hz, 1H), 2.70 (dd, J = 5.3, 15.9 Hz, 1H), 2.82 (t, J = 9.4 Hz, 1H), 3.17 (d, J = 11.2 Hz, 1H), 3.75-3.90 (m, 1H), 4.87 (d, J = 4.3 Hz, 1H) 5.68 (s, 1H), 6.29 (d, J = 7.9 Hz, 1H), 6.55 (d, J = 7.9 Hz, 1H), 6.64 (d, J = 14.5 Hz, 1H), 6.80 (t, J = 7.9 Hz, 1H), 6.99 (dd, J = 11.6, 14.5 Hz, 1H), 7.25 (d, J = 11.6 Hz, 1H), 7.46 (d, J = 7.9 Hz, 2H), 7.52 (d, J = 7.9 Hz, 2H), 7.73 (d, J = 7.9 Hz, 2H), 7.88 (d, J = 7.9 Hz, 2H), 9.37 (s, 1H); APCIMS m/z: $[M+H]^+$ 533.

Example 37

(E)-N-(1-Methyl-2-oxo-1,2-dihydroquinolin-5-yl)-5,5-bis[4-(trifluoromethyl)phenyl]-2,4-pentadienamide (Compound 38)

In a similar manner to Example 10, Compound 38 was obtained from Compound b using 5-amino-1-methyl-2-oxo-1,2-dihydroquinoline prepared according to the method described in WO2005/01691 in place of 5-aminoisoquinoline.
$^{1}$H NMR (DMSO-$d_6$, δ ppm): 3.61 (s, 3H), 6.62 (d, J = 9.9 Hz, 1H), 6.72 (d, J = 14.9 Hz, 1H), 7.07 (dd, J = 11.7, 14.9 Hz, 1H), 7.31 (d, J = 11.7 Hz, 1H), 7.36 (d, J = 8.9 Hz, 1H), 7.42 (d, J = 7.6 Hz, 1H), 7.48 (d, J = 8.3 Hz, 2H), 7.51-7.62 (m, 3H), 7.75 (d, J = 8.3 Hz, 2H), 7.88 (d, J = 8.3 Hz, 2H), 7.95 (d, J = 9.9 Hz, 1H), 10.25 (s, 1H); APCIMS m/z: $[M+H]^+$ 543.

Example 38

(E)-N-(3-Hydroxy-1-methyl-1,2,3,4-tetrahydroquinolin-5-yl)-5,5-bis[4-(trifluoromethyl)phenyl]-2,4-pentadienamide (Compound 39)

In a similar manner to Example 10, Compound 39 was obtained from Compound b using 5-amino-3-hydroxy-1-methyl-1,2,3,4-tetrahydroquinoline prepared according to the method described in WO2005/044802 in place of 5-aminoisoquinoline.
$^1$H NMR (DMSO-$d_6$, δ ppm): 2.39 (dd, J = 8.4, 16.5 Hz, 1H), 2.77 (dd, J = 5.4, 16.5 Hz, 1H), 2.81 (s, 3H), 2.89 (dd, J = 8.0, 11.0 Hz, 1H), 3.20 (dd, J = 2.3, 11.0 Hz, 1H), 3.85-3.96 (m, 1H), 4.96 (d, J = 4.5 Hz, 1H), 6.42 (d, J = 8.3 Hz, 1H), 6.58-6.70 (m, 2H), 6.90-7.06 (m, 2H), 7.26 (d, J = 11.6 Hz, 1H), 7.46 (d, J = 8.1 Hz, 2H), 7.52 (d, J = 8.1 Hz, 2H), 7.73 (d, J = 8.1 Hz, 2H), 7.88 (d, J = 8.1 Hz, 2H), 9.43 (s, 1H); APCIMS m/z: $[M+H]^+$ 547.

Example 39

(E)-N-(1,3-Dioxo-2,3-dihydro-1H-isoindol-4-yl)-5,5-bis[4-(trifluoromethyl)phenyl]-2,4-pentadienamide (Compound 40)

In a similar manner to Example 10, Compound 40 was obtained from Compound b using 4-amino-1,3-dioxo-2,3-dihydro-1H-isoindole in place of 5-aminoisoquinoline.
$^1$H NMR (DMSO-$d_6$, δ ppm): 6.72 (s, 1H), 7.00-7.24 (m, 4H), 7.42 (dd, J = 1.6, 9.2 Hz, 1H), 7.46-7.65 (m, 5H), 7.76 (d, J = 8.4 Hz, 2H), 7.90 (d, J = 8.3 Hz, 2H); APCIMS m/z: $[M+H]^+$ 531.

Example 40

(E)-N-(1,3-Dioxo-2,3-dihydro-1H-isoindol-5-yl)-5,5-bis[4-(trifluoromethyl)phenyl]-2,4-pentadienamide (Compound 41)

In a similar manner to Example 10, Compound 41 was obtained from Compound b using 5-amino-1,3-dioxo-2,3-dihydro-1H-isoindole in place of 5-aminoisoquinoline.
$^1$H NMR (DMSO-$d_6$, δ ppm) : 6.58 (d, J = 14.5 Hz, 1H), 7.11 (dd, J = 11.6, 14.5 Hz, 1H), 7.34 (d, J = 11.6 Hz, 1H), 7.48 (d, J = 7.9 Hz, 2H), 7.55 (d, J = 8.1 Hz, 2H), 7.72-7.79 (m, 3H), 7.85-7.94 (m, 3H), 8.19 (s, 1H); APCIMS m/z: $[M+H]^+$ 531.

Example 41

(E)-N-(Benzofurazan-4-yl)-5,5-bis[4-(trifluoromethyl)phenyl]-2,4-pentadienamide (Compound 42)

In a similar manner to Example 10, Compound 42 was obtained from Compound b using 4-aminobenzofurazan in place of 5-aminoisoquinoline.
$^1$H NMR (DMSO-$d_6$, δ ppm): 6.94 (d, J = 14.5 Hz, 1H), 7.13 (dd, J = 11.4, 14.5 Hz, 1H), 7.28 (d, J = 11.4 Hz, 1H), 7.50 (d, J = 8.2 Hz, 2H), 7.53-7.63 (m, 3H), 7.70 (d, J = 9.1 Hz, 1H), 7.76 (d, J = 8.2 Hz, 2H), 7.91 (d, J = 8.2 Hz, 2H), 8.19 (d, J = 7.3 Hz, 1H), 11.03 (s, 1H); APCIMS m/z: $[M+H]^+$ 504.

Example 42

(E)-N-(1-oxo-2,3-dihydro-1H-isoindol-7-yl)-5,5-bis[4-(trifluoromethyl)phenyl]-2,4-pentadienamide (Compound 43)

In a similar manner to Example 10, Compound 43 was obtained from Compound b using 7-amino-1-oxo-2,3-dihydro-1H-isoindole in place of 5-aminoisoquinoline.
$^1$H NMR (DMSO-$d_6$, δ ppm): 4.39 (s, 2H), 6.65 (d, J = 14.7 Hz, 1H), 7.09 (dd, J = 11.5, 14.7 Hz, 1H), 7.24 (d, J = 7.7 Hz, 1H), 7.42 (d, J = 11.5 Hz, 1H), 7.47-7.59 (m, 5H), 7.77 (d, J = 8.2 Hz, 2H), 7.91 (d, J = 8.2 Hz, 2H), 8.29 (d, J = 8.2 Hz, 1H), 8.88 (s, 1H), 10.66 (s, 1H); APCIMS m/z: $[M+H]^+$ 517.

Example 43

(E)-5,5-Diphenyl-N-(isoquinolin-5-yl)-2,4-pentadienamide (Compound 44)

In a similar manner to Example 10, Compound 44 was obtained from Compound c.
$^1$H NMR ($CDCl_3$, δ ppm): 6.29 (d, J = 14.8 Hz, 1H), 6.84 (d, J = 11.5 Hz, 1H), 7.22-7.25 (m, 2H), 7.32 (s, 5H), 7.38-7.42 (m, 3H), 7.53 (dd, J = 11.5, 14.8 Hz, 1H), 7.56-7.62 (m, 3H), 7.79-7.82 (m, 1H), 8.20-8.22 (m, 1H), 8.54-8.56 (m, 1H),

9.25 (s, 1H); ESIMS m/z: $[M+H]^+$ 377.

Example 44

(D)-N-(Isoquinolin-5-yl)-5,5-bis[6-(trifluoromethyl)pyridin-3-yl]-2,4-pentadienamide (Compound 45)

In a similar manner to Example 10, Compound 45 was obtained from Compound h. $^1$H NMR (DMSO-$d_6$, δ ppm): 6.94 (d, J = 14.8 Hz, 1H), 7.12 (dd, J = 11.4, 14.8 Hz, 1H), 7.57 (d, J = 11.4 Hz, 1H), 7.67 (t, J = 7.9 Hz, 1H), 7.90-7.98 (m, 5H), 8.09-8.13 (m, 3H), 8.56 (d, J = 5.9 Hz, 1H), 8.75 (s, 1H), 8.92 (s, 1H), 9.33 (s, 1H); APCIMS m/z: $[M+H]^+$ 515.

Example 45

(E)-N-(Isoquinolin-5-yl)-5,5-bis[3-(trifluoromethyl)phenyl]-2,4-pentadienamide (Compound 46)

In a similar manner to Example 10, Compound 46 was obtained from Compound d. $^1$H NMR ($EDCl_3$, δ ppm): 6.39 (d, J = 15.0 Hz, 1H), 5.92 (d, J = 11.6 Hz, 1H), 7.36-7.47 (m, 4H), 7.42 (dd, J = 11.6, 15.0 Hz, 1H), 7.56-7.63 (m, 6H), 7.68-7.71 (m, 1H), 7.82-7.85 (m, 1H), 8.18 (s, 1H), 8.55-8.58 (m, 1H), 9.26 (s, 1H); ESIMS m/z: $[M+H]^+$ 513.

Example 46

(E)-N-(Isoquinolin-5-yl)-5,5-bis[4-(trifluoromethoxy)phenyl]-2,4-pentadienamide (Compound 47)

In a similar manner to Example 10, Compound 47 was obtained from Compound e. $^1$H NMR ($CDCl_3$, δ ppm): 6.33 (d, J = 14.9 Hz, 1H), 6.83 (d, J = 11.8 Hz, 1H), 7.17-7.20 (m, 2H), 7.27-7.33 (m, 6H), 7.49 (dd, J = 11.8, 14.9 Hz, 1H), 7.56-7.64 (m, 3H), 7.82-7.85 (m, 1H), 8.21 (s, 1H), 8.56-8.58 (m, 1H), 9.27 (s, 1H); ESIMS m/z: $[M+H]^+$ 545.

Example 47

(E)-5,5-Bis(4-chlorophenyl)-N-(isoquinolin-5-yl)-2,4-pentadienamide (Compound 48)

In a similar manner to Example 10, Compound 48 was obtained from Compound f. $^1$H NMR ($CDCl_3$, δ ppm): 6.31 (d, J = 14.9 Hz, 1H), 6.80 (d, J = 11.7 Hz, 1H), 7.14-7.32 (m, 6H), 7.39-7.42 (m, 2H), 7.47 (dd, J = 11.7, 14.9 Hz, 1H), 7.56-7.63 (m, 3H), 7.81-7.84 (m, 1H), 8.20 (s, 1H), 8.55-8.57 (m, 1H), 9.26 (s, 1H); ESIMS m/z: $[M+H]^+$ 445.

Example 48

(E)-5,5-Bis(4-tert-buthylphenyl)-N-(isoquinolin-5-yl)-2,4-pentadienamide (Compound 49)

In a similar manner to Example 10, Compound 49 was obtained from Compound g. $^1$H NMR ($CDCl_3$, δ ppm) : 1.33 (s, 9H), 1.36 (s, 9H), 6.26 (d, J = 14.5 Hz, 1H), 6.81 (d, J = 11.7 Hz, 1H), 7.15-7.18 (m, 2H), 7.25-7.43 (m, 6H), 7.54-7.63 (m, 3H), 7.59 (dd, J = 11.7, 14.5 Hz, 1H), 7.79-7.82 (m, 1H), 8.22-8.24 (m, 1H), 8.54-8.56 (m, 1H), 9.25 (s, 1H); ESIMS m/z: $[M+H]^+$ 489.

Example 49

(E)-N-(Isoquinolin-5-yl)-5,5-bis(2-methylphenyl)-2,4-pentadienamide (Compound 50)

In a similar manner to Example 10, Compound 50 was obtained from Compound i. $^1$H NMR ($CDCl_3$, δ ppm): 2.08 (s, 3H), 2.25 (s, 3H), 6.23 (d, J = 14.8 Hz, 1H), 6.55 (d, J = 11.5 Hz, 1H), 7.12-7.22 (m, 8H), 7.37 (dd, J = 11.5, 14.8 Hz, 1H), 7.56-7.62 (m, 3H), 7.79-7.82 (m, 1H), 8.21-8.23 (m, 1H), 8.54-8.56 (m, 1H), 9.25 (s, 1H); ESIMS m/z: $[M+H]^+$ 405.

Example 50

(E)-N-(Isoquinolin-5-yl)-5,5-bis(4-methylphenyl)-2,4-pentadienamide (Compound 51)

In a similar manner to Example 10, Compound 51 was obtained from Compound j.
$^1$H NMR (DMSO-$d_6$, δ ppm): 2.32 (s, 3H), 2.38 (s, 3H), 6.69 (d, J = 14.7 Hz, 1H), 7.01 (d, J = 11.7 Hz, 1H), 7.09 (d, J = 7.9 Hz, 2H), 7.15-7.34 (m, 7H), 7.65 (t, J = 7.9 Hz, 1H), 7.93 (d, J = 7.9 Hz, 1H), 7.96-8.01 (m, 1H), 8.08-8.16 (m, 1H), 8.54 (d, J = 5.9 Hz, 1H), 9.31 (s, 1H), 10.19 (s, 1H); APCIMS m/z: $[M+H]^+$ 405.

Example 51

(E)-N-(Isoquinolin-5-yl)-5,5-bis(3-nitrophenyl)-2,4-pentadienamide (Compound 52)

In a similar manner to Example 10, Compound 52 was obtained from Compound k.
$^1$H NMR (DMSO-$d_6$, δ ppm) : 6.88 (d, J = 14.8 Hz, 1H), 7.10 (dd, J = 11.4, 14.8 Hz, 1H), 7.43 (d, J = 11.4 Hz, 1H), 7.60-7.90 (m, 5H), 7.90-8.00 (m, 2H), 8.04-8.15 (m, 3H), 8.23 (d, J = 7.8 Hz, 1H), 8.36 (d, J = 8.2 Hz, 1H), 8.54 (d, J = 6.2 Hz, 1H), 9.31 (s, 1H), 10.35 (s, 1H); APCIMS m/z: $[M+H]^+$ 467.

Example 52

(E)-5,5-Bis(3-fluorophenyl)-N-(isoquinolin-5-yl)-2,4-pentadienamide (Compound 53)

In a similar manner to Example 10, Compound 53 was obtained from Compound 1.
$^1$H NMR (DMSO-$d_6$, δ ppm): 6.78 (d, J = 14.5 Hz, 1H), 7.05-7.48 (m, 9H), 7.52-7.70 (m, 2H), 7.91-8.00 (m, 2H), 8.11 (d, J = 7.6 Hz, 1H), 8.55 (d, J = 6.1 Hz, 1H), 9.32 (s, 1H), 10.30 (s, 1H); APCIMS m/z: $[M+H]^+$ 413.

Example 53

(E)-5,5-Bis(4-fluorophenyl)-N-(isoquinolin-5-yl)-2,4-pentadienamide (Compound 54)

In a similar manner to Example 10, Compound 54 was obtained from Compound m.
$^1$H NMR (DMSO-$d_6$, δ ppm): 6.74 (d, J = 13.4 Hz, 1H) 7.04-7.48 (m, 10H), 7.65 (t, J = 7.7 Hz, 1H), 7.93 (d, J = 8.4 Hz, 1H), 7.97 (d, J = 5.7 Hz, 1H), 8.11 (d, J = 7.7 Hz, 1H), 8.54 (d, J = 5.7 Hz, 1H), 9.31 (s, 1H), 10.24 (s, 1H); APCIMS m/z: $[M+H]^+$ 413.

Example 54

(E)-5,5-Bis(4-cyanophenyl)-N-(isoquinolin-5-yl)-2,4-pentadienamide (Compound 55)

In a similar manner to Example 10, Compound 55 was obtained from Compound n.
$^1$H NMR (DMSO-$d_6$, δ ppm): 6.86 (d, J = 14.7 Hz, 1H), 7.07 (dd, J = 11.4, 14.7 Hz, 1H), 7.38 (d, J = 11.4 Hz, 1H), 7.46 (d, J = 8.5 Hz, 2H), 7.52 (d, J = 8.8 Hz, 2H), 7.66 (t, J = 7.8 Hz, 1H), 7.87 (d, J = 8.8 Hz, 2H), 7.91-8.04 (m, 4H), 8.11 (d, J = 7.8 Hz, 1H), 8.55 (d, J = 6.1 Hz, 1H), 9.32 (d, J = 0.7 Hz, 1H), 10.36 (s, 1H) ; APCIMS m/z: $[M+H]^+$ 427.

Example 55

(E)-N-(Isoquinolin-5-yl)-5,5-bis(4-methoxyphenyl)-2,4-pentadienamide (Compound 56)

In a similar manner to Example 10, Compound 56 was obtained from Compound o.
$^1$H NMR (DMSO-$d_6$, δ ppm): 3.78 (s, 3H), 3.83 (s, 3H), 6.57 (d, J = 14.8 Hz, 1H), 6.93 (d, J = 11.6 Hz, 1H), 6.95 (d, J = 8.9 Hz, 2H), 7.05 (d, J = 8.9 Hz, 2H), 7.13 (d, J = 8.9 Hz, 2H), 7.24 (dd, J = 11.8, 14.8 Hz, 1H), 7.29 (d, J = 8.9 Hz, 2H), 7.65 (t, J = 7.9 Hz, 1H), 7.92 (d, J = 7.9 Hz, 1H), 7.99 (d, J = 6.0 Hz, 1H), 8.13 (d, J = 7.9 Hz, 1H), 8.54 (d, J = 6.0 Hz, 1H), 9.31 (s, 1H), 10.17 (s, 1H); APCIMS m/z: $[M+H]^+$ 437.

Example 56

(E)-N-(7-Hydroxy-5,6,7,8-tetrahydronaphthalen-1-yl)-5,5-bis[6-(trifluoromethyl)pyridine-3-yl]-2,4-pentadienamide (Compound 57)

In a similar manner to Example 1, Compound 57 was obtained from Compound h using 8-amino-5,6,7,8-tetrahydro-2-naphthol in place of thiomorpholine.
$^1$H NMR (DMSO-$d_6$, δ ppm): 1.56-1.62 (m, 1H) 1.84-1.89 (m, 1H), 2.38-2.44 (m, 1H), 2.73-2.89 (m, 3H), 3.86-3.89 (m, 1H), 4.80-4.81 (m, 1H), 6.77 (d, J = 15.0 Hz, 1H), 6.91-7.09 (m, 2H), 7.02 (dd, J = 11.6, 15.0 Hz, 1H), 7.21-7.24 (m, 1H), 7.51 (d, J = 11.6 Hz, 1H), 7.88-7.94 (m, 2H), 8.03-8.10 (m, 2H), 8.72 (s, 1H), 8.89 (s, 1H), 9.56 (s, 1H); APCIMS m/z: $[M+H]^+$ 534.

Example 57

(E)-5,5-Bis[4-(difluoromethyl)phenyl]-N-(7-hydroxy-5,6,7,8-tetrahydronaphthalen-1-yl)-2,4-pentadienamide (Compound 58)

In a similar manner to Example 1, Compound 58 was obtained from Compound p using 8-amino-5,6,7,8-tetrahydro-2-naphthol in place of thiomorpholine.
$^1$H NMR (DMSO-$d_6$, δ ppm): 1.55-1.61 (m, 1H), 1.85-1.88 (m, 1H), 2.40-2.46 (m, 1H), 2.72-2.87 (m, 3H), 3.86-3.88 (m, 1H), 4.79-4.80 (m, 1H), 6.64 (d, J = 14.5 Hz, 1H), 6.87-7.32 (m, 5H), 7.05 (dd, J = 11.7, 14.5 Hz, 1H), 7.19 (d, J = 11.7 Hz, 1H), 7.38 (d, J = 8.2 Hz, 2H), 7.46 (d, J = 8.2 Hz, 2H), 7.58 (d, J = 8.2 Hz, 2H), 7.71 (d, J = 8.2 Hz, 2H), 9.43 (s, 1H); APCIMS m/z: $[M+H]^+$ 496.

Example 58

(2E,4E)-N-(Isoquinolin-5-yl)-5-phenyl-5-[4-(trifluoromethyl)phenyl]-2,4-pentadienamide (Compound 59)

In a similar manner to Example 10, Compound 59 was obtained from Compound r.
mp :114-115˚C; $^1$H NMR (DMSO-$d_6$, δ ppm): 6.81 (d, J = 13.2 Hz, 1H), 7.16-7.26 (m, 4H), 7.49-7.58 (m, 5H), 7.66 (t, J = 7.9 Hz, 1H), 7.75 (d, J = 8.2 Hz, 2H), 7.94 (d, J = 8.2 Hz, 1H), 7.98 (d, J = 6.0 Hz, 1H), 8.12 (d, J = 7.5 Hz, 1H), 8.55 (d, J = 6.0 Hz, 1H), 9.32 (s, 1H), 10.29 (s, 1H) APCIMS m/z: $[M+H]^+$ 445.

Example 59

(2E,4Z)-N-(Isoquinolin-5-yl)-5-(pyridin-3-yl)-5-[4-(trifluoromethyl)phenyl]-2,4-pentadienamide (Compound 60)

In a similar manner to Example 10, Compound 60 was obtained from Compound s.
$^1$H NMR (DMSO-$d_6$, δ ppm): 6.87 (d, J = 14.7 Hz, 1H), 7.14 (dd, J = 11.6, 14.9 Hz, 1H), 7.37 (d, J = 11.6 Hz, 1H), 7.55-7.60 (m, 3H), 7.16-7.72 (m, 2H), 7.78 (d, J = 8.1 Hz, 2H), 7.94-7.99 (m, 2H), 8.12 (d, J = 7.5 Hz, 1H), 8.49 (d, J = 1.7 Hz, 1H), 8.56 (d, J = 6.0 Hz, 1H), 8.70 (dd, J = 1.7, 4.7 Hz, 1H), 9.33 (s, 1H); APCIMS m/z: $[M+H]^+$ 446.

Example 60

(2E,4Z)-N-(Isoquinolin-5-yl)-5-(pyridin-4-yl)-5-[4-(trifluoromethyl)phenyl]-2,4-pentadienamide (Compound 61)

In a similar manner to Example 10, Compound 61 was obtained from Compound t.
$^1$H NMR (DMSO-$d_6$, δ ppm): 6.86 (d, J = 16.4 Hz, 1H), 7.11 (dd, J = 12.7, 16.4 Hz, 1H), 7.31 (dd, J = 1.6, 4.9 Hz, 2H), 7.36 (d, J = 12.7 Hz, 1H), 7.57 (d, J = 9.2 Hz, 2H), 7.56 (t, J = 8.7 Hz, 1H), 7.77 (d, J = 9.2 Hz, 2H) , 7.94-7.98 (m, 2H), 8.11 (d, J = 8.0 Hz, 1H), 8.55 (d, J = 6.8 Hz, 1H), 8.74 (dd, J = 1.6, 4.9 Hz, 2H), 9.32 (s, 1H); APCIMS m/z: $[M+H]^+$ 446.

Example 61

(2E,4Z)-N-(Isoquinolin-5-yl)-5-[4-(trifluoromethyl)phenyl]-5-[6-(trifluoromethyl)pyridin-3-yl]-2,4-pentadienamide (Compound 62)

In a similar manner to Example 10, Compound 62 was obtained from Compound u.
$^1$H NMR (DMSO-$d_6$, δ ppm) : 6.90 (d, J = 14.7 Hz, 1H), 7.08 (dd, J = 11.5, 14.7 Hz, 1H), 7.47 (d, J = 11.5 Hz, 1H), 7.61

(d, J = 8.1 Hz, 2H), 7.67 (t, J = 7.8 Hz, 1H), 7.78 (d, J = 8.1 Hz, 2H), 7.94-8.12 (m, 5H), 8.55 (d, J = 6.2 Hz, 1H), 8.08 (s, 1H) 9.33 (s, 1H); APCIMS m/z: $[M+H]^+$ 514.

Example 62

(2E,4Z)-N-(Isoquinolin-5-yl)-5-(furan-2-yl)-5-[4-(trifluoromethyl)phenyl]-2,4-pentadienamide (Compound 63)

In a similar manner to Example 10, Compound 63 was obtained from Compound v.
$^1$H NMR (DMSO-$d_6$, δ ppm): 6.55 (d, J = 3.3 Hz, 1H), 6.69-6.84 (m, 3H), 7.64 (d, J = 8.4 Hz, 2H), 7.70 (t, J = 8.0 Hz, 1H), 7.81 (d, J = 8.4 Hz, 2H), 7.92-8.03 (m, 4H), 8.19 (d, J = 7.5 Hz, 1H), 8.56 (d, J = 6.0 Hz, 1H), 9.33 (s, 1H), 10.32 (s, 1H); APCIMS m/z: $[M+H]^+$ 435.

Example 63

(2E,4Z)-N-(Isoquinolin-5-yl)-5-(furan-3-yl)-5-[4-(trifluoromethyl)phenyl]-2,4-pentadienamide (Compound 64)

In a similar manner to Example 10, Compound 64 was obtained from Compound w.
$^1$H NMR (DMSO-$d_6$, δ ppm): 6.50 (s, 1H), 6.81 (d, J = 14.1 Hz, 1H), 7.12 (d, J = 11.4 Hz, 1H), 7.54 (dd, J = 11.4, 14.1 Hz, 1H), 7.67-7.59 (m, 3H), 7.78 (d, J = 7.8 Hz, 2H), 7.90-8.01 (m, 4H), 8.16 (d, J = 6.9 Hz, 1H) 8.56 (d, J = 6.0 Hz, 1H), 9.33 (s, 1H), 10.30 (s, 1H); APCIMS m/z: $[M+H]^+$ 435.

Example 64

(2E,4Z)-N-(Isoquinolin-5-yl)-5-(thiophen-2-yl)-5-[4-(trifluoromethyl)phenyl]-2,4-pentadienamide (Compound 65)

In a similar manner to Example 10, Compound 65 was obtained from Compound x.
$^1$H NMR (DMSO-$d_6$, δ ppm): 6.86 (d, J = 15.0 Hz, 1H), 7.13 (d, J = 11.7 Hz, 1H), 7.18 (dd, J = 1.1, 3.5 Hz, 1H), 7.25 (dd, J = 3.5, 5.1 Hz, 1H), 7.58 (dd, J = 11.7, 15.0 Hz, 1H), 7.64-7.71 (m, 3H), 7.79 (d, J = 8.4 Hz, 2H), 7.82 (dd, J = 0.9, 5.1 Hz, 1H), 7.96 (d, J = 8.1 Hz, 1H), 8.00 (d, J = 6.0 Hz, 1H), 8.16 (d, J = 7.2 Hz, 1H), 8.56 (d, J = 6.0 Hz, 1H), 9.33 (s, 1H), 10.34 (s, 1H); APCIMS m/z: $[M+H]^+$ 451.

Example 65

(2E,4Z)-N-(Isoquinolin-5-yl)-5-(thiophen-3-yl)-5-[4-(trifluoromethyl)phenyl]-2,4-pentadienamide (Compound 66)

In a similar manner to Example 10, Compound 66 was obtained from Compound y.
$^1$H NMR (DMSO-$d_6$, δ ppm): 6.81 (d, J = 15.0 Hz, 1H), 7.02 (d, J = 4.8 Hz, 1H), 7.16 (d, J = 11.5 Hz, 1H), 7.41 (dd, J = 11.5, 15.0 Hz, 1H), 7.58-7.61 (m, 3H), 7.65-7.78 (m, 4H), 7.95 (d, J = 8.1 Hz, 1H), 7.99 (d, J = 5.0 Hz, 1H), 8.14 (d, J = 7.5 Hz, 1H), 8.56 (d, J = 6.0 Hz, 1H), 9.33 (s, 1H), 10.30 (s, 1H); APCIMS m/z: $[M+H]^+$ 451.

Example 66

(2E,4Z)-5-(2-Cyanophenyl)-N-(isoquinolin-5-yl)-5-[4-(trifluoromethyl)phenyl]-2,4-pentadienamide (Compound 67)

In a similar manner to Example 10, Compound 67 was obtained from Compound z.
$^1$H NMR (DMSO-$d_6$, δ ppm): 6.89-6.91 (m, 2H), 7.53-7.58 (m, 4H), 7.63-7.79 (m, 4H), 7.88-7.98 (m, 3H), 8.05-8.11 (m, 2H), 8.55 (d, J = 6.0 Hz, 1H), 9.32 (s, 1H), 10.38 (s, 1H); APCIMS m/z: $[M+H]^+$ 470.

Example 67

(2E,4Z)-5-(3-Cyanophenyl)-N-(isoquinolin-5-yl)-5-[4-(trifluoromethyl)phenyl]-2,4-pentadienamide (Compound 68)

In a similar manner to Example 10, Compound 68 was obtained from Compound aa.
mp : 118-119°C; $^1$H NMR (DMSO-$d_6$, δ ppm): 6.85 (d, J = 15.0 Hz, 1H), 7.08 (dd, J = 11.5, 15.0 Hz, 1H), 7.35 (d, J = 11.5 Hz, 1H), 7.55-7.78 (m, 8H), 7.94-7.99 (m, 3H), 8.11 (d, J = 6.9 Hz, 1H), 8.55 (d, J = 6.3 Hz, 1H), 9.32 (s, 1H); APCIMS m/z: $[M+H]^+$ 470.

Example 68

(2E,4E)-5-(4-Cyanophenyl)-N-(isoquinolin-5-yl)-5-[4-(trifluoromethyl)phenyl]-2,4-pentadienamide (Compound 69)

In a similar manner to Example 10, Compound 69 was obtained from Compound ab.
$^1$H NMR (DMSO-$d_6$, δ ppm): 6.86 (d, J = 14.9 Hz, 1H), 7.10 (dd, J = 11.6, 14.9 Hz, 1H), 7.34 (d, J = 11.6 Hz, 1H), 7.48 (d, J = 8.1 Hz, 2H), 7.56 (d, J = 8.4 Hz, 2H), 7.67 (t, J = 7.8 Hz, 1H), 7.77 (d, J = 8.7 Hz, 2H), 7.94-7.98 (m, 2H), 8.01 (d, J = 8.1 Hz, 2H), 8.11 (d, J = 6.9 Hz, 1H), 8.56 (d, J = 6.0 Hz, 1H), 9.33 (s, 1H), 10.35 (s, 1H); APCIMS m/z: $[M+H]^+$ 470.

Example 69

(2E,4Z)-N-(Isoquinolin-5-yl)-5-(3-methylphenyl)-5-[4-(trifluoromethyl)phenyl]2,4-pentadienamide (Compound 70)

In a similar manner to Example 10, Compound 70 was obtained from Compound ac.
$^1$H NMR (DMSO-$d_6$, δ ppm): 2.36 (s, 3H), 6.78-6.85 (m, 1H), 7.04-7.06 (m, 2H), 7.19-7.22 (m, 2H), 7.30 (d, J = 7.5 Hz, 1H), 7.42 (t, J = 7.9 Hz, 1H), 7.56 (d, J = 8.1 Hz, 2H), 7.66 (t, J = 7.8 Hz, 1H), 7.75 (d, J = 8.4 Hz, 2H), 7.94 (d, J = 8.1 Hz, 1H), 7.98 (d, J = 6.0 Hz, 1H), 8.12 (d, J = 7.5 Hz, 1H), 8.55 (d, J = 6.0 Hz, 1H), 9.32 (s, 1H), 10.29 (s, 1H); APCIMS m/z: $[M+H]^+$ 459.

Example 70

(2E,4E)-N-(Isoquinolin-5-yl)-5-(4-methylphenyl)-5-[4-(trifluoromethyl)phenyl]-2,4-pentadienamide (Compound 71)

In a similar manner to Example 10, Compound 71 was obtained from Compound ad.
$^1$H NMR (DMSO-$d_6$, δ ppm): 2.39 (s, 3H), 6.79 (d, J = 13.2 Hz, 1H), 7.13 (d, J = 7.8 Hz, 2H), 7.16-7.29 (m, 2H), 7.33 (d, J = 7.8 Hz, 2H), 7.56 (d, J = 8.1 Hz, 2H), 7.67 (t, J = 7.8 Hz, 1H), 7.75 (d, J = 8.1 Hz, 2H), 7.94 (d, J = 8.1 Hz, 1H), 7.98 (d, J = 6.0 Hz, 1H), 8.12 (d, J = 7.2 Hz, 1H) 8.55 (d, J = 5.7 Hz, 1H), 9.32 (s, 1H), 10.28 (s, 1H); APCIMS m/z: $[M+H]^+$ 459.

Example 71

(2E,4Z)-5-(2-Fluorophenyl)-N-(isoquinolin-5-yl)-5-[4-(trifluoromethyl)phenyl]-2,4-pentadienamide (Compound 72)

In a similar manner to Example 10, Compound 72 was obtained from Compound ae.
$^1$H NMR (DMSO-$d_6$, δ ppm): 6.85 (d, J = 15.0 Hz, 1H), 7.06 (dd, J = 12.0, 15.0 Hz, 1H), 7.32-7.45 (m, 4H), 7.58-7.61 (m, 3H), 7.66 (t, J = 7.8 Hz, 1H), 7.76 (d, J = 8.4 Hz, 2H), 7.96 (t, J = 6.9 Hz, 2H), 8.10 (d, J = 6.9 Hz, 1H), 8.55 (d, J = 5.7 Hz, 1H), 9.32 (s, 1H), 10.34 (s, 1H); APCIMS m/z: $[M+H]^+$ 463.

Example 72

(2E,4Z)-5-(3-Fluorophenyl)-N-(isoquinolin-5-yl)-5-[4-(trifluoromethyl)phenyl]-2,4-pentadienamide (Compound 73)

In a similar manner to Example 10, Compound 73 was obtained from Compound af.
$^1$H NMR (DMSO-$d_6$, δ ppm): 6.83 (d, J = 14.4 Hz, 1H), 7.10-7.38 (m, 5H), 7.57-7.62 (m, 3H), 7.67 (t, J = 7.9 Hz, 1H), 7.76 (d, J = 8.4 Hz, 2H), 7.94-7.99 (m, 2H), 8.12 (d, J = 7.2 Hz, 1H), 8.56 (d, J = 5.7 Hz, 1H), 9.33 (s, 1H), 10.33 (s, 1H) ; APCIMS m/z: $[M+H]^+$ 463.

Example 73

(2E,4Z)-5-(4-Fluorophenyl)-N-(isoquinolin-5-yl)-5-[4-(trifluoromethyl)phenyl]-2,4-pentadienamide (Compound 74)

In a similar manner to Example 10, Compound 74 was obtained from Compound ag.
mp : 142-143˚C; $^1$H NMR (DMSO-$d_6$, δ ppm) : 6.82 (d, J = 13.2 Hz, 1H), 7.13-7.40 (m, 6H), 7.56 (d, J = 8.4 Hz, 2H), 7.66 (t, J = 7.8 Hz, 1H), 7.76 (d, J = 8.6 Hz, 2H), 7.93-7.99 (m, 2H), 8.12 (d, J = 7.3 Hz, 1H), 8.55 (d, J = 5.9 Hz, 1H), 9.32 (s, 1H), 10.30 (s, 1H) ; APCIMS m/z: $[M+H]^+$ 463.

Example 74

(2E,4Z)-N-(Isoquinolin-5-yl)-5-(3-nitrophenyl)-5-[4-(trifiuoromethyl)phenyl]-2,4-pentadienamide (Compound 75)

In a similar manner to Example 10, Compound 75 was obtained from Compound ah.
$^1$H NMR (DMSO-$d_6$, δ ppm): 6.88 (d, J = 14.6 Hz, 1H), 7.12 (dd, J = 11.5, 14.6 Hz, 1H), 7.38 (d, J = 11.5 Hz, 1H), 7.59 (d, J = 8.1 Hz, 2H), 7.66 (t, J = 7.9 Hz, 1H), 7.76-7.79 (m, 3H), 7.85 (t, J = 7.9 Hz, 1H), 7.94-7.98 (m, 2H), 8.06 (t, J = 1.5 Hz, 1H), 8.10 (d, J = 6.9 Hz, 1H), 8.36 (dq, J = 1.5, 8.1 Hz, 1H), 8.55 (d, J = 6.0 Hz, 1H), 9.32 (s, 1H), 10.36 (s, 1H); APCIMS m/z: $[M+H]^+$ 490.

Example 75

(2E,4Z)-5-(4-Chlorophenyl)-N-(isoquinolin-5-yl)-5-[4-(trifluoromethyl)phenyl]-2,4-pentadienamide (Compound 76)

In a similar manner to Example 10, Compound 76 was obtained from Compound ai.
$^1$H NMR (DMSO-$d_6$, δ ppm) : 6.83 (d, J = 14.1 Hz, 1H), 7.17 (dd, J = 11.4, 14.1 Hz, 1H), 7.25-7.30 (m, 3H), 7.56-7.61 (m, 4H), 7.67 (t, J = 7.9 Hz, 1H), 7.76 (d, J = 8.4 Hz, 2H), 7.94-7.99 (m, 2H), 8.12 (d, J = 7.5 Hz, 1H), 8.55 (d, J = 5.7 Hz, 1H), 9.33 (s, 1H), 10.32 (s, 1H) APCIMS m/z: $[M+H]^+$ 479 .

Example 76

(2E,4Z)-N-(Isoquinolin-5-yl)-5-(4-methoxyphenyl)-5-[4-(trifluoromethyl)phenyl]-2,4-pentadienamide (Compound 77)

In a similar manner to Example 10, Compound 77 was obtained from Compound aj.
$^1$H NMR (DMSO-$d_6$, δ ppm): 3.83 (s, 3H), 6.79 (d, J = 14.7 Hz, 1H), 7.07 (d, J = 8.7 Hz, 2H), 7.06-7.11 (m, 1H), 7.17 (d, J = 8.7 Hz, 2H), 7.29 (dd, J = 11.7, 14.7 Hz, 1H), 7.56 (d, J = 7.8 Hz, 2H), 7.67 (t, J = 7.9 Hz, 1H), 7.75 (d, J = 8.4 Hz, 2H), 7.94 (d, J = 8.1 Hz, 1H), 7.99 (d, J = 6.0 Hz, 1H), 8.13 (d, J = 7.5 Hz, 1H), 8.55 (d, J = 6.0 Hz, 1H), 9.32 (s, 1H), 10.27 (s, 1H); APCIMS m/z: $[M+H]^+$ 475.

Example 77

(2E,4E)-5-Cyclohexyl-N-(isoquinolin-5-yl)-5-[4-(trifluoromethyl)phenyl]-2,4-pentadienamide (Compound 78)

In a similar manner to Example 10, Compound 78 was obtained from Compound ak.
$^1$H NMR (DMSO-$d_6$, δ ppm): 1.07-1.15 (m, 1H), 1.30-1.41 (m, 4H), 1.67-1.76 (m, 5H), 2.96-3.04 (m, 1H), 6.23 (d, J = 11.7 Hz, 1H), 6.60 (d, J = 15.0 Hz, 1H), 7.50 (d, J = 8.1 Hz, 2H), 7.67-7.78 (m, 4H), 7.95-8.00 (m, 2H), 8.17 (d, J = 7.8 Hz, 1H), 8.56 (d, J = 5.7 Hz, 1H), 9.34 (s, 1H), 10.25 (s, 1H) ; APCIMS m/z: $[M+H]^+$ 451.

Example 78

(2E,4E)-5-(Cyclohexen-1-yl)-N-(isoquinolin-5-yl)-5-[4-(trifluoromethyl)phenyl]-2,4-pentadienamide (Compound 79)

In a similar manner to Example 10, Compound 79 was obtained from Compound al.
$^1$H NMR (DMSO-$d_6$, δ ppm): 1.68-1.70 (m, 4H), 1.92-1.94 (m, 2H), 2.24-2.26 (m, 2H), 5.75-5.77 (m, 1H), 6.73 (d, J = 15.0 Hz, 1H), 6.89 (d, J = 11.4 Hz, 1H), 7.58 (dd, J = 11.4, 15.0 Hz, 1H), 7.66-7.79 (m, 5H), 7.96 (d, J = 8.1 Hz, 1H), 8.00 (d, J = 5.7 Hz, 1H), 8.18 (d, J = 7.2 Hz, 1H), 8.56 (d, J = 6.3 Hz, 1H), 9.33 (s, 1H), 10.27 (s, 1H); APCIMS m/z: $[M+H]^+$ 449.

Example 79

(2E,4E)-5-(3,6-Dihydro-2H-pyran-4-yl)-N-(isoquinolin-5-yl)-5-[4-(trifluoromethyl)phenyl]-2,4-pentadienamide (Compound 80)

In a similar manner to Example 10, Compound 80 was obtained from Compound am.
$^1$H NMR (DMSO-$d_6$, δ ppm): 2.03-2.05 (m, 2H), 3.81 (t, J = 5.1 Hz, 2H), 4.29-4.31 (m, 2H), 5.87-5.89 (m, 1H), 6.77 (d, J = 15.0 Hz, 1H), 6.96 (d, J = 11.6 Hz, 1H), 7.59 (dd, J = 11.6, 15.0 Hz, 1H), 7.67-7.80 (m, 5H), 7.96 (d, J = 8.1 Hz, 1H), 8.00 (d, J = 6.0 Hz, 1H), 8.18 (d, J = 7.8 Hz, 1H), 8.56 (d, J = 6.3 Hz, 1H), 9.34 (s, 1H), 10.29 (s, 1H); APCIMS m/z: $[M+H]^+$ 451 .

Example 80

(2E,4Z)-N-(Isoquinolin-5-yl)-5-phenyl-5-[4-(trifluoromethyl)phenyl]-2,4-pentadienamide (Compound 81)

In a similar manner to Example 10, Compound 81 was obtained from Compound ao.
$^1$H NMR (DMSO-$d_6$, δ ppm): 6.79 (d, J = 14.0 Hz, 1H), 7.11 (dd, J = 11.7, 14.0 Hz, 1H), 7.21 (d, J = 11.7 Hz, 1H), 7.33-7.42 (m, 5H), 7.47 (d, J = 8.1 Hz, 2H), 7.66 (t, J = 7.9 Hz, 1H), 7.88 (d, J = 8.1 Hz, 2H), 7.94 (d, J = 8.1 Hz, 1H), 7.97 (d, J = 6.1 Hz, 1H), 8.11 (d, J = 7.4 Hz, 1H), 8.55 (d, J = 5.9 Hz, 1H), 9.32 (s, 1H); APCIMS m/z: $[M+H]^+$ 445.

Example 81

(2E,4E)-5-(4-tert-Butylphenyl)-N-(isoquinolin-5-yl)-5-phenyl-2,4-pentadienamide (Compound 82)

In a similar manner to Example 10, Compound 82 was obtained from Compound aq.
$^1$H NMR (DMSO-$d_6$, δ ppm) : 1.29 (s, 9H), 6.72 (d, J = 14.4 Hz, 1H), 7.06 (d, J = 11.7 Hz, 1H), 7.15-7.23 (m, 3H), 7.28 (d, J = 8.6 Hz, 2H), 7.41 (d, J = 8.6 Hz, 2H), 7.46-7.51 (m, 3H), 7.65 (t, J = 7.8 Hz, 1H), 7.93 (d, J = 8.4 Hz, 1H), 7.99 (d, J = 6.0 Hz, 1H), 8.12 (d, J = 7.8 Hz, 1H), 8.55 (d, J = 6.3 Hz, 1H), 9.32 (s, 1H), 10.21 (s, 1H); APCIMS m/z: $[M+H]^+$ 433.

Example 82

(2E,4Z)-5-(4-tert-Butylphenyl)-5-(3-cyanophenyl)-N-(isoquinolin-5-yl)-2,4-pentadienamide (Compound 83)

In a similar manner to Example 10, Compound 83 was obtained from Compound ar.
$^1$H NMR (DMSO-$d_6$, δ ppm): 1.29 (s, 9H), 6.77 (d, J = 14.4 Hz, 1H), 7.05 (dd, J = 11.7, 14.4 Hz, 1H), 7.18 (d, J = 11.7 Hz, 1H), 7.27 (d, J = 8.4 Hz, 2H), 7.43 (d, J = 8.4 Hz, 2H), 7.58 (d, J = 7.8 Hz, 1H), 7.66 (t, J = 7.9 Hz, 1H), 7.70-7.75 (m, 2H), 7.93-7.99 (m, 3H), 8.12 (d, J = 7.5 Hz, 1H), 8.55 (d, J = 6.0 Hz, 1H), 9.32 (s, 1H), 10.26 (s, 1H); APCIMS m/z: $[M+H]^+$ 458.

Example 83

(2E,4Z)-5-(4-tert-Butylphenyl)-5-(furan-2-yl)-N-(isoquinolin-5-yl)-2,4-pentadienamide (Compound 84)

In a similar manner to Example 10, Compound 84 was obtained from Compound as.
$^1$H NMR (DMSO-$d_6$, δ ppm): 1.32 (s, 9H), 6.52 (d, J = 3.3 Hz, 1H), 6.66-6.68 (m, 1H), 6.67 (d, J = 11.7 Hz, 1H), 6.74 (d, J = 15.0 Hz, 1H), 7.35 (d, J = 8.7 Hz, 2H), 7.46 (d, J = 8.7 Hz, 2H), 7.69 (t, J = 7.9 Hz, 1H), 7.90-7.99 (m, 3H), 8.02 (d, J = 5.7 Hz, 1H), 8.19 (d, J = 7.5 Hz, 1H), 8.56 (d, J = 5.7 Hz, 1H), 9.33 (s, 1H), 10.26 (s, 1H); APCIMS m/z: $[M+H]^+$ 423.

Example 84

(2E,4E)-N-(Isoquinolin-5-yl)-5-phenyl-5-[3-(trifluoromethyl)phenyl]-2,4-pentadienamide (Compound 85)

In a similar manner to Example 10, Compound 85 was obtained from Compound au.
$^1$H NMR (DMSO-$d_6$, δ ppm): 6.79 (d, J = 13.4 Hz, 1H), 7.13-7.30 (m, 4H), 7.44-7.77 (m, 8H), 7.91-7.97 (m, 2H), 8.09 (d, J = 7.8 Hz, 1H), 8.54 (d, J = 6.1 Hz, 1H), 9.31 (s, 1H), 10.27 (s, 1H); APCIMS m/z: $[M+H]^+$ 445.

Example 85

(2E,4E)-N-(Isoquinolin-5-yl)-5-(3-cyanophenyl)-5-[3-(trifluoromethyl)phenyl]-2,4-pentadienamide (Compound 86)

In a similar manner to Example 10, Compound 86 was obtained from Compound av.
$^1$H NMR (DMSO-$d_6$, δ ppm): 6.82 (d, J = 14.8 Hz, 1H), 7.06 (dd, J = 11.4, 14.8 Hz, 1H), 7.36 (d, J = 11.4 Hz, 1H), 7.53-7.84 (m, 8H), 7.89-8.03 (m, 3H), 8.09 (d, J = 7.3 Hz, 1H), 8.54 (d, J = 5.9 Hz, 1H), 9.31 (s, 1H), 10.32 (s, 1H); APCIMS m/z: $[M+H]^+$ 470.

Example 86

(2E,4Z)-5-(Furan-2-yl)-N-(isoquinolin-5-yl)-5-[3-(trifluoromethyl)phenyl]-2,4-pentadienamide (Compound 87)

In a similar manner to Example 10, Compound 87 was obtained from Compound aw.
$^1$H NMR (DMSO-$d_6$, δ ppm): 6.52 (d, J = 3.5 Hz, 1H), 6.68 (dd, J = 1.8, 3.5 Hz, 1H), 6.77 (d, J = 11.7 Hz, 1H), 6.80 (d, J = 15.2 Hz, 1H), 7.63-7.82 (m, 5H), 7.91-8.03 (m, 4H), 8.17 (d, J = 7.3 Hz, 1H), 8.55 (d, J = 5.9 Hz, 1H), 9.32 (d, J = 0.8 Hz, 1H), 10.30 (s, 1H); APCIMS m/z: $[M+H]^+$ 435.

Example 87

(2E,4E)-N-(Isoquinolin-5-yl)-5-phenyl-5-[4-(trifluoromethoxy)phenyl] -2,4-pentadienamide (Compound 88)

In a similar manner to Example 10, Compound 88 was obtained from Compound ay.
$^1$H NMR (DMSO-$d_6$, δ ppm): 6.77 (d, J = 12.6 Hz, 1H), 7.10-7.28 (m, 4H), 7.34-7.57 (m, 7H), 7.66 (t, J = 7.8 Hz, 1H), 7.94 (d, J = 8.4 Hz, 1H), 7.98 (d, J = 6.7 Hz, 1H), 8.11 (d, J = 7.8 Hz, 1H), 8.55 (d, J = 6.0 Hz, 1H), 9.32 (s, 1H), 10.26 (s, 1H); APCIMS m/z: $[M+H]^+$ 461.

Example 88

(2E,4Z)-5-(3-Cyanophenyl)-N-(isoquinolin-5-yl)-5-[4-(tritluoromethoxy)phenyl]-2,4-pentadienamide (Compound 89)

In a similar manner to Example 10, Compound 89 was obtained from Compound az.
$^1$H NMR (DMSO-$d_6$, δ ppm): 6.81 (d, J = 14.5 Hz, 1H), 7.05 (dd, J = 11.8, 14.5 Hz, 1H), 7.25 (d, J = 11.8 Hz, 1H), 7.40 (d, J = 8.8 Hz, 2H), 7.48 (d, J = 8.8 Hz, 2H), 7.56-7.79 (m, 4H), 7.91-8.00 (m, 3H), 8.08-8.15 (m, 1H), 8.55 (d, J = 6.1 Hz, 1H), 9.32 (s, 1H), 10.31 (s, 1H); APCIMS m/z: $[M+H]^+$ 486.

Example 89

(2E,4Z)-5-(Furan-2-yl)-N-(isoquinolin-5-yl)-5-[4-(trifluoromethoxy)phenyl]-2,4-pentadienamide (Compound 90)

In a similar manner to Example 10, Compound 90 was obtained from Compound ba.
$^1$H NMR (DMSO-$d_6$, δ ppm): 6. 54 (d, J = 3.4 Hz, 1H), 6.69 (dd, J = 1.8, 3.4 Hz, 1H), 6.72 (d, J = 12.6 Hz, 1H), 6.78 (d, J = 14.7 Hz, 1H), 7.44 (d, J = 8.5 Hz, 2H), 7.55 (d, J = 8.5 Hz, 2H), 7.69 (t, J = 7.9 Hz, 1H), 7.91-8.04 (m, 4H), 8.19 (d, J = 7.2 Hz, 1H), 8.56 (d, J = 6.0 Hz, 1H), 9.34 (s, 1H), 10.31 (s, 1H); APCIMS m/z: $[M+H]^+$ 451.

Example 90

(2E,4E)-5-(4-Chlorophenyl)-N-(isoquinolin-5-yl)-5-phenyl-2,4-pentadienamide (Compound 91)

In a similar manner to Example 10, Compound 91 was obtained from Compound bc.
$^1$H NMR (DMSO-$d_6$, δ ppm): 6.75 (d, J = 13.5 Hz, 1H), 7.10-7.28 (m, 4H), 7.36 (d, J = 8.8 Hz, 2H), 7.42-7.57 (m, 5H), 7.65 (t, J = 8.0 Hz, 1H), 7.93 (d, J = 8.0 Hz, 1H), 7.97 (d, J = 6.1 Hz, 1H), 8.11 (d, J = 7.3 Hz, 1H), 8.54 (d, J = 6.1 Hz, 1H), 9.31 (s, 1H), 10.25 (s, 1H) ; APCIMS m/z: $[M+H]^+$ 411 .

Example 91

(2E,4Z)-5-(4-Chlorophenyl)-5-(3-cyanophenyl)-N-(isoquinolin-5-yl)-2,4-pentadienamide (Compound 92)

In a similar manner to Example 10, Compound 92 was obtained from Compound bd.
$^1$H NMR (DMSO-$d_6$, δ ppm): 6.79 (d, J = 14.7 Hz, 1H), 7.04 (dd, J = 11.5, 14.7 Hz, 1H), 7.25 (d, J = 11.5 Hz, 1H), 7.36 (d, J = 8.5 Hz, 2H), 7.47 (d, J = 8.5 Hz, 2H), 7.55-7.78 (m, 3H), 7.91-8.01 (m, 4H), 8.09-8.12 (m, 1H), 8.55 (d, J = 6.3 Hz, 1H), 9.31 (s, 1H), 10.30 (s, 1H) ; APCIMS m/z: $[M+H]^+$ 436.

Example 92

(2E,4Z)-5-(4-Chlorophenyl)-5-(furan-2-yl)-N- (isoquinolin-5-yl)-2,4-pentadienamide (Compound 93)

In a similar manner to Example 10, Compound 93 was obtained from Compound be.
$^1$H NMR (DMSO-$d_6$, δ ppm): 6.52 (dd, J = 0.7, 3.4 Hz, 1H), 6.68 (dd, J = 1.8, 3.4 Hz, 1H), 6.70 (d, J = 11.9 Hz, 1H), 6.77 (d, J = 14.5 Hz, 1H), 7.44 (d, J = 8.8 Hz, 2H), 7.51 (d, J = 8.8 Hz, 2H), 7.69 (t, J = 7.7 Hz, 1H), 7.89-8.03 (m, 4H), 8.19 (d, J = 7.7 Hz, 1H), 8.56 (d, J = 5.9 Hz, 1H), 9.33 (d, J = 0.8 Hz, 1H), 10.29 (s, 1H); APCIMS m/z: $[M+H]^+$ 401.

Example 93

(2E,4E)-N-(Isoquinolin-5-yl)-5-(4-methylphenyl)-5-phenyl-2,4-pentadienamide (Compound 94)

In a similar manner to Example 10, Compound 94 was obtained from Compound bg.
$^1$H NMR (DMSO-$d_6$, δ ppm): 2.32 (s, 3H), 6.71 (d, J = 14.7 Hz, 1H), 7.06 (d, J = 11.7 Hz, 1H), 7.13-7.28 (m, 7H), 7.42-7.54 (m, 3H), 7.65 (t, J = 8.1 Hz, 1H), 7.93 (d, J = 8.1 Hz, 1H), 7.98 (d, J = 5.9 Hz, 1H), 8.11 (d, J = 7.0 Hz, 1H), 8.55 (d, J = 5.9 Hz, 1H), 9.32 (s, 1H), 10.21 (s, 1H) ; APCIMS m/z: $[M+H]^+$ 391.

Example 94

(2E,4Z)-5-(3-Cyanophenyl)-N-(isoquinolin-5-yl)-5-(4-methylphenyl)-2,4-pentadienamide (Compound 95)

In a similar manner to Example 10, Compound 95 was obtained from Compound bh.
$^1$H NMR (DMSO-$d_6$, δ ppm) : 2.33 (s, 3H), 6.76 (d, J = 14.4 Hz, 1H), 7.05 (dd, J = 11.7, 14.4 Hz, 1H), 7.17 (d, J = 11.7 Hz, 1H), 7.23 (s, 4H), 7.56 (d, J = 7.9 Hz, 1H), 7.66 (t, J = 7.9 Hz, 1H), 7.69-7.76 (m, 2H), 7.91-8.00 (m, 3H), 8.08-8.15 (m, 1H), 8.55 (d, J = 6.0 Hz, 1H), 9.32 (s, 1H), 10.26 (s, 1H); APCIMS m/z: $[M+H]^+$ 416.

Example 95

(2E,4Z)-5-(Furan-2-yl)-N-(isoquinolin-5-yl)-5-(4-methylphenyl)-2,4-pentadienamide (Compound 96)

In a similar manner to Example 10, Compound 96 was obtained from Compound bi.
$^1$H NMR (DMSO-$d_6$, δ ppm): 2.38 (s, 3H), 6.18 (d, J = 3.3 Hz, 1H), 6.54-6.58 (m, 1H), 6.68 (d, J = 14.2 Hz, 1H), 7.01 (d, J = 12.0 Hz, 1H), 7.15 (dd, J = 12.0, 14.2 Hz, 1H), 7.21 (d, J = 7.9 Hz, 2H), 7.31 (d, J = 7.9 Hz, 2H), 7.64 (t, J = 7.7 Hz, 1H), 7.83 (s, 1H), 7.91 (d, J = 7.7 Hz, 1H), 7.96 (d, J = 5.9 Hz, 1H), 8.10 (d, J = 7.7 Hz, 1H), 8.53 (d, J = 5.9 Hz, 1H), 9.30 (s, 1H), 10.18 (s, 1H); APCIMS m/z: $[M+H]^+$ 381.

Example 96

(E) -N-(Isoquinolin-5-yl)-4-methyl-5,5-bis 4-(trifluoromethyl)phenyl]-2,4-pentadienamide (Compound 97)

In a similar manner to Example 10, Compound 97 was obtained from Compound bj.
$^1$H NMR (DMSO-$d_6$, δ ppm): 2.04 (s, 3H), 6.84 (d, J = 15.4 Hz, 1H), 7.34 (d, J = 15.4 Hz, 1H), 7.40 (d, J = 8.1 Hz, 2H), 7.46 (d, J = 8.1 Hz, 2H), 7.64 (t, J = 7.8 Hz, 1H), 7.78 (d, J = 8.1 Hz, 4H), 7.92 (d, J = 7.8 Hz, 1H), 8.02 (d, J = 6.1 Hz, 1H), 8.18 (d, J = 7.8 Hz, 1H), 8.55 (d, J = 6.1 Hz, 1H), 9.31 (s, 1H), 10.21 (s, 1H); APCIMS m/z: $[M+H]^+$ 527.

Example 97

(E)-N-(Isoquinolin-5-yl)-3-methyl-5,5-bis[4-(trifluoromethyl)phenyl]-2,4-pentadienamide (Compound 98)

In a similar manner to Example 10, Compound 98 was obtained from Compound bk.
$^1$H NMR (DMSO-$d_6$, δ ppm): 2.03 (s, 3H), 6.79 (s, 1H), 6.85 (s, 1H), 7.31 (d, J = 8.0 Hz, 2H), 7.39 (d, J = 8.0 Hz, 2H), 7.60 (d, J = 8.0 Hz, 2H), 7.72 (d, J = 8.0 Hz, 2H), 7.93 (d, J = 7.8 Hz, 1H), 8.02 (d, J = 6.1 Hz, 1H), 8.18 (d, J = 7.8 Hz, 1H), 8.55 (d, J = 6.1 Hz, 1H), 9.31 (s, 1H), 10.35 (brs, 1H); APCIMS m/z: $[M+H]^+$ 527.

Example 98

(E)-N-(Isoquinolin-5-yl)-2-methyl-5,5-bis(4-(trifluoromethyl)phenyl]-2,4-pentadienamide (Compound 99)

In a similar manner to Example 10, Compound 99 was obtained from Compound b1.
$^1$H NMR (DMSO-$d_6$, δ ppm): 2.03 (s, 3H), 7.01 (s, 1H), 7.31 (d, J = 8.0 Hz, 2H), 7.39 (d, J = 8.0 Hz, 2H), 7.50 (s, 1H), 7.60 (d, J = 8.0 Hz, 2H), 7.72 (d, J = 8.0 Hz, 2H), 7.93 (d, J = 7.8 Hz, 1H), 8.02 (d, J = 6.1 Hz, 1H), 8.18 (d, J = 7.8 Hz, 1H), 8.55 (d, J = 6.1 Hz, 1H), 9.31 (s, 1H), 10.35 (brs, 1H); APCIMS m/z: $[M+H]^+$ 527.

Example 99

(E)-N-[2-(2-Methylpyrimidin-4-yl)phenyl]-5,5-bis[4-(trifluoromethyl)phenyl]-2,4-pentadienamide (Compound 100)

Compound b (200 mg, 0.595 mmol) was dissolved in dimethylacetamide (2.00 mL), and pyridine (143 μL) and thionyl chloride (52.0 μL) were added thereto under ice-cooling, and then, the mixture was stirred for 30 minutes. Then, 4-(3-aminophenyl)-2-methylpyrimidine (109 mg, 0.588 mmol) was added thereto, and the mixture was stirred at room temperature for 20 hours. To the reaction mixture, saturated brine was added, and the mixture was extracted with ethyl acetate, and then, the organic layer was dried over anhydrous magnesium sulfate. After the organic layer was filtered, the solvent was evaporated under reduced pressure, and the residue was purified by silica gel column chromatography (ethyl acetate/hexane = 1/1), and then recrystallized from isopropyl ether/hexane to give Compound 100 (207 mg, 63%).
ESIMS m/z: $[M+H]^+$ 554.

Example 100

The following compounds were synthesized in a similar manner to Example 99 using Compound b and an amine corresponding to each compound.
(E)-N-[3-(2-Hydroxyethylsulfonyl)phenyl]-5,5-bis[4-(trifluoromethyl)phenyl]-2,4-pentadienamide (Compound 101) :ESIMS m/z: $[M+H]^+$ 570.
(E)-N-{4-[N-(5-Methylisooxazol-3-yl)sulfamoyl]phenyl]-5,5-bis[4-(trifluoromethyl)phenyl]-2,4-pentadienamide (Compound 102) :ESIMS m/z: $[M+H]^+$ 622.
(E)-N-[5-(4-N-itrophenyl)-[1,3,4]-thiadiazolyl-2-yl]-5,5-bis[4-(trifluoromethyl)phenyl]-2,4-pentadienamide (Compound 103) ;ESIMS m/z: $[M+H]^+$ 591 .
(E)-N-[5-(Thiophen-2-yl)-2H-pyrazol-3-yl]-5,5-bis[4-(trifluoromethyl)phenyl]-2,4-pentadienamide (Compound 104) :ESIMS m/z: $[M+H]^+$ 534.
(E)-N-[2-(Pyridin-3-yl)phenyl]-5,5-bis[4-(trifluoromethyl)phenyl]-2,4-pentadienamide (Compound 105) :ESIMS m/z: $[M+H]^+$ 539.
(E)-N-[2-(6-Methoxypyridin-3-yl)phenyl]-5,5-bis[4-(trifluoromethyl)phenyl]-2,4-pentadienamide (Compound 106) :ESIMS m/z: $[M+H]^+$ 569.
(E)-N-(1,4-Dihydro-2,3-dioxoquinoxalin-5-yl)-5,5-bis[4-(trifluoromethyl)phenyl]-2,4-pentadaenamide (Compound 107) : ESIMS m/z: $[M+H]^+$ 546.
(E)-N-(5-Trifluoromethylpyridin-2-yl)-5,5-bis[4-(trifluoromethyl)phenyl]-2,4-pentadienamide (Compound 108) :ESIMS m/z: $[M+H]^+$ 531.
(E)-N-[3-(Pyridin-3-yl)phenyl]-5,5-bis[4-(trifluoromethyl)phenyl]-2,4-pentadienamide (Compound 109) :ESIMS m/z: $[M+H]^+$ 539.
(E)-N-[2-(Pyridin-4-yl)phenyl]-5,5-bis[4-(trifluoromethyl)phenyl]-2,4-pentadienamide (Compound 110) :ESIMS m/z: $[M+H]^+$ 539.
(E)-N-(3-Phenylisooxazol-5-yl)-5,5-bis[4-(trifluoromethyl)phenyl]-2,4-pentadienamide (Compound 111) :ESIMS m/z: $[M+H]^+$ 529.
(E)-N-[3-(Pyridin-4-yl)phenyl]-5,5-bis[4-(trifluoromethyl)phenyl]-2,4-pentadienamide (Compound 112) :ESIMS m/z: $[M+H]^+$ 539.
(E)-N-(4-Formyl-3-methoxyphenyl)-5,5-bis[4-(trifluoromethyl)phenyl]-2,4-pentadienamide (Compound 113) :ESIMS m/z: $[M+H]^+$ 520.
(E)-N-(1,2,3,4-Tetrahydroisoquinolin-5-yl)-5,5-bis[4-(trifluoromethyl)phenyl]-2,4-pentadienamide (Compound 114) :ESIMS m/z: $[M+H]^+$ 517.
(E)-N-[2-(Acetylamino)benzothiazol-6-yl]-5,5-bis[4-(trifluoromethyl)phenyl]-2,4-pentadienamide (Compound 115) :ESIMS m/z: $[M+H]^+$ 576.
(E)-N-(5,6,7,8-Tetrahydroisoquinolin-5-yl)-5,5-bis[4-(trifluoromethyl)phenyl]-2,4-pentadienamide (Compound 117) :ESIMS m/z: $[M+H]^+$ 517.

(E)-N-(4-Hydroxy-3-[2-(morpholino-4-yl)ethoxylphenyl)-5,5-bis[4-(trifluoromethyl)phenyl)-2,4-pentadienamide (Compound 118) :ESIMS m/z: $[M+H]^+$ 607.

(E)-N-(2-Formylphenyl)-5,5-bis[4-(trifluoromethyl)phenyl]-2,4-pentadienamide (Compound 119) :ESIMS m/z: $[M+H]^+$ 490.

(E)-N-(2-Aminobenzothiazol-4-yl)-5,5-bis[4-(trifluoromethyl)phenyl]-2,4-pentadienamide (Compound 120) :ESIMS m/z: $[M+H]^+$ 534.

(E)-N-(Benzotriazol-4-yl)-5,5-bis[4-(trifluoromethyl)phenyl]-2,4-pentadienamide (Compound 125) :ESIMS m/z: $[M+H]^+$ 503.

(E)-N-(3-Methylcinnolin-5-yl)-5,5-bis[4-(trifluoromethyl)phenyl]-2,4-pentadienamide (Compound 126) ESIMS m/z : $[M+H]^+$ 528.

(E)-N-(2-Methylthieno[2,3-c]pyridin-3-yl)-5,5-bis[4-(trifluoromethyl)phenyl]-2,4-pentadienamide (Compound 128) :ESIMS m/z: $[M+H]^+$ 533 .

(E)-N-(Thieno[2,3-b]pyrazin-7-yl)-5,5-bis[4-(trifluoromethyl)phenyl]-2,4-pentadienamide (Compound 130) :ESIMS m/z: $[M+H]^+$ 520.

(E)-N-(2-Hydroxymethylpyrazin-3-yl)-5,5-bis[4-(trifluoromethyl)phenyl]-2,4-pentadienamide (Compound 131) :ESIMS m/z: $[M+H]^+$ 494.

(E)-N-(Quinolin-6-yl)-5,5-bis[4-(trifluoromethyl)phenyl]-2,4-pentadienamide (Compound 132) :ESIMS m/z: $[M+H]^+$ 513.

(E)-N-[2-(2-Oxo-3H-[1,3,4]oxadiazol-5-yl)phenyl]-5,5-bis[4-(trifluoromethyl)phenyl]-2,4-pentadienamide (Compound 133) :ESIMS m/z: $[M+H]^+$ 546.

(E)-N-{3-(4-Methyl-4H-[1,2,4]triazol-3-yl)phenyl]-5,5-bis[4-(trifluoromethyl)phenyl]-2,4-pentadienamide (Compound 134) :ESIMS m/z: $[M+H]^+$ 543.

(E)-1-[3-(2-Aminophenyl)pyrazol-1-yl]-5,5-bis[4-(trifluoromethyl)phenyl]-2,4-pentadien-1-one (Compound 315) :ESIMS m/z: $[M+H]^+$ 528.

(E)-1-(4-Hydroxypiperidin-1-yl)-5,5-bis[(4-trifluoromethyl)phenyl]penta-2,4-dien-1-one (Compound 316) :ESIMS m/z: $[M+H]^+$ 470.

(E)-1-[3-(Hydroxymethyl)piperidin-1-yl)-5,5-bis[(4-trifluoromethyl)phenyl]penta-2,4-dien-1-one (Compound 317) :ESIMS m/z: $[M+H]^+$ 484.

(E)-1-[2-(Hydroxymethyl)piperidin-1-yl]-5,5-bisl(4-trifluoromethyl)phenyl]penta-2,4-dien-1-one (Compound 318) :ESIMS m/z: $[M+H]^+$ 484.

(E)-1-[4-(Hydroxymethyl)piperidin-1-yl]-5,5-bis[(4-trifluoromethyl)phenyl]penta-2,4-dien-1-one (Compound 319) :ESIMS m/z: $[M+H]^+$ 484.

(E)-1-[4-(Hydroxyethyl)piperazin-1-yl]-5,5-bis[(4-trifluoromethyl)phenyl]penta-2,4-dien-1-one (Compound 320) :ESIMS m/z: $[M+H]^+$ 499.

(E)-1-(3-Hydroxypyrrolidin-1-yl)-5,5-bis[(4-trifluoromethyl)phenyl]penta-2,4-dien-1-one (Compound 321) :ESIMS m/z: $[M+H]^+$ 456.

(E)-1-(3-Hydroxypiperidin-1-yl)-5,5-bis[(4-trifluoromethyl)phenyl]penta-2,4-dien-1-one (Compound 322) :ESIMS m/z: $[M+H]^+$ 456.

(E)-1-[4-(3-Chloro-5-hydroxymethylpyridin-2-yl)piperazin-1-yl]-5,5-bis[(4-trifluoromethyl)phenyl]penta-2,4-dien-1-one (Compound 323) :ESIMS m/z: $[M+H]^+$ 596.

(E)-1-(4-Aminopiperidin-1-yl)-5,5-bis[(4-trifluoromethyl)phenyl]penta-2,4-dien-1-one (Compound 324) :ESIMS m/z: $[M+H]^+$ 469.

(E)-1-[4-(3-Hydroxymethylpyridin-2-yl)piperazin-1-yl]-5,5-bis[(4-trifluoromethyl)phenyl]penta-2,4-dien-1-one (Compound 325) :ESIMS m/z: $[M+H]^+$ 562.

Example 101

(E)-N-(4-Hydroxyimino-3-methoxyphenyl)-5,5-bis[4-(trifluoromethyl)phenyl]-2,4-pentadienamide (Compound 116)

Compound 113, hydroxylamine hydrochloride, and triethylamine were mixed, and the mixture was treated in ethanol at 70˚C to give Compound 116.
ESIMS m/z: $[M+H]^+$ 535.

Example 102

(E)-N-(2-Acetamidobenzothiazol-4-yl)-5,5-bis[4-(trifluoromethyl)phenyl]-2,4-pentadienamide (Compound 121)

Compound 120 was mixed with acetic anhydride in toluene and a reaction was allowed to proceed at 80˚C for 2 hours to give Compound 121.

ESIMS m/z: $[M+H]^+$ 576.

Example 103

(E)-N-(2-Hydroxyiminophenyl)-5,5-bis[4-(trifluoromethyl)phenyl]-2,4-pentadienamide (Compound 122)

In a similar manner to Example 101, Compound 122 was obtained from Compound 119.
ESIMS m/z: $[M+H]^+$ 505.

Example 104

(E)-N-[2-(5-Oxo-4H-[1,2,4]oxadiazol-3-yl)phenyl]-5,5-bis[4-(trifluoromethyl)phenyl]-2,4-pentadienamide

(Compound 123)

Compound 124 (104 mg, 0.20 mmol) was dissolved in THF (2.0 mL), and triethylamine (0.08 mL) and ethyl chloroformate (30 μL) were added thereto, and then, the mixture was stirred at room temperature. After it was confirmed by thin-layer chromatography that the reaction was completed, the reaction mixture was washed with water, and the organic layer was dried over anhydrous magnesium sulfate. After the solvent was evaporated, the residue was dissolved in toluene (1.0 mL), and potassium tert-butoxide (15 mg) was added thereto, and then, the mixture was stirred at room temperature. After it was confirmed by thin-layer chromatography that the reaction was completed, the reaction mixture was washed with water, and the organic layer was dried over anhydrous magnesium sulfate. The solvent was evaporated, and the residue was purified by silica gel column chromatography to give Compound 123 (10 mg, 10%).
ESIMS m/z: $[M+H]^+$ 546.

Example 105

(E)-N-[2-(N-Hydroxyamidino)phenyl]-5,5-bis[4-(trifluoromethyl)phenyl]-2,4-pentadienamide (Compound 124)

(E)-N-(2-Cyanophenyl)-5,5-bis[4-(trifluoromethyl)phenyl]-2,4-pentadienamide (170 mg, 0.35 mmol) obtained in a similar manner to Example 1 using 2-cyanoaniline was dissolved in ethanol (10 mL), and potassium carbonate (145 mg) and hydroxylamine hydrochloride (69 mg) were added thereto, and then, the mixture was stirred at 60 °C for 5 hours. Water was added to the reaction mixture, and the mixture was extracted with ethyl acetate, and then, the organic layer was dried over anhydrous magnesium sulfate. After the solvent was evaporated, the residue was purified by silica gel column chromatography to give Compound 124 (104 mg, 57%).
ESIMS m/z: $[M+H]^+$ 520.

Example 106

(E)-N-[3-(Hydroxyimino)pyridin-4-yl]-5,5-bis[4-(trifluoromethyl)phenyl]-2,4-pentadienamide (Compound 127)

(E)-N-(3-Formylpyridin-4-yl)-5,5-bis[4-(trifluoromethyl)phenyl]-2,4-pentadienamide was obtained in a similar manner to Example 1 using 4-aminopyridine-3-carboxaldehyde, and then, Compound 127 was obtained in a similar manner to Example 101.
ESIMS m/z : $[M+H]^+$ 506.

Example 107

(E)-N-[2-(2-Hydroxyethyl)-3-oxo-4H-benzo[1,4]oxazin-6-yl]-5,5-bis[4-(trifluoromethyl)phenyl)-2,4-pentadienamide (Compound 129)

In a similar manner to Example 1, 6-amino-2-[2-(tert-butoxydimethylsiloxy)ethyl]-4H-benzo[1,4]oxazin-3-one prepared according to the method described in WO 2006/058338 was reacted with Compound b, and then, a tert-butoxydimethylsilyl group was removed according to the method described in WO 2006/058338 to give Compound 129.
ESIMS m/z: $[M+H]^+$ 577.

Example 108

The following compounds were synthesized in a similar manner to Example 99 using a starting material and an amine corresponding to each compound.

(2E, 4E)-N-(2- Methylthieno [2,3- c] pyridin- 3- yl)- 5-(4- fluorophenyl)- 5-[4-(trifluoromethyl) phenyl]- 2,4- pentadienamide (Compound 135):ESIMS m/z: $[M+H]^+$ 483.

(2E, 4E)-N-[2-(2-Hydroxyethyl)-3-oxo-4H-benzo[1,4]oxazin-6-yl]-5-(4-fluorophenyl)-5-[4-(trifluoromethyl)phenyl]-2,4-pentadienamide (Compound 137) :ESIMS m/z: $[M+H]^+$ 527.

(2E,4Z)-N-(2-Methylthieno[2,3-c]pyridin-3-yl)-5-[4-(dimethylamino)phenyl]-5-[4-(trifluoromethyl)phenyl]-2,4-pentadienamide (Compound 138) :ESIMS m/z: $[M+H]^+$ 508.

(2E,4Z)-N-(1H-Pyrazolo[3,4-d]pyrimidin-4-yl)-5-[4-(dimethylamino)phenyl]-5-[4-(trifluoromethyl)phenyl]-2,4-pentadienamide (Compound 139) ESIMS m/z: $[M+H]^+$ 479.

(2E,4Z)-N-[2-(2-Hydroxyethyl)-3-oxo-4H-benzo[1,4]oxazin-6-yl]-5-[4-(dimethylamino)phenyl]-5-[4-(trifluoromethyl)phenyl]-2,4-pentadienamide (Compound 140) :ESIMS m/z: $[M+H]^+$ 552.

(2E, 4Z)-N-(3- Methylcinnolin- 5- yl)- 5-[4-(dimethylamino) phenyl]- 5-[4-(trifluoromethyl) phenyl]- 2,4- pentadienamide (Compound 141) :ESIMS m/z: $[M+H]^+$ 503.

(2E, 4Z)-N-(2- Hydroxymethylbenzimidazol- 4- yl)- 5-[4-(dimethylamino) phenyl]- 5-[4-(trifluoromethyl) phenyl]-2,4-pentadienamide (Compound 142) :ESIMS m/z: $[M+H]^+$ 507.

(2E, 4Z)-N-{2-[(2,4- Dioxothiazol- 5- ylidene) methyl] phenyl}- 5-[4-(dimethylamino) phenyl]- 5-[4-(trifluoromethyl) phenyl]-2,4-pentadienamide (Compound 143) :ESIMS m/z: $[M+H]^+$ 564.

(2E, 4Z)-N-(2- Aminoquinazolin- 8- yl)- 5-[4-(dimethylamino) phenyl]- 5-[4-(trifluoromethyl) phenyl]- 2,4- pentadienamide (Compound 144) :ESIMS m/z: $[M+H]^+$ 504.

(2E,4Z)-N-[2-(Hydroxymethyl)benzimzdazol-5-yl]-5-[4-(dimethylamino)phenyl]-5-[4-(trifluoromethyl)phenyl]-2,4-pentadienamide (Compound 145) :ESIMS m/z : $[M+H]^+$ 507.

(2E,4Z)-N-(1-Oxo-2H-isoquinolin-5-yl)-5-[4-(dimethylamino)phenyl]-5-[4-(trifluoromethyl)phenyl]-2,4-pentadienamide (Compound 146) :ESIMS m/z: $[M+H]^+$ 504.

(2E, 4Z)-N-(2- Aminoquinazolin- 8- yl)- 5-[2-(morpholino) pyridin- 5- yl]- 5-[4-(trifluoromethyl) phenyl]- 2,4- pentadienamide (Compound 147) :ESIMS m/z: $[M+H]^+$ 547.

(2E,4Z)-N-[2-(2-Hydroxyethyl)-3-oxo-4H-benzo[1,4]oxazin-6-yl]-5-[2-(morpholino)pyridin-5-yl]-5-[4-(trifluoromethyl)phenyl]-2,4-pentadienamide (Compound 148) :ESIMS m/z: $[M+H]^+$ 595.

(2E,4Z)-N-(Imidazo[1,2-a]pyrazin-3-yl)-5-[2-(morpholino)pyridin-5-yl]-5-[4-(triflucromethyl)phenyl]-2,4-pentadienamide (Compound 149) :ESIMS m/z: $[M+H]^+$ 521.

(2E,4Z)-N-[4-([1,2,3]Thiadiazol-4-yl)phenyl]-5-[2-(morpholino)pyridin-5-yl]-5-[4-(trifluoromethyl)phenyl]-2,4-pentadienamide (Compound 150) :ESIMS m/z: $[M+H]^+$ 564.

(2E,4Z)-N-(1-Cyanoisoquinolin-5-yl)-5-[2-(morpholino)pyridin-5-yl]-5-[4-(trifluoromethyl)phenyl - 2,4-pentadienamide (Compound 151) :ESIMS m/z: $[M+H]^+$ 556.

(2E, 4Z)-N-[2-(Hydroxymethyl) phenyl]- 5-[4-(dimethylamino) phenyl]- 5-[4-(trifluoromethyl) phenyl]- 2,4- pentadienamide (Compound 152) :ESIMS m/z: $[M+H]^+$ 467.

(2E,4Z)-N-[(1-Ethyl-1H-imidazol-2-yl)carbonylmethyl]-5-[2-(morpholino)pyridin-5-yl]-5-[4-(trifluoromethyl)phenyl]-2,4-pentadienamide (Compound 153) :ESIMS m/z: $[M+H]^+$ 540.

(2E,4Z)-N-[2-(Hydroxymethyl)phenyl]-5-(4-ethoxyphenyl)-5-[4-(trifluoromethyl)phenyl]-2,4-pentadienamide (Compound 154) :ESIMS m/z: $[M+H]^+$ 468.

(2E,4Z)-N-[3-(Hydroxymethyl) pyridin-4-yl]-5-[4-(dimethylamino) phenyl]-5-[4-(trifluoromethyl) phenyl]-2,4-pentadienamide (Compound 156) :ESIMS m/z: $[M+H]^+$ 468. (2E,4Z)-N-[3-(Hydroxymethyl)pyridin-4-yl]-5-(4-ethoxyphenyl)-5-[4-(trifluoromethyl)phenyl]-2,4-pentadienamide (Compound 157) :ESIMS m/z: $[M+H]^+$ 469.

(E)-N-[2-Carboxyphenyl]-5,5-bis[4-(trifluoromethyl)phenyl]-2,4-pentadienamide (Compound 158) :ESIMS m/z: $[M+H]^+$ 506.

(2E,4Z)-N-[2-Hydroxymethyl-3,4-dihydro-2H-benzo[1,4]oxazin-6-yl]-5-(4-ethoxyphenyl)-5-[4-(trifluoromethyl)phenyl]-2,4-pentadienamide (Compound 159) :ESIMS m/z: $[M+H]^+$ 525.

(E)-N-(1,1-Dioxothiomorpholin-4-yl)-5,5-bis[4-(trifluoromethyl)phenyl]-2,4-pentadienamide (Compound 160) :ESIMS m/z: $[M+H]^+$ 519.

(2E,4Z)-N-[4-(Hydroxymethyl)pyridin-3-yl]-5-(4-ethoxyphenyl)-5-[4-(trifluoromethyl)phenyl]-2,4-pentadienamide (Compound 161) :ESIMS m/z: $[M+H]^+$ 469.

(2E, 4Z)-N-{2-(1,3- Dihydroxy- 2- propyl) phenyl}- 5-(4- ethoxyphenyl)- 5-[4-(trifluoromethyl) phenyl]- 2,4- pentadienamide (Compound 162) :ESIMS m/z: $[M+H]^+$ 512.

(2E, 4Z)-N-[2-(1,2- Dihydroxyethyl) phenyl]- 5-(4- ethoxyphenyl)- 5-[4-(trifluoromethyl) phenyl]- 2,4- pentadienamide (Compound 163) :ESIMS m/z: $[M+H]^+$ 498.

(2E,4Z)-N-(3-Aminophenyl)-5-(4-ethoxyphenyl)-5-[4-(trifluoromethyl)phenyl]-2,4-pentadienamide (Compound 164) :

ESIMS m/z: $[M+H]^+$ 453.
(2E,4Z)-N-[2-Oxo-1H-quinoxalin-5-yl]-5-(4-ethoxyphenyl)-5-[4-(trifluoromethyl)phenyl]-2,4-pentadienamide (Compound 165) :ESTMS m/z: $[M+H]^+$ 506.
(2E,4Z)-N-[3-Acetamido-2-(hydroxymethyl)phenyl]-5-(4-ethoxyphenyl)-5-[4-(trifluoromethyl)phenyl]-2,4-pentadienamide (Compound 166) :ESIMS m/z: $[M+H]^+$ 525.
(2E,4Z)-N-[3-(Hydroxymethyl)pyridin-2-yl]-5-(4-ethoxyphenyl)-5-[4-(trifluoromethyl)phenyl]-2,4-pentadienamide (Compound 167) :ESIMS m/z: $[M+H]^+$ 469.
(2E,4Z)-N-[2-Chloro-4-(hydroxymethyl)pyridin-3-yl]-5-(4-ethoxyphenyl)-5-[4-(trifluoromethyl)phenyl]-2,4-pentadienamide (Compound 170) :ESIMS m/z: $[M+H]^+$ 503.
(2E,4Z)-N-[2,5-Dichloro-4-(hydroxymethyl)pyridin-3-yl]-5-(4-ethoxyphenyl)-5-[4-(trifluoromethyl)phenyl]-2,4-pentadienamide (Compound 171) :ESIMS m/z: $[M+H]^+$ 537.

(2E,4Z)-N-[3-(tert-Butoxycarbonylamino)-2-oxo-1,2,3,4-tetrahydroquinolin-5-yl]-5-(4-ethoxyphenyl)-5-[4-(trifluoromethyl)phenyl]-2,4-pentadienamide (Compound 172) :ESIMS m/z: $[M+H]^+$ 622.
(2E,4Z)-N-[2-(Hydroxymethyl)-2,3-dihydrobenzofuran-4-yl]-5-(4-ethoxyphenyl)-5-[4-(trifluoromethyl)phenyl]-2,4-pentadienamide (Compound 174) :ESIMS m/z: $[M+H]^+$ 510.
(2E,4Z)-N-(3-Hydroxychroman-5-yl)-5-(4-ethoxyphenyl)-5-[4-(trifluoromethyl)phenyl]-2,4-pentadienamide (Compound 175) :ESIMS m/z: $[M+H]^+$ 510.
(2E,4Z)-N-(1,4-Dihydro-2-oxobenzo[d][1,3]oxazin-5-yl)-5-(4-ethoxyphenyl)-5-[4-(trifluoromethyl)phenyl]-2,4-pentadienamide (Compound 176) :ESIMS m/z: $[M+H]^+$ 509.
(2E,4Z)-N-(1H-Indazol-4-yl)-5-(4-ethoxyphenyl)-5-[4-(trifluoromethyl)phenyl]-2,4-pentadienamide (Compound 177) : ESIMS m/z: $[M+H]^+$ 478.
(2E,4Z)-N-(1H-Indazol-4-yl)-5-(4-isopropoxyphenyl)-5-[4-(trifluoromethyl)phenyl]-2,4-pentadienamide (Compound 178) :ESIMS m/z: $[M+H]^+$ 492.
(2E,4Z)-N-(1H-Indazol-4-yl)-5-(4-propoxyphenyl)-5-[4-(trifluoromethyl)phenyl]-2,4-pentadienamide (Compound 179) : ESIMS m/z: $[M+H]^+$ 492.

Example 109

(E)-N-[2-(1H-Pyrazol-3-yl)phenyl]-5,5-bis[4-(trifluoromethyl)phenyl]-2,4-pentadienamide (Compound 136)

In a similar manner to Example 99, tert-butyl 3-(2-aminophenyl)pyrazole-1-carboxylate (98 mg) obtained in Reference example 179 and Compound b (116 mg, 0.4 mmol) were reacted with each other, and a 4.0 mol/L hydrogen chloride-ethyl acetate solution (1.0 mL) was added to the obtained (E)-N-[2-(1-tert-butoxycarbonylpyrazol-3-yl)phenyl]-5,5-bis[4-(trifluoromethyl)phenyl]-2,4-pentadienamide (80 mg), and the mixture was stirred at room temperature for 5 hours. The reaction mixture was poured into a saturated sodium hydrogen carbonate solution, and the mixture was extracted with ethyl acetate, and then, the organic layer was dried over anhydrous magnesium sulfate. After the solvent was evaporated, the residue was purified by silica gel column chromatography to give Compound 136 (15 mg, 22%).
ESIMS m/z: $[M+H]^+$ 528.

Example 110

(2E,4Z)-N-(2-Acetamidoquinazolin-8-yl)-5-[2-(morpholino)pyridin-5-yl]-5-[4-(trifluoromethyl)phenyl]-2,4-pentadienamide (Compound 155)

Compound 147, pyridine, and acetic anhydride were reacted with one another in dioxane at 80˚C to give Compound 155.
ESIMS m/z: $[M+H]^+$ 589.

Example 111

(2E,4Z)-N-[3-(2-Cyanoacetamido)phenyl]-5-(4-ethoxyphenyl)-5-[4-(trifluoromethyl)phenyl]-2,4-pentadienamide (Compound 168)

Compound 164 (63 mg, 0.14 mmol) was dissolved in THF (3.0 mL), and cyanoacetic acid (18 mg, 0.21 mmol), EDC hydrochloride (81 mg, 0.42 mmol), and HOBt (69 mg, 0.42 mmol) were added thereto, and then, the mixture was stirred at room temperature for 1 hour. The reaction mixture was extracted by adding water and ethyl acetate thereto, and the organic layer was dried over anhydrous magnesium sulfate. After the solvent was evaporated, the residue was purified by silica gel column chromatography to give Compound 168 (25 mg, 34%).

ESIMS my : $[M+H]^+$ 520.

Example 112

(2E,4Z)-N-[(2-Hydroxyacetamido)phenyl]-5-(4-ethoxyphenyl)-5-[4-(trifluoromethyl)phenyl]-2,4-pentadienamide (Compound 169)

Compound 164 (70 mg, 0.15 mmol) was dissolved in THF (3.0 mL), and pyridine (38 μL) and acetoxyacetyl chloride (24 μL) were added thereto, and then, the mixture was stirred at room temperature for 1 hour. The reaction mixture was extracted by adding water and ethyl acetate thereto, and the organic layer was dried over anhydrous magnesium sulfate. After the solvent was evaporated, the residue was dissolved in methanol (3.0 mL) and water (1.0 mL), and potassium carbonate (19 mg, 0.14 mmol) was added thereto, and then, the mixture was stirred at room temperature for 30 minutes. The reaction mixture was extracted by adding water and ethyl acetate thereto, and the organic layer was dried over anhydrous magnesium sulfate. After the solvent was evaporated, the residue was purified by silica gel column chromatography to give Compound 169 (61 mg, 80%) .
ESIMS m/z: [M+H] 511.

Example 113

(2E,4Z)-N-[3-Amino-2-oxo-1,2,3,4-tetrahydroquinolin-5-yl]-5-(4-ethoxyphenyl)-5-[4-(trifluoromethyl)phenyl]-2,4-pentadienamide (Compound 173)

Compound 172 (100 mg, 0.16 mmol) was dissolved in ethyl acetate (6.0 mL), and a 4.0 mol/L hydrogen chloride-ethyl acetate solution (1.5 mL) was added thereto, and the mixture was stirred overnight at room temperature. The reaction mixture was mixed with a saturated sodium hydrogen carbonate solution, and the mixture was extracted with ethyl acetate. After the organic layer was dried over anhydrous magnesium sulfate, the solvent was evaporated. The residue was purified by silica gel column chromatography to give Compound 173 (63 mg, 76%).
ESIMS m/z: $[M+H]^+$ 522.

Example 114

(2E,4E)-5-(4-Chlorophenyl)-5-(4-ethoxyphenyl)-N-(3-hydroxy-1,2,3,4-tetrahydroquinolin-5-yl)-2,4-pentadienamide monohydrochloride (Compound 180)

Compound ca (220 mg, 0.669 mmol) was dissolved in DMF (7.0 mL), and 5-amino-3-hydroxy-1,2,3,4-tetrahydroquinoline (165 mg, 1.00 mmol), EDC hydrochloride (193 mg, 1.01 mmol), and HOBt (154 mg, 1.00 mmol) were added thereto, and then, the mixture was stirred at 50˚C for 7 hours. After the reaction mixture was left to cool, a saturated sodium hydrogen carbonate solution was added thereto, and the mixture was extracted with ethyl acetate. The organic layer was washed with saturated brine and dried over anhydrous magnesium sulfate. After the organic layer was filtered, the solvent was evaporated under reduced pressure, and the residue was purified by silica gel column chromatography (ethyl acetate/hexane = 4/1) to give (2E,4E)-N-(3-hydroxy-1,2,3,4-tetrahydroquinolin-5-yl)-5-(4-chlorophenyl)-5-(4-ethoxyphenyl)-2,4-pentadienamide. The resulting compound was dissolved in ethyl acetate (3.0 mL), and a 4.0 mol/L hydrogen chloride-ethyl acetate solution (0.167 mL, 0.669 mmol) was added thereto. The solvent was evaporated under reduced pressure, and the residue was recrystallized from diethyl ether to give Compound 180 (202 mg, 59%).
$^1$H NMR (DMSO-$d_6$, δ ppm): 1.36 (t, J = 7.0 Hz, 3H), 2.31-2.41 (m, 1H), 2.68-2.89 (m, 2H), 3.17-3.21 (m, 1H), 3.83-3.84 (m, 1H), 4.09 (q, J = 7.0 Hz, 2H), 4.87 (d, J = 4.3 Hz, 1H), 5.68 (br s, 1H), 6.30 (d, J = 7.8 Hz, 1H), 6.55-5.58 (m, 2H), 6.81 (t, J = 8.0 Hz, 1H), 6.93-7.18 (m, 6H), 7.33 (d, J = 8.4 Hz, 2H), 7.43 (d, J = 8.4 Hz, 2H), 9.26 (br s, 1H) .
ESIMS m/z : $[M+H]^+$ 475.

Example 115

The following compounds were synthesized in a similar manner to Example 114 using a starting material corresponding to each compound.
(2E, 4Z)-N-(3- Hydroxy- 1,2,3,4- tetrahydroquinolin- 5- yl)- 5-(4- isopropoxyphenyl)- 5-(4- methylphenyl)- 2,4- pentadienamide monohydrochloride (Compound 181)
$^1$H NMR (DMSO-$d_6$, δ ppm): 1.31 (d, J = 5.9 Hz, 6H), 2.32 (s, 3H), 2.51-2.65 (m, 1H), 2.85-2.93 (m, 1H), 3.10-3.17 (m, 1H), 3.27-3.30 (m, 1H), 4.18 (br s, 1H), 4.67 (sept, J = 5.9 Hz, 1H), 6.56 (d, J = 14.9 Hz, 1H), 6.88-7.10 (m, 4H), 7.15-7.16 (m, 2H), 7.15-7.25 (m, 6H), 7.35 (d, J = 7.8 Hz, 1H), 9.63 (br s, 1H); ESIMS m/z: $[M+H]^+$ 469.

(2E, 4E)- 5-(4- Chlorophenyl)-N-(3- hydroxy- 1,2,3,4- tetrahydroquinolin- 5- yl)- 5-(4- isopropoxyphenyl)- 2,4- pentadienamide monohydrochloride (Compound 182)

$^1$H NMR (DMSO-$d_6$, δ ppm) : 1.31 (d, J = 5.9 Hz, 6H), 2.62 (dd, J = 5.7, 17.0 Hz, 1H), 2.86-2.94 (m, 1H), 3.11-3.17 (m, 1H), 3.27-3.31 (m, 1H), 4.20 (br s, 1H), 4.68 (sept, J = 5.9 Hz, 1H), 6.62 (d, J = 14.6 Hz, 1H), 6.95-7.15 (m, 6H), 7.19-7.25 (m, 2H), 7.33-7.45 (m, 5H), 9.70 (br s, 1H); ESIMS m/z: $[M+H]^+$ 489.

(2E, 4Z)- 5-(4- Ethoxyphenyl)-N-(3- hydroxy- 1,2,3,4- tetrahydroquinolin- 5- yl)- 5-(4- methylphenyl)- 2,4- pentadienamide monohydrochloride (Compound 187)

$^1$H NMR (DMSO-$d_6$, δ ppm): 1.35 (t, J = 7.0 Hz, 3H), 2.30 (s, 3H), 2.34-2.40 (m, 1H), 2.67-2.85 (m, 2H), 3.15-3.19 (m, 1H), 3.80-3.85 (m, 1H), 4.08 (q, J = 7.0 Hz, 2H), 4.86 (d, J = 4.3 Hz, 1H), 5.66 (br s, 1H), 6.28 (d, J = 7.8 Hz, 1H), 6.46 (d, J = 14.8 Hz, 1H), 6.56 (d, J = 8.1 Hz, 1H), 6.79 (t, J = 7.8 Hz, 1H), 6.87 (d, J = 11.6 Hz, 1H) , 6.98-7.21 (m, 9H), 9.19 (br s, 1H) ; ESIMS m/z: $[M+H]^+$ 455.

(2E,4Z)-N-(3-Hydroxy-1,2,3,4-tetrahydroquinolin-5-yl)-5-(4-isopropoxyphenyl)-5-[4-(trifluoromethyl)phenyl]-2,4-pentadienamide monohydrochloride (Compound 204)

$^1$H NMR (DMSO-$d_6$, δ ppm) : 1.31 (d, J = 6.1 Hz, 6H), 2.59 (dd, J = 5.4, 17.0 Hz, 1H), 2.87 (dd, J = 4.5, 17.0 Hz, 1H), 3.12 (dd, J = 6.6, 11.7 Hz, 1H), 3.26-3.30 (m, 1H), 4.15-4.17 (m, 1H), 4.68 (sept, J = 6.1 Hz, 1H), 6.65 (d, J = 14.6 Hz, 1H), 6.88-6.91 (m, 1H), 7.01-7.27 (m, 8H), 7.54 (d, J = 8.4 Hz, 2H), 7.73 (d, J = 8.4 Hz, 2H), 9.67 (br s, 1H) ; ESIMS m/z: $[M+H]^+$ 523.

(2E,4Z)-5-[6-(Azepan-1-yl)pyridin-3-yl]-N-(3-hydroxy-2-oxo-1,2,3,4-tetrahydroquinolin-5-yl)-5-[4-(trifluoromethyl)phenyl]-2,4-pentadienamide monohydrochloride (Compound 221)

$^1$H NMR (DMSO-$d_6$, δ ppm): 1.55-1.59 (m, 4H), 1.79-1.81 (m, 4H), 2.63 (dd, J = 12.0, 15.9 Hz, 1H), 3.01 (dd, J = 6.2, 15.9 Hz, 1H), 3.73-3.77 (m, 4H), 4.03-4.10 (m, 1H), 6.68-6.73 (m, 2H), 7.06-7.29 (m, 5H), 7.62-7.70 (m, 3H), 7.77 (d, J = 8.4 Hz, 2H), 7.86 (d, J = 2.2 Hz, 1H), 9.89 (br s, 1H), 10.20 (br s, 1H); ESIMS m/z: $[M+H]^+$ 577 .

(2E,4Z)-N-(3-Hydroxy-2-oxo-1,2,3,4-tetrahydroquinolin-5-yl)-5-{6-([1,4]oxazepan-4-yl)pyridin-3-yl}-5-[4-(trifluoromethyl)phenyl]-2,4-pentadienamide monohydrochloride (Compound 222)

$^1$H NMR (DMSO-$d_6$, δ ppm) : 1.92-1.99 (m, 2H), 2.63 (dd, J = 11.7, 15.9 Hz, 1H), 3.01 (dd, J = 6.2, 15.9 Hz, 1H), 3.65-3.90 (m, 8H), 4.03-4.10 (m, 1H), 6.66-6.73 (m, 2H), 7.08-7.28 (m, 5H), 7.61-7.64 (m, 3H), 7.77 (d, J = 8.1 Hz, 2H), 7.91 (d, J = 2.2 Hz, 1H), 9.86 (br s, 1H), 10.19 (br s, 1H) ; ESTMS m/z: $[M+H]^+$ 579.

(2E,4Z)-N-(3-Hydroxy-2-oxo-1,2,3,4-tetrahydroquinolin-5-yl)-5-[6-(3-methylpyrrolidin-1-yl)pyridin-3-yl]-5-[4-(trifluoromethyl)phenyl]-2,4-pentadienamide monohydrochloride (Compound 223)

$^1$H NMR (DMSO-$d_6$, δ ppm) : 1.11 (d, J = 6.6 Hz, 3H), 1.65-1.75 (m, 1H), 2.16-2.21 (m, 1H), 2.63 (dd, J = 11.9, 15.8 Hz, 1H), 3.80 (dd, J = 6.6, 15.8 Hz, 1H), 3.15 (dd, J = 7.8, 10.8 Hz, 1H), 3.53-3.62 (m, 1H), 3.72-3.83 (m, 2H), 4.02-4.10 (m, 2H), 6.69-6.74 (m, 2H), 7.03-7.29 (m, 5H), 7.63 (d, J = 8.3 Hz, 2H), 7.71-7.75 (m, 1H), 7.78 (d, J = 8.3 Hz, 2H), 7.87 (d, J = 2.1 Hz, 1H), 9.91 (br s, 1H), 10.20 (br s, 1H); ESIMS m/z: $[M+H]^+$ 563.

(2E,4Z)-5-(4-Ethoxyphenyl)-N-(2-oxo-1,2-dihydroquinolin-5-yl)-5-[4-(trifluoromethyl)phenyl]-2,4-pentadienamide (Compound 224)

$^1$H NMR (DMSO-$d_6$, δ ppm): 1.36 (t, J = 7.0 Hz, 3H), 4.10 (q, J = 7.0 Hz, 2H), 6.51 (d, J = 9.7 Hz, 1H), 6.67 (d, J = 14.6 Hz, 1H), 7.03-7.14 (m, 6H), 7.25 (dd, J = 11.7, 14.2 Hz, 1H), 7.38-7.48 (m, 2H), 7.54 (d, J = 8.3 Hz, 2H), 7.74 (d, J = 8.3 Hz, 2H), 7.98 (d, J = 9.7 Hz, 1H), 10.13 (br s, 1H), 11.79 (br s, 1H); ESIMS m/z: $[M+H]^+$ 505.

(2E,4Z)-5-[4-(N,N-Dimethylamino)phenyl]-N-(2-oxo-1,2-dihydroquinolin-5-yl)-5-[4-(trifluoromethyl)phenyl]-2,4-pentadienamide monohydrochloride (Compound 226)

$^1$H NMR (DMSO-$d_6$, δ ppm) : 3.03 (s, 6H), 6.51 (d, J = 9.8 Hz, 1H), 6.68 (d, J = 14.7 Hz, 1H), 7.00-7.22 (m, 6H), 7.26-7.48 (m, 3H), 7.55 (d, J = 8.3 Hz, 2H), 7.74 (d, J = 8.3 Hz, 2H), 8.01 (d, J = 9.8 Hz, 1H), 10.18 (br s, 1H), 11.80 (br s, 1H); ESIMS m/z: $[M+H]^+$ 504.

(2E,4Z)-5-[2-(Pyrrolidin-1-yl)pyrimidin-5-yl]-N-(3-hydroxy-2-oxo-1,2,3,4-tetrahydroquinolin-5-yl)-5-[4-(trifluoromethyl) phenyl]-2,4-pentadienamide monohydrochloride (Compound 230)

$^1$H NMR (DMSO-$d_6$, δ ppm): 1.95-1.99 (m, 4H), 2.63 (dd, J = 11.8, 16.0 Hz, 1H), 3.02 (dd, J = 6.3, 16.0 Hz,

1H), 3.55-3.60 (m, 4H), 4.07 (dd, J = 6.3, 11.8 Hz, 1H), 6.64-6.73 (m, 2H), 7.10-7.27 (m, 4H), 7.63 (d, J = 8.3 Hz, 2H), 7.76 (d, J = 8.3 Hz, 2H), 8.25 (s, 2H), 9.82 (br s, 1H), 10.18 (br s, 1H) ; ESIMS m/z: $[M+H]^+$ 550.

(2E,4Z)-5-[4-(N,N-Dimethylamino)phenyl]-N-(2-oxo-1,2,3,4-tetrahydroquinolin-5-yl)-5-[4-(trifluoromethyl)phenyl]-2,4-pentadienamide monohydrochloride (Compound 237)

$^1H$ NMR (DMSO-$d_6$, $\delta$ ppm): 2.36-2.42 (m, 2H), 2.72-2.77 (m, 2H), 3.07 (s, 6H), 6.61 (d, J = 14.6 Hz, 1H), 6.71 (dd, J = 2.2, 6.7 Hz, 1H), 7.02-7.26 (m, 7H), 7.53 (d, J = 8.3 Hz, 2H), 7.73 (d, J = 8.3 Hz, 2H), 9.77 (br s, 1H), 10.10 (br s, 1H); ESIMS m/z: $[M+H]^+$ 506.

(2E,4Z)-5-[4-(N,N-Dimethylamino)phenyl]-N-(3-hydroxy-2-oxo-1,2,3,4-tetrahydroquinolin-5-yl)-5-[4-(trifluoromethyl) phenyl]-2,4-pentadienamide monohydrochloride (Compound 247)

$^1H$ NMR (DMSO-$d_6$, $\delta$ ppm): 2.62 (dd, J = 11.7, 15.8 Hz, 1H), 2.98-3.06 (m, 1H), 3.02 (s, 6H), 4.00-4.10 (m, 1H), 6.60 (d, J = 14.6 Hz, 1H), 6.68-6.72 (m, 1H), 6.97-7.12 (m, 7H), 7.22-7.32 (m, 1H), 7.54 (d, J = 8.3 Hz, 2H), 7.73 (d, J = 8.3 Hz, 2H), 9.73 (br s, 1H), 10.18 (br s, 1H); ESIMS m/z: $[M+H]^+$ 522 .

(2E,4Z)-N-(3-Hydroxy-2-oxo-1,2,3,4-tetrahydroquinolin-5-yl)-5-[6-(morpholin-4-yl)pyridin-3-yl]-5-[4-(trifluoromethyl) phenyl]-2,4-pentadienamide monohydrochloride (Compound 248)

$^1H$ NMR (DMSO-$d_6$, $\delta$ ppm) : 2.63 (dd, J = 12.0, 16.0 Hz, 1H), 3.01 (dd, J = 6.1, 16.0 Hz, 1H), 3.70-3.77 (m, 8H), 4.02-4.10 (m, 1H), 5.45 (br s, 1H), 6.67-6.74 (m, 2H), 7.07-7.31 (m, 5H), 7.59-7.68 (m, 3H), 7.76 (d, J = 8.4 Hz, 2H), 7.95 (s, 1H), 9.88 (br s, 1H), 10.19 (br s, 1H) ; ESIMS m/z: $[M+H]^+$ 565.

(2E,4Z)-5-[3-(N,N-Dimethylamino)phenyl]-N-(3-hydroxy-2-oxo-1,2,3,4-tetrahydroquinolin-5-yl)-5-[4-(trifluoromethyl) phenyl]-2,4-pentadienamide monohydrochloride (Compound 249)

$^1H$ NMR (DMSO-$d_6$, $\delta$ ppm): 2.62 (dd, J = 11.9, 15.9 Hz, 1H), 2.97-3.94 (m, 1H), 3.04 (s, 6H), 3.99-4.10 (m, 1H), 5.45 (br s, 1H), 6.64-6.73 (m, 2H), 6.96-6.99 (m, 1H), 7.06-7.25 (m, 5H), 7.40-7.44 (m, 1H), 7.51-7.59 (m, 3H) , 7.74 (d, J = 8.6 Hz, 2H), 9.85 (br s, 1H), 10.18 (br s, 1H); ESIMS m/z: $[M+H]^+$ 522.

(2E,4Z)-N-(3-Hydroxy-2-oxo-1,2,3,4-tetrahydroquinolin-5-yl)-5-(pyridin-4-yl)-5-[4-(trifluoromethyl)phenyl]-2,4-pentadienamide monohydrochloride (Compound 250)

$^1H$ NMR (DMSO-$d_6$, $\delta$ ppm): 2.62 (dd, J = 11.7, 15.8 Hz, 1H), 2.99 (dd, J = 6.3, 15.8 Hz, 1H), 4.03-4.09 (m, 1H), 6.72 (d, J = 7.3 Hz, 1H), 6.78 (d, J = 15.1 Hz, 1H), 6.98-7.15 (m, 3H), 7.44 (d, J = 11.6 Hz, 1H), 7.59 (d, J = 8.3 Hz, 2H), 7.77 (d, J = 8.3 Hz, 2H), 7.84 (d, J = 5.4 Hz, 2H), 9.00 (d, J = 4.0 Hz, 2H), 9.97 (br s, 1H), 10.20 (br s, 1H); ESIMS m/z: $[M+H]^+$ 480.

(2E,4Z)-N-(3-Hydroxy-2-oxo-1,2,3,4-tetrahydroquinolin-5-yl)-5-[6-(piperidin-1-yl)pyridin-3-yl]-5-[4-(trifluoromethyl)phenyl]-2,4-pentadienamide monohydrochloride (Compound 253)

$^1H$ NMR (DMSO-$d_6$, $\delta$ ppm) : 1.58-1.60 (m, 6H), 2.57-2.67 (m, 1H), 2.97-3.06 (m, 1H), 3.60-3.62 (m, 4H), 4.02-4.07 (m, 1H), 5.45 (d, J = 4.5 Hz, 1H), 6.61 (d, J = 14.7 Hz, 1H), 6.69-6.72 (m, 1H), 6.91 (d, J = 8.7 Hz, 1H), 7.02 (d, J = 11.7 Hz, 1H), 7.11-7.13 (m, 2H), 7.23-7.32 (m, 2H), 7.57 (d, J = 8.3 Hz, 2H), 7.75 (d, J = 8.3 Hz, 2H), 7.95 (d, J = 2.4 Hz, 1H), 9.73 (br s, 1H), 10.18 (br s, 1H); ESIMS m/z: $[M+H]^+$ 563.

(2E,4Z)-5-[6-(N,N-Dimethylamino)pyridin-3-yl]-N-(3-hydroxy-2-oxo-1,2,3,4-tetrahydroquinolin-5-yl)-5-[4-(trifluoromethyl)phenyl]-2,4-pentadienamide monohydrochloride (Compound 254)

$^1H$ NMR (DMSO-$d_6$, $\delta$ ppm) : 2.63 (dd, J = 11.7, 16.0 Hz, 1H), 3.00 (dd, J = 6.1, 15.0 Hz, 1H), 3.28 (s, 6H), 4.02-4.10 (m, 1H), 6.71-6.75 (m, 2H), 7.06-7.29 (m, 5H), 7.63 (d, J = 8.4 Hz, 2H), 7.72-7.79 (m, 3H), 7.88 (d, J = 1.8 Hz, 1H), 9.93 (br s, 1H), 10.21 (br s, 1H); ESIMS m/z: $[M+H]^+$ 523.

(2E,4Z)-N-(3-Hydroxy-2-oxo-1,2,3,4-tetrahydroquinolin-5-yl)-5-[6-(pyrrolidin-1-yl)pyridin-3-yl]-5-[4-(trifluoromethyl) phenyl]-2,4-pentadienamide monohydrochloride (Compound 261)

$^1$H NMR (DMSO-$d_6$, δ ppm): 1.99-2.05 (m, 4H), 2.63 (dd, J = 12.0, 16.0 Hz, 1H), 3.00 (dd, J = 6.2, 16.0 Hz, 1H), 3.60-3.62 (m, 4H), 4.03-4.09 (m, 1H), 6.71-6.75 (m, 2H), 7.03-7.29 (m, 5H), 7.63 (d, J = 8.1 Hz, 2H), 7.72-7.79 (m, 3H), 7.87 (d, J = 1.9 Hz, 1H), 9.93 (br s, 1H), 10.20 (br s, 1H); ESIMS m/z: $[M+H]^+$ 549.

(2E,4Z)-N-(3-Hydroxy-2-oxo-1,2,3,4-tetrahydroquinolin-5-yl)-5-[6-(2-methylpyrrolidin-1-yl)pyridin-3-yl]-5-[4-(trifluoromethyl)phenyl]-2,4-pentadienamide monohydrochloride (Compound 262)

$^1$H NMR (DMSO-$d_6$, δ ppm): 1.23 (d, J = 6.3 Hz, 3H), 1.78-1.79 (m, 1H), 2.10-2.12 (m, 2H), 2.63 (dd, J = 11.8, 15.8 Hz, 1H), 3.00 (dd, J = 5.8, 15.8 Hz, 1H), 3.34-3.73 (m, 3H), 4.06 (dd, J = 6.3, 11.8 Hz, 1H), 4.34-4.38 (m, 1H), 6.68-6.73 (m, 2H), 7.05-7.28 (m, 5H), 7.49-7.79 (m, 5H), 7.86-7.87 (m, 1H), 9.90 (br s, 1H) 10.20 (br s, 1H); ESIMS m/z: $[M+H]^+$ 563 .

(2E,4Z)-N-(3-Hydroxy-2-oxo-1,2,3,4-tetrahydroquinolin-5-yl)-5-[2-(piperidin-1-yl)pyrimidin-5-yl]-5-[4-(trifluoromethyl) phenyl]-2,4-pentadienamide monohydrochloride (Compound 263)

$^1$H NMR (DMSO-$d_6$, δ ppm) : 1.57-1.64 (m, 6H), 2.64 (dd, J = 11.6, 15.8 Hz, 1H), 3.03 (dd, J = 6.0, 15.8 Hz, 1H), 3.81-3.83 (m, 4H), 4.07 (dd, J = 6.0, 11.6 Hz, 1H), 6.63-6.73 (m, 2H), 7.09-7.16 (m, 3H), 7.23 (dd, J = 11.5, 14.2 Hz, 1H), 7.63 (d, J = 8.1 Hz, 2H), 7.76 (d, J = 8.1 Hz, 2H), 8.21 (s, 2H), 9.83 (br s, 1H), 10.19 (br s, 1H); ESIMS m/z: $[M+H]^+$ 564.

(2E,4Z)-5-(3-Cyano-4-fluorophenyl)-N-(isoquinolin-5-yl)-5-[4-(tritluoromethyl)phenyl]-2,4-pentadienamide monohydrochloride (Compound 264)

$^1$H NMR (DMSO-$d_6$, δ ppm): 6.88-6.95 (m, 1H), 7.06-7.42 (m, 2H), 7.58 (d, J = 8.4 Hz, 2H), 7.66-7.71 (m, 2H), 7.76 (d, J = 8.4 Hz, 2H) , 7.91-7.99 (m, 2H), 8.30 (d, J = 8.1 Hz, 1H), 8.39-8.43 (m, 1H), 8.50-8.54 (m, 1H), 8.71 (d, J = 6.8 Hz, 1H), 9.83 (s, 1H), 10.81 (br s, 1H); ESIMS m/z: $[M+H]^+$ 488.

(2E,4Z)-N-(Isoquinolin-5-yl)-5-(5-methylthiophen-2-yl)-5-[4-(trifluoromethyl)phenyl]-2,4-pentadienamide monohydrochloride (Compound 265)

$^1$H NMR (DMSO-$d_6$, δ ppm) : 2.52 (s, 3H), 6.84, (d, J = 14.7 Hz, 1H), 6.94-6.97 (m, 3H), 7.03 (d, J = 11.7 Hz, 1H), 7.64-7.72 (m, 3H), 7.79 (d, J = 8.4 Hz, 2H), 7.95 (t, J = 7.9 Hz, 1H) , 8.26 (d, J = 8.4 Hz, 1H), 8.41 (d, J = 7.2 Hz, 2H), 8.68 (d, J = 6.6 Hz, 1H), 9.76 (br s, 1H); ESIMS m/z: $[M+H]^+$ 465.

(2E,4Z)-N-(Isoquinolin-5-yl)-5-[4-(piperidin-1-yl)phenyl]-5-[4-(trifluoromethyl)phenyl]-2,4-pentadienamide dihydrochloride (Compound 267)

$^1$H NMR (DMSO-$d_6$, δ ppm): 1.64-1.66 (m, 2H), 1.91-1.94 (m, 4H), 3.48-3.52 (m, 4H), 6.94 (d, J = 13.8 Hz, 1H), 7.20-7.48 (m, 5H), 7.56 (d, J = 8.1 Hz, 2H), 7.76-7.78 (m, 3H), 7.99 (t, J = 7.9 Hz, 1H), 8.33 (d, J = 8.1 Hz, 1H), 8.46 (dd, J = 4.5, 6.9 Hz, 1H), 8.62 (d, J = 6.6 Hz, 1H), 8.73 (d, J = 6.9 Hz, 1H), 9.90 (s, 1H), 10.91 (br s, 1H); ESIMS m/z: $[M+H]^+$ 528.

(2E,4Z)-5-[4-(N,N-Dimethylamino)phenyl]-N-(3-hydroxy-1,2,3,4-tetrahydroquinolin-5-yl)-5-[4-(trifluoromethyl)phenyl]-2,4-pentadienamide dihydrochloride (Compound 270)

$^1$H NMR (DMSO-$d_6$, δ ppm): 2.63-2.65 (m, 1H), 2.84-2.86 (m, 1H), 3.03 (s, 6H), 3.10-3.17 (m, 1H), 3.27-3.31 (m, 1H), 4.17-4.18 (m, 1H), 6.63 (d, J = 15.1 Hz, 1H), 6.89-7.32 (m, 9H), 7.54 (d, J = 8.4 Hz, 2H), 7.74 (d, J = 8.4 Hz, 2H), 9.65 (br s, 1H); ESIMS m/z: $[M+H]^+$ 508.

(2E,4Z)-N-(3-Hydroxy-2-oxo-1,2,3,4-tetrahydroquinolin-5-yl)-5-[4-(piperidin-1-yl)phenyl]-5-[4-(trifluoromethyl)phenyl]-2,4-pentadienamide monohydrochloride (Compound 278)

$^1$H NMR (DMSO-$d_6$, δ ppm): 2.61-2.67 (m, 2H), 1.91-2.01 (m, 4H), 2.63 (dd, J = 12.0, 15.8 Hz, 1H), 3.01 (dd,

J = 6.3, 15.8 Hz, 1H), 3.53-3.57 (m, 4H), 3.96-4.07 (m, 1H), 6.66-6.73 (m, 2H), 7.06-7.22 (m, 4H), 7.40 (d, J = 8.4 Hz, 2H), 7.53 (d, J = 8.1 Hz, 2H), 7.75 (d, J = 8.4 Hz, 2H), 7.89 (d, J = 5.1 Hz, 2H), 9.88 (s, 1H), 10.19 (br s, 1H); ESIMS m/z : $[M+H]^+$ 562.

(2E,4Z)-N-(3-Hydroxy-2-oxo-1,2,3,4-tetrahydroquinolin-5-yl)-5-[6-(N-methyl(2-methoxyethyl)amino)pyridin-3-yl]-5-[4-(trifluoromethyl)phenyl]-2,4-pentadienamide monohydrochloride (Compound 301)

$^1$H NMR (DMSO-$d_6$, δ ppm) : 2.61 (dd, J = 12.2, 16.0 Hz, 1H), 2.95-3.03 (m, 1H), 3.22 (s, 3H), 3.28 (s, 3H), 3.82-3.83 (m, 2H), 3.98-4.08 (m, 3H), 6.69-6.71 (m, 2H), 7.08-7.20 (m, 5H), 7.59-7.62 (m, 3H), 7.76 (d, J = 8.3 Hz, 2H), 7.88 (s, 1H), 9.86 (br s, 1H), 10.19 (br s, 1H); ESIMS m/z:
$[M+H]^+$ 567.

(2E,4E)-5-(4-Chlorophenyl)-N-(3-hydroxy-2-oxo-1,2,3,4-tetrahydroquinolin-5-yl)-5-[4-(piperidin-1-yl)phenyl]-2,4-pentadienamide monohydrochloride (Compound 303)

$^1$H NMR (DMSO-$d_6$, δ ppm): 1.63-1.64 (m, 2H), 1.85-1.86 (m, 4H), 2.61 (dd, J = 12.0, 16.0 Hz, 1H), 3.00 (dd, J = 6.2, 16.0 Hz, 1H), 3.47-3.48 (m, 4H), 3.99-4.09 (m, 1H), 4.28 (br s, 1H), 6.59 (d, J = 14.3 Hz, 1H), 6.70 (d, J = 8.6 Hz, 1H), 7.02-7.32 (m, 6H), 7.33 (d, J = 8.6 Hz, 2H), 7.45 (d, J = 8.6 Hz, 2H), 7.55-7.58 (m, 2H), 9.77 (br s, 1H), 10.18 (br s, 1H); ESIMS m/z: $[M+H]^+$ 529.

(E)-N-(3-Hydroxy-2-oxo-1,2,3,4-tetrahydroquinolin-5-yl)-5,5-bis[6-(piperidin-1-yl)pyridin-3-yl]-2,4-pentadienamide dihydrochloride (Compound 305)

$^1$H NMR (DMSO-$d_6$, δ ppm): 1.66-1.67 (m, 12H), 2.62 (dd, J = 11.9, 16.0 Hz, 1H), 3.00 (dd, J = 6.2, 16.0 Hz, 1H), 3.74-3.75 (m, 8H), 4.06 (dd, J = 6.2, 11.9 Hz, 1H), 4.09 (br s, 1H), 6.62 (d, J = 13.3 Hz, 1H), 6.71 (d, J = 8.9 Hz, 1H), 7.06-7.20 (m, 4H), 7.32 (d, J = 8.1 Hz, 1H), 7.41 (d, J = 9.3 Hz, 1H), 7.69-7.76 (m, 2H), 7.86 (d, J = 2.0 Hz, 1H), 8.02 (d, J = 9.9 Hz, 1H), 9.84 (br s, 1H), 10.19 (br s, 1H); ESIMS m/z: $[M+H]^+$ 579.

Example 116

The following compounds were synthesized in a similar manner to Example 10 using a starting material and an amine corresponding to each compound.

(2E,4Z)-5-(3-Cyanophenyl)-N-(3-hydroxy-2-oxo-1,2,3,4-tetrahydroquinolin-5-yl)-5-[4-(trifluoromethyl)phenyl]-2,4-pentadienamide (Compound 183)

$^1$H NMR (DMSO-$d_6$, δ ppm) : 2.54-2.67 (m, 1H), 3.01 (dd, J = 6.3, 15.8 Hz, 1H), 4.02-4.10 (m, 1H), 5.45 (d, J = 4.6 Hz, 1H), 6.68-6.72 (m, 2H), 6.94-7.15 (m, 3H), 7.30 (d, J = 11.6 Hz, 1H), 7.54 (d, J = 8.1 Hz, 2H), 7.58-7.61 (m, 1H), 7.71-7.77 (m, 4H), 7.97 (td, J = 1.6, 8.1 Hz, 1H), 9.79 (br s, 1H), 10.17 (br s, 1H); ESIMS m/z: $[M+H]^+$ 504.

(2E,4E)-5-(4-Cyanophenyl)-N-(3-hydroxy-2-oxo-1,2,3,4-tetrahydroquinolin-5-yl)-5-[4-(trifluoromethyl)phenyl]-2,4-pentadienamide (Compound 184)

$^1$H NMR (DMSO-$d_6$, δ ppm): 2.62 (dd, J = 12.0, 16.0 Hz, 1H), 3.01 (dd, J = 6.1, 16.0 Hz, 1H), 4.02-4.11 (m, 1H), 5.45 (d, J = 4.6 Hz, 1H), 6.65-6.72 (m, 1H), 6.97-7.15 (m, 3H), 7.29 (d, J = 11.6 Hz, 1H), 7.43-7.54 (m, 5H), 7.75 (d, J = 8.4 Hz, 2H), 7.99 (d, J = 8.4 Hz, 2H), 9.79 (br s, 1H), 10.18 (br s, 1H); ESIMS m/z: $[M+H]^+$ 504.

(2E,4Z)-N-(3-Hydroxy-2-oxo-1,2,3,4-tetrahydroquinolin-5-yl)-5-(4-isopropoxyphenyl)-5-(4-methylphenyl)-2,4-pentadienamide (Compound 185)

$^1$H NMR (DMSO-$d_6$, δ ppm): 1.31 (d, J = 6.0 Hz, 6H), 2.32 (s, 3H), 2.62 (dd, J = 11.9, 15.9 Hz, 1H), 3.02 (dd, J = 6.2, 15.9 Hz, 1H), 4.02-4.10 (m, 1H), 4.67 (sept, J = 6.0 Hz, 1H) 5.43 (d, J = 4.3 Hz, 1H), 6.50 (d, J = 14.8 Hz, 1H), 6.67-6.70 (m, 1H), 6.90 (d, J = 11.3 Hz, 1H), 6.99 (d, J = 8.6 Hz, 2H), 7.06-7.23 (m, 9H), 9.63 (br s, 1H), 10.16 (br s, 1H); ESIMS m/z: $[M+H]^+$ 483.

(2E,4E)-5-(4-Chlorophenyl)-N-(3-hydroxy-2-oxo-1,2,3,4-tetrahydroquinolin-5-yl)-5-(4-isopropoxyphenyl)-2,4-pentadienamide (Compound 186)

$^1$H NMR (DMSO-$d_6$, δ ppm): 1.31 (d, J = 5.9 Hz, 6H), 2.62 (dd, J = 11.9, 15.9 Hz, 1H), 3.02 (dd, J = 6.1, 15.9 Hz, 1H), 4.02-4.10 (m, 1H), 4.67 (sept, J = 5.9 Hz, 1H), 5.44 (d, J = 4.3 Hz, 1H), 6.55 (d, J = 14.8 Hz, 1H), 6.68-6.71 (m, 1H), 6.95-7.23 (m, 8H), 7.34 (d, J = 8.4 Hz, 2H), 7.43 (d, J = 8.4 Hz, 2H), 9.68 (br s, 1H), 10.17 (br s, 1H); ESIMS m/z: $[M+H]^+$ 503.

(2E,4Z)-N-(3-Hydroxy-2-oxo-1,2,3,4-tetrahydroquinolin-5-yl)-5-(3-methylphenyl)-5-[4-(trifluoromethyl)phenyl]-2,4-pentadienamide (Compound 188)

$^1$H NMR (DMSO-$d_6$, ppm): 2.35 (s, 3H), 2.62 (dd, J = 11.9, 16.0 Hz, 1H), 3.01 (dd, J = 6.3, 16.0 Hz, 1H), 4.02-4.10 (m, 1H), 5.43 (d, J = 4.6 Hz, 1H), 6.61 (d, J = 12.7 Hz, 1H), 6.70 (dd, J = 2.6, 6.3 Hz, 1H), 7.02-7.19 (m, 6H), 7.28 (d, J = 7.8 Hz, 1H), 7.41 (t, J = 7.8 Hz, 1H), 7.54 (d, J = 8.3 Hz, 2H), 7.73 (d, J = 8.3 Hz, 2H), 9.73 (br s, 1H), 10.16 (br s, 1H); ESIMS m/z: $[M+H]^+$ 493.

(2E,4E)-N-(3-Hydroxy-2-oxo-1,2,3,4-tetrahydroquinolin-5-yl)-5-[4-(hydroxymethyl)phenyl]-5-[4-(trifluoromethyl)phenyl]-2,4-pentadienamide (Compound 189)

$^1$H NMR (DMSO-$d_6$, δ ppm): 2.62 (dd, J = 11.9, 16.0 Hz, 1H), 3.01 (dd, J = 6.1, 16.0 Hz, 1H), 4.03-4.08 (m, 1H), 4.55 (d, J = 5.7 Hz, 2H), 5.27 (t, J = 5.7 Hz, 1H), 5.44 (d, J = 4.5 Hz, 1H), 6.62 (d, J = 13.5 Hz, 1H), 6.70 (dd, J = 2.6, 6.1 Hz, 1H), 7.07-7.21 (m, 6H), 7.41 (d, J = 7.5 Hz, 1H), 7.48 (t, J = 7.5 Hz, 1H), 7.54 (d, J = 8.0 Hz, 2H), 7.74 (d, J = 8.0 Hz, 2H), 9.74 (br s, 1H), 10.17 (br s, 1H); ESIMS m/z: $[M+H]^+$ 509.

(2E,4Z)-N-(3-Hydroxy-2-oxo-1,2,3,4-tetrahydroquinolin-5-yl)-5-[3-(hydroxymethyl)phenyl]-5-[4-(trifluoromethyl)phenyl]-2,4-pentadienamide (Compound 190)

$^1$H NMR (DMSO-$d_6$, δ ppm): 2.62 (dd, J = 11.9, 16.0 Hz, 1H), 3.01 (dd, J = 6.3, 16.0 Hz, 1H), 4.02-4.08 (m, 1H), 4.58 (d, J = 5.7 Hz, 2H), 5.29 (t, J = 5.7 Hz, 1H), 5.45 (d, J = 4.5 Hz, 1H), 6.60-6.65 (m, 1H), 6.70 (dd, J = 2.3, 6.7 Hz, 1H), 7.09-7.20 (m, 6H), 7.46 (d, J = 8.1 Hz, 2H), 7.54 (d, J = 8.1 Hz, 2H), 7.74 (d, J = 8.1 Hz, 2H), 9.74 (br s, 1H), 10.17 (br s, 1H); ESIMS m/z: $[M+H]^+$ 509.

(2E,4Z)-5-(4-tert-Butoxyphenyl)-N-(3-hydroxy-2-oxo-1,2,3,4-tetrahydroquinolin-5-yl)-5-[4-(trifluo romethyl)phenyl]-2,4-pentadienamide (Compound 191).

$^1$H NMR (DMSO-$d_6$, δ ppm): 1.37 (s, 9H), 2.62 (dd, J = 11.9, 15.9 Hz, 1H), 3.02 (dd, J = 6.2, 15.9 Hz, 1H), 4.03-4.09 (m, 1H), 5.44 (d, J = 4.6 Hz, 1H), 6.61 (d, J = 14.0 Hz, 1H), 6.70 (dd, J = 3.4, 5.5 Hz, 1H), 7.07-7.24 (m, 8H), 7.54 (d, J = 8.3 Hz, 2H), 7.74 (d, J = 8.3 Hz, 2H), 9.72 (br s, 1H), 10.17 (br s, 1H); ESIMS m/z: $[M+H]^+$ 551.

(2E,4Z)-5-(4-Cyclobutoxyphenyl)-N-(3-hydroxy-2-oxo-1,2,3,4-tetrahydroquinolin-5-yl)-5-[4-(trifluoromethyl)phenyl]-2,4-pentadienamide (Compound 192)

$^1$H NMR (DMSO-$d_6$, δ ppm): 1.60-1.71 (m, 1H), 1.74-1.86 (m, 1H), 2.02-2.17 (m, 2H), 2.45-2.67 (m, 3H), 3.02 (dd, J = 6.3, 15.8 Hz, 1H), 4.02-4.11 (m, 1H), 4.75 (quint, J = 7.0 Hz, 1H), 5.44 (d, J = 4.6 Hz, 1H), 6.60 (d, J = 14.3 Hz, 1H), 6.70 (dd, J = 3.1, 5.8 Hz, 1H), 6.96 (d, J = 8.6 Hz, 2H), 7.05-7.24 (m, 6H), 7.53 (d, J = 8.1 Hz, 2H), 7.73 (d, J = 8.1 Hz, 2H), 9.72 (br s, 1H), 10.17 (br s, 1H); ESIMS m/z: $[M+H]^+$ 549.

(2E,4Z)-N-(3-Hydroxy-2-oxo-1,2,3,4-tetrahydroquinolin-5-yl)-5-[4-(1-methylcyclopropylmethoxy)phenyl]-5-[4-(trifluoromethyl)phenyl]-2,4-pentadienamide (Compound 193)

$^1$H NMR (DMSO-$d_6$, δ ppm): 0.39-0.42 (m, 2H), 0.54-0.57 (m, 2H), 1.21 (s, 3H), 2.63 (dd, J = 12.0, 16.0 Hz, 1H), 3.03 (dd, J = 6.3, 16.0 Hz, 1H), 3.82 (s, 2H), 4.03-4.12 (m, 1H), 5.44 (d, J = 4.3 Hz, 1H), 6.61 (d, J = 14.3 Hz, 1H), 6.69-6.73 (m, 1H), 7.02-7.26 (m, 8H), 7.54 (d, J = 8.3 Hz, 2H), 7.73 (d, J = 8.3 Hz, 2H), 9.72 (br s, 1H), 10.17 (br s, 1H); ESIMS m/z: $[M+H]^+$ 563.

(2E,4Z)-5-{4-[(3-Ethyloxetan-3-yl)methoxy]phenyl}-N-(3-hydroxy-2-oxo-1,2,3,4-tetrahydroquinolin-5-yl)-5-[4-(trifluoromethyl)phenyl]-2,4-pentadienamide (Compound 194)

$^1$H NMR (DMSO-$d_6$, δ ppm): 0.92 (t, J = 7.4 Hz, 3H), 1.81 (q, J = 7.4 Hz, 2H), 2.62 (dd, J = 12.2, 15.9 Hz, 1H), 3.01 (dd, J = 6.3, 15.9 Hz, 1H), 4.02-4.10 (m, 1H), 4.17 (s, 2H), 4.36 (d, J = 5.9 Hz, 2H), 4.48 (d, J = 5.9 Hz, 2H), 5.44 (d, J = 4.0 Hz, 1H), 6.61 (d, J = 14.3 Hz, 1H), 6.68-6.71 (m, 1H), 7.16 (m, 8H), 7.54 (d, J = 8.3 Hz, 2H), 7.73 (d, J = 8.3 Hz, 2H), 9.72 (br s, 1H), 10.17 (br s, 1H); ESIMS m/z: $[M+H]^+$ 593.

(2E,4Z)-5-[3-(Cyclopropylmethoxy)phenyl]-N-(3-hydroxy-2-oxo-1,2,3,4-tetrahydroquinolin-5-yl)-5-[4-(trifluoromethyl) phenyl]-2,4-pentadienamide (Compound 195)

$^1$H NMR (DMSO-$d_6$, δ ppm): 0.28-0.33 (m, 2H), 0.51-0.58 (m, 2H), 1.18-1.25 (m, 1H), 2.62 (dd, J = 11.9, 16.0 Hz, 1H), 3.01 (dd, J = 6.0, 16.0 Hz, 1H), 3.82 (d, J = 6.9 Hz, 2H), 4.02-4.10 (m, 1H), 5.44 (d, J = 4.2 Hz, 1H), 6.59-6.78 (m, 4H), 7.01-7.19 (m, 5H), 7.41 (t, J = 7.9 Hz, 1H), 7.55 (d, J = 8.4 Hz, 2H), 7.73 (d, J = 8.4 Hz, 2H), 9.74 (br s, 1H), 10.17 (br s, 1H); ESIMS m/z: $[M+H]^+$ 549.

(2E,4Z)-N-(3-Hydroxy-2-oxo-1,2,3,4-tetrahydroquinolin-5-yl)-5-[3-(1-methylcyclopropylmethoxy)phenyl]-5-[4-(trifluoromethyl)phenyl]-2,4-pentadienamide (Compound 196)

$^1$H NMR (DMSO-$d_6$, δ ppm): 0.34-0.38 (m, 2H), 0.48-0.52 (m, 2H), 1.16 (s, 3H), 2.62 (dd, J = 11.9, 15.9 Hz, 1H), 3.01 (dd, J = 6.1, 15.9 Hz, 1H), 3.76 (s, 2H), 4.02-4.10 (m, 1H), 5.43 (d, J = 4.3 Hz, 1H), 6.59-6.79 (m, 4H), 7.00-7.19 (m, 5H), 7.41 (t, J = 7.8 Hz, 1H), 7.55 (d, J = 8.3 Hz, 2H), 7.73 (d, J = 8.3 Hz, 2H), 9.74 (br s, 1H), 10.16 (br s, 1H); ESIMS m/z: $[M+H]^+$ 563.

(2E,4Z)-5-[3-(2-Cyclopropylethoxy)phenyl]-N-(3-hydroxy-2-oxo-1,2,3,4-tetrahydroquinolin-5-yl)-5-[4-(trifluoromethyl) phenyl]-2,4-pentadienamide (Compound 197)

$^1$H NMR (DMSO-$d_6$, δ ppm): 0.07-0.12 (m, 2H), 0.38-0.44 (m, 2H), 0.77-0.84 (m, 1H), 1.61 (q, J = 6.8 Hz, 2H), 2.62 (dd, J = 11.9, 16.0 Hz, 1H), 3.01 (dd, J = 6.3, 16.0 Hz, 1H), 4.01-4.05 (m, 3H), 5.43 (d, J = 3.5 Hz, 1H), 6.55-6.79 (m, 4H), 7.02-7.17 (m, 5H), 7.42 (t, J = 7.8 Hz, 1H), 7.55 (d, J = 8.1 Hz, 2H), 7.73 (d, J = 8.1 Hz, 2H), 9.74 (br s, 1H), 10.16 (br s, 1H); ESIMS m/z: $[M+H]^+$ 563.

(2E,4Z)-5-{3-[(3-Ethyloxetan-3-yl)methoxy]phenyl}-N-(3-hydroxy-2-oxo-1,2,3,4-tetrahydroquinolin-5-yl)-5-[4-(trifluoromethyl)phenyl]-2,4-pentadienamide (Compound 198)

$^1$H NMR (DMSO-$d_6$, δ ppm): 0.88 (t, J = 7.4 Hz, 3H), 1.77 (q, J = 7.4 Hz, 2H), 2.61 (dd, J = 12.0, 15.9 Hz, 1H), 3.01 (dd, J = 6.2, 15.9 Hz, 1H), 4.04-4.06 (m, 1H), 4.11 (s, 2H), 4.32 (d, J = 5.9 Hz, 2H), 4.44 (d, J = 5.9 Hz, 2H), 5.42 (d, J = 3.0 Hz, 1H), 6.62 (d, J = 12.4 Hz, 1H), 6.68-6.71 (m, 1H), 6.81-6.84 (m, 2H), 7.09-7.21 (m, 5H), 7.45 (t, J = 7.8 Hz, 1H), 7.56 (d, J = 8.3 Hz, 2H), 7.74 (d, J = 8.3 Hz, 2H), 9.74 (br s, 1H), 10.16 (br s, 1H); ESIMS m/z: $[M+H]^+$ 593.

(2E,4Z)-5-{3-[(3-Methyloxetan-3-yl)methoxy]phenyl}-N-(3-hydroxy-2-oxo-1,2,3,4-tetrahydroquinolin-5-yl)-5-[4-(trifluoromethyl)phenyl]-2,4-pentadienamide (Compound 199)

$^1$H NMR (DMSO-$d_6$, δ ppm): 1.35 (s, 3H), 2.61 (dd, J = 12.2, 15.8 Hz, 1H), 3.01 (dd, J = 5.8, 15.8 Hz, 1H), 4.05-4.06 (m, 3H), 4.28 (d, J = 5.8 Hz, 2H), 4.48 (d, J = 5.8 Hz, 2H), 5.44 (d, J = 4.3 Hz, 1H), 6.62 (d, J = 13.5 Hz, 1H), 6.68-6.71 (m, 1H), 6.79-6.84 (m, 2H), 7.09-7.21 (m, 5H), 7.45 (t, J = 8.0 Hz, 1H), 7.56 (d, J = 8.0 Hz, 2H), 7. 74 (d, J = 8.0 Hz, 2H), 9.75 (br s, 1H), 10.17 (br s, 1H); ESIMS m/z: $[M+H]^+$ 579.

(2E,4Z)-N-(3-Hydroxy-2-oxo-1,2,3,4-tetrahydroquinolin-5-yl)-5-{4-[(3-methyloxetan-3-yl)methoxy]phenyl}-5-[4-(trifluoromethyl)phenyl]-2,4-pentadienamide (Compound 201)

$^1$H NMR (DMSO-$d_6$, δ ppm): 1.39 (s, 3H), 2.62 (dd, J = 12.2, 15.8 Hz, 1H), 3.02 (dd, J = 6.3, 15.8 Hz, 1H), 4.03-4.10 (m, 1H), 4.13 (s, 2H), 4.33 (d, J = 4.8 Hz, 2H), 4.53 (d, J = 4.5 Hz, 2H), 5.45 (d, J = 4.5 Hz, 1H), 6.61 (d, J = 14.4 Hz, 1H), 6.70 (dd, J = 2.9, 6.2 Hz, 1H), 7.07-7.24 (m, 8H), 7.54 (d, J = 8.3 Hz, 2H), 7.74 (d, J = 8.3 Hz, 2H), 9.73 (br s, 1H), 10.18 (br s, 1H); ESIMS m/z: $[M+H]^+$ 579.

(2E,4Z)-N-(3-Hydroxy-2-oxo-1,2,3,4-tetrahydroquinolin-5-yl)-5-[4-(tetrahydropyran-4-ylmethoxy)phenyl]-5-[4-(trifluoromethyl)phenyl]-2,4-pentadienamide (Compound 202)

$^1$H NMR (DMSO-$d_6$, δ ppm): 1.28-1.43 (m, 2H), 1.68-1.73 (m, 2H), 2.01-2.05 (m, 1H), 2.62 (dd, J = 12.0, 15.9 Hz, 1H), 3.01 (dd, J = 6.2, 15.9 Hz, 1H), 3.32-3.39 (m, 2H), 3.86-3.91 (m, 4H), 4.02-4.10 (m, 1H), 5.44 (d, J = 4.6 Hz, 1H), 6.60 (d, J = 14.6 Hz, 1H), 6.70 (dd, J = 3.0, 5.7 Hz, 1H), 7.04-7.25 (m, 8H), 7.53 (d, J = 8.5 Hz, 2H), 7.73 (d, J = 8.5 Hz, 2H), 9.72 (br s, 1H), 10.17 (br s, 1H); ESIMS m/z:
$[M+H]^+$ 593.

(2E,4Z)-N-(3-Hydroxy-2-oxo-1,2,3,4-tetrahydroquinolin-5-yl)-5-[3-(tetrahydropyran-4-ylmethoxy)phenyl]-5-[4-(trifluoromethyl)phenyl]-2,4-pentadienamide (Compound 203)

$^1$H NMR (DMSO-$d_6$, δ ppm): 1.24-1.39 (m, 2H), 1.64-1.68 (m, 2H), 1.95-2.03 (m, 1H), 2.62 (dd, J = 11.9, 15.8 Hz, 1H), 3.01 (dd, J = 6.2, 15.8 Hz, 1H), 3.27-3.42 (m, 2H), 3.83-3.88 (m, 4H), 4.02-4.10 (m, 1H), 5.43 (d, J = 4.6 Hz, 1H), 6.59-6.80 (m, 4H), 7.02-7.19 (m, 5H), 7.42 (t, J = 7.8 Hz, 1H), 7.55 (d, J = 8.3 Hz, 2H), 7.73 (d, J = 8.3 Hz, 2H), 9.73 (br s, 1H), 10.16 (br s, 1H); ESTMS m/z: $[M+H]^+$ 593.

(2E,4E)-N-(3-Hydroxy-2-oxo-1,2,3,4-tetrahydroquinolin-5-yl)-5-(4-methylphenyl)-5-[4-(trifluoromethyl)phenyl]-2,4-pentadienamide (Compound 206)

$^1$H NMR (DMSO-$d_6$, δ ppm): 2.39 (s, 3H), 2.61 (dd, J = 11.9, 16.0 Hz, 1H), 3.01 (dd, J = 6.1, 16.0 Hz, 1H), 4.02-4.10 (m, 1H), 5.44 (d, J = 4.8 Hz, 1H), 6.59-6.63 (m, 1H), 6.70 (dd, J = 2.3, 6.4 Hz, 1H), 7.10-7.20 (m, 6H), 7.32 (d, J = 7.8 Hz, 2H), 7.53 (d, J = 8.3 Hz, 2H), 7.73 (d, J = 8.3 Hz, 2H), 9.73 (br s, 1H), 10.17 (br s, 1H); ESIMS m/z: $[M+H]^+$ 493.

(2E,4E)-N-(3-Hydroxy-2-oxo-1,2,3,4-tetrahydroquinolin-5-yl)-5-phenyl-5-[4-(trifluoromethyl)phenyl]-2,4-pentadienamide (Compound 207)

$^1$H NMR (DMSO-$d_6$, δ ppm) : 2.62 (dd, J = 11.9, 15.9 Hz, 1H), 3.02 (dd, J = 6.3, 15.9 Hz, 1H), 4.03-4.11 (m, 1H), 5.45 (d, J = 4.3 Hz, 1H), 6.61-6.72 (m, 2H), 7.08-7.25 (m, 6H), 7.45-7.55 (m, 5H), 7.74 (d, J = 8.1 Hz, 2H), 9.74 (br s, 1H), 10.17 (br s, 1H); ESIMS m/z: $[M+H]^+$ 479.

(2E,4Z)-N-(3-Hydroxy-2-oxo-1,2,3,4-tetrahydroquinolin-5-yl)-5-(1-methyl-1H-pyrazol-4-yl)-5-[4-(trifluoromethyl)phenyl]-2,4-pentadienamide (Compound 208)

$^1$H NMR (DMSO-$d_6$, δ ppm) : 2.64 (dd, J = 11.9, 15.9 Hz, 1H), 3.04 (dd, J = 6.3, 15.9 Hz, 1H), 3.91 (s, 3H), 4.04-4.12 (m, 1H), 5.45 (d, J = 4.6 Hz, 1H), 6.59 (d, J = 14. 9 Hz, 1H), 6.71 (t, J = 4.5 Hz, 1H) 6.88 (d, J = 11.3 Hz, 1H), 7.13 (d, J = 4.6 Hz, 2H), 7.44-7.54 (m, 2H), 7.63 (d, J = 8.4 Hz, 2H), 7.75 (d, J = 8.4 Hz, 2H), 7.84 (s, 1H), 9.72 (br s, 1H), 10.18 (br s, 1H); ESIMS m/z: $[M+H]^+$ 483.

(2E,4E)-5-(4-Ethylphenyl)-N-(3-hydroxy-2-oxo-1,2,3,4-tetrahydroquinolin-5-yl)-5-[4-(trifluoromethyl)phenyl]-2,4-pentadienamide (Compound 209)

$^1$H NMR (DMSO-$d_6$, δ ppm) : 1.25 (t, J = 7.6 Hz, 3H), 2.57-2.65 (m, 1H), 2.70 (q, J = 7.6 Hz, 2H), 3.01 (dd, J = 6.2, 15.9 Hz, 1H), 4.02-4.10 (m, 1H), 5.44 (d, J = 4.3 Hz, 1H), 6.61 (d, J = 12.7 Hz, 1H), 6.70 (dd, J = 3.0, 6.2 Hz, 1H), 7.09-7.22 (m, 6H), 7.35 (d, J = 7.8 Hz, 2H), 7.53 (d, J = 8.3 Hz, 2H), 7.73 (d, J = 8.3 Hz, 2H), 9.73 (br s, 1H), 10.17 (br s, 1H); ESIMS m/z: $[M+H]^+$ 507.

(2E,42)-N-(3-Hydroxy-2-oxo-1,2,3,4-tetrahydroquinolin-5-yl)-5-(4-hydroxyphenyl)-5-[4-(trifluoromethyl)phenyl]-2,4-pentadienamide (Compound 210)

$^1$H NMR (DMSO-$d_6$, δ ppm): 2.62 (dd, J = 12.0, 16.0 Hz, 1H), 3.02 (dd, J = 6.2, 16.0 Hz, 1H), 4.03-4.11 (m, 1H), 5.42 (d, J = 4.6 Hz, 1H), 6.58 (d, J = 14.8 Hz, 1H), 6.68-6.72 (m, 1H), 6.87 (d, J = 8.4 Hz, 2H), 7.00-7.12 (m, 5H), 7.23 (dd, J = 11.7, 14.7 Hz, 1H), 7.54 (d, J = 8.3 Hz, 2H), 7.72 (d, J = 8.3 Hz, 2H), 9.69 (br s, 1H), 9.75 (br s, 1H), 10.16 (br s, 1H); ESIMS m/z: $[M+H]^+$ 495.

(2E,4Z)-5-[4-(2-Cyclopropylethoxy)phenyl]-N-(3-hydroxy-2-oxo-1,2,3,4-tetrahydroquinolin-5-yl)-5-[4-(trifluoromethyl) phenyl]-2,4-pentadienamide (Compound 211)

$^1$H NMR (DMSO-$d_6$, δ ppm): 0.12-0.17 (m, 2H), 0.42-0.48 (m, 2H), 0.82-0.89 (m, 1H), 1.66 (q, J = 6.6 Hz, 2H), 2.63 (dd, J = 12.0, 15.9 Hz, 1H), 3.03 (dd, J = 6.2, 15.9 Hz, 1H), 4.03-4.11 (m, 3H), 5.45 (d, J = 4.3 Hz, 1H), 6.61 (d, J = 14.6 Hz, 1H), 6.69-6.72 (m, 1H), 7.04-7.26 (m, 8H), 7.54 (d, J = 8.3 Hz, 2H), 7.73 (d, J = 8.3 Hz, 2H), 9.72 (br s, 1H), 10.18 (br s, 1H); ESIMS m/z: $[M+H]^+$ 563.

(2E,4Z)-5-[4-(Cyclopropylmethoxy)phenyl]-N-(3-hydroxy-2-oxo-1,2,3,4-tetrahydroquinolin-5-yl)-5-[4-(trifluoromethyl) phenyl]-2,4-pentadienamide (Compound 212)

$^1$H NMR (DMSO-$d_6$, δ ppm): 0.32-0.38 (m, 2H), 0.56-0.62 (m, 2H), 1.23-1.28 (m, 1H), 2.52 (dd, J = 11.9, 16.0 Hz, 1H), 3.02 (dd, J = 6.3, 16.0 Hz, 1H), 3.88 (d, J = 7.0 Hz, 2H), 4.03-4.11 (m, 1H), 5.44 (d, J = 4.3 Hz, 1H), 6.60 (d, J = 14.3 Hz, 1H), 6.68-6.72 (m, 1H), 7.02-7.25 (m, 8H), 7.53 (d, J = 8.3 Hz, 2H), 7.73 (d, J = 8.3 Hz, 2H), 9.72 (br s, 1H), 10.17 (br s, 1H); ESIMS m/z: $[M+H]^+$ 549.

(2E,4Z)-N-(3-Hydroxy-2-oxo-1,2,3,4-tetrahydroquinolin-5-yl)-5-[4-(methoxycarbonylmethoxy)phenyl]-5-[4-(trifluoromethyl)phenyl]-2,4-pentadienamide (Compound 213)

$^1$H NMR (DMSO-$d_6$, δ ppm): 2.62 (dd, J = 11.9, 15.9 Hz, 1H), 3.02 (dd, J = 6.2, 15.9 Hz, 1H), 3.73 (s, 3H), 4.02-4.11 (m, 1H), 4.88 (s, 2H), 5.44 (d, J = 4.6 Hz, 1H), 6.61 (d, J = 14.0 Hz, 1H), 6.68-6.72 (m, 1H), 7.04-7.23 (m, 8H), 7.53 (d, J = 8.3 Hz, 2H), 7.73 (d, J = 8.3 Hz, 2H), 9.72 (br s, 1H), 10.17 (br s, 1H); ESIMS m/z: $[M+H]^+$ 567.

(2E,4Z)-N-(3-Hydroxy-2-oxo-1,2,3,4-tetrahydroquinolin-5-yl)-5-[2-(piperidin-1-yl)pyridin-4-yl]-5-[4-(trifluoromethyl)phenyl]-2,4-pentadienamide (Compound 214)

$^1$H NMR (DMSO-$d_6$, δ ppm): 1.54-1.56 (m, 6H), 2.61 (dd, J = 11.7, 15.9 Hz, 1H), 3.00 (dd, J = 6.2, 15.9 Hz, 1H), 3.52-3.54 (m, 4H), 4.01-4.10 (m, 1H), 5.41 (d, J = 4.6 Hz, 1H), 6.41 (d, J = 4.9 Hz, 1H), 6.60-6.71 (m, 3H), 7.06-7.23 (m, 4H), 7.58 (d, J = 8.4 Hz, 2H), 7.73 (d, J = 8.4 Hz, 2H), 8.19 (d, J = 4.9 Hz, 1H), 9.75 (br s, 1H), 10.15 (br s, 1H); ESIMS m/z: $[M+H]^+$ 563.

(2E,4Z)-N-(3-Hydroxy-2-oxo-1,2,3,4-tetrahydroquinolin-5-yl)-5-(4-methylthiophenyl)-5-[4-(trifluoromethyl)phenyl]-2,4-pentadienamide (Compound 215)

$^1$H NMR (DMSO-$d_6$, δ ppm): 2.54 (s, 3H), 2.62 (dd, J = 11.7, 15.8 Hz, 1H), 3.02 (dd, J = 6.2, 15.8 Hz, 1H), 4.03-4.11 (m, 1H), 5.45 (d, J = 4.3 Hz, 1H), 6.60-6.72 (m, 2H), 7.10-7.26 (m, 6H), 7.38. (d, J = 8.4 Hz, 2H), 7.54 (d, J = 8.3 Hz, 2H), 7.73 (d, J = 8.3 Hz, 2H), 9.74 (br s, 1H), 10.17 (br s, 1H); ESIMS m/z: $[M+H]^+$ 525.

(2E,4Z)-N-(3-Hydroxy-2-oxo-1,2,3,4-tetrahydroquinolin-5-yl)-5-[2-(pyrrolidin-1-yl)pyridin-4-yl]-5-[4-(trifluoromethyl) phenyl]-2,4-pentadienamide (Compound 216)

$^1$H NMR (DMSO-$d_6$, δ ppm): 1.91-1.96 (m, 4H), 2.62 (dd, J = 11.9, 15.8Hz, 1H), 3.01 (dd, J = 6.2, 15.8Hz, 1H), 3.36-3.42 (m, 4H), 4.02-4.10 (m, 1H), 5.42 (d, J = 4.6 Hz, 1H), 6.23 (s, 1H), 6.36 (d, J = 5.0 Hz, 1H), 6.62 (d, J = 14.0 Hz, 1H), 6.70 (dd, J = 2.0, 6.9 Hz, 1H), 7.06-7.25 (m, 4H), 7.60 (d, J = 8.3 Hz, 2H), 7.73 (d, J = 8.3 Hz, 2H), 8.16 (d, J = 5.0 Hz, 1H), 9.75 (br s, 1H), 10.16 (br s, 1H); ESIMS m/z: $[M+H]^+$ 549.

(2E,4Z)-N-(3-Hydroxy-2-oxo-1,2,3,4-tetrahydroquinolin-5-yl)-5-(2-isopropoxyphenyl)-5-[4-(trifluoromethyl)phenyl]-2,4-pentadienamide (Compound 217)

$^1$H NMR (DMSO-$d_6$, δ ppm) : 0.85-0.95 (m, 6H), 2.61 (dd, J = 12.0, 15.9 Hz, 1H), 3.01 (dd, J = 6.2, 15.9 Hz, 1H), 4.02-4.11 (m, 1H), 4.48 (sept, J = 5.9 Hz, 1H), 5.43 (d, J = 4.6 Hz, 1H), 6.58 (d, J = 13.8 Hz, 1H), 6.70 (dd, J = 2.4, 6.5 Hz, 1H), 7.00-7.18 (m, 7H), 7.39-7.46 (m, 1H), 7.49 (d, J = 8.3 Hz, 2H), 7.69 (d, J = 8.3 Hz, 2H), 9.71 (br s, 1H), 10.16 (br s, 1H); ESIMS m/z: $[M+H]^+$ 537.

(2E,4Z)-5-(2-Ethoxyphenyl)-N-(3-hydroxy-2-oxo-1,2,3,4-tetrahydroquinolin-5-yl)-5-[4-(trifluoromethyl)phenyl]-2,4-pentadienamide (Compound 218)

$^1$H NMR (DMSO-$d_6$, δ ppm): 0.91 (t, J = 6.9 Hz, 3H), 2.60 (dd, J = 11.9, 15.9 Hz, 1H), 3.00 (dd, J = 6.2, 15.9

Hz, 1H), 3.86-3.90 (m, 2H), 4.01-4.09 (m, 1H), 5.41 (d, J = 4.6 Hz, 1H), 6.58 (d, J = 14.6 Hz, 1H), 6.69 (dd, J = 2.3, 6.9 Hz, 1H), 6.97-7.18 (m, 7H), 7.40-7.47 (m, 1H), 7.49 (d, J = 8.2 Hz, 2H), 7.68 (d, J = 8.2 Hz, 2H), 9.70 (br s, 1H), 10.15 (br s, 1H); ESIMS m/z: $[M+H]^+$ 523.

(2E,4Z)-N-(3-Hydroxy-2-oxo-1,2,3,4-tetrahydroquinolin-5-yl)-5-(2-methoxyphenyl)-5-[4-(trifluoromethyl)phenyl]-2,4-pentadienamide (Compound 219)

$^1$H NMR (DMSO-$d_6$, δ ppm): 2.61 (dd, J = 11.7, 15.9 Hz, 1H), 3.00 (dd, J = 6.3, 15.9 Hz, 1H), 3.62 (s, 3H), 4.02-4.09 (m, 1H), 5.44 (d, J = 4.5 Hz, 1H), 6.58 (d, J = 15.0 Hz, 1H), 6.69 (dd, J = 2.0, 7.0 Hz, 1H), 6.96 (dd, J = 11.6, 15.0 Hz, 1H), 7.08-7.27 (m, 6H), 7.44-7.50 (m, 1H), 7.52 (d, J = 8.6 Hz, 2H), 7.69 (d, J = 8.6 Hz, 2H), 9.73 (br s, 1H), 10.17 (br s, 1H); ESIMS m/z: [M+H] + 509.

(2E,4Z)-N-(3-Hydroxy-2-oxo-1,2,3,4-tetrahydroquinolin-5-yl)-5-(2-methoxypyrimidin-5-yl)-5-[4-(trifluoromethyl)phenyl]-2,4-pentadienamide (Compound 220)

$^1$H NMR (DMSO-$d_6$, δ ppm): 2.63 (dd, J = 11.7, 15.9 Hz, 1H), 3.02 (dd, J = 6.2, 15.9 Hz, 1H), 4.01 (s, 3H), 4.03-4.11 (m, 1H), 5.43 (d, J = 4.6 Hz, 1H), 6.66-6.73 (m, 2H), 7.04-7.16 (m, 3H), 7.32 (d, J = 11.3 Hz, 1H), 7.62 (d, J = 8.4 Hz, 2H), 7.76 (d, J = 8.4 Hz, 2H), 8.52 (s, 2H), 9.79 (br s, 1H), 10.17 (br s, 1H) ; ESIMS m/z: $[M+H]^+$ 511.

(2E,4Z)-5-(6-Ethoxypyridin-3-yl)-N-(3-hydroxy-2-oxo-1,2,3,4-tetrahydroquinolin-5-yl)-5-[4-(trifluoromethyl)phenyl]-2,4-pentadienamide (Compound 225)

$^1$H NMR (DMSO-$d_6$, δ ppm): 1.29-1.38 (m, 3H), 2.57-2.67 (m, 1H), 2.96-3.05 (m, 1H), 4.03-4.08 (m, 1H), 4.28-4.42 (m, 2H), 5.44-5.46 (m, 1H), 6.56-6.71 (m, 2H), 6.80-6.94 (m, 1H), 7.06-7.23 (m, 4H), 7.45-7.58 (m, 3H), 7.73-7.76 (m, 1H), 7.86-7.89 (m, 1H), 8.04-8.06 (m, 1H), 9.77 (br s, 1H), 10.17 (br s, 1H); ESIMS m/z: $[M+H]^+$ 524.

(2E,4Z)-N-(3-Hydroxy-2-oxo-1,2,3,4-tetrahydroquinolin-5-yl)-5-(4-methoxyphenyl)-5-[4-(trifluoromethyl)phenyl]-2,4-pentadienamide. (Compound 227)

$^1$H NMR (DMSO-$d_6$, δ ppm): 2.63 (dd, J = 12.2, 15.9 Hz, 1H), 3.02 (dd, J = 6.2, 15.9 Hz, 1H), 3.83 (s, 3H), 4.03-4.11 (m, 1H), 5.44 (d, J = 4.6 Hz, 1H), 6.61 (d, J = 14.3 Hz, 1H), 6.69-6.72 (m, 1H), 7.05-7.25 (m, 8H), 7.54 (d, J = 8.5 Hz, 2H), 7.73 (d, J = 8.5 Hz, 2H), 9.72 (s, 1H), 10.17 (s, 1H); ESIMS m/z: $[M+H]^+$ 509.

(2E,4Z)-5-[6-(Azetidin-1-yl)pyridin-3-yl]-N-(3-hydroxy-2-oxo-1,2,3,4-tetrahydroquinolin-5-yl)-5-[4-(trifluoromethyl)phenyl]-2,4-pentadienamide (Compound 228)

$^1$H NMR (DMSO-$d_6$, δ ppm): 2.30-2.41. (m, 2H), 2.63 (dd, J= 11.9, 15.9 Hz, 1H); 3.02 (dd, J = 6.2, 15.9 Hz, 1H), 3.99-4.09 (m, 5H), 5.44 (d, J = 4.3 Hz, 1H), 6.44 (d, J = 8.4 Hz, 1H), 6.60 (d, J = 14.6 Hz, 1H), 6.68-6.72 (m, 1H), 7.01-7.31 (m, 5H), 7.55 (d, J = 8.1 Hz, 2H), 7.74 (d, J = 8.1 Hz, 2H), 7.91 (d, J = 2.2 Hz, 1H), 9.72 (br s, 1H), 10.17 (br s, 1H); ESIMS m/z: $[M+H]^+$ 535.

(2E,4Z)-N-(3-Hydroxy-2-oxo-1,2,3,4-tetrahydroquinolin-5-yl)-5-(4-isopropoxyphenyl)-5-[4-(trifluoromethyl)phenyl]-2,4-pentadienamide (Compound 229)

$^1$H NMR (DMSO-$d_6$, δ ppm): 1.31 (d, J = 6.0 Hz, 6H), 2.62 (dd, J = 12.0, 15.9 Hz, 1H), 3.02 (dd, J = 6.2, 15.9 Hz, 1H), 4.03-4.11 (m, 1H), 4.68 (sept, J = 6.0 Hz, 1H), 5.44 (d, J = 4.6 Hz, 1H), 6.60 (d, J = 14.8 Hz, 1H), 6.68-5.72 (m, 1H), 7.01-7.13 (m, 7H), 7.21 (dd, J = 11.6, 14.8 Hz, 1H), 7.54 (d, J = 8.4 Hz, 2H), 7.73 (d, J = 8.4 Hz, 2H), 9.71 (br s, 1H), 10.17 (br s, 1H) ; ESIMS m/z: $[M+H]^+$ 537.

(2E,4Z)-N-(3-Hydroxy-2-oxo-1,2,3,4-tetrahydroquinolin-5-yl)-5-[4-(tetrahydropyran-4-yloxy)phenyl]-5-[4-(trifluoromethyl)phenyl]-2,4-pentadienamide (Compound 231)

$^1$H NMR (DMSO-$d_6$, δ ppm): 1.56-1.69 (m, 2H), 1.99-2.05 (m, 2H), 2.62 (dd, J = 11.9, 15.9 Hz, 1H), 3.02 (dd, J = 6.3, 15.9 Hz, 1H), 3.47-3.54 (m, 2H), 3.87 (td, J = 4.6, 11.6 Hz, 2H), 4.02-4.11 (m, 1H), 4.61-4.70 (m, 1H), 5.44 (d, J = 4.3 Hz, 1H), 6.60 (d, J = 14.8 Hz, 1H), 6.70 (dd, J = 3.1, 6.1 Hz, 1H), 7.05-7.15 (m, 7H), 7.21 (dd, J = 11.6, 14.8 Hz, 1H), 7.54 (d, J = 8.1 Hz, 2H), 7.73 (d, J = 8.1 Hz, 2H), 9.72 (br s, 1H), 10.17 (br s, 1H); ESIMS m/z: $[M+H]^+$ 579.

(2E,4Z)-N-(3-Hydroxy-2-oxo-1,2,3,4-tetrahydroquinolin-5-yl)-5-(3-methoxyphenyl)-5-[4-(trifluoromethyl)phenyl]-2,4-pentadienamide (Compound 232)

$^1$H NMR (DMSO-$d_6$, δ ppm) : 2.61 (dd, J = 11.7, 15.9 Hz, 1H), 3.01 (dd, J = 6.2, 15.9 Hz, 1H), 3.77 (s, 3H), 4.02-4.10 (m, 1H), 5.44 (d, J = 4.3 Hz, 1H), 6.60-6.68 (m, 4H), 7.03-7.17 (m, 5H), 7.44 (t, J = 8.0 Hz, 1H), 7.55 (d, J = 8.3 Hz, 2H), 7.73 (d, J = 8.3 Hz, 2H), 9.74 (br s, 1H), 10.16 (br s, 1H); ESIMS m/z: $[M+H]^+$ 509.

(2E,4Z)-N-(3-Hydroxy-2-oxo-1,2,3,4-tetrahydroquinolin-5-yl)-5-(3-propoxyphenyl)-5-[4-(trifluoromethyl)phenyl]-2,4-pentadienamide (Compound 233)

$^1$H NMR (DMSO-$d_6$, δ ppm): 0.96 (t, J = 6.9 Hz, 3H), 1.72 (sext, J = 6.9 Hz, 2H), 2.62 (dd, J = 12.0, 16.0 Hz, 1H), 3.01 (dd, J = 6.2, 16.0 Hz, 1H), 3.93 (t, J = 6.9 Hz, 2H), 4.02-4.11 (m, 1H), 5.44 (d, J = 4.3 Hz, 1H), 6.59-6.79 (m, 4H), 7.02-7.17 (m, 5H), 7.42 (t, J = 8.0 Hz, 1H), 7.55 (d, J = 8.3 Hz, 2H), 7.73 (d, J = 8.3 Hz, 2H), 9.74 (br s, 1H), 10.17 (br s, 1H) ; ESIMS m/z: $[M+H]^+$ 537.

(2E,4Z)-N-(3-Hydroxy-2-oxo-1,2,3,4-tetrahydroquinolin-5-yl)-5-(3-isopropoxyphenyl)-5-[4-(trifluoromethyl)phenyl]-2,4-pentadienamide (Compound 234)

$^1$H NMR (DMSO-$d_6$, δ ppm): 1.26 (d, J = 6.1 Hz, 6H), 2.62 (dd, J = 11.9, 16.0 Hz, 1H) , 3.01 (dd, J = 6.1, 16.0 Hz, 1H), 4.02-4.11 (m, 1H), 4.63 (sept, J = 6.1 Hz, 1H), 5.43 (d, J = 4.6 Hz, 1H), 6.59-6.76 (m, 4H), 7.00-7.03 (m, 1H), 7.09-7.18 (m, 4H), 7.40 (t, J = 7.8 Hz, 1H), 7.55 (d, J = 8.4 Hz, 2H), 7.73 (d, J = 8.4 Hz, 2H), 9.74 (br s, 1H), 10.16 (br s, 1H) ; ESIMS m/z: $[M+H]^+$ 537.

(2E,4Z)-N-(3-Hydroxy-2-oxo-1,2,3,4-tetrahydroquinolin-5-yl)-5-[3-(tetrahydropyran-4-yloxy)phenyl]-5-[4-(trifluoromethyl)phenyl]-2,4-pentadienamide (Compound 235)

$^1$H NMR (DMSO-$d_6$, δ ppm): 1.52-1.64 (m, 2H), 1.95-1.99 (m, 2H), 2.62 (dd, J = 11.7, 16.0 Hz, 1H), 3.01 (dd, J = 6.3, 16.0 Hz, 1H), 3.45 (dt, J = 2.4, 9.5 Hz, 2H), 3.82 (td, J = 4.3, 11.6 Hz, 2H), 4.02-4.10 (m, 1H), 4.58-4.64 (m, 1H), 5.43 (d, J = 4.6 Hz, 1H), 6.59-6.80 (m, 4H), 7.07-7.17 (m, 5H), 7.42 (t, J = 7.8 Hz, 1H), 7.55 (d, J = 8.3 Hz, 2H), 7.74 (d, J = 8.3 Hz, 2H), 9.73 (br s, 1H), 10.17 (br s, 1H) ; ESIMS m/z: $[M+H]^+$ 579 .

(2E,4Z)-5-(3-Ethoxyphenyl)-N-(3-hydroxy-2-oxo-1,2,3,4-tetrahydroquinolin-5-yl)-5-[4-(trifluoromethyl)phenyl]-2,4-pentadienamide (Compound 236)

$^1$H NMR (DMSO-$d_6$, δ ppm): 1.32 (t, J = 6.9 Hz, 3H), 2.62 (dd, J = 12.0, 15.9 Hz, 1H) , 3.02 (dd, J = 6.2, 15.9 Hz, 1H), 4.00-4.11 (m, 3H), 5.45 (d, J = 4.6 Hz, 1H), 6.60-6.78 (m, 4H), 7.01-7.17 (m, 5H), 7.42 (t, J = 7.8 Hz, 1H), 7.55 (d, J = 8.3 Hz, 2H), 7.73 (d, J = 8.3 Hz, 2H), 9.75 (br s, 1H), 10.17 (br s, 1H) ; ESIMS m/z: $[M+H]^+$ 523. (2E,4Z)-5-(Benzo[1,3]dioxol-5-yl)-N-(3-hydroxy-2-oxo-1,2,3,4-tetrahydroquinolin-5-yl)-5-[4-(trifluoromethyl)phenyl]-2,4-pentadienamide (Compound 238)

$^1$H NMR (DMSO-$d_6$, δ ppm): 2.63 (dd, J = 11.7, 15.9 Hz, 1H), 3.02 (dd, J = 6.3, 15.9 Hz, 1H), 4.03-4.11 (m, 1H), 5.45 (d, J = 4.5 Hz, 1H), 6.11 (s, 2H), 6.61 (d, J = 14.4 Hz, 1H), 6.68-6.77 (m, 3H), 7.04-7.24 (m, 5H), 7.56 (d, J = 8.4 Hz, 2H), 7.73 (d, J = 8.4 Hz, 2H), 9.74 (br s, 1H), 10.18 (br s, 1H); ESIMS m/z: $[M+H]^+$ 523.

(2E,4Z)-5-(2,3-Dihydrpbenzo[1,4]dioxan-6-yl)-N-(3-hydroxy-2-oxo-1,2,3,4-tetrahydroquinolin-5-yl)-5-[4-(trifluoromethyl)phenyl]-2,4-pentadienamide (Compound 239)

$^1$H NMR (DMSO-$d_6$, δ ppm) : 2.62 (dd, J = 11.7, 15.9 Hz, 1H), 3.02 (dd, J = 6.3, 15.9 Hz, 1H), 4.03-4.11 (m, 1H), 4.30-4.31 (m, 4H), 5.44-5.45 (m, 1H), 6.58-6.72 (m, 4H), 6.98 (d, J = 7.8 Hz, 1H), 7.05-7.12 (m, 3H), 7.20 (dd, J = 11.6, 14.5 Hz, 1H), 7.54 (d, J = 8.5 Hz, 2H), 7.73 (d, J = 8.5 Hz, 2H), 9.73 (br s, 1H), 10.17 (br s, 1H); ESIMS m/z: $[M+H]^+$ 537.

(2E,4Z)-5-(4-Ethoxyphenyl)-N-(3-hydroxy-1,2,3,4-tetrahydroquinolin-5-yl)-5 [4-(trifluoromethyl)phenyl]-2,4-pentadienamide monohydrochloride (Compound 240)

$^1$H NMR (DMSO-$d_6$, δ ppm): 1.36 (t, J = 6.9 Hz, 3H), 2.50-2.55 (m, 1H), 2.79-2.85 (m, 1H), 3.02-3.10 (m, 1H), 3.24-3.28 (m, 1H), 4.06-4.14 (m, 1H), 4.10 (q, J = 6.9 Hz, 2H), 6.59-6.72 (m, 2H), 7.02-7.24 (m, 8H), 7.53 (d, J = 8.3 Hz, 2H), 7.73 (d, J = 8.3 Hz, 2H), 9.55 (br s, 1H); ESIMS m/z: $[M+H]^+$ 509.

(2E,4Z)-5-(4-Ethoxyphenyl)-N-(2-oxo-1,2,3,4-tetrahydroquinolin-5-yl)-5-[4-(trifluoromethyl)phenyll-2,4-pentadienamide (Compound 241)

$^1$H NMR (DMSO-$d_6$, δ ppm): 1.36 (t, J = 6.9 Hz, 3H), 2.36-2.41 (m, 2H), 2.71-2.76 (m, 2H), 4.09 (q, J = 6.9 Hz, 2H), 6.57 (d, J = 14.3 Hz, 1H), 6.70 (dd, J = 2.0, 6.9 Hz, 1H), 7.02-7.24 (m, 8H), 7.53 (d, J = 8.3 Hz, 2H), 7.73 (d, J = 8.3 Hz, 2H), 9.68 (br s, 1H), 10.09 (br s, 1H); ESIMS m/z: $[M+H]^+$ 507.

(2E,4Z)-5-(4-Ethoxyphenyl)-N-(2-oxo-2,3-dihydro-1H-indol-4-yl)-5-[4-(trifluoromethyl)phenyl]-2,4-pentadienamide (Compound 242)

$^1$H NMR (DMSO-$d_6$, δ ppm): 1.37 (t, J = 7.0 Hz, 3H), 3.42 (s, 2H), 4.10 (q, J = 6.9 Hz, 2H), 6.57 (d, J = 5.7 Hz, 1H), 6.61-6.62 (m, 1H), 7.03-7.34 (m, 8H), 7.53 (d, J = 8.4 Hz, 2H), 7.73 (d, J = 8.4 Hz, 2H), 9.72 (br s, 1H), 10.38 (br s, 1H); ESIMS m/z: $[M+H]^+$ 493.

(2E,4Z)-5-[4-(N,N-Dimethylamino)phenyl]-N-(2-oxo-2,3-dihydro-1H-indol-4-yl)-5-[4-(trifluoromethyl)phenyl]-2,4-pentadienamide (Compound 243)

$^1$H NMR (DMSO-$d_6$, δ ppm): 2.98 (s, 6H), 3.43 (s, 2H), 6.52-6.61 (m, 2H), 6.81 (d, J = 8.5 Hz, 2H), 6.92 (d, J = 11.6 Hz, 1H), 7.02 (d, J = 8.5 Hz, 2H), 7.12 (t, J = 8.0 Hz, 1H), 7.29-7.38 (m, 2H), 7.54 (d, J = 8.3 Hz, 2H), 7.73 (d, J = 8.3 Hz, 2H), 9.67 (br s, 1H), 10.38 (br s, 1H) ; ESIMS m/z: $[M+H]^+$ 492.

(2E,4Z)-N-(3-Hydroxy-2-oxo-1,2,3,4-tetrahydroquinolin-5-yl)-5-(6-methylpyridin-3-yl)-5-[4-(trifluoromethyl)phenyl]-2,4-pentadienamide (Compound 244)

$^1$H NMR (DMSO-$d_6$, δ ppm): 2.56 (s, 3H), 2.62 (dd, J = 11.9, 15.8 Hz, 1H), 3.01 (dd, J = 6.3, 15.8 Hz, 1H), 4.03-4.11 (m, 1H), 5.44 (d, J = 4.6 Hz, 1H), 6.64-6.72 (m, 2H), 7.04-7.13 (m, 3H), 7.27 (d, J = 11.6 Hz, 1H), 7.41 (d, J = 7.8 Hz, 1H), 7.53-7.57 (m, 3H), 7.75 (d, J = 8.4 Hz, 2H), 8.32 (d, J = 1.9 Hz, 1H), 9.78 (br s, 1H), 10.17 (br s, 1H); ESIMS m/z: $[M+H]^+$ 494.

(2H,4Z)-N-(3-Hydroxy-2-oxo-1,2,3,4-tetrahydroquinolin-5-yl)-5-(2-methylpyridin-4-yl)-5-[4-(trifluoromethyl)phenyl]-2,4-pentadienamide (Compound 245)

$^1$H NMR (DMSO-$d_6$, δ ppm) : 2.53 (s, 3H), 2.56-2.67 (m, 1H), 3.01 (dd, J = 6.2, 15.9 Hz, 1H), 4.02-4.11 (m, 1H), 5.44 (d, J = 4.6 Hz, 1H), 6.63-6.72 (m, 2H), 6.97-7.14 (m, 5H), 7.28 (d, J = 11.6 Hz, 1H), 7.55 (d, J = 8.3 Hz, 2H), 7.75 (d, J = 8.3 Hz, 2H), 8.59 (d, J = 4.9 Hz, 1H), 9.78 (br s, 1H), 10.17 (br s, 1H); ESIMS m/z: $[M+H]^+$ 494.

(2E,4Z)-N-(3-Hydroxy-2-oxo-1,2,3,4-tetrahydroquinclin-5-yl)-5-[3-(trifluoromethoxy)phenyl]-5-[4-(trifluoromethyl)phenyl]-2,4-pentadienamide (Compound 246)

$^1$H NMR (DMSO-$d_6$, δ ppm) : 2.62 (dd, J = 11.9, 15.9 Hz, 1H), 3.02 (dd, J = 6.2, 15.9 Hz, 1H), 4.02-4.11 (m, 1H), 5.45 (d, J = 4.6 Hz, 1H), 6.64-6.72 (m, 2H), 7.03-7.15 (m, 3H), 7.24-7.31 (m, 3H), 7.48-7.56 (m, 3H), 7.67 (t, J = 8.0 Hz, 1H), 7.75 (d, J = 8.4 Hz, 2H), 9.78 (br s, 1H), 10.18 (br s, 1H) ; ESIMS m/z: $[M+H]^+$ 563.

(2E,4Z)-N-(3-Hydroxy-2-oxo-1,2,3,4-tetrahydroquinolin-5-yl)-5-(1-methyl-1H-indol-5-yl)-5-[4-(trifluoromethyl)phenyl]-2,4-pentadienamide (Compound 252)

$^1$H NMR (DMSO-$d_6$, δ ppm): 2.61 (dd, J = 11.9, 15.9 Hz, 1H), 3.01 (dd, J = 6.2, 15.9 Hz, 1H), 3.83 (s, 3H), 3.99-4.10 (m, 1H) 5.45 (br s, 1H), 6.48 (d, J = 3.2 Hz, 1H), 6.60 (d, J = 14.6 Hz, 1H), 6.68 (dd, J = 3.2, 5.8 Hz, 1H), 6.95 (dd, J = 1.3, 8.4 Hz, 1H), 7.08-7.27 (m, 4H), 7.41-7.42 (m, 2H), 7.53-7.56 (m, 3H), 7.71 (d, J = 8.4 Hz, 2H), 9.70 (br s, 1H), 10.16 (br s, 1H); ESIMS m/z: $[M+H]^+$ 532.

(2E,4Z)-5-[2-(N,N-Dimethylamino)pyrimidin-5-yl]-N-(3-hydroxy-2-oxo-1,2,3,4-tetrahydroquinolin-5-yl)-5-[4-(trifluoromethyl)phenyl]-2,4-pentadienamide (Compound 255)

$^1$H NMR (DMSO-$d_6$, δ ppm): 2.63 (dd, J = 12.2, 15.9 Hz, 1H) 3.03 (dd, J = 6.1, 15.9 Hz, 1H), 3.19 (s, 6H), 4.05-4.09 (m, 1H), 5.45 (d, J = 4.6 Hz, 1H), 6.64 (d, J = 14.3 Hz, 1H), 6.69-6.72 (m, 1H), 7.09-7.27 (m, 4H), 7.62 (d, J = 8.0 Hz, 2H), 7.76 (d, J = 8.0 Hz, 2H), 8.20 (s, 2H), 9.77 (br s, 1H), 10.18 (br s, 1H); ESIMS m/z: $[M+H]^+$ 524.

(2E,4Z)-N-(3-Hydroxy-2-oxo-1,2,3,4-tetrahydroquinolin-5-yl)-5-(3-hydroxyphenyl)-5-[4-(trifluoromethyl)phenyl]-2,4-pentadienamide (Compound 256)

$^1$H NMR (DMSO-$d_6$, δ ppm) : 2.63 (dd, J = 12.0, 15.9 Hz, 1H), 3.02 (dd, J = 6.0, 15.9 Hz, 1H), 4.03-4.11 (m, 1H), 5.46 (d, J = 4.5 Hz, 1H), 6.59-6.72 (m, 4H), 6.84-6.88 (m, 1H), 7.10-7.23 (m, 4H), 7.31 (t, J = 7.9 Hz, 1H), 7.55 (d, J = 8.3 Hz, 2H), 7.74 (d, J = 8.3 Hz, 2H), 9.65 (br s, 1H), 9.75 (br s, 1H), 10.18 (br s, 1H); ESIMS m/z: $[M+H]^+$ 495.

(2E,4Z)-N-(3-Hydroxy-2-oxo-1,2,3,4-tetrahydroquinolin-5-yl)-5-(4-ethoxyphenyl)-5-[4-(trifluoromethyl)phenyl]-2,4-pentadienamide (Compound 257)

$^1$H NMR (DMSO-$d_6$, δ ppm): 1.36 (t, J = 6.9 Hz, 3H), 2.62 (dd, J = 12.0, 15.9 Hz, 1H), 3.02 (dd, J = 6.3, 15.9 Hz, 1H), 4.03-4.11 (m, 1H), 4.10 (q, J = 6.9 Hz, 2H) 5.45 (d, J = 3.9 Hz, 1H), 6.61 (d, J = 14.4 Hz, 1H), 6.70 (dd, J = 3.1, 5.9 Hz, 1H), 7.03-7.24 (m, 8H), 7.54 (d, J = 8.4 Hz, 2H), 7.73 (d, J = 8.4 Hz, 2H), 9.73 (br s, 1H), 10.18 (br s, 1H) ; ESIMS m/z: $[M+H]^+$ 523.

(2E,4Z)-N-(3-Hydroxy-2-oxo-1,2,3,4-tetrahydroquinolin-5-yl)-5-[4-(trifluoromethoxy)phenyl]-5-[4-(trifluoromethyl)phenyl]-2,4-pentadienamide (Compound 258)

$^1$H NMR (DMSO-$d_6$, δ ppm) : 2.63 (dd, J = 12.0, 15.8 Hz, 1H), 3.02 (dd, J = 6.1, 15.8 Hz, 1H), 4.03-4.11 (m, 1H), 5.45-5.47 (m, 1H), 6.64-6.73 (m, 2H), 7.04-7.15 (m, 3H), 7.23 (d, J = 11.3 Hz, 1H), 7.38 (d, J = 8.6 Hz, 2H), 7.53 (d, J = 14.6 Hz, 4H), 7.75 (d, J = 8.4 Hz, 2H), 9.78 (br s, 1H), 10.18 (br s, 1H); :ESIMS m/z: $[M+H]^+$ 563.

(2E,4Z)-5-[4-(2,5-Dimethylpyrrol-1-yl)phenyl]-N-(3-hydroxy-2-oxo-1,2,3,4-tetrahydroquinolin-5-yl)-5-[4-(trifluoromethyl) phenyl]-2,4-pentadienamide (Compound 259)

$^1$H NMR (DMSO-$d_6$, δ ppm) : 2.04 (s, 6H), 2.63 (dd, J = 11.7, 15.8 Hz, 1H), 3.02 (dd, J = 6.3, 15.8 Hz, 1H), 4.03-4.10 (m, 1H), 5.46 (d, J = 4.8 Hz, 1H), 5.83 (s, 2H), 6.65-6.72 (m, 2H), 7.10-7.25 (m, 4H), 7.34-7.42 (m, 4H), 7.58 (d, J = 8.3 Hz, 2H), 7.78 (d, J = 8.3 Hz, 2H), 9.78 (br s, 1H), 10.18 (br s, 1H); ESIMS m/z: $[M+H]^+$ 572.

(2E,4Z)-N-(3-Hydroxy-2-oxo-1,2,3,4-tetrahydroquinolin-5-yl)-5-[4-(pyrrol-1-yl)phenyl]-5-[4-(trifluoromethyl)phenyl]-2,4-pentadienamide (Compound 260)

$^1$H NMR (DMSO-$d_6$, δ ppm) : 2.63 (dd, J = 12.0, 16.0 Hz, 1H), 3.02 (dd, J = 6.1, 16.0 Hz, 1H), 4.03-4.11 (m, 1H), 5.47 (d, J = 4.5 Hz, 1H), 6.30-6.32 (m, 2H), 6.64-6.72 (m, 2H), 7.10-7.26 (m, 4H), 7.30 (d, J = 8.4 Hz, 2H), 7.47-7.48 (m, 2H), 7.58 (d, J = 7.8 Hz, 2H), 7.71-7.77 (m, 4H), 9.77 (br s, 1H), 10.18 (br s, 1H); ESIMS m/z: $[M+H]^+$ 544.

(2E,4Z)-N-(Isoquinolin-5-yl)-5-[4-(pyrrolidin-1-yl)phenyl]-5-[4-(trifluoromethyl)phenyl]-2,4-pentadienamide (Compound 266)

$^1$H NMR (DMSO-$d_6$, δ ppm) : 1.00-1.04 (m, 4H), 1.96-1.99 (m, 4H), 6.52-6.78 (m, 3H), 6.93-7.17 (m, 3H), 7.38-7.58 (m, 3H), 7.62-8.03 (m, 5H), 8.17 (d, J = 7.5 Hz, 1H), 8.56 (d, J = 5.7 Hz, 1H), 9.34 (s, 1H), 10.24 (br s, 1H); ESIMS m/z: $[M+H]^+$ 514.

(2E,4Z)-5-(3,4-Difluorophenyl)-N-(isoquinolin-5-yl)-5-[4-(trifluoromethyl)phenyl]-2,4-pentadienamide (Compound 271)

$^1$H NMR (DMSO-$d_6$, δ ppm): 6.84 (d, J = 14.7 Hz, 1H), 7.08-7.20 (m, 2H), 7.29 (d, J = 11.7 Hz, 1H), 7.37-7.44 (m, 1H), 7.57-7.70 (m, 4H), 7.76 (d, J = 8.4 Hz, 2H), 7.94-7.99 (m, 2H), 8.12 (d, J = 7.5 Hz, 1H), 8.56 (d, J = 6.0 Hz, 1H), 9.33 (s, 1H), 10.33 (br s, 1H) ; ESIMS m/z: $[M+H]^+$ 481.

(2E,4E)-5-[4-(Hydroxymethyl)phenyl]-N-(isoquinolin-5-yl)-5-[4-(trifluoromethyl)phenyl]-2,4-pentadienamide (Compound 272)

$^1$H NMR (DMSO-$d_6$, δ ppm): 4.59 (d, J = 5.7 Hz, 2H), 5.31 (t, J = 5.7 Hz, 1H), 6.81 (d, J = 13.8 Hz, 1H), 7.19-7.25 (m, 4H), 7.47 (d, J = 8.1 Hz, 2H), 7.56 (d, J = 8.1 Hz, 2H), 7.66 (t, J = 7.8 Hz, 1H), 7.75 (d, J = 8.1 Hz, 2H), 7.94 (d, J = 8.1 Hz, 1H), 7.98 (d, J = 6.3 Hz, 1H), 8.12 (d, J = 7.5 Hz, 1H), 8.55 (d, J = 6.0 Hz, 1H), 9.32 (s, 1H), 10.29 (br s, 1H); ESIMS m/z: $[M+H]^+$ 475.

(2E,4Z)-N-(Isoquinolin-5-yl)-5-(4-methyl-3,4-dihydro-2H-benzo[1,4]oxazin-7-yl)-5-[4-(trifluoromethyl)phenyl]-2,4-pentadienamide (Compound 273)

$^1$H-NMR (DMSO-$d_6$, δ ppm) : 2.91 (s, 3H), 3.33-3.35 (m, 2H), 4.25-4.28 (m, 2H), 6.50 (s, 1H), 6.66 (dd, J = 1.8, 8.1 Hz, 1H), 6.73-6.81 (m, 2H), 6.98 (d, J = 11.7 Hz, 1H), 7.40 (dd, J = 11.7, 14.9 Hz, 1H), 7.56 (d, J = 8.1 Hz, 2H), 7.67 (t, J = 7.8 Hz, 1H), 7.74 (d, J = 8.4 Hz, 2H), 7.94 (d, J = 8.1 Hz, 1H), 8.00 (d, J = 6.0 Hz, 1H), 8.16 (d, J = 7.2 Hz, 1H), 8.56 (d, J = 6.0 Hz, 1H), 9.33 (s, 1H), 10.25 (br s, 1H); ESIMS m/z: $[M+H]^+$ 516.

(2E,4Z)-5-[5-(N,N-Dimethylaminomethyl)furan-2-yl]-N-(isoquinolin-5-yl)-5-[4-(trifluoromethyl)phenyl]-2,4-pentadienamide (Compound 275)

$^1$H NMR (DMSO-$d_6$, δ ppm): 2.20 (s, 6H), 3.52 (s, 2H), 6.49-6.49 (m, 2H), 6.73 (d, J = 12.2 Hz, 1H), 6.80 (d, J = 15.1 Hz, 1H), 7.63-7.73 (m, 3H), 7.80 (d, J = 8.1 Hz, 2H), 7.95-8.08 (m, 3H), 8.18-8.21 (m, 1H), 8.56 (d, J = 4.9 Hz, 1H), 9.34 (s, 1H), 10.30 (br s, 1H); ESIMS m/z: $[M+H]^+$ 492.

(2E,4Z)-5-[6-(N,N-Dimethylamino)pyridin-3-yl-N-(isoquinolin-5-yl)-5-[4-(trifluoromethyl)phenyl]-2,4-pentadienamide (Compound 276)

$^1$H NMR (DMSO-$d_6$, δ ppm): 3.10 (s, 6H), 6.73-6.82 (m, 2H), 7.07 (d, J = 11.7 Hz, 1H), 7.30-7.42 (m, 2H), 7.59 (d, J = 8.3 Hz, 2H), 7.68 (t, J = 7.8 Hz, 1H), 7.76 (d, J = 8.3 Hz, 2H), 7.93-8.01 (m, 3H), 8.16 (d, J = 7.5 Hz, 1H) 8.56 (d, J = 6.0 Hz, 1H), 9.33 (s, 1H), 10.28 (br s, 1H); ESIMS m/z: $[M+H]^+$ 489.

(2E,4Z)-N-(Isoquinolin-5-yl)-5-[5-(morpholin-4-ylmethyl)furan-2-yl]-5-[4-(trifluoromethyl)phenyl]-2,4-pentadienamide (Compound 277)

$^1$H NMR (DMSO-$d_6$, δ ppm): 2.43-2.46 (m, 4H), 3.55-3.58 (m, 4H), 3.59 (s, 2H), 6.46 (d, J = 3.8 Hz, 1H), 6.49 (d, J = 3.8 Hz, 1H), 6.70 (d, J = 13.2 Hz, 1H), 6.78 (d, J = 16.5 Hz, 1H), 7.63-7.71 (m, 3H), 7.77-7.80 (m, 2H), 7.93-8.08 (m, 3H), 8.18 (d, J = 8.4 Hz, 1H), 8.54 (d, J = 6.6 Hz, 1H), 9.32 (s, 1H), 10.18 (br s, 1H); ESIMS m/z: $[M+H]^+$ 534.

(2E,4Z)-N-(Isoquinolin-5-yl)-5-(5-methylfuran-2-yl)-5-[4-(trifluoromethyl)phenyl]-2,4-pentadienamide (Compound 279)

$^1$H NMR (DMSO-$d_6$, δ ppm): 2.36 (s, 3H), 6.31 (d, J = 3.3 Hz, 1H), 6.41 (d, J = 3.3 Hz, 1H) 6.64 (d, J = 11.7 Hz, 1H), 6.77 (d, J = 15.0 Hz, 1H), 7.64 (d, J = 8.1 Hz, 2H), 7.69 (t, J = 7.9 Hz, 1H), 7.80 (d, J = 8.1 Hz, 2H), 7.96 (d, J = 8.1 Hz, 1H), 8.01-8.10 (m, 2H), 8.20 (d, J = 7.5 Hz, 1H), 8.56 (d, J = 5.7 Hz, 1H), 9.33 (s, 1H), 10.29 (br s, 1H); ESIMS m/z: $[M+H]^+$ 449.

(2E,4Z)-5-(4-Hydroxyphenyl)-N-(isoquinolin-5-yl)-5-[4-(trifluoromethyl)phenyl]-2,4-pentadienamide (Compound 280)

$^1$H NMR (DMSO-$d_6$, δ ppm): 6.77 (d, J = 14.9 Hz, 1H), 6.88 (d, J = 8.7 Hz, 2H), 7.03-7.08 (m, 3H), 7.32 (dd, J = 11.7, 14.9 Hz, 1H), 7.55 (d, J = 7.8 Hz, 2H), 7.67 (t, J = 7.8 Hz, 1H), 7.74 (d, J = 8.1 Hz, 2H), 7.94 (d, J = 8.1 Hz, 1H), 7.99 (d, J = 5.7 Hz, 1H), 8.14 (d, J = 7.8 Hz, 1H), 8.55 (d, J = 6.0 Hz, 1H), 9.32 (s, 1H), 9.78 (br s, 1H), 10.24 (br s, 1H); ESIMS m/z: $[M+H]^+$ 461.

(2E,4Z)-5-(4-Fluorophenyl)-N-(3-hydroxy-2-oxo-1,2,3,4-tetrahydroquinolin-5-yl)-5-[4-(trifluoromethyl)phenyl]-2,4-pentadienamide (Compound 281)

$^1$H NMR (DMSO-$d_6$, δ ppm): 2.62 (dd, J = 11.8, 15.9 Hz, 1H), 3.01 (dd, J = 6.2, 15.9 Hz, 1H), 4.02-4.10 (m, 1H), 5.45 (d, J = 4.6 Hz, 1H), 6.61-6.71 (m, 2H), 7.05-7.38 (m, 8H), 7.54 (d, J = 8.3 Hz, 2H), 7.74 (d, J = 8.3 Hz, 2H), 9.76 (br s, 1H), 10.17 (br s, 1H); ESIMS m/z: $[M+H]^+$ 497.

(2E,4Z)-5-(4-Fluorophenyl)-N-(3-hydroxy-1,2,3,4-tetrahydroquinolin-5-yl)-5-[4-(trifluoromethyl)phenyl]-2,4-pentadienamide (Compound 282)

$^1$H NMR (DMSO-$d_6$, δ ppm) : 2.36 (dd, J = 8.5, 16.2 Hz, 1H), 2.72 (dd, J = 4.8, 16.2 Hz, 1H), 2.80-2.87 (m, 1H), 3.17-3.20 (m, 1H), 3.82-3.86 (m, 1H), 4.88 (d, J = 4.4 Hz, 1H), 5.69 (br s, 1H), 6.31 (d, J = 7.9 Hz, 1H), 6.56-6.64 (m, 2H), 6.81 (t, J = 7.9 Hz, 1H), 7.02-7.19 (m, 2H), 7.25-7.38 (m, 4H), 7.53 (d, J = 8.1 Hz, 2H), 7.73 (d, J = 8.1 Hz, 2H), 9.34 (br s; 1H) ; ESIMS m/z: $[M+H]^+$ 483 .

(2E,4E)-5-(4-Fluorophenyl)-N-(isoquinolin-5-yl)-5-[4-(methanesulfonyl)phenyl]-2,4-pentadienamide (Compound 283)

$^1$H NMR (DMSO-$d_6$, δ ppm): 3.24 (s, 3H), 6.83 (d, J = 14.1 Hz, 1H), 7.14-7.40 (m, 6H), 7.60 (d, J = 8.4 Hz, 2H), 7.67 (t, J = 7.9 Hz, 1H), 7.92-7.99 (m, 4H), 8.12 (d, J = 7.5 Hz, 1H), 8.55 (d, J = 5.8 Hz, 1H), 9.32 (s, 1H), 10.30 (br s, 1H); ESIMS m/z: $[M+H]^+$ 473.

(2E,4Z)-5-(4-Fluorophenyl)-N-(2-oxo-1,2-dihydroquinolin-5-yl)-5-[4-(trifluoromethyl)phenyl]-2,4-pentadienamide (Compound 284)

$^1$H NMR (DMSO-$d_6$, δ ppm): 6.51 (d, J = 9.9 Hz, 1H), 6.70 (d, J = 13.5 Hz, 1H), 7.09-7.48 (m, 9H), 7.55 (d, J = 8.4 Hz, 2H), 7.75 (d, J = 8.4 Hz, 2H), 7.97 (d, J = 9.7 Hz, 1H), 10.16 (br s, 1H), 11.78 (br s, 1H); ESIMS m/z: $[M+H]^+$ 479.

(2E,4Z)-5-(4-Fluorophenyl)-N-(7-hydroxy-5,6,7,8-tetrahydronaphthalen-1-yl)-5-[4-(trifluoromethyl)phenyl]-2,4-pentadienamide (Compound 285)

$^1$H NMR (DMSO-$d_6$, δ ppm): 1.52-1.65 (m, 1H), 1.85-1.91 (m, 1H), 2.42 (dd, J = 7.8, 17.1 Hz, 1H), 2.67-2.91 (m, 3H), 3.87-3.90 (m, 1H), 6.66 (d, J = 14.5 Hz, 1H), 6.93 (d, J = 7.3 Hz, 1H), 7.04-7.40 (m, 8H), 7.55 (d, J = 8.2 Hz, 2H), 7.75 (d, J = 8.2 Hz, 2H), 9.44 (br s, 1H); ESIMS m/z: $[M+H]^+$ 482.

(2E,4Z)-N-(Isoquinolin-5-yl)-5-(pyrimidin-5-yl)-5-[4-(trifluoromethyl)phenyl]-2,4-pentadienamide (Compound 286)

$^1$H NMR (DMSO-$d_6$, δ ppm): 6.89 (d, J = 14.8 Hz, 1H), 7.10 (dd, J = 11.6, 14.8 Hz, 1H), 7.47 (d, J = 11.6 Hz, 1H), 7.62-7.70 (m, 3H), 7.79 (d, J = 8.4 Hz, 2H), 7.96 (d, J = 2.7 Hz, 1H), 7.98 (s, 1H), 8.11 (d, J = 7.5 Hz, 1H), 8.56 (d, J = 6.1 Hz, 1H), 8.78 (s, 2H), 9.32-9.33 (m, 2H), 10.37 (br s, 1H) ; ESIMS m/z: $[M+H]^+$ 447.

(2E,4E)-5-(4-Acetylphenyl)-N-(isoquinolin-5-yl)-5-[4-(trifluoromethyl)phenyl]-2,4-pentadienamide (Compound 287)

$^1$H NMR (DMSO-$d_6$, δ ppm): 2.65 (s, 3H), 6.84 (d, J = 14.6 Hz, 1H), 7.16 (dd, J = 11.6, 14.6 Hz, 1H), 7.31 (d, J = 11.6 Hz, 1H), 7.41 (d, J = 8.2 Hz, 2H), 7.56 (d, J = 8.4 Hz, 2H), 7.66 (t, J = 7.8 Hz, 1H) , 7.76 (d, J = 8.4 Hz, 2H), 7.93-7.98 (m, 2H), 8.09-8.12 (m, 3H), 8.55 (d, J = 6.1 Hz, 1H), 9.32 (s, 1H), 10.32 (br s, 1H); ESIMS m/z $[M+H]^+$ 487.

(2E,4Z)-5-(1-Acetyl-1,2,3,6-tetrahydropyridin-4-yl)-N-(isoquinolin-5-yl)-5-[4-(trifluoromethyl)phenyl]-2,4-pentadienamide (Compound 289)

$^1$H NMR ($CD_3OD$, δ ppm): 2.15-2.22 (m, 5H), 3.72-3.78 (m, 2H), 4.29-4.32 (m, 2H), 5.16-5.17 (m, 1H), 5.87-5.88 (m, 1H), 6.64 (d, J = 14.5 Hz, 1H), 6.86-6.90 (m, 1H), 7.65-7.80 (m, 6H), 7.93-8.10 (m, 3H), 8.48-8.49 (m, 1H), 9.27 (s, 1H); ESIMS m/z: $[M+H]^+$ 492.

(2E,4Z)-5-[4-(N,N-Dimethylamino)phenyl]-N-(isoquinolin-5-yl)-5-[4-(trifluoromethyl)phenyl]-2,4-pentadienamide (Compound 293)

$^1$H NMR (DMSO-$d_6$, δ ppm): 2.98 (s, 6H), 6.75 (d, J = 15.1 Hz, 1H), 6.81 (d, J = 8.8 Hz, 2H), 6.97 (d, J = 11.6 Hz, 1H), 7.04 (d, J = 8.6 Hz, 2H), 7.41 (dd, J = 11.6, 15.1 Hz, 1H), 7.56 (d, J = 8.2 Hz, 2H), 7.67 (t, J = 7.9 Hz, 1H), 7.74 (d, J = 8.2 Hz, 2H), 7.94 (d, J = 8.1 Hz, 1H), 8.00 (d, J = 6.0 Hz, 1H), 8.15 (d, J = 7.5 Hz, 1H), 8.55 (d, J = 5.9 Hz, 1H) , 9.32 (s, 1H), 10.23 (br s, 1H); ESIMS m/z: $[M+H]^+$ 488.

(2E,4Z)-N-(Isoquinolin-5-yl)-5-[4-(morpholin-4-yl)phenyl]-5-[4-(trifluoromethyl)phenyl]-2,4-pentadienamide (Compound 295)

$^1$H NMR (DMSO-$d_6$, δ ppm): 3.18-3.23 (m, 4H), 3.74-3.77 (m, 4H), 6.77 (d, J = 14.9 Hz, 1H), 7.03-7.11 (m, 5H), 7.36 (dd, J = 11.6, 14.9 Hz, 1H), 7.56 (d, J = 8.4 Hz, 2H), 7.67 (t, J = 7.9 Hz, 1H), 7.75 (d, J = 8.4 Hz, 2H), 7.94 (d, J = 8.2 Hz, 1H), 7.99 (d, J = 5.8 Hz, 1H), 8.14 (d, J = 8.1 Hz, 1H), 8.55 (d, J = 6.0 Hz, 1H), 9.32 (s, 1H), 10.25 (br s, 1H); ESIMS m/z: $[M+H]^4$ 530.

(2E,4Z)-N-(Isoquinolin-5-yl)-5-[4-(methansulfonyl)phenyl]-5-[4-(trifluoromethyl)phenyl]-2,4-pentadienamide (Compound 297)

$^1$H NMR (DMSO-$d_6$, δ ppm): 3.26 (s, 3H), 6.87 (d, J = 14.7 Hz, 1H), 7.14 (dd, J = 11.7, 14.7 Hz, 1H), 7.34 (d,

J = 11.7 Hz, 1H), 7.54-7.58 (m, 4H), 7.66 (t, J = 7.9 Hz, 1H), 7.77 (d, J = 8.2 Hz, 2H), 7.94-7.98 (m, 2H), 8.07-8.12 (m, 3H), 8.55 (d, J = 6.0 Hz, 1H), 9.32 (s, 1H), 10.35 (br s, 1H) ; ESIMS m/z: $[M+H]^+$ 523 .

(2E,4E)-N-(Isoquinolin-5-yl)-5-phenyl-5-[4-(trifluoromethansulfonyl)phenyl]-2,4-pentadienamide (Compound 298)

$^1$H NMR (DMSO-$d_6$, δ ppm): 6.83 (d, J = 14.6 Hz, 1H), 7.07 (dd, J = 11.5, 14.6 Hz, 1H), 7.27 (d, J = 11.5 Hz, 1H), 7.34-7.43 (m, 5H), 7.66 (t, J = 7.9 Hz, 1H), 7.72 (d, J = 8.4 Hz, 2H), 7.93-7.98 (m, 2H), 8.11 (d, J = 6.4 Hz, 1H), 8.28 (d, J = 8.2 Hz, 2H), 8.55 (d, J = 6.1 Hz, 1H), 9.32 (s, 1H) , 10.32 (br s, 1H); ESIMS m/z: $[M+H]^+$ 509.

(2E,4Z)-N-(3-Hydroxy-2-oxo-1,2,3,4-tetrahydroquinolin-5 yl)-5-[2-(piperidin-1-yl)thiazol-4-yl]-5-[4-(trifluoromethyl)phenyl]-2,4-pentadienamide (Compound 299)

$^1$H NMR (DMSO-$d_6$, δ ppm): 1.62-1.63 (m, 6H), 2.60 (dd, J = 11.7, 16.0 Hz, 1H), 3.00 (dd, J = 6.4, 16.0 Hz, 1H), 3.48-3.49 (m, 4H), 4.05 (dd, J = 6.4, 1.7 Hz, 1H) , 6.22 (s, 1H), 6.57 (d, J = 14.6 Hz, 1H), 6.68 (t, J = 4.7 Hz, 1H), 6.90 (dd, J = 12.2, 14.6 Hz, 1H), 7.10 (d, J = 4.7 Hz, 2H), 7.36 (d, J = 12.2 Hz, 1H), 7.52 (d, J = 8.0 Hz, 2H), 7.86 (d, J = 8.0 Hz, 2H), 9.63 (br s, 1H), 10.17 (br s, 1H) ; ESIMS m/z: $[M+H]^+$ 569.

(2E,4E)-5-(4-Fluorophenyl)-N-(3-hydroxy-2-oxo-1,2,3,4-tetrahydroquinolin-5-yl)-5-[2-(piperidin-1-yl)-6-(trifluoromethyl) pyridin-3-yl]-2,4-pentadienamide (Compound 300)

$^1$H NMR (DMSO-$d_6$, δ ppm): 1.21-1.38 (m, 6H), 2.57-2.73 (m, 1H), 2.99-3.05 (m, 1H), 3.17-3.22 (m, 4H), 4.02-4.08 (m, 1H), 5.45-5.46 (m, 1H), 6.59-6.64 (m, 1H), 6.69-6.71 (m, 1H) 7.10-7.47 (m, 9H), 7.58 (d, J = 7.1 Hz, 1H), 9.76 (br s, 1H), 10.18 (br s, 1H) ; ESIMS m/z: $[M+H]^+$ 581.

(2E,4Z)-5-[4-(2-Dimethylaminoethoxy)phenyl]-N-(3-hydroxy-2-oxo-1,2,3,4-tetrahydroquinolin-5-yl)-5-[4-(trifluoromethyl)phenyl]-2,4-pentadienamide (Compound 302)

$^1$H NMR (DMSO-$d_6$, δ ppm): 2.24 (s, 6H), 2.56-2.68 (m, 3H), 3.00 (dd, J = 6.3, 16.0 Hz, 1H), 4.04 (dd, J = 6.3, 11.4 Hz, 1H), 4.11 (t, J = 5.7 Hz, 2H), 5.45 (d, J = 4.1 Hz, 1H) , 6.59 (d, J = 15.0 Hz, 1H), 6.67-6.70 (m, 1H), 7.04-7.23 (m, 8H), 7.52 (d, J = 8.3 Hz, 2H), 7.72 (d, J = 8.3 Hz, 2H), 9.72 (br s, 1H), 10.16 (br s, 1H); ESIMS m/z: $[M+H]^+$ 566.

(2E,4Z)-N-(3-Hydroxy-2-oxo-1,2,3,4-tetrahydroquinolin-5-yl)-5-[4-(2-methoxyethoxy)phenyl]-5-[4-(trifluoromethyl)phenyl]-2,4-pentadienamide (Compound 304)

$^1$H NMR (DMSO-$d_6$, δ ppm): 2.62 (dd, J = 11.8, 16.1 Hz, 1H), 3.01 (dd, J = 6.4, 16.1 Hz, 1H), 3.30 (s, 3H), 3.67-3.71 (m, 2H), 4.03-4.09 (m, 1H), 4.15-4.18 (m, 2H), 5.45 (br s, 1H), 6.60 (d, J = 14.4 Hz, 1H) , 6.68-6.71 (m, 1H) , 7.05-7.24 (m, 8H), 7.53 (d, J = 8.3 Hz, 2H), 7.73 (d, J = 8.3 Hz, 2H), 9.73 (br s, 1H), 10.17 (br s, 1H); ESIMS m/z: $[M+H]^+$ 553.

(2E,4Z)-N-(3-Hydroxy-2-oxo-1,2,3,4-tetrahydroquinolin-5-yl)-5-(4-propoxyphenyl)-5-[4-(trifluoromethyl)phenyl]-2,4-pentadienamide (Compound 306)

$^1$H NMR (DMSO-$d_6$, δ ppm) : 1.00 (t, J = 6.8 Hz, 3H), 1.76 (sext, J = 6.8 Hz, 2H), 2.62 (dd, J = 11.9, 16.0 Hz, 1H), 3.01 (dd, J = 6.2, 16.0 Hz, 1H), 4.00 (t, J = 6.8 Hz, 2H), 4.02-4.06 (m, 1H), 5.44 (br s, 1H), 6.60 (d, J = 14.3 Hz, 1H), 6.69 (dd, J = 3.3, 5.6 Hz, 1H), 7.03-7.19 (m, 8H), 7.53 (d, J = 8.3 Hz, 2H), 7.73 (d, J = 8.3 Hz, 2H), 9.72 (br s, 1H), 10.17 (br s, 1H); ESIMS m/z: $[M+H]^+$ 537.

(2E,4E)-5-[2-(Dimethylamino)-6-(trifluoromethyl)pyridin-3-yl]-5-(4-fluorophenyl)-N-(3-hydroxy-2-oxo-1,2,3,4-tetrahydroquinolin-5-yl)-2,4-pentadienamide (Compound 307)

$^1$H NMR (DMSO-$d_6$, δ ppm): 2.57-2.66 (m, 1H), 2.97-3.04 (m, 1H), 3.33 (s, 6H), 4.04-4.08 (m, 1H), 5.45 (d, J = 4.2 Hz, 1H), 6.60 (d, J = 12.5 Hz, 1H), 6.68-6.71 (m, 1H), 7.04-7.16 (m, 4H), 7.20-7.29 (m, 4H), 7.35-7.40 (m, 2H), 9.76 (br s, 1H), 10.18 (br s, 1H) ; ESIMS m/z: $[M+H]^+$ 541.

(2E,4E)-5-(4-Chlorophenyl)-N-(3-hydroxy-2-oxo-1,2,3,4-tetrahydroquinolin-5-yl)-5-[4-(trifluoromethoxy)phenyl]-2,4-pentadienamide (Compound 308)

$^1$H NMR (DMSO-$d_6$, δ ppm) : 2.61 (dd, J = 11.9, 15.9 Hz, 1H). 3.00 (dd, J = 6.3, 15.9 Hz, 1H), 3.99-4.07 (m,

1H), 5.45 (d, J = 3.8 Hz, 1H), 6.60 (d, J = 14.0 Hz, 1H), 6.69 (dd, J = 2.3, 6.8 Hz, 1H), 6.99-7.25 (m, 4H), 7.32-7.36 (m, 4H), 7.43-7.58 (m, 4H), 9.74 (br s, 1H), 10.17 (br s, 1H); ESIMS m/z: $[M+H]^+$ 529.

(2E,4Z)-N-(3-Hydroxy-2-oxo-1,2,3,4-tetrahydroquinolin-5-yl)-5-(6-propoxypyridin-3-yl)-5-[4-(trifluoromethyl)phenyl]-2,4-pentadienamide (Compound 309)

$^1$H NMR (DMSO-$d_6$, δ ppm) : 0.93-1.01 (m, 3H), 1.72-1.77 (m, 2H), 2.56-2.66 (m, 1H), 2.99-3.03 (m, 1H), 4.02-4.06 (m, 2H), 4.20-4.30 (m, 1H), 5.44 (br s, 1H), 6.55-6.70 (m, 2H), 6.81-6.95 (m, 1H), 7.01-7.23 (m, 4H), 7.45-7.58 (m, 3H), 7.74 (d, J = 8.8 8 Hz, 1H), 7.87 (d, J = 7. 7 Hz, 1H), 8.05 (d, J = 6.2 Hz, 1H), 9.76 (br s, 1H), 10.17 (br s, 1H); ESIMS m/z: $[M+H]^+$ 538.

(2E,4E)-5-(4-Chlorophenyl)-5-(4-ethoxyphenyl)-N-(3-hydroxy-2-oxo-1,2,3,4-tetrahydroquinolin-5-yl)-2,4-pentadienamide (Compound 310)

$^1$H NMR (DMSO-$d_6$, δ ppm) : 1.36 (t, J = 7.0 Hz, 3H), 2.61. (dd, J = 11.8, 15.9 Hz, 1H), 3.01 (dd, J = 6.4, 15.9 Hz, 1H), 4.06-4.13 (m, 1H), 4.09 (q, J = 6.9 Hz, 2H), 5.44 (br s, 1H), 6.54 (d, J = 14.6 Hz, 1H), 6.68-6.71 (m, 1H), 6.96-7.12 (m, 7H), 7.16 (dd, J = 11.5, 14.6 Hz, 1H), 7.34 (d, J = 8.6 Hz, 2H), 7.43 (d, J = 8.6 Hz, 2H), 9.67 (br s, 1H), 10.16 (br s, 1H); ESIMS m/z: $[M+H]^+$ 489.

(2E,4Z)-5-(4-Ethoxyphenyl)-N-(3-hydroxy-2-oxo-1,2,3,4-tetrahydroquinolin-5-yl)-5-(4-methylphenyl)-2,4-pentadienamide (Compound 311)

$^1$H NMR (DMSO-$d_6$, δ ppm) : 1.36 (t, J = 7.0 Hz, 3H), 2.31 (s, 3H), 2.61 (dd, J = 11.9, 15.9 Hz, 1H), 3.01 (dd, J = 6.3, 15.9 Hz, 1H), 4.02-4.13 (m, 1H), 4.09 (q, J = 7.0 Hz, 2H), 5.42 (d, J = 4.3 Hz, 1H), 6.50 (d, J = 15.0 Hz, 1H), 6.67-6.70 (m, 1H), 6.88-7.23 (m, 12H), 9.62. (br s, 1H), 10.15 (br s, 1H); ESIMS m/z: $[M+H]^+$ 469.

(2E,4Z)-N-(3-Hydroxy-2-oxo-1,2,3,4-tetrahydroquinolin-5-yl)-5-(1-methyl-6-oxo-1,6-dihydropyridin-3-yl)-5-[4-(trifluoromethyl)phenyl]-2,4-pentadienamide (Compound 312)

$^1$H NMR (DMSO-$d_6$, δ ppm) : 2.62 (dd, J = 12.1, 16.0 Hz, 1H), 3.02 (dd, J = 6.2, 16.0 Hz, 1H), 3.47 (s, 3H), 4.02-4.10 (m, 1H), 5.44 (d, J = 3.1 Hz, 1H), 6.48 (d, J = 9.3 Hz, 1H), 6.61 (d, J = 15.0 Hz, 1H), 6.68-6.71 (m, 1H), 7.05-7.12 (m, 3H), 7.19-7.32 (m, 2H), 7.63 (d, J = 8.2 Hz, 2H), 7.74 (s, 1H), 7.75 (d, J = 8.2 Hz, 2H), 9.74 (br s, 1H), 10.16 (br s, 1H) ; ESIMS m/z: $[M+H]^+$ 510.

(2E,4Z)-N-(3-Hydroxy-2-oxo-1,2,3,4-tetrahydroquinolin-5-yl)-5-[4-(thietan-3-yloxy)phenyl]-5-[4-(trifluoromethyl)phenyl]-2,4-pentadienamide (Compound 313)

$^1$H NMR (DMSO-$d_6$, δ ppm) : 2.61 (dd, J = 11.8, 15.9 Hz, 1H), 3.00 (dd, J = 6.4, 15.9 Hz, 1H), 3.46-3.51 (m, 4H), 4.01-4.09 (m, 1H), 5.39-5.49 (m, 2H), 6.59 (d, J = 14.6 Hz, 1H), 6.69 (dd, J = 2.4, 6.2 Hz, 1H), 7.01-7.24 (m, 8H), 7.51 (d, J = 8.2 Hz, 2H), 7.72 (d, J = 8.2 Hz, 2H), 9.71 (br s, 1H), 10.15 (br s, 1H) ; ESIMS m/z: $[M+H]^+$ 567.

(2E,4Z)-N-(3-Hydroxy-2-oxo-1,2,3,4-tetrahydroquinolin-5-yl)-5-[4-(oxetan-3-yloxy)phenyl]-5-[4-(trifluoromethyl)phenyl]-2,4-pentadienamide (Compound 314)

$^1$H NMR (DMSO-$d_6$, δ ppm) : 2.60 (dd, J = 11.9, 15.9 Hz, 1H), 3.00 (dd, J = 6.3, 15.9 Hz, 1H), 4.01-4.09 (m, 1H), 4.60 (dd, J = 5.5, 7.5 Hz, 2H), 4.92-4.96 (m, 2H), 5.35 (quint, J = 5.5 Hz, 1H), 5.43 (d, J = 4.5 Hz, 1H), 6.59 (d, J = 14.1 Hz, 1H), 6.69 (dd, J = 2.6, 6.5 Hz, 1H), 6.91 (d, J = 8.4 Hz, 2H), 7.05-7.20 (m, 6H), 7.51 (d, J = 8.1 Hz, 2H), 7.72 (d, J = 8.1 Hz, 2H), 9.71 (br s, 1H), 10.16 (br s, 1H); ESIMS m/z: $[M+H]^+$ 551.

(2E,4Z)-5-(3-Cyclobutoxyphenyl)-N-(3-hydroxy-2-oxo-1,2,3,4-tetrahydroquinolin-5-yl)-5-[4-(trifluoromethyl)phenyl]-2,4-pentadienamide (Compound 326)

$^1$H NMR (DMSO-$d_6$, δ ppm): 1.56-1.78 (m, 1H), 2.01-2.08 (m, 2H), 2.33-2.43 (m, 2H), 2.54-2.66 (m, 1H), 2.97-3.05 (m, 1H), 4.02-4.10 (m, 1H), 4.68-4.74 (m, 1H), 5.44 (d, J = 4.5 Hz, 1H), 6.59-6.77 (m, 4H), 6.94 (dd, J = 2.3, 7.9 Hz, 1H), 7.09-7.17 (m, 4H), 7.40 (t, J = 7.9 Hz, 1H), 7.55 (d, J = 8.4 Hz, 2H), 7.73 (d, J = 8.4 Hz, 2H), 9.74 (br s, 1H), 10.17 (br s, 1H); ESIMS m/z: $[M+H]^+$ 549.

(2E,4Z)-5-(4-Cyclopropoxyphenyl)-N-(3-hydroxy-2-oxo-1,2,3,4-tetrahydroquinolin-5-yl)-5-[4-(trifluoromethyl)phenyl]-2,4-pentadienamide (Compound 327)

$^1$H NMR (DMSO-$d_6$, δ ppm): 0.69-0.76 (m, 2H), 0.78-0.85 (m, 2H), 2.62 (dd, J = 11.8, 15.9 Hz, 1H), 3.02 (dd, J = 6.3, 15.9 Hz, 1H), 3.89-3.95 (m, 1H), 4.03-4.10 (m, 2H), 5.45 (d, J = 4.6 Hz, 1H), 6.61 (d, J = 14.4 Hz, 1H), 6.70 (dd, J = 3.1, 5.9 Hz, 1H), 7.07-7.24 (m, 8H), 7.54 (d, J = 8.2 Hz, 2H), 7.74 (d, J = 8.2 Hz, 2H), 9.73 (br s, 1H), 10.17 (br s, 1H) ; ESIMS m/z: $[M+H]^+$ 535 .

(2E,4Z)-N-(3-Hydroxy-1,2,3,4-tetrahydroquinolin-5-yl)-5-[4-(pentafluorosulfanyl)phenyl]-5-[4-(trifluoromethyl)phenyl]-2,4-pentadienamide (Compound 328)

$^1$H NMR (DMSO-$d_6$, δ ppm): 2.52 (dd, J = 11.7, 15.8 Hz, 1H), 3.01 (dd, J = 6.0, 15.8 Hz, 1H), 4.03-4.10 (m, 1H), 5.46 (d, J = 4.4 Hz, 1H), 6.66-6.71 (m, 2H), 6.99-7.14 (m, 3H), 7.31 (d, J = 11.7 Hz, 1H), 7.48-7.56 (m, 4H), 7.76 (d, J = 8.5 Hz, 2H), 8.06 (d, J = 8.5 Hz, 2H), 9.81 (br s, 1H), 10.18 (br s, 1H); ESIMS m/z: $[M+H]^+$ 605.

(2E,4Z)-5-[4-(1-Ethylpropoxy)phenyl]-N-(3-hydroxy-2-oxo-1,2,3,4-tetrahydroquinolin-5-yl)-5-[4-(trifluornmethyl)phenyl]-2,4-pentadienamide (Compound 329)

$^1$H NMR (DMSO-$d_6$, δ ppm) : 0.93 (t, J = 6.6 Hz, 6H), 1.65 (quint, J = 6.6 Hz, 4H), 2.63 (dd, J = 11.9, 15.9 Hz, 1H), 3.02 (dd, J = 6.3, 15.9 Hz, 1H), 4.03-4.11 (m, 1H), 4.30 (quint, J = 6.6 Hz, 1H), 5.45 (d, J = 4.6 Hz, 1H), 6.55-6.72 (m, 2H), 7.02-7.13 (m, 7H), 7.23 (dd, J = 11. 5, 14.7 Hz, 1H), 7.54 (d, J = 8.4 Hz, 2H), 7.74 (d, J = 8.4 Hz, 2H), 9.73 (br s, 1H), 10.18 (br s, 1H) ; APCIMS m/z: $[M+H]^+$ 565.

(E)-5,5-Bis(4-ethoxyphenyl)-N-(3-hydroxy-2-oxo-1,2,3,4-tetrahydroquinolin-5-yl)-2,4-pentadienamide (Compound 330)

$^1$H NMR (DMSO-$d_6$, δ ppm) : 1.33 (t, J = 7.1 Hz, 3H), 1.36 (t, J = 7.1 Hz, 3H), 2.61 (dd, J = 12.0, 15.9 Hz, 1H), 3.01 (dd, J = 6.3, 15.9 Hz, 1H), 4.00-4.13 (m, 5H), 5.45 (d, J = 4.3 Hz, 1H), 6.47 (d, J = 14.5 Hz, 1H), 6.67-6.70 (m, 1H), 6.85-6.93 (m, 3H), 7.00-7.26 (m, 9H), 9.62 (br s, 1H), 10.17 (br s, 1H) ; APCIMS m/z: $[M+H]^+$ 499.

(2E,4E)-5-(4-Ethoxyphenyl)-N-(3-hydroxy-2-oxo-1,2,3,4-tetrahydroquinolin-5-yl)-5-[4-(trifluoromethoxy)phenyl]-2,4-pentadienamide (Compound 331)

$^1$H NMR (DMSO-$d_6$, δ ppm) : 1.36 (t, J = 7.0 Hz, 3H), 2.62 (dd, J = 12.0, 15.9 Hz, 1H), 3.02 (dd, J = 6.3, 15.9 Hz, 1H), 4.03-4.11 (m, 1H), 4.09 (q, J = 7.0 Hz, 2H), 5.45 (d, J = 4.6 Hz, 1H), 6.57 (d, J = 14.5 Hz, 1H) , 6.68-6.72 (m, 1H), 6.97-7.14 (m, 7H), 7.18 (dd, J = 11.2, 14.5 Hz, 1H), 7.36 (d, J = 8.2 Hz, 2H), 7.45 (d, J = 8.9 Hz, 2H), 9.70 (br s, 1H) , 10.18 (br s, 1H) ; APCIMS m/z: $[M+H]^+$ 539.

Example 117

(2E,4Z)-5-[4-(Cyanomethoxy)phenyl]-N-(3-hydroxy-2-oxo-1,2,3,4-tetrahydroquinolin-5-yl)-5-[4-(trifluoromethyl)phenyl]-2,4-pentadienamide (Compound 200)

Compound 210 (110 mg, 0.222 mmol), chloroacetonitrile (0.0150 mL, 0.245 mmol), and potassium carbonate (34.0 mg, 0.245 mmol) were dissolved in DMF (2 mL), and the mixture was stirred at room temperature for 3 hours. Water was added to the reaction mixture, and the mixture was extracted three times with ethyl acetate. The organic layer was washed with saturated brine and dried over anhydrous magnesium sulfate. Then, the solvent was evaporated under reduced pressure, and the residue was purified by silica gel column chromatography (chloroform/methanol = 5/1) to give Compound 200 (57.0 mg, 48%) .
$^1$H NMR (DMSO-$d_6$, δ ppm) : 2.62 (dd, J = 12.0, 15.9 Hz, 1H), 3.02 (dd, J = 6.2, 15.9 Hz, 1H), 4.02-4.11 (m, 1H), 5.25 (s, 2H), 5.44 (d, J = 4.6 Hz, 1H), 6.60-6.72 (m, 2H), 7.09-7.26 (m, 8H), 7.54 (d, J = 8.3 Hz, 2H), 7.74 (d, J = 8.3 Hz, 2H), 9.73 (br s, 1H), 10.16 (br s, 1H) ; ESIMS m/z: $[M+H]^+$ 534.

Example 118

(2E,4Z)-5-[3-(Cyanomethoxy)phenyl]-N-(3-hydroxy-2-oxo-1,2,3,4-tetrahydroquinolin-5-yl)-5-[4-(trifluoromethyl)phenyl]-2,4-pentadienamide (Compound 205)

In a similar manner to Example 117, Compound 205 was obtained from Compound 256.

$^1$H NMR (DMSO-$d_6$, δ ppm): 2.62 (dd, J = 11.9, 15.9 Hz, 1H), 3.01 (dd, J = 6.3, 15.9 Hz, 1H), 4.02-4.11 (m, 1H), 5.20 (s, 2H), 5.45 (d, J = 4.5 Hz, 1H), 6.61-6.72 (m, 2H), 6.89-6.90 (m, 1H) , 6.95 (d, J = 7.5 Hz, 1H), 7.05-7.25 (m, 5H), 7.50-7.57 (m, 3H), 7.74 (d, J = 8.4 Hz, 2H), 9.76 (br s, 1H), 10.18 (br s, 1H); ESIMS m/z: $[M+H]^+$ 534.

Example 119

(2E,4Z)-N-(3-Hydroxy-2-oxo-1,2,3,4-tetrahydroquinolin-5-yl)-5-(1-oxypyridin-4-yl)-5-[4-(trifluoromethyl)phenyl]-2,4-pentadienamide monohydrochloride (Compound 251)

Step 1

Compound t (280 mg, 0.883 mmol) was dissolved in DMF (8.0 mL), and 5-amino-3-hydroxy-2-oxo-1,2,3,4-tetrahydroquinoline (315 mg, 1.77 mmol), EDC hydrochloride (339 mg, 1.77 mmol), and HOBt (270 mg, 1.76 mmol) were added thereto, and then, the mixture was stirred at 65˚C for 8 hours. After the reaction mixture was left to cool, a saturated sodium hydrogen carbonate solution was added thereto, and the mixture was extracted with ethyl acetate. The organic layer was washed with saturated brine and dried over anhydrous magnesium sulfate. After the organic layer was filtered, the solvent was evaporated under reduced pressure, and the residue was purified by silica gel column chromatography (ethyl acetate/hexane = 1/1) to give (2E,4Z)-N-(3-hydroxy-2-oxo-1,2,3,4-tetrahydroquinolin-5-yl)-5-(pyridin-4-yl)-5-[4-(trifluoromethyl)phenyl]-2,4-pentadienamide (225 mg, 54%).

Step 2

The product of Step 1 (170 mg, 0.355 mmol) was dissolved in DMF (3.0 mL), and 3-chloroperbenzoic acid (92.0 mg, 0.533 mmol) was added thereto, and then, the mixture was stirred at 0˚C for 7 hours. Water was added to the reaction mixture and the precipitated crystal was filtered, washed with water and dried to give (2E,4Z)-N-(3-hydroxy-2-oxo-1,2,3,4-tetrahydroquinolin-5-yl)-5-(1-oxypyridin-4-yl)-5-[4-(trifluoromethyl)phenyl]-2,4-pentadienamide. The resulting compound was suspended in ethanol (4.0 mL), and a 4 mol/L hydrogen chloride-ethyl acetate solution (0.067 mL, 0.268 mmol) was added thereto. The solvent was evaporated under reduced pressure, and the residue was recrystallized from diethyl ether to give Compound 251 (107 mg, 57%).
$^1$H NMR (DMSO-$d_6$, δ ppm): 2.62 (dd, J = 11.8, 15.9 Hz, 1H), 3.00 (dd, J = 6.3, 15.9 Hz, 1H), 4.05 (dd, J = 6.3, 11.8 Hz, 1H), 6.69-6.74 (m, 2H), 7.06-7.17 (m, 3H), 7.26 (d, J = 11.7 Hz, 1H), 7.42 (d, J = 7.1 Hz, 2H), 7.60 (d, J = 8.3 Hz, 2H), 7.76 (d, J = 8.3 Hz, 2H), 8.48 (d, J = 7.1 Hz, 2H), 9.89 (br s, 1H), 10.19 (br s, 1H); ESIMS m/z: $[M+H]^+$ 496.

Example 120

(2E,4Z)-5-(4-Aminophenyl)-N-(isoquinolin-5-yl)-5-[4-(trifluoromethyl)phenyl]-2,4-pentadienamide (Compound 268)

Step 1

In a similar manner to Example 10, (2E,4Z)-5-bromo-N-(isoquinolin-5-yl)-5-[4-(trifluoromethyl)phenyl]-2,4-pentadienamide was synthesized from Compound ew.
$^1$H NMR (DMSO-$d_6$, δ ppm): 6.92-7.02 (m, 1H), 7.54-7.74 (m, 3H), 7.84 (d, J = 8.6 Hz, 2H), 7.97-8.02 (m, 4H), 8.17-8.22 (m, 1H), 8.57 (d, J = 6.2 Hz, 1H), 9.35 (s, 1H), 10.45 (br s, 1H).

Step 2

In a similar manner to Step 1 of Reference example 18, Compound 268 was obtained from the product of step 1 using 4-aminophenylboronic acid.
$^1$H NMR (DMSO-$d_6$, δ ppm): 5.45 (br s, 2H), 6.63 (d, J = 8.1 Hz, 2H), 6.72 (d, J = 15.0 Hz, 1H), 6.85-6.92 (m, 3H), 7.41 (dd, J = 11.6, 15.0 Hz, 1H), 7.55 (d, J = 8.1 Hz, 2H), 7.66 (t, J = 7.9 Hz, 1H), 7.72 (d, J = 8.4 Hz, 2H), 7.92 (d, J = 8.1 Hz, 1H), 7.99 (d, J = 5.7 He, 1H), 8.15 (d, J = 6.9 Hz, 1H), 8.54 (d, J = 6.0 Hz, 1H), 9.31 (s, 1H), 10.21 (br s, 1H); ESIMS m/z: $[M+H]^+$ 460.

Example 121

(2E,4Z)-N-(Isoquinolin-5-yl)-5-[4-(methanesulfonylamino)phenyl]-5-[4-(trifluoromethyl)phenyl]-2,4-pentadienamide (Compound 269)

Compound 268 (75.0 mg, 0.163 mmol) was dissolved in THF (1.0 mL), and methanesulfonyl chloride (28.0 mg, 0.244 mmol) and triethylamine (33.0 mg, 0.326 mmol) were added thereto, and then, the mixture was stirred at room temperature for 17 hours. After the reaction mixture was concentrated, water and ethyl acetate were added to the residue. Then, the precipitated crystal was filtered, and washed with water and ethyl acetate to give Compound 269 (37.0 mg, 42%).
$^1$H NMR (DMSO-$d_6$, δ ppm): 3.08 (s, 3H), 6.80 (d, J = 13.8 Hz, 1H), 7.14-7.27 (m, 2H), 7.20 (d, J = 7.6 Hz, 2H), 7.31 (d, J = 8.4 Hz, 2H), 7.56 (d, J = 8.1 Hz, 2H), 7.73-7.81 (m, 3H), 8.08 (d, J = 7.8 Hz, 1H), 8-18-8.25 (m, 2H), 8.61 (d, J = 6.5 Hz, 1H), 9.53 (br s, 1H), 10.04 (s, 1H), 10.43 (br s, 1H); ESIMS m/z: $[M+H]^+$ 538.

Example 122

(2E,4Z)-N-(Isoquinolin-5-yl)-5-[4-(4-methylpiperazin-1-yl)phenyl]-5-[4-(trifluoromethyl)phenyl]-2,4-pentadienamide (Compound 274)

In a similar manner to Step 1 of Reference example 18, Compound 274 was obtained from (2E,4Z)-5-bromo-N-(isoquinolin-5-yl)-5-[4-(trifluoromethyl)phenyl]-2,4-pentadienamide obtained in Step 1 of Example 120 using 1-methyl-4-[4-(4,4,5,5-tetramethyl-1,3,2-dioxaborolan-2-yl)phenyl]piperazine.
$^1$H NMR (DMSO-$d_6$, δ ppm): 2.23 (s, 3H), 2.46-2.50 (m, 4H), 3.22-3.25 (m, pH), 6.77 (d, J = 14.8 Hz, 1H), 7.01-7.09 (m, 5H), 7.37 (dd, J = 11.6, 14.8 Hz, 1H), 7.56 (d, J = 8.1 Hz, 2H), 7.67 (t, J = 7.8 Hz, 1H), 7.74 (d, J = 8.4 Hz, 2H), 7.94 (d, J = 8.1 Hz, 1H), 8.00 (d, J = 5.9 Hz, 1H), 8.15 (d, J = 7.6 Hz, 1H), 8.55 (d, J = 6.2 Hz, 1H), 9.32 (s, 1H), 10.25 (br s, 1H); ESIMS m/z: $[M+H]^+$ 543.

Example 123

(2E,4E)-5-[4-(2-Hydroxy-2-propyl)phenyl]-N-(isoquinolin-5-yl)-5-[4-(trifluoromethyl)phenyl]-2,4-pentadienamide (Compound 288)

Compound 287 (48.9 mg, 0.101 mmol) was dissolved in THF (2.5 mL), and a 0.87 mol/L methylmagnesium bromide-THF solution (0.600 mL, 0.522 mmol) was added thereto, and then, the mixture was stirred at 0˚C for 7 hours. To the reaction mixture, a saturated aqueous ammonium chloride solution was added, and the mixture was extracted with chloroform. The organic layer was washed with saturated brine and dried over anhydrous magnesium sulfate. After the organic layer was filtered, the solvent was evaporated under reduced pressure, and the residue was purified by silica gel column chromatography (chloroform/methanol = 97/3) to give Compound 288 (11.0 mg, 35%).
$^1$H NMR ($CD_3OD$, δ ppm): 1.52 (s, 6H), 6.62 (d, J = 14.6 Hz, 1H), 7.02 (d, J = 11.3 Hz, 1H), 7.15 (d, J = 7.7 Hz, 2H), 7.40-7.64 (m, 8H), 7.84-7.99 (m, 3H), 8.39 (d, J = 5.7 Hz, 1H), 9.18 (s, 1H); ESIMS m/z: $[M+H]^+$ 503.

Example 124

(2E,4Z)-5-[4-(2-Aminoethoxy)phenyl]-N-(isoquinolin-5-yl)-5-[4-(trifluoromethyl)phenyl]-2,4-pentadienamide (Compound 290)

Step 1

In a similar manner to example 10, (2E,4Z)-5-[4-(2-tert-butoxycarbonylaminoethoxy)phenyl-N-(isoquinolin-5-yl)-5-[4-(trifluoromethyl)phenyl]-2,4-pentadienamide was obtained from Compound fi.

Step 2

In a similar manner to Step 2 of Reference example 150, Compound 290 was obtained from the product of step 1. $^1$H NMR (DMSO-$d_6$, δ ppm): 3.18 (t, J = 5.2 Hz, 2H), 4.20 (t, J = 5.2 Hz, 2H), 6.82 (d, J = 14.1 Hz, 1H), 7.10-7.31 (m, 6H), 7.56 (d, J = 8.0 Hz, 2H), 7.67 (t, J = 7.9 Hz, 1H), 7.76 (d, J = 8.4 Hz, 2H), 7.95 (d, J = 8.0 Hz, 1H), 7.99 (d, J = 6.0 Hz, 1H), 8.111 (d, J = 7.5 Hz, 1H), 8.55 (d, J = 6.0 Hz, 1H), 9.32 (s, 1H), 10.33 (br s, 1H) ; ESIMS m/z: $[M+H]^+$ 504.

Example 125

(2E,4E)-5-[4-((E)-1-Hydroxyiminoethyl)phenyl]-N-(isoquinolin-5-yl)-5-[4-(trifluoromethyl)phenyl]-2,4-pentadienamide (Compound 291)

Compound 287 (79.0 mg, 0.162 mmol) was dissolved in ethanol (2.0 mL), and hydroxylamine monohydrochloride (13.5 mg, 0.194 mmol) and pyridine (25.7 mg, 0.325 mmol) were added thereto, and then, the mixture was stirred at room temperature for 1 hour. Water was added to the reaction mixture, and the mixture was extracted with ethyl acetate, and then, the organic layer was dried over anhydrous magnesium sulfate. After the organic layer was filtered, the solvent was evaporated under reduced pressure, and the residue was purified by silica gel column chromatography (chloroform/ methanol = 10/1) to give Compound 291 (59.0 mg, 73%).
$^1$H NMR (DMSO-$d_6$, δ ppm): 2.21 (s, 3H), 6.80-6.85 (m, 1H), 7.23-7.28 (m, 4H), 7.58 (d, J = 8.1 Hz, 2H), 7.66 (t, J = 7.8 Hz, 1H), 7.76 (d, J = 8.6 Hz, 2H), 7.80 (d, J = 8.1 Hz, 2H), 7.94 (d, J = 8.1 Hz, 1H), 7.99 (d, J = 5.9 Hz, 1H), 8.13 (d, J = 7.3 Hz, 1H), 8.55 (d, J = 5.9 Hz, 1H), 9.32 (s, 1H), 10.30 (br s, 1H), 11.34 (br s, 1H); ESIMS m/z: $[M+H]^+$ 487.

Example 126

(2E,4E)-5-(4-Formylphenyl)-N-(isoquinolin-5-yl)-5-[4-(trifluoromethyl)phenyl]-2,4-pentadienamide (Compound 292)

Step 1

In a similar manner to example 10, (2E,4Z)-5-[4-(isoquinolin-5-yliminomethyl)phenyl]-N-(isoquinolin-5-yl)-5-[4-(trifluoromethyl)phenyl]-2,4-pentadienamide was obtained from Compound fj.

Step 2

In a similar manner to Step 2 of Reference example 150, Compound 292 was obtained from the product of step 1.
$^1$H NMR (DMSO-$d_6$, δ ppm): 6.85 (d, J = 14.6 Hz, 1H), 7.16 (dd, J = 11.5, 14.6 Hz, 1H), 7.33 (d, J = 11.5 Hz, 1H), 7.50 (d, J = 8.1 Hz, 2H), 7.57 (d, J = 8.0 Hz, 2H), 7.66 (t, J = 7.9 Hz, 1H), 7.77 (d, J = 8.4 Hz, 2H), 7.93-7.98 (m, 2H), 8.06 (d, J = 8.0 Hz, 2H), 8.11 (d, J = 7.3 Hz, 1H), 8.55 (d, J = 5.9 Hz, 1H), 9.32 (s, 1H), 10.10 (s, 1H), 10.32 (br s, 1H); ESIMS m/z: $[M+H]^+$ 473.

Example 127

(2E,4E)-N-(Isoquinolin-5-yl)-5-[4-(pyrrolidin-1-ylmethyl)phenyl]-5-[4-(trifluoromethyl)phenyl]-2,4-pentadienamide (Compound 294)

Compound 292 (66.6 mg, 0.141 mmol) was dissolved in dichloromethane (2.0 mL), and pyrrolidine (34.9 mg, 0.491 mmol) and sodium triacetoxyborohydride (63.0 mg, 0.297 mmol) were added thereto, and then, the mixture was stirred at room temperature for 8 hours. Water was added to the reaction mixture, and the mixture was extracted with chloroform. The organic layer was washed with saturated brine and dried over anhydrous magnesium sulfate. After the organic layer was filtered, the solvent was evaporated under reduced pressure, and the residue was purified by silica gel column chromatography (chloroform/methanol = 9/1) to give Compound 294 (47.1 mg, 63%).
$^1$H NMR ($CD_3OD$, δ ppm) : 1.96-1.97 (m, 4H), 2.99-3.01 (m, 4H), 4.08 (s, 2H), 6.69 (d, J = 14.7 Hz, 1H), 7.15 (d, J = 11.6 Hz, 1H), 7.31 (d, J = 8.2 Hz, 2H), 7.42 (dd, J = 11.6, 14.7 Hz, 1H), 7.52-7.57 (m, 4H), 7.65-7.72 (m, 3H), 7.91 (d, J = 6.2 Hz, 1H), 7.98-8.04 (m, 2H), 8.47 (d, J = 6.0 Hz, 1H), 9.26 (d, J = 0.9 Hz, 1H) ; ESIMS m/z: $[M+H]^+$ 528.

Example 128

(2E,4E)-N-(Isoquinolin-5-yl)-5-[4-(morpholin-4-ylmethyl)phenyl]-5-4-(trifluoromethyl)phenyl]-2,4-pentadienamide (Compound 295)

In a similar manner to Reference example 127, Compound 295 was obtained using morpholine in place of pyrrolidine.
$^1$H NMR (DMSO-$d_6$, δ ppm): 2.41-2.42 (m, 4H), 3.55 (s, 2H), 3.60-3.62 (m, 4H), 6.81 (d, J = 13.2 Hz, 1H), 7.19-7.29 (m, 4H), 7.45 (d, J = 7.9 Hz, 2H), 7.56 (d, J = 8.2 Hz, 2H), 7.66 (t, J = 7.9 Hz, 1H), 7.75 (d, J = 8.2 Hz, 2H), 7.93-7.99 (m, 2H), 8.11 (d, J = 6.8 Hz, 1H), 8.55 (d, J = 6.1 Hz, 1H), 9.32 (s, 1H), 10.29 (br s, 1H) ; ESIMS m/z: $[M+H]^+$ 544.

Example 129

Preparation Example 1: Tablet

A tablet having the following formulation is prepared according to the conventional manner.

| Formulation | |
|---|---|
| Compound 1 | 20 mg |
| Lactose | 143.4 mg |
| Potato starch | 30 mg |
| Hydroxypropyl cellulose | 6 mg |
| Magnesium stearate | 0.6 mg |
| Total | 200 mg |

Example 130

Preparation Example 2: Injection Preparation

An injection preparation having the following formulation is prepared according to the conventional manner.

| Formulation | |
|---|---|
| Compound 7 | 2 mg |
| D-mannitol | 10 mg |
| Aqueous hydrochloric acid solution | proper amount |
| Aqueous sodium hydroxide solution | proper amount |
| Distilled water for injection | proper amount |
| Total | 2.00 mL |

Industrial Applicability

According to the present invention, a pentadienamide derivative or a pharmaceutically acceptable salt thereof having an activity to modify the function of TRPV1, and the like are provided.

**Claims**

**1.** A pentadienamide derivative represented by the formula (I):

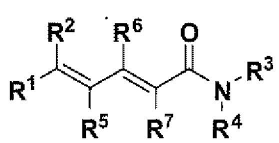

(I)

(wherein $R^1$ represents substituted or unsubstituted aryl or a substituted or unsubstituted aromatic heterocyclic group;
$R^2$ represents substituted or unsubstituted cycloalkyl, substituted or unsubstituted cycloalkenyl, substituted or unsubstituted aryl, a substituted or unsubstituted aromatic heterocyclic group, or a substituted or unsubstituted heteroalicyclic group;
$R^3$ represents a hydrogen atom or is combined together with $R^4$ and the adjacent nitrogen atom thereto to form a substituted or unsubstituted heterocyclic group;
$R^4$ represents substituted or unsubstituted lower alkyl, substituted or unsubstituted cycloalkyl, substituted or unsubstituted aryl, a substituted or unsubstituted aromatic heterocyclic group, or a substituted or unsubstituted heteroalicyclic group, or is combined together with $R^3$ and the adjacent nitrogen atom thereto to form a substituted

or unsubstituted heterocyclic group; and
$R^5$, $R^6$, and $R^7$ may be the same or different, and each represents a hydrogen atom or methyl), or a pharmaceutically acceptable salt thereof.

**2.** The pentadienamide derivative or the pharmaceutically acceptable salt thereof according to claim 1, wherein each of $R^5$, $R^6$, and $R^7$ is a hydrogen atom.

**3.** The pentadienamide derivative or the pharmaceutically acceptable salt thereof according to claim 1 or 2, wherein $R^3$ and $R^4$ are combined together with the adjacent nitrogen atom thereto to form a substituted or unsubstituted heterocyclic group.

**4.** The pentadienamide derivative or the pharmaceutically acceptable salt thereof according to claim 1 or 2, wherein $R^3$ and $R^4$ are combined together with the adjacent nitrogen atom thereto to form substituted or unsubstituted thiomorpholino.

**5.** The pentadienamide derivative or the pharmaceutically acceptable salt thereof according to claim 1 or 2, wherein $R^3$ and $R^4$ are combined together with the adjacent nitrogen atom thereto to form substituted or unsubstituted piperidino, or substituted or unsubstituted piperazinyl.

**6.** The pentadienamide derivative or the pharmaceutically acceptable salt thereof according to claim 1 or 2, wherein $R^3$ is a hydrogen atom.

**7.** The pentadienamide derivative or the pharmaceutically acceptable salt thereof according to claim 6, wherein $R^4$ is substituted or unsubstituted aryl.

**8.** The pentadienamide derivative or the pharmaceutically acceptable salt thereof according to claim 6, wherein $R^4$ is substituted or unsubstituted dihydrobenzoxazinyl.

**9.** The pentadienamide derivative or the pharmaceutically acceptable salt thereof according to claim 6, wherein $R^4$ is substituted or unsubstituted tetrahydroisoquinolyl.

**10.** The pentadienamide derivative or the pharmaceutically acceptable salt thereof according to claim 6, wherein $R^4$ is a substituted or unsubstituted aromatic heterocyclic group.

**11.** The pentadienamide derivative or the pharmaceutically acceptable salt thereof according to claim 6, wherein $R^4$ is substituted or unsubstituted indazolyl.

**12.** The pentadienamide derivative or the pharmaceutically acceptable salt thereof according to claim 6, wherein $R^4$ is substituted or unsubstituted isoquinolyl.

**13.** The pentadienamide derivative or the pharmaceutically acceptable salt thereof according to any one of claims 1 to 12, wherein $R^1$ is substituted or unsubstituted aryl.

**14.** The pentadienamide derivative or the pharmaceutically acceptable salt thereof according to any one of claims 1 to 12, wherein $R^1$ is 4-(trifluoromethyl)phenyl.

**15.** The pentadienamide derivative or the pharmaceutically acceptable salt thereof according to any one of claims 1 to 14, wherein $R^2$ is substituted or unsubstituted aryl.

**16.** The pentadienamide derivative or the pharmaceutically acceptable salt thereof according to any one of claims 1 to 14, wherein $R^2$ is substituted or unsubstituted phenyl.

**17.** The pentadienamide derivative or the pharmaceutically acceptable salt thereof according to any one of claims 1 to 14, wherein $R^2$ is phenyl substituted with substituted or unsubstituted lower alkoxy.

**18.** The pentadienamide derivative or the pharmaceutically acceptable salt thereof according to any one of claims 1 to 14, wherein $R^2$ is 4-(trifluoromethyl)phenyl.

**19.** The pentadienamide derivative or the pharmaceutically acceptable salt thereof according to any one of claims 1 to 14, wherein $R^2$ is a substituted or unsubstituted aromatic heterocyclic group.

**20.** The pentadienamide derivative or the pharmaceutically acceptable salt thereof according to any one of claims 1 to 14, wherein $R^2$ is substituted or unsubstituted pyridyl or substituted or unsubstituted pyrimidinyl.

**21.** A pharmaceutical composition, which comprises, as an active ingredient, the pentadienamide derivative or the pharmaceutically acceptable salt thereof described in any one of claims 1 to 20.

**22.** A vanilloid receptor (TRPV1) agonist, which comprises, as an active ingredient, the pentadienamide derivative or the pharmaceutically acceptable salt thereof described in any one of claims 1 to 20.

**23.** A vanilloid receptor (TRPV1) antagonist, which comprises, as an active ingredient, the pentadienamide derivative or the pharmaceutically acceptable salt thereof described in any one of claims 1 to 20.

**24.** A preventive and/or therapeutic agent for a disease associated with the function of a vanilloid receptor (TRPV1), which comprises, as an active ingredient, the pentadienamide derivative or the pharmaceutically acceptable salt thereof described in any one of claims 1 to 20.

**25.** A preventive and/or therapeutic agent for a pain, which comprises, as an active ingredient, the pentadienamide derivative or the pharmaceutically acceptable salt thereof described in any one of claims 1 to 20.

**26.** The preventive and/or therapeutic agent according to claim 25, wherein the pain is neuropathic pain.

**27.** A method for agonizing a vanilloid receptor (TRPV1), which comprises the step of administering an effective amount of the pentadienamide derivative or the pharmaceutically acceptable salt thereof described in any one of claims 1 to 20.

**28.** A method for antagonizing a vanilloid receptor (TRPV1), which comprises the step of administering an effective amount of the pentadienamide derivative or the pharmaceutically acceptable salt thereof described in any one of claims 1 to 20.

**29.** A method for preventing and/or treating a disease associated with the function of a vanilloid receptor (TRPV1), which comprises the step of administering an effective amount of the pentadienamide derivative or the pharmaceutically acceptable salt thereof described in any one of claims 1 to 20.

**30.** A method for preventing and/or treating a pain, which comprises the step of administering an effective amount of the pentadienamide derivative or the pharmaceutically acceptable salt thereof described in any one of claims 1 to 20.

**31.** The method for preventing and/or treating according to claim 30, wherein the pain is neuropathic pain.

**32.** Use of the pentadienamide derivative or the pharmaceutically acceptable salt thereof described in any one of claims 1 to 20, for the manufacture of a vanilloid receptor (TRPV1) agonist.

**33.** Use of the pentadienamide derivative or the pharmaceutically acceptable salt thereof described in any one of claims 1 to 20, for the manufacture of a vanilloid receptor (TRPV1) antagonist.

**34.** Use of the pentadienamide derivative or the pharmaceutically acceptable salt thereof described in any one of claims 1 to 20, for the manufacture of a preventive and/or therapeutic agent for a disease associated with the function of a vanilloid receptor (TRPV1).

**35.** Use of the pentadienamide derivative or the pharmaceutically acceptable salt thereof described in any one of claims 1 to 20, for the manufacture of a preventive and/or therapeutic agent for a pain.

**36.** The use according to claim 35, wherein the pain is neuropathic pain.

**INTERNATIONAL SEARCH REPORT**

International application No.
PCT/JP2007/064007

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
See extra sheet.

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2007 |
| Kokai Jitsuyo Shinan Koho | 1971-2007 | Toroku Jitsuyo Shinan Koho | 1994-2007 |

Electronic data base consulted during the international search (name of data base and, where practicable, search terms used)
CA(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X | JP 64-31766 A (F. Hoffmann-La Roche & Co., AG.), 02 February, 1989 (02.02.89), Claims; examples & EP 299379 A1 & US 4788206 A | 1,2,6,7,10, 12-21 |
| X | JP 5-230069 A (Yamanouchi Pharmaceutical Co., Ltd.), 07 September, 1993 (07.09.93), Claims; examples (Family: none) | 1,2,6,7,10, 13,15-17,21 |
| X | Robert W. G. et al., 'Pentadienyl Carboxamide Derivatives as Antagonists of Platelet-Activating Factor' J. Med. Chem., 1989, Vol.32, No.8, p.1820-1835 | 1,2,6,7,13, 15-17,21 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 August, 2007 (07.08.07) | 21 August, 2007 (21.08.07) |
| Name and mailing address of the ISA/ Japanese Patent Office | Authorized officer |
| Facsimile No. | Telephone No. |

Form PCT/ISA/210 (second sheet) (April 2005)

**INTERNATIONAL SEARCH REPORT**

International application No.
PCT/JP2007/064007

C (Continuation). DOCUMENTS CONSIDERED TO BE RELEVANT

| Category* | Citation of document, with indication, where appropriate, of the relevant passages | Relevant to claim No. |
|---|---|---|
| X | William T. C. et al., '5,5-Diarylpenta-2,4-dienoic Acid Amides as Potential Antimalarial Agents' J. Med. Chem., 1968, Vol.11, p.749-752 | 1-3,5,6,13, 15-17,21 |
| X | Judy H. L. et al., '5,5-Diarylpenta-2,4-dienoic Acid Amides as Potential Antimalarial Agents. II' J. Med. Chem., 1969, Vol.12, p.946-947 | 1-3,5-7,13, 15,16,21 |
| X | US 3244701 A (Farbwerke Hoechst Aktiengesellschaft vormals Meister Lucius & Bruning), 05 April, 1966 (05.04.66), Full text & JP 41-11620 B1 | 1-3,5,13, 15-17 |
| A | JP 2005-518371 A (Amgem Inc.), 23 June, 2005 (23.06.05), & WO 2003/49702 A2 & EP 1463714 A2 & US 2003/195201 A1 | 1-26,32-36 |
| A | WO 2005/44786 A1 (BAYER HEALTHCARE AG.), 19 May, 2005 (19.05.05), & JP 2007-511479 A & EP 1687262 A1 | 1-26,32-36 |
| A | JP 2005-517737 A (Council of Scientific and Industrial Research), 16 June, 2005 (16.06.05), & WO 2003/70713 A1 & EP 1479632 A1 & US 2003/161860 A1 | 1-26,32-36 |
| A | Arpad Szallasi 'Piperine:researchers discover new flavor in an ancient spice' Trends Pharmacol. Sci., 2005, Vol.26, No.9, p.437-439 | 1-26,32-36 |
| A | Michael J. G. et al., 'Stimulation of Dichlorofluorescin Oxidation by Capsaicin and Analogues in RAW 264 Monocyte/Macrophages: Lack of Invement of the Vanilloid Receptor' Biochemical Pharmacology, 2000, Vol.59, No.5, p.563-572 | 1-26,32-36 |
| A | Giovanni A. et al., 'The Taming of Capsaicin. Reversal of the Vanilloid Activity of N-Acylvanillamines by Aromatic Iodination' J. Med. Chem., 2005, Vol.48, No.14, p.4663-4669 | 1-26,32-36 |

Form PCT/ISA/210 (continuation of second sheet) (April 2005)

**INTERNATIONAL SEARCH REPORT**

International application No.
PCT/JP2007/064007

Continuation of A. CLASSIFICATION OF SUBJECT MATTER
(International Patent Classification (IPC))

*C07C233/11*(2006.01)i, *A61K31/167*(2006.01)i, *A61K31/18*(2006.01)i, *A61K31/277*(2006.01)i, *A61K31/416*(2006.01)i, *A61K31/4184*(2006.01)i, *A61K31/4245*(2006.01)i, *A61K31/428*(2006.01)i, *A61K31/433*(2006.01)i, *A61K31/44*(2006.01)i, *A61K31/4406*(2006.01)i, *A61K31/4409*(2006.01)i, *A61K31/4427*(2006.01)i, *A61K31/443*(2006.01)i, *A61K31/47*(2006.01)i, *A61K31/4709*(2006.01)i, *A61K31/472*(2006.01)i, *A61K31/4725*(2006.01)i, *A61K31/496*(2006.01)i, *A61K31/506*(2006.01)i, *A61K31/536*(2006.01)i, *A61K31/538*(2006.01)i, *A61K31/54*(2006.01)i, *A61P25/00*(2006.01)i, *A61P43/00*(2006.01)i, *C07C233/27*(2006.01)i, *C07C233/30*(2006.01)i, *C07C255/58*(2006.01)i, *C07C307/02*(2006.01)i, *C07C311/46*(2006.01)i, *C07C317/40*(2006.01)i, *C07D209/08*(2006.01)i, *C07D213/40*(2006.01)i, *C07D213/64*(2006.01)i, *C07D213/74*(2006.01)i, *C07D213/75*(2006.01)i, *C07D215/38*(2006.01)i, *C07D217/02*(2006.01)i, *C07D217/24*(2006.01)i, *C07D217/26*(2006.01)i, *C07D231/56*(2006.01)i, *C07D235/12*(2006.01)i, *C07D265/18*(2006.01)i, *C07D265/36*(2006.01)i, *C07D271/06*(2006.01)i, *C07D277/62*(2006.01)i, *C07D277/82*(2006.01)i, *C07D279/12*(2006.01)i, *C07D285/135*(2006.01)i, *C07D295/18*(2006.01)i, *C07D311/04*(2006.01)i, *C07D319/18*(2006.01)i, *C07D401/06*(2006.01)i, *C07D401/12*(2006.01)i, *C07D405/12*(2006.01)i, *C07D409/12*(2006.01)i

(According to International Patent Classification (IPC) or to both national classification and IPC)

Continuation of B. FIELDS SEARCHED
Minimum documentation searched (International Patent Classification (IPC))

C07C233/11, A61K31/167, A61K31/18, A61K31/277, A61K31/416, A61K31/4184, A61K31/4245, A61K31/428, A61K31/433, A61K31/44, A61K31/4406, A61K31/4409, A61K31/4427, A61K31/443, A61K31/47, A61K31/4709, A61K31/472, A61K31/4725, A61K31/496, A61K31/506, A61K31/536, A61K31/538, A61K31/54, A61P25/00, A61P43/00, C07C233/27, C07C233/30, C07C255/58, C07C307/02, C07C311/46, C07C317/40, C07D209/08, C07D213/40, C07D213/64, C07D213/74, C07D213/75, C07D215/38, C07D217/02, C07D217/24, C07D217/26, C07D231/56, C07D235/12, C07D265/18, C07D265/36, C07D271/06, C07D277/62, C07D277/82, C07D279/12, C07D285/135, C07D295/18, C07D311/04, C07D319/18, C07D401/06, C07D401/12, C07D405/12, C07D409/12

Minimum documentation searched (classification system followed by classification symbols)

Form PCT/ISA/210 (extra sheet) (April 2005)

**INTERNATIONAL SEARCH REPORT**

International application No.
PCT/JP2007/064007

**Box No. II Observations where certain claims were found unsearchable (Continuation of item 2 of first sheet)**

This international search report has not been established in respect of certain claims under Article 17(2)(a) for the following reasons:

1. [x] Claims Nos.: 27-31
because they relate to subject matter not required to be searched by this Authority, namely:
Claims 27 to 31 pertain to methods for treatment of the human body by surgery or therapy and diagnostic methods.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

1. [ ] As all required additional search fees were timely paid by the applicant, this international search report covers all searchable claims.
2. [ ] As all searchable claims could be searched without effort justifying an additional fee, this Authority did not invite payment of any additional fee.
3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** the
- [ ] The additional search fees were accompanied by the applicant's protest and, where applicable, payment of a protest fee..
- [ ] The additional search fees were accompanied by the applicant's protest but the applicable protest fee was not paid within the time limit specified in the invitation.
- [ ] No protest accompanied the payment of additional search fees.

Form PCT/ISA/210 (continuation of first sheet (2)) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 03049702 A
- US 4788206 A
- JP 7316144 A
- EP 0124791 A
- WO 2005044802 A
- WO 200407874 A
- WO 200504480 A
- WO 200501691 A
- WO 2006058338 A


### Non-patent literature cited in the description


- *Nature,* 1997, vol. 389, 816
- *Nature,* 2000, vol. 405, 183
- *Pharmacological Reviews,* 1999, vol. 51, 159
- *Life Sciences,* 1992, vol. 51, 1777
- *Journal of Urology,* 1997, vol. 158, 2087
- *The Journal of Pharmacology and Experimental Therapeutics,* 2003, vol. 304, 56
- *The Journal of Pharmacology and Experimental Therapeutics,* 2003, vol. 306, 387
- *Journal of Medicinal Chemistry,* 1969, vol. 12, 946
- *Journal of Medicinal Chemistry,* 1968, vol. 11, 749
- **T. W. GREENE.** Protective Groups in Organic Synthesis. John Wiley & Sons Inc, 1999
- The Experimental Chemical Course. Synthesis of Organic Compounds I, Hydrocarbons/Halides. Maruzen, 2005, 283
- **R. C. LAROCK.** *Comprehensive Organic Transformations,* 1989
- *The Journal of Pharmacology and Experimental Therapeutics,* 1993, vol. 267, 728-733
- **KRUGER et al.** *Pain,* vol. 64, 37-57
- **DIXON.** *Annual Review of Pharmacology and Toxicology,* 1980, vol. 20, 441-462
- *Chemical Reviews,* 2002, vol. 102, 29-60
- *Chemical & Pharmaceutical Bulltin,* 1995, vol. 43, 493-498
- *Journal of Organic Chemistry,* 1986, vol. 51, 3143-3147
- *Journal of Heterocyclic Chemistry,* 1986, vol. 23, 1253-1255
- *Bioorganic and Medicinal Chemistry Letters,* 2000, vol. 10, 1459-1462
- *Journal of Medicinal Chemistry,* 1968, vol. 11, 700-703